# EUROPEAN PATENT SPECIFICATION

(11) **EP 1 848 815 B1**
(45) Date of publication and mention of the grant of the patent: **19.05.2010**
(21) Application number: 06706596.1
(22) Date of filing: 02.02.2006
(51) Int. Cl.: C12Q 1/44, G01N 33/68

(54) **ESTERASES FOR MONITORING PROTEIN BIOSYNTHESIS IN VITRO**
ESTERASEN ZUR ÜBERWACHUNG DER PROTEINBIOSYNTHESE IN VITRO
ESTERASES DESTINEES A SURVEILLER LA BIOSYNTHESE IN VITRO DE PROTEINES

(30) Priority: 02.02.2005 EP 05002200
(43) Date of publication of application: 31.10.2007
(73) Proprietor: Universität Bayreuth, 95440 Bayreuth (DE)
(72) Inventor: SPRINZL, Mathias, 95445 Bayreuth (DE); AGAFONOV, Dmitry, 37077 Göttingen (DE); RABE, Kersten, 44145 Dortmund (DE)
(74) Representative: Vossius & Partner
(86) International application number: PCT/EP2006/000927
(87) International publication number: WO 2006/082059

(56) References cited:
- WO-A-01/32847
- US-B1- 6 602 658
- BONNING BRYONY C ET AL: "Use of juvenile hormone esterase as a novel reporter enzyme in the baculovirus expression system" JOURNAL OF VIROLOGICAL METHODS, vol. 51, no. 1, 1995, pages 103-113, XP002378263 ISSN: 0166-0934
- LOOMES KERRY M ET AL: "Functional protective role for mucin glycosylated repetitive domains" EUROPEAN JOURNAL OF BIOCHEMISTRY, vol. 266, no. 1, November 1999 (1999-11), pages 105-111, XP002378264 ISSN: 0014-2956
- FARRELL P J ET AL: "SECRETION OF CYTOPLASMIC AND NUCLEAR PROTEINS FROM ANIMAL CELLS USING NOVEL SECRETION MODULES" PROTEINS: STRUCTURE, FUNCTION AND GENETICS, ALAN R. LISS, US, vol. 41, no. 1, 1 October 2000 (2000-10-01), pages 144-153, XP008019861 ISSN: 0887-3585
- BONNING B C ET AL: "Superior expression of juvenile hormone esterase and beta-galactosidase from the basic protein promoter of Autographa californica nuclear polyhedrosis virus compared to the p10 protein and polyhedrin promoters" JOURNAL OF GENERAL VIROLOGY, SOCIETY FOR GENERAL MICROBIOLOGY, SPENCERS WOOD, GB, vol. 75, 1995, pages 1551-1556, XP002089683 ISSN: 0022-1317
- MANCO GIUSEPPE ET AL: "Overexpression and properties of a new thermophilic and thermostable esterase from Bacillus acidocaldarius with sequence similarity to hormone-sensitive lipase subfamily" BIOCHEMICAL JOURNAL, vol. 332, no. 1, 15 May 1998 (1998-05-15), pages 203-212, XP002378265 ISSN: 0264-6021
- MANCO GIUSEPPE ET AL: "Cloning, overexpression, and properties of a new thermophilic and thermostable esterase with sequence similarity to hormone-sensitive lipase subfamily from the archaeon Archaeoglobus fulgidus" ARCHIVES OF BIOCHEMISTRY AND BIOPHYSICS, vol. 373, no. 1, 1 January 2000 (2000-01-01), pages 182-192, XP002378266 ISSN: 0003-9861
- AGAFONOV D E ET AL: "The esterase from Alicyclobacillus acidocaldarius as a reporter enzyme and affinity tag for protein biosynthesis" FEBS LETTERS, ELSEVIER, AMSTERDAM, NL, vol. 579, no. 10, 11 April 2005 (2005-04-11), pages 2082-2086, XP004850708 ISSN: 0014-5793
- LEE J H; RHEE S K; KIM C H: "Expression and activation of an esterase from Pseudomonas aeruginosa 1001 in Escherichia coli" ENZYME AND MICROBIAL TECHNOLOGY, vol. 35, no. 6-7, 1 December 2004 (2004-12-01), pages 563-567, XP004619952
- KWON OH HYEONG; ITO YOSHIHIRO; UEDA MITSUYOSHI; TANAKA ATSUO; IMANISHI YUKIO: "Hydrophobilization of esterase by genetic combination with polyproline or polytyrosine at the carboxyl terminal" BIOCHIMICA ET BIOPHYSICA ACTA, vol. 1388, no. 1, 14 October 1998 (1998-10-14), pages 239-246,
- LIU ET AL.: "Crystallization and preliminary X-ray diffraction data for the carboxylesterase Est30 from Bacillus stearothermophilus" ACTA CRYSTALLOGRAPHICA SECTION D, vol. D59, 2003, pages 1472-1473, Denmark
- SCHMIDT-DANNERT ET AL.: "Thermoalkalophilic lipase of Bacillus thermocatenulatus: I. Molecular cloning, nucleotide sequence, purification and some properties" BIOCHIMICA ET BIOPHYSICA ACTA, vol. 1301, no. 1-2, 1 January 1996 (1996-01-01), pages 105-114, XP009096389 AMSTERDAM

### SEQUENCE LISTING

<110> Universität Bayreuth
<120> Esterases for monitoring protein biosynthesis in vitro
<130> K2520 PCT S3
<160> 62
<170> Patent In version 3.3
<210> 1
   <211> 933
   <212> DNA
   <213> Alicyclobacillus acidocaldarius
<400> 1
   atgccgctcg atcccgtcat tcagcaggtg ctcgatcaac tcaaccgcat gcctgccccg     60
   gactacaaac atctctccgc ccagcaattt cgttcccaac agtcgctgtt tcctcctgtc    120
   aagaaggagc ccgtggccga ggtccgagag tttgacatgg atctgcctgg ccgcacgctc    180
   aaggtgcgca tgtaccgccc ggagggcgtc gaaccgccct accccgcgct cgtgtattat    240
   cacggcggcg gttgggtcgt cggagacctc gagacgcacg atcccgtctg ccgcgtcctc    300
   gcgaaagacg gccgcgcggt cgtgttctcc gtcgactacc gcctggcgcc ggagcacaag    360
   ttccctgccg ccgtggaaga cgcctacgac gcgcttcagt ggatcgcgga gcgcgcagcg    420
   gactttcatc tcgatccagc ccgcatcgcg gtcggcggag acagcgccgg agggaatctt    480
   gccgctgtga cgagcatcct tgccaaagag cgcggcgggc cggccatcgc gttccagctg    540
   ctcatctacc cttccacggg gtacgatccg gctcatcctc ccgcatctat cgaagaaaat    600
   gcggaaggct atctcctgac cggcggcatg atgctctggt tccgggatca atacttgaac    660
   agcctggagg aactcacgca tccgtggttt tcacccgtcc tctacccgga cttgagcggc    720
   ttgcctccgg cgtacatcgc gacggcgcag tacgatccgc tgcgcgacgt cggcaagctt    780
   tacgcggaag cgctgaacaa ggcgggcgtc aaggtcgaga tcgagaactt cgaagatctg    840
   atccacggat tcgcacagtt ttacagcctt tcgcctggcg cgacgaaggc gctcgtccgc    900
   attgcggaga aacttcgaga cgcgctggcc tga                                 933
<210> 2
   <211> 310
   <212> PRT
   <213> Alicyclobacillus acidocaldarius
<220>
   <221> misc_feature
   <222> (53)..(53)
   <223> Xaa can be any naturally occurring amino acid
<220>
   <221> misc_feature
   <222> (64)..(64)
   <223> Xaa can be any naturally occurring amino acid
<220>
   <221> misc_feature
   <222> (210)..(211)
   <223> Xaa can be any naturally occurring amino acid
<400> 2
   Met Pro Leu Asp Pro Val Ile Gln Gln Val Leu Asp Gln Leu Asn Arg
   1               5                   10                  15
   Met Pro Ala Pro Asp Tyr Lys His Leu Ser Ala Gln Gln Phe Arg Ser
               20                  25                  30
   Gln Gln Ser Leu Phe Pro Pro Val Lys Lys Glu Pro Val Ala Glu Val
           35                  40                  45
   Arg Glu Phe Asp Xaa Asp Leu Pro Gly Arg Thr Leu Lys Val Arg Xaa
       50                  55                  60
   Tyr Arg Pro Glu Gly Val Glu Pro Pro Tyr Pro Ala Leu Val Tyr Tyr
   65                  70                  75                  80
   His Gly Gly Gly Trp Val Val Gly Asp Leu Glu Thr His Asp Pro Val
                   85                  90                  95
   Cys Arg Val Leu Ala Lys Asp Gly Arg Ala Val Val Phe Ser Val Asp
               100                 105                 110
   Tyr Arg Leu Ala Pro Glu His Lys Phe Pro Ala Ala Val Glu Asp Ala
           115                 120                 125
   Tyr Asp Ala Leu Gln Trp Ile Ala Glu Arg Ala Ala Asp Phe His Leu
       130                 135                 140
   Asp Pro Ala Arg Ile Ala Val Gly Gly Asp Ser Ala Gly Gly Asn Leu
   145                 150                 155                 160
   Ala Ala Val Thr Ser Ile Leu Ala Lys Glu Arg Gly Gly Pro Ala Leu
                   165                 170                 175
   Ala Phe Gln Leu Leu Ile Tyr Pro Ser Thr Gly Tyr Asp Pro Ala His
               180                 185                 190
   Pro Pro Ala Ser Ile Glu Glu Asn Ala Glu Gly Tyr Leu Leu Thr Gly
   195 200 205
   Gly Xaa Xaa Leu Trp Phe Arg Asp Gln Tyr Leu Asn Ser Leu Glu Glu
   210 215 220
   Leu Thr His Pro Trp Phe Ser Pro Val Leu Tyr Pro Asp Leu Ser Gly
   225 230 235 240
   Leu Pro Pro Ala Tyr Ile Ala Thr Ala Gln Tyr Asp Pro Leu Arg Asp
   245 250 255
   Val Gly Lys Leu Tyr Ala Glu Ala Leu Asn Lys Ala Gly Val Lys Val
   260 265 270
   Glu Ile Glu Asn Phe Glu Asp Leu Ile His Gly Phe Ala Gln Phe Tyr
   275 280 285
   Ser Leu Ser Pro Gly Ala Thr Lys Ala Leu Val Arg Ile Ala Glu Lys
   290 295 300
   Leu Arg Asp Ala Leu Ala
   305 310
<210> 3
   <211> 1065
   <212> DNA
   <213> Thermus thermophilus
<400> 3
   atgctggaca agcttgaccg cctagaggaa gagtaccggg agctggaggc gctcctctcc 60
   gacccggagg tgctgaagga caaggggcgc taccagagcc tctcccgccg ctacgccgag 120
   atgggggagg tgatcggcct catccgggag taccggaagg tgctggagga cctggagcag 180
   gcggaaagcc ttcttgacga ccccgagctc aaggagatgg ccaaggcgga gcgggaggcc 240
   ctcctcgccc gcaaggaggc cctggagaag gagctggagc gccacctcct gcctaaggac 300
   cccatggacg aaagggacgc catcgtagag atccgggcgg ggacgggagg ggaggaggcc 360
   gccctcttcg cccgcgacct tttcaacatg tacctccgct tcgccgagga gatgggcttt 420
   gagacggagg tcctggactc ccaccccacg gacctcgggg gcttctccaa ggtggtcttt 480
   gaggtgcggg gcccgggggc ctacggcacc ttcaagtacg agagcggggt ccaccgggtg 540
   caacgggtgc ccgtcaccga gacccagggg cggatccaca cctccaccgc cacggtggcc 600
   gtcctcccca aggcggagga ggaggacttc gccctcaaca tggacgagat ccgcattgac 660
   gtgatgcggg cctcggggcc cggggggcag ggggtgaaca ccacggactc ggcggtgcgg 720
   gtggtccacc tgcccacggg gatcatggtc acctgccagg actcccgcag ccagatcaag 780
   aaccgggaga aggccctcat gatcctaaga agccgtctcc tggagatgaa gcgggcggag 840
   gaggcggaaa ggctccggaa gacccgcctt gcccagatcg gcaccgggga gcgctcggag 900
   aagatccgca cctacaactt cccccagtcc cgggtcacgg accaccgcat cgggttcacc 960
   acccacgacc tcgagggcgt cctctccggc cacctgaccc ccatcctgga ggcgctcaag 1020
   cgggccgacc aggagcgcca gctcgcggcg ctggcggaag ggtga 1065
<210> 4
   <211> 354
   <212> PRT
   <213> Thermus thermophilus
<400> 4
   Met Leu Asp Lys Leu Asp Arg Leu Glu Glu Glu Tyr Arg Glu Leu Glu
   1               5                   10                  15
   Ala Leu Leu Ser Asp Pro Glu Val Leu Lys Asp Lys Gly Arg Tyr Gln
               20                  25                  30
   Ser Leu Ser Arg Arg Tyr Ala Glu Met Gly Glu Val Ile Gly Leu Ile
           35                  40                  45
   Arg Glu Tyr Arg Lys Val Leu Glu Asp Leu Glu Gln Ala Glu Ser Leu
       50                  55                  60
   Leu Asp Asp Pro Glu Leu Lys Glu Met Ala Lys Ala Glu Arg Glu Ala
   65                  70                  75                  80
   Leu Leu Ala Arg Lys Glu Ala Leu Glu Lys Glu Leu Glu Arg His Leu
                   85                  90                  95
   Leu Pro Lys Asp Pro Met Asp Glu Arg Asp Ala Ile Val Glu Ile Arg
               100                 105                 110
   Ala Gly Thr Gly Gly Glu Glu Ala Ala Leu Phe Ala Arg Asp Leu Phe
           115                 120                 125
   Asn Met Tyr Leu Arg Phe Ala Glu Glu Met Gly Phe Glu Thr Glu Val
       130                 135                 140
   Leu Asp Ser His Pro Thr Asp Leu Gly Gly Phe Ser Lys Val Val Phe
   145 150 155 160
   Glu Val Arg Gly Pro Gly Ala Tyr Gly Thr Phe Lys Tyr Glu Ser Gly
   165 170 175
   Val His Arg Val Gln Arg Val Pro Val Thr Glu Thr Gln Gly Arg Ile
   180 185 190
   His Thr Ser Thr Ala Thr Val Ala Val Leu Pro Lys Ala Glu Glu Glu
   195 200 205
   Asp Phe Ala Leu Asn Met Asp Glu Ile Arg Ile Asp Val Met Arg Ala
   210 215 220
   Ser Gly Pro Gly Gly Gln Gly Val Asn Thr Thr Asp Ser Ala Val Arg
   225 230 235 240
   Val Val His Leu Pro Thr Gly Ile Met Val Thr Cys Gln Asp Ser Arg
   245 250 255
   Ser Gln Ile Lys Asn Arg Glu Lys Ala Leu Met Ile Leu Arg Ser Arg
   260 265 270
   Leu Leu Glu Met Lys Arg Ala Glu Glu Ala Glu Arg Leu Arg Lys Thr
   275 280 285
   Arg Leu Ala Gln Ile Gly Thr Gly Glu Arg Ser Glu Lys Ile Arg Thr
   290 295 300
   Tyr Asn Phe Pro Gln Ser Arg Val Thr Asp His Arg Ile Gly Phe Thr
   305 310 315 320
   Thr His Asp Leu Glu Gly Val Leu Ser Gly His Leu Thr Pro Ile Leu
   325 330 335
   Glu Ala Leu Lys Arg Ala Asp Gln Glu Arg Gln Leu Ala Ala Leu Ala
   340 345 350
   Glu Gly
<210> 5
   <211> 1083
   <212> DNA
   <213> Escherichia coli
<400> 5
   atgaagcctt ctatcgttgc caaactggaa gccctgcatg aacgccatga agaagttcag 60
   gcgttgctgg gtgacgcgca aactatcgcc gaccaggaac gttttcgcgc attatcacgc 120
   gaatatgcgc agttaagtga tgtttcgcgc tgttttaccg actggcaaca ggttcaggaa 180
   gatatcgaaa ccgcacagat gatgctcgat gatcctgaaa tgcgtgagat ggcgcaggat 240
   gaactgcgcg aagctaaaga aaaaagcgag caactggaac agcaattaca ggttctgtta 300
   ctgccaaaag atcctgatga cgaacgtaac gccttcctcg aagtccgagc cggaaccggc 360
   ggcgacgaag cggcgctgtt cgcgggcgat ctgttccgta tgtacagccg ttatgccgaa 420
   gcccgccgct ggcgggtaga aatcatgagc gccagcgagg gtgaacatgg tggttataaa 480
   gagatcatcg ccaaaattag cggtgatggt gtgtatggtc gtctgaaatt tgaatccggc 540
   ggtcatcgcg tgcaacgtgt tcctgctacg gaatcgcagg gtcgtattca tacttctgct 600
   tgtaccgttg cggtaatgcc agaactgcct gacgcagaac tgccggacat caacccagca 660
   gatttacgca ttgatacttt ccgctcgtca ggggcgggtg gtcagcacgt taacaccacc 720
   ggttcggcaa ttcgtattac tcacttgccg accgggattg ttgttgaatg tcaggacgaa 780
   cgttcacaac ataaaaacaa agctaaagca ctttctgttc tcggtgctcg catccacgct 840
   gctgaaatgg caaaacgcca acaggccgaa gcgtctaccc gtcgtaacct gctggggagt 900
   ggcgatcgca gcgaccgtaa ccgtacttac aacttcccgc aggggcgcgt taccgatcac 960
   cgcatcaacc tgacgctcta ccgcctggat gaagtgatgg aaggtaagct ggatatgctg 1020
   attgaaccga ttatccagga acatcaggcc gaccaactgg cggcgttgtc cgagcaggaa 1080
   taa 1083
<210> 6
   <211> 360
   <212> PRT
   <213> Escherichia coli
<400> 6
   Met Lys Pro Ser Ile Val Ala Lys Leu Glu Ala Leu His Glu Arg His
   1               5                   10                  15
   Glu Glu Val Gln Ala Leu Leu Gly Asp Ala Gln Thr Ile Ala Asp Gln
               20                  25                  30
   Glu Arg Phe Arg Ala Leu Ser Arg Glu Tyr Ala Gln Leu Ser Asp Val
           35                  40                  45
   Ser Arg Cys Phe Thr Asp Trp Gln Gln Val Gln Glu Asp Ile Glu Thr
   50 55 60
   Ala Gln Met Met Leu Asp Asp Pro Glu Met Arg Glu Met Ala Gln Asp
   65 70 75 80
   Glu Leu Arg Glu Ala Lys Glu Lys Ser Glu Gln Leu Glu Gln Gln Leu
   85 90 95
   Gln Val Leu Leu Leu Pro Lys Asp Pro Asp Asp Glu Arg Asn Ala Phe
   100 105 110
   Leu Glu Val Arg Ala Gly Thr Gly Gly Asp Glu Ala Ala Leu Phe Ala
   115 120 125
   Gly Asp Leu Phe Arg Met Tyr Ser Arg Tyr Ala Glu Ala Arg Arg Trp
   130 135 140
   Arg Val Glu Ile Met Ser Ala Ser Glu Gly Glu His Gly Gly Tyr Lys
   145 150 155 160
   Glu Ile Ile Ala Lys Ile Ser Gly Asp Gly Val Tyr Gly Arg Leu Lys
   165 170 175
   Phe Glu Ser Gly Gly His Arg Val Gln Arg Val Pro Ala Thr Glu Ser
   180 185 190
   Gln Gly Arg Ile His Thr Ser Ala Cys Thr Val Ala Val Met Pro Glu
   195 200 205
   Leu Pro Asp Ala Glu Leu Pro Asp Ile Asn Pro Ala Asp Leu Arg Ile
   210 215 220
   Asp Thr Phe Arg Ser Ser Gly Ala Gly Gly Gln His Val Asn Thr Thr
   225 230 235 240
   Asp Ser Ala Ile Arg Ile Thr His Leu Pro Thr Gly Ile Val Val Glu
   245 250 255
   Cys Gln Asp Glu Arg Ser Gln His Lys Asn Lys Ala Lys Ala Leu Ser
   260 265 270
   Val Leu Gly Ala Arg Ile His Ala Ala Glu Met Ala Lys Arg Gln Gln
   275 280 285
   Ala Glu Ala Ser Thr Arg Arg Asn Leu Leu Gly Ser Gly Asp Arg Ser
   290 295 300
   Asp Arg Asn Arg Thr Tyr Asn Phe Pro Gln Gly Arg Val Thr Asp His
   305 310 315 320
   Arg Ile Asn Leu Thr Leu Tyr Arg Leu Asp Glu Val Met Glu Gly Lys
   325 330 335
   Leu Asp Met Leu Ile Glu Pro Ile Ile Gln Glu His Gln Ala Asp Gln
   340 345 350
   Leu Ala Ala Leu Ser Glu Gln Glu
   355 360
<210> 7
   <211> 39
   <212> DNA
   <213> artificial
<220>
   <223> artificial
<400> 7
   gagctcggta ccattgaggg tcgcggttcc ggcggtggt 39
<210> 8
   <211> 5156
   <212> DNA
   <213> artificial
<220>
   <223> artificial
<400> 8
   cggtaccatt gagggtcgcg gttccggcgg tggtatggcg ctcgatcccg tcattcagca    60
   ggtgctcgat caactcaacc gcatgcctgc cccggactac aaacatctct ccgcccagca   120
   atttcgttcc caacagtcgc tgtttcctcc tgtcaagaag gagcccgtgg ccgaggtccg   180
   agagtttgac atggatctgc ctggccgcac gctcaaggtg cgcatgtacc gcccggaggg   240
   cgtcgaaccg ccctaccccg cgctcgtgta ttatcacggc ggcggttggg tcgtcggaga   300
   cctcgagacg cacgatcccg tctgccgcgt cctcgcgaaa gacggccgcg cggtcgtgtt   360
   ctccgtcgac taccgcctgg cgccggagca caagttccct gccgccgtgg aagacgccta   420
   cgacgcgctt cagtggatcg cggagcgcgc agcggacttt catctcgatc cagcccgcat   480
   cgcggtcggc ggagacagcg ccggagggaa tcttgccgct gtgacgagca tccttgccaa     540
   agagcgcggc gggccggcca tcgcgttcca gctgctcatc tacccttcca cggggtacga     600
   tccggctcat cctcccgcat ctatcgaaga aaatgcggaa ggctatctcc tgaccggcgg    660
   catgatgctc tggttccggg atcaatactt gaacagcctg gaggaactca cgcatccgtg    720
   gttttcaccc gtcctctacc cggacttgag cggcttgcct ccggcgtaca tcgcgacggc    780
   gcagtacgat ccgctgcgcg acgtcggcaa gctttacgcg gaagcgctga acaaggcggg    840
   cgtcaaggtc gagatcgaga acttcgaaga tctgatccac ggattcgcac agttttacag    900
   cctttcgcct ggcgcgacga aggcgctcgt ccgcattgcg gagaaacttc gagacgcgct    960
   ggcctgagga tccggctgct aacaaagccc gaaaggaagc tgagttggct gctgccaccg   1020
   ctgagcaata actagcataa ccccttgggg cctctaaacg ggtcttgagg ggttttttgc   1080
   tgaaaggagg aactatatcc ggatatccac aggacgggtg tggtcgccat gatcgcgtag   1140
   tcgatagtgg ctccaagtag cgaagcgagc aggactgggc ggcggccaaa gcggtcggac   1200
   agtgctccga gaacgggtgc gcatagaaat tgcatcaacg catatagcgc tagcagcacg   1260
   ccatagtgac tggcgatgct gtcggaatgg acgatatccc gcaagaggcc cggcagtacc   1320
   ggcataacca agcctatgcc tacagcatcc agggtgacgg tgccgaggat gacgatgagc   1380
   gcattgttag atttcataca cggtgcctga ctgcgttagc aatttaactg tgataaacta   1440
   ccgcattaaa gcttatcgat gataagctgt caaacatgag aattcgtaat catgtcatag   1500
   ctgtttcctg tgtgaaattg ttatccgctc acaattccac acaacatacg agccggaagc   1560
   ataaagtgta aagcctgggg tgcctaatga gtgagctaac tcacattaat tgcgttgcgc   1620
   tcactgcccg ctttccagtc gggaaacctg tcgtgccagc tgcattaatg aatcggccaa   1680
   cgcgcgggga gaggcggttt gcgtattggg cgctcttccg cttcctcgct cactgactcg   1740
   ctgcgctcgg tcgttcggct gcggcgagcg gtatcagctc actcaaaggc ggtaatacgg   1800
   ttatccacag aatcagggga taacgcagga aagaacatgt gagcaaaagg ccagcaaaag   1860
   gccaggaacc gtaaaaaggc cgcgttgctg gcgtttttcc ataggctccg cccccctgac   1920
   gagcatcaca aaaatcgacg ctcaagtcag aggtggcgaa acccgacagg actataaaga   1980
   taccaggcgt ttccccctgg aagctccctc gtgcgctctc ctgttccgac cctgccgctt   2040
   accggatacc tgtccgcctt tctcccttcg ggaagcgtgg cgctttctca tagctcacgc   2100
   tgtaggtatc tcagttcggt gtaggtcgtt cgctccaagc tgggctgtgt gcacgaaccc   2160
   cccgttcagc ccgaccgctg cgccttatcc ggtaactatc gtcttgagtc caacccggta   2220
   agacacgact tatcgccact ggcagcagcc actggtaaca ggattagcag agcgaggtat   2280
   gtaggcggtg ctacagagtt cttgaagtgg tggcctaact acggctacac tagaaggaca   2340
   gtatttggta tctgcgctct gctgaagcca gttaccttcg gaaaaagagt tggtagctct 2400
   tgatccggca aacaaaccac cgctggtagc ggtggttttt ttgtttgcaa gcagcagatt 2460
   acgcgcagaa aaaaaggatc tcaagaagat cctttgatct tttctacggg gtctgacgct 2520
   cagtggaacg aaaactcacg ttaagggatt ttggtcatga gattatcaaa aaggatcttc 2580
   acctagatcc ttttaaatta aaaatgaagt tttaaatcaa tctaaagtat atatgagtaa 2640
   acttggtctg acagttacca atgcttaatc agtgaggcac ctatctcagc gatctgtcta 2700
   tttcgttcat ccatagttgc ctgactcccc gtcgtgtaga taactacgat acgggagggc 2760
   ttaccatctg gccccagtgc tgcaatgata ccgcgagacc cacgctcacc ggctccagat 2820
   ttatcagcaa taaaccagcc agccggaagg gccgagcgca gaagtggtcc tgcaacttta 2880
   tccgcctcca tccagtctat taattgttgc cgggaagcta gagtaagtag ttcgccagtt 2940
   aatagtttgc gcaacgttgt tgccattgct acaggcatcg tggtgtcacg ctcgtcgttt 3000
   ggtatggctt cattcagctc cggttcccaa cgatcaaggc gagttacatg atcccccatg 3060
   ttgtgcaaaa aagcggttag ctccttcggt cctccgatcg ttgtcagaag taagttggcc 3120
   gcagtgttat cactcatggt tatggcagca ctgcataatt ctcttactgt catgccatcc 3180
   gtaagatgct tttctgtgac tggtgagtac tcaaccaagt cattctgaga atagtgtatg 3240
   cggcgaccga gttgctcttg cccggcgtca atacgggata ataccgcgcc acatagcaga 3300
   actttaaaag tgctcatcat tggaaaacgt tcttcggggc gaaaactctc aaggatctta 3360
   ccgctgttga gatccagttc gatgtaaccc actcgtgcac ccaactgatc ttcagcatct 3420
   tttactttca ccagcgtttc tgggtgagca aaaacaggaa ggcaaaatgc cgcaaaaaag 3480
   ggaataaggg cgacacggaa atgttgaata ctcatactct tcctttttca atattattga 3540
   agcatttatc agggttattg tctcatgagc ggatacatat ttgaatgtat ttagaaaaat 3600
   aaacaaatag gggttccgcg cacatttccc cgaaaagtgc cacctgacgt ctaagaaacc 3660
   attattatca tgacattaac ctataaaaat aggcgtatca cgaggccctt tcgtctcgcg 3720
   cgtttcggtg atgacggtga aaacctctga cacatgcagc tcccggagac ggtcacagct 3780
   tgtctgtaag cggatgccgg gagcagacaa gcccgtcagg gcgcgtcagc gggtgttggc 3840
   gggtgtcggg gctggcttaa ctatgcggca tcagagcaga ttgtactgag agtgcaccat 3900
   atatgcggtg tgaaataccg cacagatgcg taaggagaaa ataccgcatc aggcgccatt 3960
   cgccattcag gctgcgcaac tgttgggaag ggcgatcggt gcgggcctct tcgctattac 4020
   gccagctggc gaaaggggga tgtgctgcaa ggcgattaag ttgggtaacg ccagggtttt 4080
   cccagtcacg acgttgtaaa acgacggcca gtgccaagct tgcatgcaag gagatggcgc 4140
   ccaacagtcc cccggccacg gggcctgcca ccatacccac gccgaaacaa gcgctcatga 4200
   gcccgaagtg gcgagcccga tcttccccat cggtgatgtc ggcgatatag gcgccagcaa 4260
   ccgcacctgt ggcgccggtg atgccggcca cgatgcgtcc ggcgtagagg atcgagatct 4320
   cgatcccgcg aaattaatac gactcactat agggagacca caacggtttc cctctagaaa 4380
   taattttgtt taactttaag aaggagatat accatggtga gcaagggcga ggagctgttc 4440
   accggggtgg tgcccatcct ggtcgagctg gacggcgacg taaacggcca caagttcagc 4500
   gtgtccggcg agggcgaggg cgatgccacc tacggcaagc tgaccctgaa gttcatctgc 4560
   accaccggca agctgcccgt gccctggccc accctcgtga ccaccctgac ctacggcgtg 4620
   cagtgcttca gccgctaccc cgaccacatg aagcagcacg acttcttcaa gtccgccatg 4680
   cccgaaggct acgtccagga gcgcaccatc ttcttcaagg acgacggcaa ctacaagacc 4740
   cgcgccgagg tgaagttcga gggcgacacc ctggtgaacc gcatcgagct gaagggcatc 4800
   gacttcaagg aggacggcaa catcctgggg cacaagctgg agtacaacta caacagccac 4860
   aacgtctata tcatggccga caagcagaag aacggcatca aggtgaactt caagatccgc 4920
   cacaacatcg aggacggcag cgtgcagctc gccgaccact accagcagaa cacccccatc 4980
   ggcgacggcc ccgtgctgct gcccgacaac cactacctga gcacccagtc cgccctgagc 5040
   aaagacccca acgagaagcg cgatcacatg gtcctgctgg agttcgtgac cgccgccggg 5100
   atcactctcg gcatggacga gctgtacaaa ggcggccgcg tcgactcgag cgagct 5156
<210> 9
   <211> 4460
   <212> DNA
   <213> artificial
<220>
   <223> artificial
<400> 9
   catggcgctc gatcccgtca ttcagcaggt gctcgatcaa ctcaaccgca tgcctgcccc     60
   ggactacaaa catctctccg cccagcaatt tcgttcccaa cagtcgctgt ttcctcctgt    120
   caagaaggag cccgtggccg aggtccgaga gtttgacatg gatctgcctg gccgcacgct    180
   caaggtgcgc atgtaccgcc cggagggcgt cgaaccgccc taccccgcgc tcgtgtatta    240
   tcacggcggc ggttgggtcg tcggagacct cgagacgcac gatcccgtct gccgcgtcct    300
   cgcgaaagac ggccgcgcgg tcgtgttctc cgtcgactac cgcctggcgc cggagcacaa    360
   gttccctgcc gccgtggaag acgcctacga cgcgcttcag tggatcgcgg agcgcgcagc    420
   ggactttcat ctcgatccag cccgcatcgc ggtcggcgga gacagcgccg gagggaatct 480
   tgccgctgtg acgagcatcc ttgccaaaga gcgcggcggg ccggccatcg cgttccagct 540
   gctcatctac ccttccacgg ggtacgatcc ggctcatcct cccgcatcta tcgaagaaaa 600
   tgcggaaggc tatctcctga ccggcggcat gatgctctgg ttccgggatc aatacttgaa 660
   cagcctggag gaactcacgc atccgtggtt ttaccccgtc ctctacccgg acttgagcgg 720
   cttgcctccg gcgtacatcg cgacggcgca gtacgatccg ctgcgcgacg tcggcaagct 780
   ttacgcggaa gcgctgaaca aggcgggcgt caaggtcgag atcgagaact tcgaagatct 840
   gatcctcgga ttcgcacagt tttacagcct ttcgcctggc gcgacgaagg cgctcgtccg 900
   cattgcggag aaacttcgag acgcgctggc ctgagagctc ccgggggggg ttctcatcat 960
   catcatcatc attaataaaa gggcgaattc cagcacactg gcggccgtta ctagtggatc 1020
   cggctgctaa caaagcccga aaggaagctg agttggctgc tgccaccgct gagcaataac 1080
   tagcataacc ccttggggcc tctaaacggg tcttgagggg ttttttgctg aaaggaggaa 1140
   ctatatccgg atatccacag gacgggtgtg gtcgccatga tcgcgtagtc gatagtggct 1200
   ccaagtagcg aagcgagcag gactgggcgg cggccaaagc ggtcggacag tgctccgaga 1260
   acgggtgcgc atagaaattg catcaacgca tatagcgcta gcagcacgcc atagtgactg 1320
   gcgatgctgt cggaatggac gatatcccgc aagaggcccg gcagtaccgg cataaccaag 1380
   cctatgccta cagcatccag ggtgacggtg ccgaggatga cgatgagcgc attgttagat 1440
   ttcatacacg gtgcctgact gcgttagcaa tttaactgtg ataaactacc gcattaaagc 1500
   ttatcgatga taagctgtca aacatgagaa ttcgtaatca tgtcatagct gtttcctgtg 1560
   tgaaattgtt atccgctcac aattccacac aacatacgag ccggaagcat aaagtgtaaa 1620
   gcctggggtg cctaatgagt gagctaactc acattaattg cgttgcgctc actgcccgct 1680
   ttccagtcgg gaaacctgtc gtgccagctg cattaatgaa tcggccaacg cgcggggaga 1740
   ggcggtttgc gtattgggcg ctcttccgct tcctcgctca ctgactcgct gcgctcggtc 1800
   gttcggctgc ggcgagcggt atcagctcac tcaaaggcgg taatacggtt atccacagaa 1860
   tcaggggata acgcaggaaa gaacatgtga gcaaaaggcc agcaaaaggc caggaaccgt 1920
   aaaaaggccg cgttgctggc gtttttccat aggctccgcc cccctgacga gcatcacaaa 1980
   aatcgacgct caagtcagag gtggcgaaac ccgacaggac tataaagata ccaggcgttt 2040
   ccccctggaa gctccctcgt gcgctctcct gttccgaccc tgccgcttac cggatacctg 2100
   tccgcctttc tcccttcggg aagcgtggcg ctttctcata gctcacgctg taggtatctc 2160
   agttcggtgt aggtcgttcg ctccaagctg ggctgtgtgc acgaaccccc cgttcagccc 2220
   gaccgctgcg ccttatccgg taactatcgt cttgagtcca acccggtaag acacgactta 2280
   tcgccactgg cagcagccac tggtaacagg attagcagag cgaggtatgt aggcggtgct 2340
   acagagttct tgaagtggtg gcctaactac ggctacacta gaaggacagt atttggtatc 2400
   tgcgctctgc tgaagccagt taccttcgga aaaagagttg gtagctcttg atccggcaaa 2460
   caaaccaccg ctggtagcgg tggttttttt gtttgcaagc agcagattac gcgcagaaaa 2520
   aaaggatctc aagaagatcc tttgatcttt tctacggggt ctgacgctca gtggaacgaa 2580
   aactcacgtt aagggatttt ggtcatgaga ttatcaaaaa ggatcttcac ctagatcctt 2640
   ttaaattaaa aatgaagttt taaatcaatc taaagtatat atgagtaaac ttggtctgac 2700
   agttaccaat gcttaatcag tgaggcacct atctcagcga tctgtctatt tcgttcatcc 2760
   atagttgcct gactccccgt cgtgtagata actacgatac gggagggctt accatctggc 2820
   cccagtgctg caatgatacc gcgagaccca cgctcaccgg ctccagattt atcagcaata 2880
   aaccagccag ccggaagggc cgagcgcaga agtggtcctg caactttatc cgcctccatc 2940
   cagtctatta attgttgccg ggaagctaga gtaagtagtt cgccagttaa tagtttgcgc 3000
   aacgttgttg ccattgctac aggcatcgtg gtgtcacgct cgtcgtttgg tatggcttca 3060
   ttcagctccg gttcccaacg atcaaggcga gttacatgat cccccatgtt gtgcaaaaaa 3120
   gcggttagct ccttcggtcc tccgatcgtt gtcagaagta agttggccgc agtgttatca 3180
   ctcatggtta tggcagcact gcataattct cttactgtca tgccatccgt aagatgcttt 3240
   tctgtgactg gtgagtactc aaccaagtca ttctgagaat agtgtatgcg gcgaccgagt 3300
   tgctcttgcc cggcgtcaat acgggataat accgcgccac atagcagaac tttaaaagtg 3360
   ctcatcattg gaaaacgttc ttcggggcga aaactctcaa ggatcttacc gctgttgaga 3420
   tccagttcga tgtaacccac tcgtgcaccc aactgatctt cagcatcttt tactttcacc 3480
   agcgtttctg ggtgagcaaa aacaggaagg caaaatgccg caaaaaaggg aataagggcg 3540
   acacggaaat gttgaatact catactcttc ctttttcaat attattgaag catttatcag 3600
   ggttattgtc tcatgagcgg atacatattt gaatgtattt agaaaaataa acaaataggg 3660
   gttccgcgca catttccccg aaaagtgcca cctgacgtct aagaaaccat tattatcatg 3720
   acattaacct ataaaaatag gcgtatcacg aggccctttc gtctcgcgcg tttcggtgat 3780
   gacggtgaaa acctctgaca catgcagctc ccggagacgg tcacagcttg tctgtaagcg 3840
   gatgccggga gcagacaagc ccgtcagggc gcgtcagcgg gtgttggcgg gtgtcggggc 3900
   tggcttaact atgcggcatc agagcagatt gtactgagag tgcaccatat atgcggtgtg 3960
   aaataccgca cagatgcgta aggagaaaat accgcatcag gcgccattcg ccattcaggc 4020
   tgcgcaactg ttgggaaggg cgatcggtgc gggcctcttc gctattacgc cagctggcga   4080
   aagggggatg tgctgcaagg cgattaagtt gggtaacgcc agggttttcc cagtcacgac   4140
   gttgtaaaac gacggccagt gccaagcttg catgcaagga gatggcgccc aacagtcccc   4200
   cggccacggg gcctgccacc atacccacgc cgaaacaagc gctcatgagc ccgaagtggc   4260
   gagcccgatc ttccccatcg gtgatgtcgg cgatataggc gccagcaacc gcacctgtgg   4320
   cgccggtgat gccggccacg atgcgtccgg cgtagaggat cgagatctcg atcccgcgaa   4380
   attaatacga ctcactatag ggagaccaca acggtttccc tctagaaata attttgttta   4440
   actttaagaa ggagatatac                                                4460
<210> 10
   <211> 4460
   <212> DNA
   <213> artificial
<220>
   <223> artificial
<400> 10
   catggcgctc gatcccgtca ttcagcaggt gctcgatcaa ctcaaccgca tgcctgcccc      60
   ggactacaaa catctctccg cccagcaatt tcgttcccaa cagtcgctgt ttcctcctgt     120
   caagaaggag cccgtggccg aggtccgaga gtttgacatg gatctgcctg gccgcacgct     180
   caaggtgcgc atgtaccgcc cggagggcgt cgaaccgccc taccccgcgc tcgtgtatta     240
   tcacggcggc ggttgggtcg tcggagacct cgagacgcac gatcccgtct gccgcgtcct     300
   cgcgaaagac ggccgcgcgg tcgtgttctc cgtcgactac cgcctggcgc cggagcacaa     360
   gttccctgcc gccgtggaag acgcctacga cgcgcttcag tggatcgcgg agcgcgcagc     420
   ggactttcat ctcgatccag cccgcatcgc ggtcggcgga gactaggccg gagggaatct     480
   tgccgctgtg acgagcatcc ttgccaaaga gcgcggcggg ccggccatcg cgttccagct     540
   gctcatctac ccttccacgg ggtacgatcc ggctcatcct cccgcatcta tcgaagaaaa     600
   tgcggaaggc tatctcctga ccggcggcat gatgctctgg ttccgggatc aatacttgaa     660
   cagcctggag gaactcacgc atccgtggtt ttaccccgtc ctctacccgg acttgagcgg     720
   cttgcctccg gcgtacatcg cgacggcgca gtacgatccg ctgcgcgacg tcggcaagct     780
   ttacgcggaa gcgctgaaca aggcgggcgt caaggtcgag atcgagaact tcgaagatct     840
   gatcctcgga ttcgcacagt tttacagcct ttcgcctggc gcgacgaagg cgctcgtccg     900
   cattgcggag aaacttcgag acgcgctggc ctgagagctc ccgggggggg ttctcatcat     960
   catcatcatc attaataaaa gggcgaattc cagcacactg gcggccgtta ctagtggatc    1020
   cggctgctaa caaagcccga aaggaagctg agttggctgc tgccaccgct gagcaataac 1080
   tagcataacc ccttggggcc tctaaacggg tcttgagggg ttttttgctg aaaggaggaa 1140
   ctatatccgg atatccacag gacgggtgtg gtcgccatga tcgcgtagtc gatagtggct 1200
   ccaagtagcg aagcgagcag gactgggcgg cggccaaagc ggtcggacag tgctccgaga 1260
   acgggtgcgc atagaaattg catcaacgca tatagcgcta gcagcacgcc atagtgactg 1320
   gcgatgctgt cggaatggac gatatcccgc aagaggcccg gcagtaccgg cataaccaag 1380
   cctatgccta cagcatccag ggtgacggtg ccgaggatga cgatgagcgc attgttagat 1440
   ttcatacacg gtgcctgact gcgttagcaa tttaactgtg ataaactacc gcattaaagc 1500
   ttatcgatga taagctgtca aacatgagaa ttcgtaatca tgtcatagct gtttcctgtg 1560
   tgaaattgtt atccgctcac aattccacac aacatacgag ccggaagcat aaagtgtaaa 1620
   gcctggggtg cctaatgagt gagctaactc acattaattg cgttgcgctc actgcccgct 1680
   ttccagtcgg gaaacctgtc gtgccagctg cattaatgaa tcggccaacg cgcggggaga 1740
   ggcggtttgc gtattgggcg ctcttccgct tcctcgctca ctgactcgct gcgctcggtc 1800
   gttcggctgc ggcgagcggt atcagctcac tcaaaggcgg taatacggtt atccacagaa 1860
   tcaggggata acgcaggaaa gaacatgtga gcaaaaggcc agcaaaaggc caggaaccgt 1920
   aaaaaggccg cgttgctggc gtttttccat aggctccgcc cccctgacga gcatcacaaa 1980
   aatcgacgct caagtcagag gtggcgaaac ccgacaggac tataaagata ccaggcgttt 2040
   ccccctggaa gctccctcgt gcgctctcct gttccgaccc tgccgcttac cggatacctg 2100
   tccgcctttc tcccttcggg aagcgtggcg ctttctcata gctcacgctg taggtatctc 2160
   agttcggtgt aggtcgttcg ctccaagctg ggctgtgtgc acgaaccccc cgttcagccc 2220
   gaccgctgcg ccttatccgg taactatcgt cttgagtcca acccggtaag acacgactta 2280
   tcgccactgg cagcagccac tggtaacagg attagcagag cgaggtatgt aggcggtgct 2340
   acagagttct tgaagtggtg gcctaactac ggctacacta gaaggacagt atttggtatc 2400
   tgcgctctgc tgaagccagt taccttcgga aaaagagttg gtagctcttg atccggcaaa 2460
   caaaccaccg ctggtagcgg tggttttttt gtttgcaagc agcagattac gcgcagaaaa 2520
   aaaggatctc aagaagatcc tttgatcttt tctacggggt ctgacgctca gtggaacgaa 2580
   aactcacgtt aagggatttt ggtcatgaga ttatcaaaaa ggatcttcac ctagatcctt 2640
   ttaaattaaa aatgaagttt taaatcaatc taaagtatat atgagtaaac ttggtctgac 2700
   agttaccaat gcttaatcag tgaggcacct atctcagcga tctgtctatt tcgttcatcc 2760
   atagttgcct gactccccgt cgtgtagata actacgatac gggagggctt accatctggc 2820
   cccagtgctg caatgatacc gcgagaccca cgctcaccgg ctccagattt atcagcaata 2880
   aaccagccag ccggaagggc cgagcgcaga agtggtcctg caactttatc cgcctccatc 2940
   cagtctatta attgttgccg ggaagctaga gtaagtagtt cgccagttaa tagtttgcgc 3000
   aacgttgttg ccattgctac aggcatcgtg gtgtcacgct cgtcgtttgg tatggcttca 3060
   ttcagctccg gttcccaacg atcaaggcga gttacatgat cccccatgtt gtgcaaaaaa 3120
   gcggttagct ccttcggtcc tccgatcgtt gtcagaagta agttggccgc agtgttatca 3180
   ctcatggtta tggcagcact gcataattct cttactgtca tgccatccgt aagatgcttt 3240
   tctgtgactg gtgagtactc aaccaagtca ttctgagaat agtgtatgcg gcgaccgagt 3300
   tgctcttgcc cggcgtcaat acgggataat accgcgccac atagcagaac tttaaaagtg 3360
   ctcatcattg gaaaacgttc ttcggggcga aaactctcaa ggatcttacc gctgttgaga 3420
   tccagttcga tgtaacccac tcgtgcaccc aactgatctt cagcatcttt tactttcacc 3480
   agcgtttctg ggtgagcaaa aacaggaagg caaaatgccg caaaaaaggg aataagggcg 3540
   acacggaaat gttgaatact catactcttc ctttttcaat attattgaag catttatcag 3600
   ggttattgtc tcatgagcgg atacatattt gaatgtattt agaaaaataa acaaataggg 3660
   gttccgcgca catttccccg aaaagtgcca cctgacgtct aagaaaccat tattatcatg 3720
   acattaacct ataaaaatag gcgtatcacg aggccctttc gtctcgcgcg tttcggtgat 3780
   gacggtgaaa acctctgaca catgcagctc ccggagacgg tcacagcttg tctgtaagcg 3840
   gatgccggga gcagacaagc ccgtcagggc gcgtcagcgg gtgttggcgg gtgtcggggc 3900
   tggcttaact atgcggcatc agagcagatt gtactgagag tgcaccatat atgcggtgtg 3960
   aaataccgca cagatgcgta aggagaaaat accgcatcag gcgccattcg ccattcaggc 4020
   tgcgcaactg ttgggaaggg cgatcggtgc gggcctcttc gctattacgc cagctggcga 4080
   aagggggatg tgctgcaagg cgattaagtt gggtaacgcc agggttttcc cagtcacgac 4140
   gttgtaaaac gacggccagt gccaagcttg catgcaagga gatggcgccc aacagtcccc 4200
   cggccacggg gcctgccacc atacccacgc cgaaacaagc gctcatgagc ccgaagtggc 4260
   gagcccgatc ttccccatcg gtgatgtcgg cgatataggc gccagcaacc gcacctgtgg 4320
   cgccggtgat gccggccacg atgcgtccgg cgtagaggat cgagatctcg atcccgcgaa 4380
   attaatacga ctcactatag ggagaccaca acggtttccc tctagaaata attttgttta 4440
   actttaagaa ggagatatac 4460
<210> 11
   <211> 5042
   <212> DNA
   <213> artificial
<220>
   <223> artificial
<400> 11
   tatggaggcg acccttcccg ttttggacgc gaagacggcg gccctaaaga ggcgttccat 60
   ccggcgttac cggaaggacc ccgtacccga ggggcttctc cgggaaatcc tcgaggccgc 120
   cctccgggcg ccctcggcct ggaacctcca gccctggcgg atcgtggtgg tgcgggaccc 180
   cgccaccaaa cgggccctga gggaggcggc cttcggccag gcccacgtgg aggaggcccc 240
   cgtggtcctg gtcctctacg ccgacctcga ggacgctctc gcccacctgg acgaggtcat 300
   ccaccccggg gtccaggggg aaaggcgtga ggcgcagaag caggccatcc aacgggcctt 360
   cgccgccatg ggcaagaggg cgcgaaaggc ctgggcctcc gggcagagct acatcctctt 420
   gggctacctc cttctcctcc tggaggctta tggcctcgga agcgtcccca tgctggggtt 480
   tgaccccgag agggtgaggg cgatcctggg gcttccttcc cgcgccgcca tccccgccct 540
   ggtggccttg ggctacccgg cggaggaggg ctacccctcc caccgcctgc ccctggagcg 600
   ggtggtcctc tggcgcgagc tcggtaccat tgagggtcgc ggttccggcg gtggtatggc 660
   gctcgatccc gtcattcagc aggtgctcga tcaactcaac cgcatgcctg ccccggacta 720
   caaacatctc tccgcccagc aatttcgttc ccaacagtcg ctgtttcctc ctgtcaagaa 780
   ggagcccgtg gccgaggtcc gagagtttga catggatctg cctggccgca cgctcaaggt 840
   gcgcatgtac cgcccggagg gcgtcgaacc gccctacccc gcgctcgtgt attatcacgg 900
   cggcggttgg gtcgtcggag acctcgagac gcacgatccc gtctgccgcg tcctcgcgaa 960
   agacggccgc gcggtcgtgt tctccgtcga ctaccgcctg gcgccggagc acaagttccc 1020
   tgccgccgtg gaagacgcct acgacgcgct tcagtggatc gcggagcgcg cagcggactt 1080
   tcatctcgat ccagcccgca tcgcggtcgg cggagacagc gccggaggga atcttgccgc 1140
   tgtgacgagc atccttgcca aagagcgcgg cgggccggcc atcgcgttcc agctgctcat 1200
   ctacccttcc acggggtacg atccggctca tcctcccgca tctatcgaag aaaatgcgga 1260
   aggctatctc ctgaccggcg gcatgatgct ctggttccgg gatcaatact tgaacagcct 1320
   ggaggaactc acgcatccgt ggttttcacc cgtcctctac ccggacttga gcggcttgcc 1380
   tccggcgtac atcgcgacgg cgcagtacga tccgctgcgc gacgtcggca agctttacgc 1440
   ggaagcgctg aacaaggcgg gcgtcaaggt cgagatcgag aacttcgaag atctgatcca 1500
   cggattcgca cagttttaca gcctttcgcc tggcgcgacg aaggcgctcg tccgcattgc 1560
   ggagaaactt cgagacgcgc tggcctgagg atccggctgc taacaaagcc cgaaaggaag 1620
   ctgagttggc tgctgccacc gctgagcaat aactagcata accccttggg gcctctaaac 1680
   gggtcttgag gggttttttg ctgaaaggag gaactatatc cggatatcca caggacgggt 1740
   gtggtcgcca tgatcgcgta gtcgatagtg gctccaagta gcgaagcgag caggactggg 1800
   cggcggccaa agcggtcgga cagtgctccg agaacgggtg cgcatagaaa ttgcatcaac 1860
   gcatatagcg ctagcagcac gccatagtga ctggcgatgc tgtcggaatg gacgatatcc 1920
   cgcaagaggc ccggcagtac cggcataacc aagcctatgc ctacagcatc cagggtgacg 1980
   gtgccgagga tgacgatgag cgcattgtta gatttcatac acggtgcctg actgcgttag 2040
   caatttaact gtgataaact accgcattaa agcttatcga tgataagctg tcaaacatga 2100
   gaattcgtaa tcatgtcata gctgtttcct gtgtgaaatt gttatccgct cacaattcca 2160
   cacaacatac gagccggaag cataaagtgt aaagcctggg gtgcctaatg agtgagctaa 2220
   ctcacattaa ttgcgttgcg ctcactgccc gctttccagt cgggaaacct gtcgtgccag 2280
   ctgcattaat gaatcggcca acgcgcgggg agaggcggtt tgcgtattgg gcgctcttcc 2340
   gcttcctcgc tcactgactc gctgcgctcg gtcgttcggc tgcggcgagc ggtatcagct 2400
   cactcaaagg cggtaatacg gttatccaca gaatcagggg ataacgcagg aaagaacatg 2460
   tgagcaaaag gccagcaaaa ggccaggaac cgtaaaaagg ccgcgttgct ggcgtttttc 2520
   cataggctcc gcccccctga cgagcatcac aaaaatcgac gctcaagtca gaggtggcga 2580
   aacccgacag gactataaag ataccaggcg tttccccctg gaagctccct cgtgcgctct 2640
   cctgttccga ccctgccgct taccggatac ctgtccgcct ttctcccttc gggaagcgtg 2700
   gcgctttctc atagctcacg ctgtaggtat ctcagttcgg tgtaggtcgt tcgctccaag 2760
   ctgggctgtg tgcacgaacc ccccgttcag cccgaccgct gcgccttatc cggtaactat 2820
   cgtcttgagt ccaacccggt aagacacgac ttatcgccac tggcagcagc cactggtaac 2880
   aggattagca gagcgaggta tgtaggcggt gctacagagt tcttgaagtg gtggcctaac 2940
   tacggctaca ctagaaggac agtatttggt atctgcgctc tgctgaagcc agttaccttc 3000
   ggaaaaagag ttggtagctc ttgatccggc aaacaaacca ccgctggtag cggtggtttt 3060
   tttgtttgca agcagcagat tacgcgcaga aaaaaaggat ctcaagaaga tcctttgatc 3120
   ttttctacgg ggtctgacgc tcagtggaac gaaaactcac gttaagggat tttggtcatg 3180
   agattatcaa aaaggatctt cacctagatc cttttaaatt aaaaatgaag ttttaaatca 3240
   atctaaagta tatatgagta aacttggtct gacagttacc aatgcttaat cagtgaggca 3300
   cctatctcag cgatctgtct atttcgttca tccatagttg cctgactccc cgtcgtgtag 3360
   ataactacga tacgggaggg cttaccatct ggccccagtg ctgcaatgat accgcgagac 3420
   ccacgctcac cggctccaga tttatcagca ataaaccagc cagccggaag ggccgagcgc 3480
   agaagtggtc ctgcaacttt atccgcctcc atccagtcta ttaattgttg ccgggaagct 3540
   agagtaagta gttcgccagt taatagtttg cgcaacgttg ttgccattgc tacaggcatc 3600
   gtggtgtcac gctcgtcgtt tggtatggct tcattcagct ccggttccca acgatcaagg 3660
   cgagttacat gatcccccat gttgtgcaaa aaagcggtta gctccttcgg tcctccgatc 3720
   gttgtcagaa gtaagttggc cgcagtgtta tcactcatgg ttatggcagc actgcataat 3780
   tctcttactg tcatgccatc cgtaagatgc ttttctgtga ctggtgagta ctcaaccaag 3840
   tcattctgag aatagtgtat gcggcgaccg agttgctctt gcccggcgtc aatacgggat 3900
   aataccgcgc cacatagcag aactttaaaa gtgctcatca ttggaaaacg ttcttcgggg 3960
   cgaaaactct caaggatctt accgctgttg agatccagtt cgatgtaacc cactcgtgca 4020
   cccaactgat cttcagcatc ttttactttc accagcgttt ctgggtgagc aaaaacagga 4080
   aggcaaaatg ccgcaaaaaa gggaataagg gcgacacgga aatgttgaat actcatactc 4140
   ttcctttttc aatattattg aagcatttat cagggttatt gtctcatgag cggatacata 4200
   tttgaatgta tttagaaaaa taaacaaata ggggttccgc gcacatttcc ccgaaaagtg 4260
   ccacctgacg tctaagaaac cattattatc atgacattaa cctataaaaa taggcgtatc 4320
   acgaggccct ttcgtctcgc gcgtttcggt gatgacggtg aaaacctctg acacatgcag 4380
   ctcccggaga cggtcacagc ttgtctgtaa gcggatgccg ggagcagaca agcccgtcag 4440
   ggcgcgtcag cgggtgttgg cgggtgtcgg ggctggctta actatgcggc atcagagcag 4500
   attgtactga gagtgcacca tatatgcggt gtgaaatacc gcacagatgc gtaaggagaa 4560
   aataccgcat caggcgccat tcgccattca ggctgcgcaa ctgttgggaa gggcgatcgg 4620
   tgcgggcctc ttcgctatta cgccagctgg cgaaaggggg atgtgctgca aggcgattaa 4680
   gttgggtaac gccagggttt tcccagtcac gacgttgtaa aacgacggcc agtgccaagc 4740
   ttgcatgcaa ggagatggcg cccaacagtc ccccggccac ggggcctgcc accataccca 4800
   cgccgaaaca agcgctcatg agcccgaagt ggcgagcccg atcttcccca tcggtgatgt 4860
   cggcgatata ggcgccagca accgcacctg tggcgccggt gatgccggcc acgatgcgtc 4920
   cggcgtagag gatcgagatc tcgatcccgc gaaattaata cgactcacta tagggagacc 4980
   acaacggttt ccctctagaa ataattttgt ttaactttaa gaaggagata taccatggca 5040
   ca 5042
<210> 12
   <211> 5633
   <212> DNA
   <213> artificial
<220>
   <223> artificial
<400> 12
   catggcgaag ggcgagtttg ttcggacgaa gcctcacgtg aacgtgggga cgattgggca 60
   cgtggaccac gggaagacga cgctgacggc ggcgttaacg tatgtggcgg cggcggagaa 120
   cccgaatgta gaggttaagg actacgggga cattgacaag gcgccggagg agcgtgcgcg 180
   ggggattacg atcaacacgg cgcacgtgga gtacgagacg gcgaagcggc actattccca 240
   cgtggattgc cctgggcacg cggactacat caagaacatg atcacgggtg ccgcgcagat 300
   ggacggggcg atccttgtgg tgtcggcggc ggacgggccg atgccgcaga cgcgggagca 360
   cattttgctg gcgcggcagg tgggggtgcc gtacattgtg gtgttcatga acaaggtgga 420
   catggtggac gaccccgagt tgctggacct ggtggagatg gaggtgcggg accttttgaa 480
   ccagtacgag tttcctgggg acgaggttcc ggtgattcgg gggagtgctc ttttggcgct 540
   tgagcagatg cacaggaacc cgaagacgag gcgtggggag aacgagtggg tggacaagat 600
   ttgggagctg ttggacgcga ttgacgagta cattcccacg ccggtgcggg acgtggacaa 660
   gccgttcttg atgccggtgg aggacgtgtt tacgatcacg ggtcgtggga cggtggccac 720
   gggtcggatt gagcggggca aggtgaaggt tggggacgag gtggagattg tgggccttgc 780
   tccggagacg cggaggacgg tggtgacggg tgtggagatg caccggaaga ccttgcagga 840
   ggggattgct ggggacaatg tgggggtgct cctgcggggt gtgagccggg aggaggtgga 900
   gcgggggcag gtgctggcga agcctgggag cattacgccg cacacgaagt ttgaggcctc 960
   ggtgtatgtg ttgaagaagg aggagggtgg acggcacacg gggttttttt cggggtaccg 1020
   tccgcagttt tactttcgga cgacggacgt gacgggggtg gtgcagttgc ctccgggcgt 1080
   ggagatggtg atgcctgggg acaacgtgac gtttacggtg gagctgatca agccggtggg 1140
   cctggaggag ggtttgcggt ttgccatccg tgagggtggg cggaccgtgg gcgccggcgt 1200
   cgtcaccaag atcctggagc tcggtaccat tgagggtcgc ggttccggcg gtggtatggc 1260
   gctcgatccc gtcattcagc aggtgctcga tcaactcaac cgcatgcctg ccccggacta 1320
   caaacatctc tccgcccagc aatttcgttc ccaacagtcg ctgtttcctc ctgtcaagaa 1380
   ggagcccgtg gccgaggtcc gagagtttga catggatctg cctggccgca cgctcaaggt 1440
   gcgcatgtac cgcccggagg gcgtcgaacc gccctacccc gcgctcgtgt attatcacgg 1500
   cggcggttgg gtcgtcggag acctcgagac gcacgatccc gtctgccgcg tcctcgcgaa 1560
   agacggccgc gcggtcgtgt tctccgtcga ctaccgcctg gcgccggagc acaagttccc 1620
   tgccgccgtg gaagacgcct acgacgcgct tcagtggatc gcggagcgcg cagcggactt 1680
   tcatctcgat ccagcccgca tcgcggtcgg cggagacagc gccggaggga atcttgccgc 1740
   tgtgacgagc atccttgcca aagagcgcgg cgggccggcc atcgcgttcc agctgctcat 1800
   ctacccttcc acggggtacg atccggctca tcctcccgca tctatcgaag aaaatgcgga 1860
   aggctatctc ctgaccggcg gcatgatgct ctggttccgg gatcaatact tgaacagcct 1920
   ggaggaactc acgcatccgt ggttttcacc cgtcctctac ccggacttga gcggcttgcc 1980
   tccggcgtac atcgcgacgg cgcagtacga tccgctgcgc gacgtcggca agctttacgc 2040
   ggaagcgctg aacaaggcgg gcgtcaaggt cgagatcgag aacttcgaag atctgatcca 2100
   cggattcgca cagttttaca gcctttcgcc tggcgcgacg aaggcgctcg tccgcattgc 2160
   ggagaaactt cgagacgcgc tggcctgagg atccggctgc taacaaagcc cgaaaggaag 2220
   ctgagttggc tgctgccacc gctgagcaat aactagcata accccttggg gcctctaaac 2280
   gggtcttgag gggttttttg ctgaaaggag gaactatatc cggatatcca caggacgggt 2340
   gtggtcgcca tgatcgcgta gtcgatagtg gctccaagta gcgaagcgag caggactggg 2400
   cggcggccaa agcggtcgga cagtgctccg agaacgggtg cgcatagaaa ttgcatcaac 2460
   gcatatagcg ctagcagcac gccatagtga ctggcgatgc tgtcggaatg gacgatatcc 2520
   cgcaagaggc ccggcagtac cggcataacc aagcctatgc ctacagcatc cagggtgacg 2580
   gtgccgagga tgacgatgag cgcattgtta gatttcatac acggtgcctg actgcgttag 2640
   caatttaact gtgataaact accgcattaa agcttatcga tgataagctg tcaaacatga 2700
   gaattcgtaa tcatgtcata gctgtttcct gtgtgaaatt gttatccgct cacaattcca 2760
   cacaacatac gagccggaag cataaagtgt aaagcctggg gtgcctaatg agtgagctaa 2820
   ctcacattaa ttgcgttgcg ctcactgccc gctttccagt cgggaaacct gtcgtgccag 2880
   ctgcattaat gaatcggcca acgcgcgggg agaggcggtt tgcgtattgg gcgctcttcc 2940
   gcttcctcgc tcactgactc gctgcgctcg gtcgttcggc tgcggcgagc ggtatcagct 3000
   cactcaaagg cggtaatacg gttatccaca gaatcagggg ataacgcagg aaagaacatg 3060
   tgagcaaaag gccagcaaaa ggccaggaac cgtaaaaagg ccgcgttgct ggcgtttttc 3120
   cataggctcc gcccccctga cgagcatcac aaaaatcgac gctcaagtca gaggtggcga 3180
   aacccgacag gactataaag ataccaggcg tttccccctg gaagctccct cgtgcgctct 3240
   cctgttccga ccctgccgct taccggatac ctgtccgcct ttctcccttc gggaagcgtg 3300
   gcgctttctc atagctcacg ctgtaggtat ctcagttcgg tgtaggtcgt tcgctccaag 3360
   ctgggctgtg tgcacgaacc ccccgttcag cccgaccgct gcgccttatc cggtaactat 3420
   cgtcttgagt ccaacccggt aagacacgac ttatcgccac tggcagcagc cactggtaac 3480
   aggattagca gagcgaggta tgtaggcggt gctacagagt tcttgaagtg gtggcctaac 3540
   tacggctaca ctagaaggac agtatttggt atctgcgctc tgctgaagcc agttaccttc 3600
   ggaaaaagag ttggtagctc ttgatccggc aaacaaacca ccgctggtag cggtggtttt 3660
   tttgtttgca agcagcagat tacgcgcaga aaaaaaggat ctcaagaaga tcctttgatc 3720
   ttttctacgg ggtctgacgc tcagtggaac gaaaactcac gttaagggat tttggtcatg 3780
   agattatcaa aaaggatctt cacctagatc cttttaaatt aaaaatgaag ttttaaatca 3840
   atctaaagta tatatgagta aacttggtct gacagttacc aatgcttaat cagtgaggca 3900
   cctatctcag cgatctgtct atttcgttca tccatagttg cctgactccc cgtcgtgtag 3960
   ataactacga tacgggaggg cttaccatct ggccccagtg ctgcaatgat accgcgagac 4020
   ccacgctcac cggctccaga tttatcagca ataaaccagc cagccggaag ggccgagcgc 4080
   agaagtggtc ctgcaacttt atccgcctcc atccagtcta ttaattgttg ccgggaagct 4140
   agagtaagta gttcgccagt taatagtttg cgcaacgttg ttgccattgc tacaggcatc 4200
   gtggtgtcac gctcgtcgtt tggtatggct tcattcagct ccggttccca acgatcaagg 4260
   cgagttacat gatcccccat gttgtgcaaa aaagcggtta gctccttcgg tcctccgatc 4320
   gttgtcagaa gtaagttggc cgcagtgtta tcactcatgg ttatggcagc actgcataat 4380
   tctcttactg tcatgccatc cgtaagatgc ttttctgtga ctggtgagta ctcaaccaag 4440
   tcattctgag aatagtgtat gcggcgaccg agttgctctt gcccggcgtc aatacgggat 4500
   aataccgcgc cacatagcag aactttaaaa gtgctcatca ttggaaaacg ttcttcgggg 4560
   cgaaaactct caaggatctt accgctgttg agatccagtt cgatgtaacc cactcgtgca 4620
   cccaactgat cttcagcatc ttttactttc accagcgttt ctgggtgagc aaaaacagga 4680
   aggcaaaatg ccgcaaaaaa gggaataagg gcgacacgga aatgttgaat actcatactc 4740
   ttcctttttc aatattattg aagcatttat cagggttatt gtctcatgag cggatacata 4800
   tttgaatgta tttagaaaaa taaacaaata ggggttccgc gcacatttcc ccgaaaagtg 4860
   ccacctgacg tctaagaaac cattattatc atgacattaa cctataaaaa taggcgtatc 4920
   acgaggccct ttcgtctcgc gcgtttcggt gatgacggtg aaaacctctg acacatgcag 4980
   ctcccggaga cggtcacagc ttgtctgtaa gcggatgccg ggagcagaca agcccgtcag 5040
   ggcgcgtcag cgggtgttgg cgggtgtcgg ggctggctta actatgcggc atcagagcag 5100
   attgtactga gagtgcacca tatatgcggt gtgaaatacc gcacagatgc gtaaggagaa 5160
   aataccgcat caggcgccat tcgccattca ggctgcgcaa ctgttgggaa gggcgatcgg 5220
   tgcgggcctc ttcgctatta cgccagctgg cgaaaggggg atgtgctgca aggcgattaa 5280
   gttgggtaac gccagggttt tcccagtcac gacgttgtaa aacgacggcc agtgccaagc 5340
   ttgcatgcaa ggagatggcg cccaacagtc ccccggccac ggggcctgcc accataccca 5400
   cgccgaaaca agcgctcatg agcccgaagt ggcgagcccg atcttcccca tcggtgatgt 5460
   cggcgatata ggcgccagca accgcacctg tggcgccggt gatgccggcc acgatgcgtc 5520
   cggcgtagag gatcgagatc tcgatcccgc gaaattaata cgactcacta tagggagacc 5580
   acaacggttt ccctctagaa ataattttgt ttaactttaa gaaggagata tac 5633
<210> 13
   <211> 5009
   <212> DNA
   <213> artificial
<220>
   <223> artificial
<400> 13
   tatgagccaa atggaactca tcaagaagct acgcgaagcc acgggggccg ggatgatgga      60
   cgtgaagcgg gccctcgagg acgccggctg ggacgaggag aaggcggtcc agctcctccg     120
   ggagcggggg gcgatgaagg ccgccaagaa ggcggaccgg gaagcccgcg agggcatcat     180
   cggccactac atccaccaca accagcgggt gggggtcctg gtggagctca actgcgaaac     240
   ggacttcgtg gcccggaacg agctcttcca gaacctggcc aaggacctcg ccatgcacat     300
   cgccatgatg aacccccgct acgtctccgc cgaggagatc cccgccgagg agctggaaaa     360
   agagcggcag atctacattc aggccgccct gaacgagggg aagccccagc agatcgccga     420
   gaagatcgcc gaaggccgcc tcaagaagta cctggaggag gtggtcctgc tggagcagcc     480
   cttcgtcaag gacgacaagg tcaaggtgaa ggagctcatc cagcaggcca tcgccaagat     540
   cggcgagaac atcgtggtcc gacgcttctg ccgctttgag ctgggggcgg gtaccattga     600
   gggtcgcggt tccggcggtg gtatggcgct cgatcccgtc attcagcagg tgctcgatca     660
   actcaaccgc atgcctgccc cggactacaa acatctctcc gcccagcaat ttcgttccca     720
   acagtcgctg tttcctcctg tcaagaagga gcccgtggcc gaggtccgag agtttgacat     780
   ggatctgcct ggccgcacgc tcaaggtgcg catgtaccgc ccggagggcg tcgaaccgcc 840
   ctaccccgcg ctcgtgtatt atcacggcgg cggttgggtc gtcggagacc tcgagacgca 900
   cgatcccgtc tgccgcgtcc tcgcgaaaga cggccgcgcg gtcgtgttct ccgtcgacta     960
   ccgcctggcg ccggagcaca agttccctgc cgccgtggaa gacgcctacg acgcgcttca 1020
   gtggatcgcg gagcgcgcag cggactttca tctcgatcca gcccgcatcg cggtcggcgg 1080
   agacagcgcc ggagggaatc ttgccgctgt gacgagcatc cttgccaaag agcgcggcgg 1140
   gccggccatc gcgttccagc tgctcatcta cccttccacg gggtacgatc cggctcatcc 1200
   tcccgcatct atcgaagaaa atgcggaagg ctatctcctg accggcggca tgatgctctg 1260
   gttccgggat caatacttga acagcctgga ggaactcacg catccgtggt tttcacccgt 1320
   cctctacccg gacttgagcg gcttgcctcc ggcgtacatc gcgacggcgc agtacgatcc 1380
   gctgcgcgac gtcggcaagc tttacgcgga agcgctgaac aaggcgggcg tcaaggtcga 1440
   gatcgagaac ttcgaagatc tgatccacgg attcgcacag ttttacagcc tttcgcctgg 1500
   cgcgacgaag gcgctcgtcc gcattgcgga gaaacttcga gacgcgctgg cctgaggatc 1560
   cggctgctaa caaagcccga aaggaagctg agttggctgc tgccaccgct gagcaataac 1620
   tagcataacc ccttggggcc tctaaacggg tcttgagggg ttttttgctg aaaggaggaa 1680
   ctatatccgg atatccacag gacgggtgtg gtcgccatga tcgcgtagtc gatagtggct 1740
   ccaagtagcg aagcgagcag gactgggcgg cggccaaagc ggtcggacag tgctccgaga 1800
   acgggtgcgc atagaaattg catcaacgca tatagcgcta gcagcacgcc atagtgactg 1860
   gcgatgctgt cggaatggac gatatcccgc aagaggcccg gcagtaccgg cataaccaag 1920
   cctatgccta cagcatccag ggtgacggtg ccgaggatga cgatgagcgc attgttagat 1980
   ttcatacacg gtgcctgact gcgttagcaa tttaactgtg ataaactacc gcattaaagc 2040
   ttatcgatga taagctgtca aacatgagaa ttcgtaatca tgtcatagct gtttcctgtg 2100
   tgaaattgtt atccgctcac aattccacac aacatacgag ccggaagcat aaagtgtaaa 2160
   gcctggggtg cctaatgagt gagctaactc acattaattg cgttgcgctc actgcccgct 2220
   ttccagtcgg gaaacctgtc gtgccagctg cattaatgaa tcggccaacg cgcggggaga 2280
   ggcggtttgc gtattgggcg ctcttccgct tcctcgctca ctgactcgct gcgctcggtc 2340
   gttcggctgc ggcgagcggt atcagctcac tcaaaggcgg taatacggtt atccacagaa 2400
   tcaggggata acgcaggaaa gaacatgtga gcaaaaggcc agcaaaaggc caggaaccgt 2460
   aaaaaggccg cgttgctggc gtttttccat aggctccgcc cccctgacga gcatcacaaa 2520
   aatcgacgct caagtcagag gtggcgaaac ccgacaggac tataaagata ccaggcgttt 2580
   ccccctggaa gctccctcgt gcgctctcct gttccgaccc tgccgcttac cggatacctg 2640
   tccgcctttc tcccttcggg aagcgtggcg ctttctcata gctcacgctg taggtatctc 2700
   agttcggtgt aggtcgttcg ctccaagctg ggctgtgtgc acgaaccccc cgttcagccc 2760
   gaccgctgcg ccttatccgg taactatcgt cttgagtcca acccggtaag acacgactta 2820
   tcgccactgg cagcagccac tggtaacagg attagcagag cgaggtatgt aggcggtgct 2880
   acagagttct tgaagtggtg gcctaactac ggctacacta gaaggacagt atttggtatc 2940
   tgcgctctgc tgaagccagt taccttcgga aaaagagttg gtagctcttg atccggcaaa 3000
   caaaccaccg ctggtagcgg tggttttttt gtttgcaagc agcagattac gcgcagaaaa 3060
   aaaggatctc aagaagatcc tttgatcttt tctacggggt ctgacgctca gtggaacgaa 3120
   aactcacgtt aagggatttt ggtcatgaga ttatcaaaaa ggatcttcac ctagatcctt 3180
   ttaaattaaa aatgaagttt taaatcaatc taaagtatat atgagtaaac ttggtctgac 3240
   agttaccaat gcttaatcag tgaggcacct atctcagcga tctgtctatt tcgttcatcc 3300
   atagttgcct gactccccgt cgtgtagata actacgatac gggagggctt accatctggc 3360
   cccagtgctg caatgatacc gcgagaccca cgctcaccgg ctccagattt atcagcaata 3420
   aaccagccag ccggaagggc cgagcgcaga agtggtcctg caactttatc cgcctccatc 3480
   cagtctatta attgttgccg ggaagctaga gtaagtagtt cgccagttaa tagtttgcgc 3540
   aacgttgttg ccattgctac aggcatcgtg gtgtcacgct cgtcgtttgg tatggcttca 3600
   ttcagctccg gttcccaacg atcaaggcga gttacatgat cccccatgtt gtgcaaaaaa 3660
   gcggttagct ccttcggtcc tccgatcgtt gtcagaagta agttggccgc agtgttatca 3720
   ctcatggtta tggcagcact gcataattct cttactgtca tgccatccgt aagatgcttt 3780
   tctgtgactg gtgagtactc aaccaagtca ttctgagaat agtgtatgcg gcgaccgagt 3840
   tgctcttgcc cggcgtcaat acgggataat accgcgccac atagcagaac tttaaaagtg 3900
   ctcatcattg gaaaacgttc ttcggggcga aaactctcaa ggatcttacc gctgttgaga 3960
   tccagttcga tgtaacccac tcgtgcaccc aactgatctt cagcatcttt tactttcacc 4020
   agcgtttctg ggtgagcaaa aacaggaagg caaaatgccg caaaaaaggg aataagggcg 4080
   acacggaaat gttgaatact catactcttc ctttttcaat attattgaag catttatcag 4140
   ggttattgtc tcatgagcgg atacatattt gaatgtattt agaaaaataa acaaataggg 4200
   gttccgcgca catttccccg aaaagtgcca cctgacgtct aagaaaccat tattatcatg 4260
   acattaacct ataaaaatag gcgtatcacg aggccctttc gtctcgcgcg tttcggtgat 4320
   gacggtgaaa acctctgaca catgcagctc ccggagacgg tcacagcttg tctgtaagcg 4380
   gatgccggga gcagacaagc ccgtcagggc gcgtcagcgg gtgttggcgg gtgtcggggc 4440
   tggcttaact atgcggcatc agagcagatt gtactgagag tgcaccatat atgcggtgtg 4500
   aaataccgca cagatgcgta aggagaaaat accgcatcag gcgccattcg ccattcaggc   4560
   tgcgcaactg ttgggaaggg cgatcggtgc gggcctcttc gctattacgc cagctggcga   4620
   aagggggatg tgctgcaagg cgattaagtt gggtaacgcc agggttttcc cagtcacgac   4680
   gttgtaaaac gacggccagt gccaagcttg catgcaagga gatggcgccc aacagtcccc   4740
   cggccacggg gcctgccacc atacccacgc cgaaacaagc gctcatgagc ccgaagtggc   4800
   gagcccgatc ttccccatcg gtgatgtcgg cgatataggc gccagcaacc gcacctgtgg   4860
   cgccggtgat gccggccacg atgcgtccgg cgtagaggat cgagatctcg atcccgcgaa   4920
   attaatacga ctcactatag ggagaccaca acggtttccc tctagaaata attttgttta   4980
   actttaagaa ggagatatac catggcaca                                      5009
<210> 14
   <211> 7304
   <212> DNA
   <213> artificial
<220>
   <223> artificial
<400> 14
   catggatgaa caggctctat tagggctaaa tccaaatgct gattcagact ttagacaaag     60
   ggccctggcc tattttgagc agttaaaaat ttccccagat gcctggcagg tgtgtgcaga    120
   agctctagcc cagaggacat acagtgatga tcatgtgaag tttttctgct ttcaagtact    180
   ggaacatcaa gttaaataca aatactcaga actaaccact gttcaacaac agctaattag    240
   ggagacgctc atatcatggc tgcaagctca gatgctgaat ccccaaccag agaagacctt    300
   tatacgaaat aaagccgccc aagtcttcgc cttgcttttt gttacagagt atctcactaa    360
   gtggcccaag tttttttttg acattctctc agtagtggac ctaaatccaa ggggagtaga    420
   tctctacctg cgaatcctca tggctattga ttcagagttg gtggatcgtg atgtggtgca    480
   tacatcagag gaggctcgta ggaatactct cataaaagat accatgaggg aacagtgcat    540
   tccaaatctg gtggaatcat ggtaccaaat attacaaaat tatcagttta ctaattctga    600
   agtgacgtgt cagtgccttg aagtagttgg ggcttatgtc tcttggatag acttatccct    660
   tatagccaat gataggttta taaatatgct gctaggtcat atgtcaatag aagttctacg    720
   ggaagaagca tgtgactgtt tatttgaagt tgtaaataaa ggaatggacc ctgttgataa    780
   aatgaaacta gtggaatctt tgtgtcaagt attacagtct gctgggtttt tcagcattga    840
   ccaggaagaa gatgttgact tcctggccag attttctaag ttggtaaatg gaatgggaca    900
   gtcattgata gttagttgga gtaaattaat taagaatggg gatattaaga atgctcaaga    960
   ggcactacaa gctattgaaa caaaagtggc actgatgttg cagctactaa ttcatgagga 1020
   tgatgatatt tcttctaata ttattggatt ttgttacgat tatcttcata ttttgaaaca 1080
   gcttacagtg ctctcggatc agcaaaaagc taatgtagag gcaatcatgt tggccgttat 1140
   gaaaaaattg acttacgatg aagaatataa ctttgaaaat gagggtgaag atgaagccat 1200
   gtttgtagaa tatagaaaac aactgaagtt actgttggac aggcttgctc aagtttcacc 1260
   agagttacta ctggcctctg ttcgcagagt ttttagttct acactgcaga attggcagac 1320
   tacacggttt atggaagttg aagtagcaat aagattgctg tatatgttgg cagaagctct 1380
   tccagtatct catggtgctc acttctcagg tgatgtttca aaagctagtg ctttgcagga 1440
   tatgatgcga actctggtaa catcaggagt cagttcctat cagcatacat ctgtgacatt 1500
   ggagttcttc gaaactgttg ttagatatga aaagtttttc acagttgaac ctcagcacat 1560
   tccatgtgta ctaatggctt tcttagatca cagaggtctg cggcattcca gtgcaaaagt 1620
   tcggagcagg acggcttacc tgttttctag atttgtcaaa tctctcaata agcaaatgaa 1680
   tcctttcatt gaggatattt tgaatagaat acaagattta ttagagcttt ctccacctga 1740
   gaatggccac cagtccttac tgagcagcga tgatcaactt tttatttatg agacagctgg 1800
   agtgctgatt gttaatagtg aatatccggc agaaaggaaa caagccttaa tgaggaatct 1860
   gttgactcca ctaatggaga agtttaaaat tctgttagaa aagttgatgc tggcacaaga 1920
   tgaagaaagg caagcctctc tagcagactg tcttaaccat gctgttggat ttgcaagtcg 1980
   aaccagtaaa gctttcagca acaaacagac tgtgaaacaa tgtggctgtt ccgaagttta 2040
   tctggactgt ttacagacat tcttgccagc cctcagttgt cccttacaaa aggatattct 2100
   cagaagtgga gtccgtactt tccttcatcg aatgattatt tgcctggagg aagaagttct 2160
   tccgttcatt ccatctgctt cagaacatat gctcaaagat tgtgaagcaa aagatctcca 2220
   ggagttcatt cctcttatca accagattac ggccaaattc aagatacagg tatccccgtt 2280
   tttacaacag atgttcatgc ccctgcttca tgcaattttt gaagtgctgc tccggccagc 2340
   agaagaaaat gaccagtctg ctgctttaga gaagcagatg ttgcggagga gttactttgc 2400
   tttcctgcaa acagtcacag gcagtgggat gagcgaagtt atagcaaatc aaggtgcaga 2460
   gaatgtagaa agagtgttgg ttactgttat ccaaggagca gttgaatatc cagatccaat 2520
   tgcacagaaa acatgtttta tcatcctctc aaagttggta gaactctggg gaggtaaaga 2580
   tggaccagtg ggatttgctg attttgttta taagcacatt gtccccgcat gtttcctagc 2640
   acctttaaaa caaacctttg acctggcaga tgcacaaaca gtattggctt tatctgagtg 2700
   tgcagtgaca ctgaaaacaa ttcatctcaa acggggccca gaatgtgttc agtatcttca 2760
   acaagaatac ctgccctcct tgcaagtagc tccagaaata attcaggagt tttgtcaagc 2820
   gcttcagcag cctgatgcta aagtttttaa aaattactta aaggtgttct tccagagagc 2880
   aaagcccgag ctcggtacca ttgagggtcg cggttccggc ggtggtatgg cgctcgatcc 2940
   cgtcattcag caggtgctcg atcaactcaa ccgcatgcct gccccggact acaaacatct 3000
   ctccgcccag caatttcgtt cccaacagtc gctgtttcct cctgtcaaga aggagcccgt 3060
   ggccgaggtc cgagagtttg acatggatct gcctggccgc acgctcaagg tgcgcatgta 3120
   ccgcccggag ggcgtcgaac cgccctaccc cgcgctcgtg tattatcacg gcggcggttg 3180
   ggtcgtcgga gacctcgaga cgcacgatcc cgtctgccgc gtcctcgcga aagacggccg 3240
   cgcggtcgtg ttctccgtcg actaccgcct ggcgccggag cacaagttcc ctgccgccgt 3300
   ggaagacgcc tacgacgcgc ttcagtggat cgcggagcgc gcagcggact ttcatctcga 3360
   tccagcccgc atcgcggtcg gcggagacag cgccggaggg aatcttgccg ctgtgacgag 3420
   catccttgcc aaagagcgcg gcgggccggc catcgcgttc cagctgctca tctacccttc 3480
   cacggggtac gatccggctc atcctcccgc atctatcgaa gaaaatgcgg aaggctatct 3540
   cctgaccggc ggcatgatgc tctggttccg ggatcaatac ttgaacagcc tggaggaact 3600
   cacgcatccg tggttttcac ccgtcctcta cccggacttg agcggcttgc ctccggcgta 3660
   catcgcgacg gcgcagtacg atccgctgcg cgacgtcggc aagctttacg cggaagcgct 3720
   gaacaaggcg ggcgtcaagg tcgagatcga gaacttcgaa gatctgatcc acggattcgc 3780
   acagttttac agcctttcgc ctggcgcgac gaaggcgctc gtccgcattg cggagaaact 3840
   tcgagacgcg ctggcctgag gatccggctg ctaacaaagc ccgaaaggaa gctgagttgg 3900
   ctgctgccac cgctgagcaa taactagcat aaccccttgg ggcctctaaa cgggtcttga 3960
   ggggtttttt gctgaaagga ggaactatat ccggatatcc acaggacggg tgtggtcgcc 4020
   atgatcgcgt agtcgatagt ggctccaagt agcgaagcga gcaggactgg gcggcggcca 4080
   aagcggtcgg acagtgctcc gagaacgggt gcgcatagaa attgcatcaa cgcatatagc 4140
   gctagcagca cgccatagtg actggcgatg ctgtcggaat ggacgatatc ccgcaagagg 4200
   cccggcagta ccggcataac caagcctatg cctacagcat ccagggtgac ggtgccgagg 4260
   atgacgatga gcgcattgtt agatttcata cacggtgcct gactgcgtta gcaatttaac 4320
   tgtgataaac taccgcatta aagcttatcg atgataagct gtcaaacatg agaattcgta 4380
   atcatgtcat agctgtttcc tgtgtgaaat tgttatccgc tcacaattcc acacaacata 4440
   cgagccggaa gcataaagtg taaagcctgg ggtgcctaat gagtgagcta actcacatta 4500
   attgcgttgc gctcactgcc cgctttccag tcgggaaacc tgtcgtgcca gctgcattaa 4560
   tgaatcggcc aacgcgcggg gagaggcggt ttgcgtattg ggcgctcttc cgcttcctcg 4620
   ctcactgact cgctgcgctc ggtcgttcgg ctgcggcgag cggtatcagc tcactcaaag 4680
   gcggtaatac ggttatccac agaatcaggg gataacgcag gaaagaacat gtgagcaaaa 4740
   ggccagcaaa aggccaggaa ccgtaaaaag gccgcgttgc tggcgttttt ccataggctc 4800
   cgcccccctg acgagcatca caaaaatcga cgctcaagtc agaggtggcg aaacccgaca 4860
   ggactataaa gataccaggc gtttccccct ggaagctccc tcgtgcgctc tcctgttccg 4920
   accctgccgc ttaccggata cctgtccgcc tttctccctt cgggaagcgt ggcgctttct 4980
   catagctcac gctgtaggta tctcagttcg gtgtaggtcg ttcgctccaa gctgggctgt 5040
   gtgcacgaac cccccgttca gcccgaccgc tgcgccttat ccggtaacta tcgtcttgag 5100
   tccaacccgg taagacacga cttatcgcca ctggcagcag ccactggtaa caggattagc 5160
   agagcgaggt atgtaggcgg tgctacagag ttcttgaagt ggtggcctaa ctacggctac 5220
   actagaagga cagtatttgg tatctgcgct ctgctgaagc cagttacctt cggaaaaaga 5280
   gttggtagct cttgatccgg caaacaaacc accgctggta gcggtggttt ttttgtttgc 5340
   aagcagcaga ttacgcgcag aaaaaaagga tctcaagaag atcctttgat cttttctacg 5400
   gggtctgacg ctcagtggaa cgaaaactca cgttaaggga ttttggtcat gagattatca 5460
   aaaaggatct tcacctagat ccttttaaat taaaaatgaa gttttaaatc aatctaaagt 5520
   atatatgagt aaacttggtc tgacagttac caatgcttaa tcagtgaggc acctatctca 5580
   gcgatctgtc tatttcgttc atccatagtt gcctgactcc ccgtcgtgta gataactacg 5640
   atacgggagg gcttaccatc tggccccagt gctgcaatga taccgcgaga cccacgctca 5700
   ccggctccag atttatcagc aataaaccag ccagccggaa gggccgagcg cagaagtggt 5760
   cctgcaactt tatccgcctc catccagtct attaattgtt gccgggaagc tagagtaagt 5820
   agttcgccag ttaatagttt gcgcaacgtt gttgccattg ctacaggcat cgtggtgtca 5880
   cgctcgtcgt ttggtatggc ttcattcagc tccggttccc aacgatcaag gcgagttaca 5940
   tgatccccca tgttgtgcaa aaaagcggtt agctccttcg gtcctccgat cgttgtcaga 6000
   agtaagttgg ccgcagtgtt atcactcatg gttatggcag cactgcataa ttctcttact 6060
   gtcatgccat ccgtaagatg cttttctgtg actggtgagt actcaaccaa gtcattctga 6120
   gaatagtgta tgcggcgacc gagttgctct tgcccggcgt caatacggga taataccgcg 6180
   ccacatagca gaactttaaa agtgctcatc attggaaaac gttcttcggg gcgaaaactc 6240
   tcaaggatct taccgctgtt gagatccagt tcgatgtaac ccactcgtgc acccaactga 6300
   tcttcagcat cttttacttt caccagcgtt tctgggtgag caaaaacagg aaggcaaaat 6360
   gccgcaaaaa agggaataag ggcgacacgg aaatgttgaa tactcatact cttccttttt 6420
   caatattatt gaagcattta tcagggttat tgtctcatga gcggatacat atttgaatgt 6480
   atttagaaaa ataaacaaat aggggttccg cgcacatttc cccgaaaagt gccacctgac 6540
   gtctaagaaa ccattattat catgacatta acctataaaa ataggcgtat cacgaggccc 6600
   tttcgtctcg cgcgtttcgg tgatgacggt gaaaacctct gacacatgca gctcccggag 6660
   acggtcacag cttgtctgta agcggatgcc gggagcagac aagcccgtca gggcgcgtca 6720
   gcgggtgttg gcgggtgtcg gggctggctt aactatgcgg catcagagca gattgtactg 6780
   agagtgcacc atatatgcgg tgtgaaatac cgcacagatg cgtaaggaga aaataccgca 6840
   tcaggcgcca ttcgccattc aggctgcgca actgttggga agggcgatcg gtgcgggcct 6900
   cttcgctatt acgccagctg gcgaaagggg gatgtgctgc aaggcgatta agttgggtaa 6960
   cgccagggtt ttcccagtca cgacgttgta aaacgacggc cagtgccaag cttgcatgca 7020
   aggagatggc gcccaacagt cccccggcca cggggcctgc caccataccc acgccgaaac 7080
   aagcgctcat gagcccgaag tggcgagccc gatcttcccc atcggtgatg tcggcgatat 7140
   aggcgccagc aaccgcacct gtggcgccgg tgatgccggc cacgatgcgt ccggcgtaga 7200
   ggatcgagat ctcgatcccg cgaaattaat acgactcact atagggagac cacaacggtt 7260
   tccctctaga aataattttg tttaacttta agaaggagat atac 7304
<210> 15
   <211> 7025
   <212> DNA
   <213> artificial
<220>
   <223> artificial
<400> 15
   atccggatat agttcctcct ttcagcaaaa aacccctcaa gacccgttta gaggccccaa 60
   ggggttatgc tagttattgc tcagcggtgg cagcagccaa ctcagcttcc tttcgggctt 120
   tgttagcagc cggatctcag tggtggtggt ggtggtgctc gagtgcggcc gcaagcttgt 180
   cgacggagct cgaattcgga tcctcaggcc agcgcgtctc gaagtttctc cgcaatgcgg 240
   acgagcgcct tcgtcgcgcc aggcgaaagg ctgtaaaact gtgcgaatcc gtggatcaga 300
   tcttcgaagt tctcgatctc gaccttgacg cccgccttgt tcagcgcttc cgcgtaaagc 360
   ttgccgacgt cgcgcagcgg atcgtactgc gccgtcgcga tgtacgccgg aggcaagccg 420
   ctcaagtccg ggtagaggac gggtgaaaac cacggatgcg tgagttcctc caggctgttc 480
   aagtattgat cccggaacca gagcatcatg ccgccggtca ggagatagcc ttccgcattt 540
   tcttcgatag atgcgggagg atgagccgga tcgtaccccg tggaagggta gatgagcagc 600
   tggaacgcga tggccggccc gccgcgctct ttggcaagga tgctcgtcac agcggcaaga 660
   ttccctccgg cgctgtctcc gccgaccgcg atgcgggctg gatcgagatg aaagtccgct 720
   gcgcgctccg cgatccactg aagcgcgtcg taggcgtctt ccacggcggc agggaacttg 780
   tgctccggcg ccaggcggta gtcgacggag aacacgaccg cgcggccgtc tttcgcgagg 840
   acgcggcaga cgggatcgtg cgtctcgagg tctccgacga cccaaccgcc gccgtgataa 900
   tacacgagcg cggggtaggg cggttcgacg ccctccgggc ggtacatgcg caccttgagc 960
   gtgcggccag gcagatccat gtcaaactct cggacctcgg ccacgggctc cttcttgaca 1020
   ggaggaaaca gcgactgttg ggaacgaaat tgctgggcgg agagatgttt gtagtccggg 1080
   gcaggcatgc ggttgagttg atcgagcacc tgctgaatga cgggatcgag cgccatacca 1140
   ccgccggaac cgcgaccctc aatggtaccg agctctagag tggaacctcc atttaactca 1200
   ccctccaata tacgaacaaa tccggtttta cttccattat ttcgaactaa actactatct 1260
   aaattatccc ccaacattat tggggctaaa aaaagattat acaactttaa atatttatca 1320
   ctcttcttag aaaacgcata cagcatatca tggtagtcat caaaagtgta atccctaact 1380
   tttaagctct tggaaatcac acctcttagg ttttgcaaca taatactccc gtacttatta 1440
   agctttatca tcctttcatc aaaattgtct ggaaacttat tcactagata atcagatatc 1500
   accctaattg cgttcaagtg atttagaaca gctaaagtca acaaagactt aacctcctca 1560
   gctttctcga cgtcaatagg ttcataatca taatagaaaa atagagccga accgagttca 1620
   tgatatatga aagagaagtt actctttaga ggattacaat tatcgtcgaa ctgattctgg 1680
   taatactcgc cggcttttcc catatcgtga aggacaacga cgtccttaac catttcctta 1740
   acaccgttta gatcaagaac tattccatat ctctccaatc ttctcgagat aatcttataa 1800
   taagactcac ttattttacc atctaaaacc ctataagaac caatagcgtg atcaattaaa 1860
   ccttgtttct cataagcgca aggcttgatc aacatatggc tgccgcgcgg caccaggccg 1920
   ctgctgtgat gatgatgatg atggctgctg cccatggtat atctccttct taaagttaaa 1980
   caaaattatt tctagagggg aattgttatc cgctcacaat tcccctatag tgagtcgtat 2040
   taatttcgcg ggatcgagat ctcgatcctc tacgccggac gcatcgtggc cggcatcacc 2100
   ggcgccacag gtgcggttgc tggcgcctat atcgccgaca tcaccgatgg ggaagatcgg 2160
   gctcgccact tcgggctcat gagcgcttgt ttcggcgtgg gtatggtggc aggccccgtg 2220
   gccgggggac tgttgggcgc catctccttg catgcaccat tccttgcggc ggcggtgctc 2280
   aacggcctca acctactact gggctgcttc ctaatgcagg agtcgcataa gggagagcgt 2340
   cgagatcccg gacaccatcg aatggcgcaa aacctttcgc ggtatggcat gatagcgccc 2400
   ggaagagagt caattcaggg tggtgaatgt gaaaccagta acgttatacg atgtcgcaga 2460
   gtatgccggt gtctcttatc agaccgtttc ccgcgtggtg aaccaggcca gccacgtttc 2520
   tgcgaaaacg cgggaaaaag tggaagcggc gatggcggag ctgaattaca ttcccaaccg 2580
   cgtggcacaa caactggcgg gcaaacagtc gttgctgatt ggcgttgcca cctccagtct 2640
   ggccctgcac gcgccgtcgc aaattgtcgc ggcgattaaa tctcgcgccg atcaactggg 2700
   tgccagcgtg gtggtgtcga tggtagaacg aagcggcgtc gaagcctgta aagcggcggt 2760
   gcacaatctt ctcgcgcaac gcgtcagtgg gctgatcatt aactatccgc tggatgacca 2820
   ggatgccatt gctgtggaag ctgcctgcac taatgttccg gcgttatttc ttgatgtctc 2880
   tgaccagaca cccatcaaca gtattatttt ctcccatgaa gacggtacgc gactgggcgt 2940
   ggagcatctg gtcgcattgg gtcaccagca aatcgcgctg ttagcgggcc cattaagttc 3000
   tgtctcggcg cgtctgcgtc tggctggctg gcataaatat ctcactcgca atcaaattca 3060
   gccgatagcg gaacgggaag gcgactggag tgccatgtcc ggttttcaac aaaccatgca 3120
   aatgctgaat gagggcatcg ttcccactgc gatgctggtt gccaacgatc agatggcgct 3180
   gggcgcaatg cgcgccatta ccgagtccgg gctgcgcgtt ggtgcggata tctcggtagt 3240
   gggatacgac gataccgaag acagctcatg ttatatcccg ccgttaacca ccatcaaaca 3300
   ggattttcgc ctgctggggc aaaccagcgt ggaccgcttg ctgcaactct ctcagggcca 3360
   ggcggtgaag ggcaatcagc tgttgcccgt ctcactggtg aaaagaaaaa ccaccctggc 3420
   gcccaatacg caaaccgcct ctccccgcgc gttggccgat tcattaatgc agctggcacg 3480
   acaggtttcc cgactggaaa gcgggcagtg agcgcaacgc aattaatgta agttagctca 3540
   ctcattaggc accgggatct cgaccgatgc ccttgagagc cttcaaccca gtcagctcct 3600
   tccggtgggc gcggggcatg actatcgtcg ccgcacttat gactgtcttc tttatcatgc 3660
   aactcgtagg acaggtgccg gcagcgctct gggtcatttt cggcgaggac cgctttcgct 3720
   ggagcgcgac gatgatcggc ctgtcgcttg cggtattcgg aatcttgcac gccctcgctc 3780
   aagccttcgt cactggtccc gccaccaaac gtttcggcga gaagcaggcc attatcgccg 3840
   gcatggcggc cccacgggtg cgcatgatcg tgctcctgtc gttgaggacc cggctaggct 3900
   ggcggggttg ccttactggt tagcagaatg aatcaccgat acgcgagcga acgtgaagcg 3960
   actgctgctg caaaacgtct gcgacctgag caacaacatg aatggtcttc ggtttccgtg 4020
   tttcgtaaag tctggaaacg cggaagtcag cgccctgcac cattatgttc cggatctgca 4080
   tcgcaggatg ctgctggcta ccctgtggaa cacctacatc tgtattaacg aagcgctggc 4140
   attgaccctg agtgattttt ctctggtccc gccgcatcca taccgccagt tgtttaccct 4200
   cacaacgttc cagtaaccgg gcatgttcat catcagtaac ccgtatcgtg agcatcctct 4260
   ctcgtttcat cggtatcatt acccccatga acagaaatcc cccttacacg gaggcatcag 4320
   tgaccaaaca ggaaaaaacc gcccttaaca tggcccgctt tatcagaagc cagacattaa 4380
   cgcttctgga gaaactcaac gagctggacg cggatgaaca ggcagacatc tgtgaatcgc 4440
   ttcacgacca cgctgatgag ctttaccgca gctgcctcgc gcgtttcggt gatgacggtg 4500
   aaaacctctg acacatgcag ctcccggaga cggtcacagc ttgtctgtaa gcggatgccg 4560
   ggagcagaca agcccgtcag ggcgcgtcag cgggtgttgg cgggtgtcgg ggcgcagcca 4620
   tgacccagtc acgtagcgat agcggagtgt atactggctt aactatgcgg catcagagca 4680
   gattgtactg agagtgcacc atatatgcgg tgtgaaatac cgcacagatg cgtaaggaga 4740
   aaataccgca tcaggcgctc ttccgcttcc tcgctcactg actcgctgcg ctcggtcgtt 4800
   cggctgcggc gagcggtatc agctcactca aaggcggtaa tacggttatc cacagaatca 4860
   ggggataacg caggaaagaa catgtgagca aaaggccagc aaaaggccag gaaccgtaaa 4920
   aaggccgcgt tgctggcgtt tttccatagg ctccgccccc ctgacgagca tcacaaaaat 4980
   cgacgctcaa gtcagaggtg gcgaaacccg acaggactat aaagatacca ggcgtttccc 5040
   cctggaagct ccctcgtgcg ctctcctgtt ccgaccctgc cgcttaccgg atacctgtcc 5100
   gcctttctcc cttcgggaag cgtggcgctt tctcatagct cacgctgtag gtatctcagt 5160
   tcggtgtagg tcgttcgctc caagctgggc tgtgtgcacg aaccccccgt tcagcccgac 5220
   cgctgcgcct tatccggtaa ctatcgtctt gagtccaacc cggtaagaca cgacttatcg 5280
   ccactggcag cagccactgg taacaggatt agcagagcga ggtatgtagg cggtgctaca 5340
   gagttcttga agtggtggcc taactacggc tacactagaa ggacagtatt tggtatctgc 5400
   gctctgctga agccagttac cttcggaaaa agagttggta gctcttgatc cggcaaacaa 5460
   accaccgctg gtagcggtgg tttttttgtt tgcaagcagc agattacgcg cagaaaaaaa 5520
   ggatctcaag aagatccttt gatcttttct acggggtctg acgctcagtg gaacgaaaac 5580
   tcacgttaag ggattttggt catgaacaat aaaactgtct gcttacataa acagtaatac 5640
   aaggggtgtt atgagccata ttcaacggga aacgtcttgc tctaggccgc gattaaattc 5700
   caacatggat gctgatttat atgggtataa atgggctcgc gataatgtcg ggcaatcagg 5760
   tgcgacaatc tatcgattgt atgggaagcc cgatgcgcca gagttgtttc tgaaacatgg 5820
   caaaggtagc gttgccaatg atgttacaga tgagatggtc agactaaact ggctgacgga 5880
   atttatgcct cttccgacca tcaagcattt tatccgtact cctgatgatg catggttact   5940
   caccactgcg atccccggga aaacagcatt ccaggtatta gaagaatatc ctgattcagg   6000
   tgaaaatatt gttgatgcgc tggcagtgtt cctgcgccgg ttgcattcga ttcctgtttg   6060
   taattgtcct tttaacagcg atcgcgtatt tcgtctcgct caggcgcaat cacgaatgaa   6120
   taacggtttg gttgatgcga gtgattttga tgacgagcgt aatggctggc ctgttgaaca   6180
   agtctggaaa gaaatgcata aacttttgcc attctcaccg gattcagtcg tcactcatgg   6240
   tgatttctca cttgataacc ttatttttga cgaggggaaa ttaataggtt gtattgatgt   6300
   tggacgagtc ggaatcgcag accgatacca ggatcttgcc atcctatgga actgcctcgg   6360
   tgagttttct ccttcattac agaaacggct ttttcaaaaa tatggtattg ataatcctga   6420
   tatgaataaa ttgcagtttc atttgatgct cgatgagttt ttctaagaat taattcatga   6480
   gcggatacat atttgaatgt atttagaaaa ataaacaaat aggggttccg cgcacatttc   6540
   cccgaaaagt gccacctgaa attgtaaacg ttaatatttt gttaaaattc gcgttaaatt   6600
   tttgttaaat cagctcattt tttaaccaat aggccgaaat cggcaaaatc ccttataaat   6660
   caaaagaata gaccgagata gggttgagtg ttgttccagt ttggaacaag agtccactat   6720
   taaagaacgt ggactccaac gtcaaagggc gaaaaaccgt ctatcagggc gatggcccac   6780
   tacgtgaacc atcaccctaa tcaagttttt tggggtcgag gtgccgtaaa gcactaaatc   6840
   ggaaccctaa agggagcccc cgatttagag cttgacgggg aaagccggcg aacgtggcga   6900
   gaaaggaagg gaagaaagcg aaaggagcgg gcgctagggc gctggcaagt gtagcggtca   6960
   cgctgcgcgt aaccaccaca cccgccgcgc ttaatgcgcc gctacagggc gcgtcccatt   7020
   cgcca                                                               7025
<210> 16
   <211> 27
   <212> DNA
   <213> artificial
<220>
   <223> artificial
<400> 16
   ccatggcgct cgatcccgtc attcagc 27
<210> 17
   <211> 24
   <212> DNA
   <213> artificial
<220>
   <223> artificial
<400> 17
   gagctcctag gccagcgcgt ctcg 24
<210> 18
   <211> 18
   <212> DNA
   <213> artificial
<220>
   <223> artificial
<400> 18
   ccatggtgag caagggcg 18
<210> 19
   <211> 27
   <212> DNA
   <213> artificial
<220>
   <223> artificial
<400> 19
   gcggccgcct ttgtacagct cgtccat 27
<210> 20
   <211> 54
   <212> DNA
   <213> artificial
<220>
   <223> artificial
<400> 20
   gagctcggta ccattgaggg tcgcggttcc ggcggtggta tggcgctcga tccc 54
<210> 21
   <211> 18
   <212> DNA
   <213> artificial
<220>
   <223> artificial
<400> 21
   ggatcctcag gccagcgc 18
<210> 22
   <211> 20
   <212> DNA
   <213> artificial
<220>
   <223> artificial
<400> 22
   taatacgact cactataggg 20
<210> 23
   <211> 35
   <212> DNA
   <213> artificial
<220>
   <223> artificial
<400> 23
   attccctccg gcctagtctc cgccgaccgc gatgc 35
<210> 24
   <211> 35
   <212> DNA
   <213> artificial
<220>
   <223> artificial
<400> 24
   cggtcggcgg agactaggcc ggagggaatc ttgcc 35
<210> 25
   <211> 20
   <212> DNA
<213> artificial
<220>
   <223> artificial
<400> 25
   ctagttattg ctcagcggtg 20
<210> 26
   <211> 35
   <212> DNA
   <213> artificial
<220>
   <223> artificial
<400> 26
   ggaattctaa tacgactcac tataggagag atgcc 35
<210> 27
   <211> 35
   <212> DNA
   <213> artificial
<220>
   <223> artificial
<400> 27
   gtccgttcag ccgctccggc atctctccta tagtg 35
<210> 28
   <211> 35
   <212> DNA
   <213> artificial
<220>
   <223> artificial
<400> 28
   ctccggtttt agagaccggt ccgttcagcc gctcc 35
<210> 29
   <211> 35
   <212> DNA
   <213> artificial
<220>
   <223> artificial
<400> 29
   ccggtagagt tgcccctact ccggttttag agacc 35
<210> 30
   <211> 35
   <212> DNA
   <213> artificial
<220>
   <223> artificial
<400> 30
   gagaggggga tttgaacccc cggtagagtt gcccc 35
<210> 31
   <211> 42
   <212> DNA
   <213> artificial
<220>
   <223> artificial
<400> 31
   aagcttggat ggatcacctg gcggagagag ggggatttga ac 42
<210> 32
   <211> 18
   <212> DNA
   <213> artificial
<220>
   <223> artificial
<400> 32
   caggaaacag ctatgacc 18
<210> 33
   <211> 27
   <212> DNA
   <213> artificial
<220>
   <223> artificial
<400> 33
   catatggagg cgacccttcc cgttttg 27
<210> 34
   <211> 32
<212> DNA
   <213> artificial
<220>
   <223> artificial
<400> 34
   gagctcgcgc cagaggacca cccgctccag gg 32
<210> 35
   <211> 29
   <212> DNA
   <213> artificial
<220>
   <223> artificial
<400> 35
   ccatggcgaa gggcgagttt gttcggacg 29
<210> 36
   <211> 32
   <212> DNA
   <213> artificial
<220>
   <223> artificial
<400> 36
   gagctccagg atcttggtga cgacgccggc gc 32
<210> 37
   <211> 31
   <212> DNA
   <213> artificial
<220>
   <223> artificial
<400> 37
   catatgagcc aaatggaact catcaagaag c 31
<210> 38
   <211> 26
   <212> DNA
   <213> artificial
<220>
   <223> artificial
<400> 38
   ggtacccgcc cccagctcaa agcggc 26
<210> 39
   <211> 27
   <212> DNA
   <213> artificial
<220>
   <223> artificial
<400> 39
   ccatggatga acaggctcta ttagggc 27
<210> 40
   <211> 30
   <212> DNA
   <213> artificial
<220>
   <223> artificial
<400> 40
gagctcgggc tttgctctct ggaagaacac 30
   <210> 41
   <211> 58
   <212> DNA
   <213> artificial
<220>
   <223> artificial
<400> 41
   ccatgggcag cagccatcat catcatcatc acagcagcgg cctggtgccg cgcggcag 58
<210> 42
   <211> 27
   <212> DNA
   <213> artificial
<220>
   <223> artificial
<400> 42
   ggtaccgagc tctagagtgg aacctcc 27
<210> 43
   <211> 1098
   <212> DNA
   <213> Escherichia coli
<400> 43
   atgtttgaaa ttaatccggt aaataatcgc attcaggacc tcacggaacg ctccgacgtt 60
   cttagggggt atcttgacta cgacgccaag aaagagcgtc tggaagaagt aaacgccgag 120
   ctggaacagc cggatgtctg gaacgaaccc gaacgcgcac aggcgctggg taaagagcgt 180
   tcctccctcg aagccgttgt cgacaccctc gaccaaatga aacaggggct ggaagatgtt 240
   tctggtctgc tggaactggc tgtagaagct gacgacgaag aaacctttaa cgaagccgtt 300
   gctgaactcg acgccctgga agaaaaactg gcgcagcttg agttccgccg tatgttctct 360
   ggcgaatatg acagcgccga ctgctacctc gatattcagg cggggtctgg cggtacggaa 420
   gcacaggact gggcgagcat gcttgagcgt atgtatctgc gctgggcaga atcgcgtggt 480
   ttcaaaactg aaatcatcga agagtcggaa ggtgaagtgg cgggtattaa atccgtgacg 540
   atcaaaatct ccggcgatta cgcttacggc tggctgcgta cagaaaccgg cgttcaccgc 600
   gtggtgcgta aaagcccgtt tgactccggc ggtcgtcgcc acacgtcgtt cagctccgcg 660
   tttgtttatc cggaagttga tgatgatatt gatatcgaaa tcaacccggc ggatctgcgc 720
   attgacgttt atcgcacgtc cggcgcgggc ggtcagcacg ttaaccgtac cgaatctgcg 780
   gtgcgtatta cccacatccc gaccgggatc gtgacccagt gccagaacga ccgttcccag 840
   cacaagaaca aagatcaggc catgaagcag atgaaagcga agctttatga actggagatg 900
   cagaagaaaa atgccgagaa acaggcgatg gaagataaca aatccgacat cggctggggc 960
   agccagattc gttcttatgt ccttgatgac tcccgcatta aagatctgcg caccggggta 1020
   gaaacccgca acacgcaggc cgtgctggac ggcagcctgg atcaatttat cgaagcaagt 1080
   ttgaaagcag ggttatga 1098
<210> 44
   <211> 365
   <212> PRT
   <213> Escherichia coli
<400> 44
   Met Phe Glu Ile Asn Pro Val Asn Asn Arg Ile Gln Asp Leu Thr Glu
   1 5 10 15
   Arg Ser Asp Val Leu Arg Gly Tyr Leu Asp Tyr Asp Ala Lys Lys Glu
   20 25 30
   Arg Leu Glu Glu Val Asn Ala Glu Leu Glu Gln Pro Asp Val Trp Asn
   35 40 45
   Glu Pro Glu Arg Ala Gln Ala Leu Gly Lys Glu Arg Ser Ser Leu Glu
   50 55 60
   Ala Val Val Asp Thr Leu Asp Gln Met Lys Gln Gly Leu Glu Asp Val
   65 70 75 80
   Ser Gly Leu Leu Glu Leu Ala Val Glu Ala Asp Asp Glu Glu Thr Phe
   85 90 95
   Asn Glu Ala Val Ala Glu Leu Asp Ala Leu Glu Glu Lys Leu Ala Gln
   100 105 110
   Leu Glu Phe Arg Arg Met Phe Ser Gly Glu Tyr Asp Ser Ala Asp Cys
   115 120 125
   Tyr Leu Asp Ile Gln Ala Gly Ser Gly Gly Thr Glu Ala Gln Asp Trp
   130 135 140
   Ala Ser Met Leu Glu Arg Met Tyr Leu Arg Trp Ala Glu Ser Arg Gly
   145 150 155 160
   Phe Lys Thr Glu Ile Ile Glu Glu Ser Glu Gly Glu Val Ala Gly Ile
   165 170 175
   Lys Ser Val Thr Ile Lys Ile Ser Gly Asp Tyr Ala Tyr Gly Trp Leu
   180 185 190
   Arg Thr Glu Thr Gly Val His Arg Leu Val Arg Lys Ser Pro Phe Asp
   195 200 205
   Ser Gly Gly Arg Arg His Thr Ser Phe Ser Ser Ala Phe Val Tyr Pro
   210 215 220
   Glu Val Asp Asp Asp Ile Asp Ile Glu Ile Asn Pro Ala Asp Leu Arg
   225 230 235 240
   Ile Asp Val Tyr Arg Thr Ser Gly Ala Gly Gly Gln His Val Asn Arg
   245 250 255
   Thr Glu Ser Ala Val Arg Ile Thr His Ile Pro Thr Gly Ile Val Thr
   260 265 270
   Gln Cys Gln Asn Asp Arg Ser Gln His Lys Asn Lys Asp Gln Ala Met
   275 280 285
   Lys Gln Met Lys Ala Lys Leu Tyr Glu Leu Glu Met Gln Lys Lys Asn
   290 295 300
   Ala Glu Lys Gln Ala Met Glu Asp Asn Lys Ser Asp Ile Gly Trp Gly
   305 310 315 320
   Ser Gln Ile Arg Ser Tyr Val Leu Asp Asp Ser Arg Ile Lys Asp Leu
   325 330 335
   Arg Thr Gly Val Glu Thr Arg Asn Thr Gln Ala Val Leu Asp Gly Ser
   340 345 350
   Leu Asp Gln Phe Ile Glu Ala Ser Leu Lys Ala Gly Leu
   355 360 365
<210> 45
   <211> 426
   <212> DNA
   <213> Escherichia coli
<400> 45
   atgggcatta agcgaaagtg gtgtggcact attcagtgga tttgtccgag tccgtatcgg 60
   cctcatcatg ggcgcaaaaa ctggtttctg ggcattgggc gttttaccgc tgatgagcag 120
   gaacaatcgg atgcaatccg ttatgagacg ctgcgttcgt cggggccggg cggtcaacat 180
   gtcaataaaa ccgactcggc ggtacgcgcc acgcatttgg catccggtat tagcgtgaag 240
   gttcagtcag agcgtagtca gcatgctaac aagcggctgg cacgattgct gattgcctgg 300
   aagctggagc aacagcaaca ggaaaatagc gcggcgctga aatcgcagcg gcgaatgttc 360
   catcaccaga ttgaacgtgg caacccgcga cggacattta cagggatggc ttttatcgaa 420
   ggataa 426
<210> 46
   <211> 141
   <212> PRT
   <213> Escherichia coli
<400> 46
   Met Gly Ile Lys Arg Lys Trp Cys Gly Thr Ile Gln Trp Ile Cys Pro
   1               5                   10                  15
   Ser Pro Tyr Arg Pro His His Gly Arg Lys Asn Trp Phe Leu Gly Ile
               20                  25                  30
   Gly Arg Phe Thr Ala Asp Glu Gln Glu Gln Ser Asp Ala Ile Arg Tyr
           35                  40                  45
   Glu Thr Leu Arg Ser Ser Gly Pro Gly Gly Gln His Val Asn Lys Thr
       50                  55                  60
   Asp Ser Ala Val Arg Ala Thr His Leu Ala Ser Gly Ile Ser Val Lys
   65                  70                  75                  80
   Val Gln Ser Glu Arg Ser Gln His Ala Asn Lys Arg Leu Ala Arg Leu
                   85                  90                  95
   Leu Ile Ala Trp Lys Leu Glu Gln Gln Gln Gln Glu Asn Ser Ala Ala
               100                 105                 110
   Leu Lys Ser Gln Arg Arg Met Phe His His Gln Ile Glu Arg Gly Asn
           115                 120                 125
   Pro Arg Arg Thr Phe Thr Gly Met Ala Phe Ile Glu Gly
       130                 135                 140
<210> 47
   <211> 615
   <212> DNA
   <213> Escherichia coli
<400> 47
   atgatcttgc tacaactctc ctctgctcag gggccggaag aatgttgtct cgcagtgaga 60
   aaagcactgg acaggctgat taaagaagct acccgacagg acgtcgcggt aacggtgctg 120
   gaaacagaaa cgggtcgcta ctctgacaca ctgcgttcgg cgctgatttc tctggatggc 180
   gataacgcat gggctctaag cgaaagctgg tgcggcacta ttcagtggat ttgtccgagt 240
   ccgtatcggc ctcatcatgg gcgcaaaaac tggtttctgg gcattgggcg ttttaccgct 300
   gatgagcagg aacaatcgga tgcaatccgt tatgagacgc tgcgttcgtc ggggccgggc 360
   ggtcaacatg tcaataaaac cgactcggcg gtacgcgcca cgcatctggc atccggtatt 420
   agcgtgaagg ttcagtcaga gcgcagtcag catgctaaca aacggctggc gcgattactg 480
   attgcctgga agctggaaca acagcaacag gaaaatagcg cggcgctgaa atcgcagcgg 540
   cgaatgttcc atcaccagat tgaacgtggc aacccgcgac gaacgtttac agggatggcc 600
   tttatcgaag ggtaa 615
<210> 48
   <211> 204
   <212> PRT
   <213> Escherichia coli
<400> 48
   Met Ile Leu Leu Gln Leu Ser Ser Ala Gln Gly Pro Glu Glu Cys Cys
   1               5                   10                  15
   Leu Ala Val Arg Lys Ala Leu Asp Arg Leu Ile Lys Glu Ala Thr Arg
               20                  25                  30
   Gln Asp Val Ala Val Thr Val Leu Glu Thr Glu Thr Gly Arg Tyr Ser
           35                  40                  45
   Asp Thr Leu Arg Ser Ala Leu Ile Ser Leu Asp Gly Asp Asn Ala Trp
       50                  55                  60
   Ala Leu Ser Glu Ser Trp Cys Gly Thr Ile Gln Trp Ile Cys Pro Ser
   65                  70                  75                  80
   Pro Tyr Arg Pro His His Gly Arg Lys Asn Trp Phe Leu Gly Ile Gly
                   85                  90                  95
   Arg Phe Thr Ala Asp Glu Gln Glu Gln Ser Asp Ala Ile Arg Tyr Glu
               100                 105                 110
   Thr Leu Arg Ser Ser Gly Pro Gly Gly Gln His Val Asn Lys Thr Asp
           115                 120                 125
   Ser Ala Val Arg Ala Thr His Leu Ala Ser Gly Ile Ser Val Lys Val
       130                 135                 140
   Gln Ser Glu Arg Ser Gln His Ala Asn Lys Arg Leu Ala Arg Leu Leu
   145                 150                 155                 160
   Ile Ala Trp Lys Leu Glu Gln Gln Gln Gln Glu Asn Ser Ala Ala Leu
                   165                 170                 175
   Lys Ser Gln Arg Arg Met Phe His His Gln Ile Glu Arg Gly Asn Pro
               180                 185                 190
   Arg Arg Thr Phe Thr Gly Met Ala Phe Ile Glu Gly
           195                 200
<210> 49
   <211> 501
   <212> DNA
   <213> Escherichia coli
<400> 49
   gtgctggaaa cagaaacggg ccgctactct gacacgctgc gttcggcgct gatttctctg 60
   gatggcgaca acgcatgggc gttaagcgaa agttggtgcg gcactattca gtggatttgt 120
   ctgagtccgt atcggcctca tcatgggcgc aaaaactggt ttctgggcat tgggcgtttt 180
   accgctgatg agcaggaaca atcggatgca atccgttatg agacgctgcg tccgtcgggg 240
   ccgggcggtc aacatgtcaa taaaaccgac tcggcggtac gcgccacgca tctggcaacc 300
   gggattagcg tgaaggttca gtcagaacgc agccagcatg ctaacaaacg gctggcgcga 360
   ttgctgattg cctggaagct ggagcagcag caacaggaaa atagcgcagt gctgaaatcg 420
   cagcggcgaa tgttccatca ccagattgaa cgtggcaacc cgcgacgaac gttcacaggg 480
   atggctttta tcgaaggata a 501
<210> 50
   <211> 166
   <212> PRT
   <213> Escherichia coli
<400> 50
   Met Leu Glu Thr Glu Thr Gly Arg Tyr Ser Asp Thr Leu Arg Ser Ala
   1 5 10 15
   Leu Ile Ser Leu Asp Gly Asp Asn Ala Trp Ala Leu Ser Glu Ser Trp
   20 25 30
   Cys Gly Thr Ile Gln Trp Ile Cys Leu Ser Pro Tyr Arg Pro His His
   35 40 45
   Gly Arg Lys Asn Trp Phe Leu Gly Ile Gly Arg Phe Thr Ala Asp Glu
   50 55 60
   Gln Glu Gln Ser Asp Ala Ile Arg Tyr Glu Thr Leu Arg Pro Ser Gly
   65 70 75 80
   Pro Gly Gly Gln His Val Asn Lys Thr Asp Ser Ala Val Arg Ala Thr
                   85                  90                  95
   His Leu Ala Thr Gly Ile Ser Val Lys Val Gln Ser Glu Arg Ser Gln
               100                 105                 110
   His Ala Asn Lys Arg Leu Ala Arg Leu Leu Ile Ala Trp Lys Leu Glu
           115                 120                 125
   Gln Gln Gln Gln Glu Asn Ser Ala Val Leu Lys Ser Gln Arg Arg Met
       130                 135                 140
   Phe His His Gln Ile Glu Arg Gly Asn Pro Arg Arg Thr Phe Thr Gly
   145                 150                 155                 160
   Met Ala Phe Ile Glu Gly
                   165
<210> 51
   <211> 166
   <212> PRT
   <213> Escherichia coli
<400> 51
   Met Leu Glu Thr Glu Thr Gly Arg Tyr Ser Asp Thr Leu Arg Ser Ala
   1               5                   10                  15
   Leu Val Ser Leu Asp Gly Asp Asn Ala Trp Ala Leu Ser Glu Ser Trp
               20                  25                  30
   Cys Gly Thr Ile Gln Trp Ile Cys Pro Ser Pro Tyr Arg Pro His His
           35                  40                  45
   Gly Arg Lys Asn Trp Phe Leu Gly Ile Gly Arg Phe Thr Ala Asp Glu
       50                  55                  60
   Gln Glu Gln Ser Asp Ala Ile Arg Tyr Glu Thr Leu Arg Ser Ser Gly
   65                  70                  75                  80
   Pro Gly Gly Gln His Val Asn Lys Thr Asp Ser Ala Val Arg Ala Thr
                   85                  90                  95
   His Leu Ala Ser Gly Ile Ser Val Lys Val Gln Ser Glu Arg Ser Gln
               100                 105                 110
   His Ala Asn Lys Arg Leu Ala Arg Leu Leu Ile Ala Trp Lys Leu Glu
           115                 120                 125
   Gln Gln Gln Gln Glu Asn Ser Ala Ala Leu Lys Ser Gln Arg Arg Met
       130                 135                 140
   Phe His His Gln Ile Glu Arg Gly Asn Pro Arg Arg Thr Phe Thr Gly
   145                 150                 155                 160
   Met Ala Phe Ile Glu Gly
                   165
<210> 52
   <211> 1314
   <212> DNA
   <213> Oryctolagus cuniculus
<400> 52
   atggcggacg accccagtgc tgccgacagg aacgtggaaa tctggaagat caagaagctc      60
   attaagagct tggaggcggc ccgcggcaat ggcaccagca tgatatcatt gatcattcct     120
   cccaaagacc agatttcccg agtggcaaaa atgttagcag atgaatttgg aactgcatcc     180
   aacattaagt cacgagtaaa ccgcctttca gtcctgggag ccattacatc tgtacaacaa     240
   agactcaaac tttataacaa agtacctcca aatggtctgg ttgtttactg tggaacaatt     300
   gtaacagaag aaggaaagga aaagaaagtc aacattgact ttgaaccttt caaaccaatt     360
   aatacgtcat tgtatttgtg tgacaacaaa ttccatacag aggctcttac agcactactt     420
   tcagatgata gcaagtttgg cttcattgta atagatggta gtggtgcact ttttggcaca      480
   ctgcagggaa atacaagaga agtcctgcac aaattcactg tggatctccc aaagaaacac     540
   ggtagaggag gtcagtcagc cttgcgtttt gcccgtttaa gaatggaaaa gcgacacaac     600
   tatgttcgga aagtagcaga gactgctgta cagctgttta tttctgggga caaagtgaat     660
   gtggctggtc tcgttttagc tggatcagct gactttaaaa ctgaactaag tcaatctgat     720
   atgtttgacc agaggttgca atcaaaagtt ttaaaattag ttgatatatc ctatggcggt     780
   gaaaatggat tcaaccaagc tattgagtta tctactgagg tcctctccaa cgtgaaattc     840
   attcaagaga agaaattaat aggacgatac tttgatgaaa tcagtcaaga cacgggcaag     900
   tactgttttg gagttgaaga tacgctaaaa gctttggaaa tgggagccgt agaaattcta     960
   atagtctatg aaaatttgga tataatgaga tacgttcttc attgccaagg cacagaagag    1020
   gagaaaattc tttacctaac tccagaacaa gagaaggata aatctcattt cacagacaaa    1080
   gagacaggac aggaacatga gctgattgag agcatgcccc tgttggaatg gtttgctaac    1140
   aactataaaa aatttggagc tacattggaa attgtcacag ataagtcaca agaaggatcc    1200
   cagtttgtga aaggatttgg tggaattgga ggtatcttgc ggtaccgagt agatttccag    1260
   ggaatggaat atcaaggagg agacgatgaa ttttttgacc ttgatgacta ctag          1314
<210> 53
   <211> 437
   <212> PRT
   <213> Oryctolagus cuniculus
<400> 53
   Met Ala Asp Asp Pro Ser Ala Ala Asp Arg Asn Val Glu Ile Trp Lys
   1               5                   10                  15
   Ile Lys Lys Leu Ile Lys Ser Leu Glu Ala Ala Arg Gly Asn Gly Thr
               20                  25                  30
   Ser Met Ile Ser Leu Ile Ile Pro Pro Lys Asp Gln Ile Ser Arg Val
           35                  40                  45
   Ala Lys Met Leu Ala Asp Glu Phe Gly Thr Ala Ser Asn Ile Lys Ser
       50                  55                  60
   Arg Val Asn Arg Leu Ser Val Leu Gly Ala Ile Thr Ser Val Gln Gln
   65                  70                  75                  80
   Arg Leu Lys Leu Tyr Asn Lys Val Pro Pro Asn Gly Leu Val Val Tyr
                   85                  90                  95
   Cys Gly Thr Ile Val Thr Glu Glu Gly Lys Glu Lys Lys Val Asn Ile
               100                 105                 110
   Asp Phe Glu Pro Phe Lys Pro Ile Asn Thr Ser Leu Tyr Leu Cys Asp
           115                 120                 125
   Asn Lys Phe His Thr Glu Ala Leu Thr Ala Leu Leu Ser Asp Asp Ser
       130                 135                 140
   Lys Phe Gly Phe Ile Val Ile Asp Gly Ser Gly Ala Leu Phe Gly Thr
   145                 150                 155                 160
   Leu Gln Gly Asn Thr Arg Glu Val Leu His Lys Phe Thr Val Asp Leu
                   165                 170                 175
   Pro Lys Lys His Gly Arg Gly Gly Gln Ser Ala Leu Arg Phe Ala Arg
               180                 185                 190
   Leu Arg Met Glu Lys Arg His Asn Tyr Val Arg Lys Val Ala Glu Thr
           195                 200                 205
   Ala Val Gln Leu Phe Ile Ser Gly Asp Lys Val Asn Val Ala Gly Leu
       210                 215                 220
   Val Leu Ala Gly Ser Ala Asp Phe Lys Thr Glu Leu Ser Gln Ser Asp
   225                 230                 235                 240
   Met Phe Asp Gln Arg Leu Gln Ser Lys Val Leu Lys Leu Val Asp Ile
                   245                 250                 255
   Ser Tyr Gly Gly Glu Asn Gly Phe Asn Gln Ala Ile Glu Leu Ser Thr
               260                 265                 270
   Glu Val Leu Ser Asn Val Lys Phe Ile Gln Glu Lys Lys Leu Ile Gly
           275                 280                 285
   Arg Tyr Phe Asp Glu Ile Ser Gln Asp Thr Gly Lys Tyr Cys Phe Gly
       290                 295                 300
   Val Glu Asp Thr Leu Lys Ala Leu Glu Met Gly Ala Val Glu Ile Leu
   305                 310                 315                 320
   Ile Val Tyr Glu Asn Leu Asp Ile Met Arg Tyr Val Leu His Cys Gln
                   325                 330                 335
   Gly Thr Glu Glu Glu Lys Ile Leu Tyr Leu Thr Pro Glu Gln Glu Lys
               340                 345                 350
   Asp Lys Ser His Phe Thr Asp Lys Glu Thr Gly Gln Glu His Glu Leu
           355                 360                 365
   Ile Glu Ser Met Pro Leu Leu Glu Trp Phe Ala Asn Asn Tyr Lys Lys
       370                 375                 380
   Phe Gly Ala Thr Leu Glu Ile Val Thr Asp Lys Ser Gln Glu Gly Ser
   385                 390                 395                 400
   Gln Phe Val Lys Gly Phe Gly Gly Ile Gly Gly Ile Leu Arg Tyr Arg
                   405                 410                 415
   Val Asp Phe Gln Gly Met Glu Tyr Gln Gly Gly Asp Asp Glu Phe Phe
               420                 425                 430
   Asp Leu Asp Asp Tyr
   435
<210> 54
   <211> 1767
   <212> DNA
   <213> Oryctolagus cuniculus
<400> 54
   ctggcggcgg cggccgaggc ccagcgtgac cacctcagcg cggccttcag ccggcagctc 60
   aacgtcaacg ccaaaccttt cgtgcccaac gtccacgccg cggagttcgt accgtctttc 120
   ctgcggggcc cggccccgcc tccagccccg gctggcgccg ccggcaacaa ccacggagcg 180
   ggcagcgtcg cgggaggccc ttcggcacct gtggaatcct ctcaagagga acagtcattg 240
   tgtgaaggct ccatttcagc tgttagcatg gaactttcag aacctgttgt agagaacgga 300
   gagacagaaa tgtccccaga agaatcatgg gagcacaaag aagaaataag tgaggcagag 360
   ccagggggtg gctccctggg agatggaagg ccaccggagg aaggtgccca agaaatgatg 420
   gaggaggaag aggaaatgcc aaagcccaaa tctgtagctg cgcctcctgg tgcccctaaa 480
   aaagaacatg taaatgtagt gtttattggg catgtagatg ctggcaagtc aaccattgga 540
   ggccaaataa tgtatttgac tggaatggtt gataaaagga cacttgagaa atatgaaaga 600
   gaagctaaag aaaaaaacag agaaacttgg tacttgtctt gggccctaga tacaaatcag 660
   gaagaacgag acaaaggtaa aacagtcgaa gtgggtcgtg cctactttga aacagaaaag 720
   aagcatttca caattctaga cgcccctggc cacaagagtt ttgtcccaaa tatgattggt 780
   ggcgcctctc aagctgattt ggctgtgctg gtcatctctg ccaggaaagg agagtttgaa 840
   actggatttg aaaaaggagg acagacaaga gaacacgcaa tgttggcaaa gacagcaggt 900
   gtaaagcact taattgtgct tattaataag atggatgacc caacagtgaa ttggagcaac 960
   gagagatatg aagaatgtaa agagaaacta gtgccatttt tgaaaaaagt tggcttcaat 1020
   cccaaaaagg acattcactt tatgccctgc tcaggactga ctggagcaaa tctcaaagag 1080
   caatcagatt tctgtccttg gtacattgga ttaccattta ttccatatct ggataatttg 1140
   ccaaacttca atagatcagt tgatggacca atcagactgc cgattgtgga taagtacaag 1200
   gatatgggca ctgtggtcct gggaaagctg gaatcgggat ctatttgtaa aggccagcag 1260
   ctagtgatga tgccgaacaa gcacaacgtg gaagttcttg gaatactttc tgatgatgta 1320
   gaaactgatt ctgtagcccc aggtgagaac ctgaaaatca gactcaaagg aattgaggaa 1380
   gaagagattc ttccaggatt catcctttgt gatcttaata atctttgcca ttctggacgc 1440
   acatttgatg cccagatagt gattatagag cacaaatcca tcatctgccc agggtacaat 1500
   gcggtgctgc atattcatac ctgtattgag gaagtcgaga taacagcctt aatctgcttg 1560
   gtagacaaaa agtcaggaga gaaaagcaag actcggcccc gttttgtgaa acaagatcaa 1620
   gtgtgcattg cccgtcttcg gacagcagga accatctgcc ttgagacctt taaggacttc 1680
   cctcagatgg gtcgttttac cttaagagat gagggtaaga ccattgcaat tggaaaagtt 1740
   ctgaaactgg ttccagaaaa agactaa 1767
<210> 55
   <211> 588
   <212> PRT
   <213> Oryctolagus cuniculus
<400> 55
   Leu Ala Ala Ala Ala Glu Ala Gln Arg Asp His Leu Ser Ala Ala Phe
   1               5                   10                  15
   Ser Arg Gln Leu Asn Val Asn Ala Lys Pro Phe Val Pro Asn Val His
               20                  25                  30
   Ala Ala Glu Phe Val Pro Ser Phe Leu Arg Gly Pro Ala Pro Pro Pro
           35                  40                  45
   Ala Pro Ala Gly Ala Ala Gly Asn Asn His Gly Ala Gly Ser Val Ala
       50                  55                  60
   Gly Gly Pro Ser Ala Pro Val Glu Ser Ser Gln Glu Glu Gln Ser Leu
   65                  70                  75                  80
   Cys Glu Gly Ser Ile Ser Ala Val Ser Met Glu Leu Ser Glu Pro Val
                   85                  90                  95
   Val Glu Asn Gly Glu Thr Glu Met Ser Pro Glu Glu Ser Trp Glu His
               100                 105                 110
   Lys Glu Glu Ile Ser Glu Ala Glu Pro Gly Gly Gly Ser Leu Gly Asp
           115                 120                 125
   Gly Arg Pro Pro Glu Glu Gly Ala Gln Glu Met Met Glu Glu Glu Glu
       130                 135                 140
   Glu Met Pro Lys Pro Lys Ser Val Ala Ala Pro Pro Gly Ala Pro Lys
   145                 150                 155                 160
   Lys Glu His Val Asn Val Val Phe Ile Gly His Val Asp Ala Gly Lys
   165 170 175
   Ser Thr Ile Gly Gly Gln Ile Met Tyr Leu Thr Gly Met Val Asp Lys
   180 185 190
   Arg Thr Leu Glu Lys Tyr Glu Arg Glu Ala Lys Glu Lys Asn Arg Glu
   195 200 205
   Thr Trp Tyr Leu Ser Trp Ala Leu Asp Thr Asn Gln Glu Glu Arg Asp
   210 215 220
   Lys Gly Lys Thr Val Glu Val Gly Arg Ala Tyr Phe Glu Thr Glu Lys
   225 230 235 240
   Lys His Phe Thr Ile Leu Asp Ala Pro Gly His Lys Ser Phe Val Pro
   245 250 255
   Asn Met Ile Gly Gly Ala Ser Gln Ala Asp Leu Ala Val Leu Val Ile
   260 265 270
   Ser Ala Arg Lys Gly Glu Phe Glu Thr Gly Phe Glu Lys Gly Gly Gln
   275 280 285
   Thr Arg Glu His Ala Met Leu Ala Lys Thr Ala Gly Val Lys His Leu
   290 295 300
   Ile Val Leu Ile Asn Lys Met Asp Asp Pro Thr Val Asn Trp Ser Asn
   305 310 315 320
   Glu Arg Tyr Glu Glu Cys Lys Glu Lys Leu Val Pro Phe Leu Lys Lys
   325 330 335
   Val Gly Phe Asn Pro Lys Lys Asp Ile His Phe Met Pro Cys Ser Gly
   340 345 350
   Leu Thr Gly Ala Asn Leu Lys Glu Gln Ser Asp Phe Cys Pro Trp Tyr
   355 360 365
   Ile Gly Leu Pro Phe Ile Pro Tyr Leu Asp Asn Leu Pro Asn Phe Asn
   370 375 380
   Arg Ser Val Asp Gly Pro Ile Arg Leu Pro Ile Val Asp Lys Tyr Lys
   385 390 395 400
   Asp Met Gly Thr Val Val Leu Gly Lys Leu Glu Ser Gly Ser Ile Cys
                   405                 410                 415
   Lys Gly Gln Gln Leu Val Met Met Pro Asn Lys His Asn Val Glu Val
               420                 425                 430
   Leu Gly Ile Leu Ser Asp Asp Val Glu Thr Asp Ser Val Ala Pro Gly
           435                 440                 445
   Glu Asn Leu Lys Ile Arg Leu Lys Gly Ile Glu Glu Glu Glu Ile Leu
       450                 455                 460
   Pro Gly Phe Ile Leu Cys Asp Leu Asn Asn Leu Cys His Ser Gly Arg
   465                 470                 475                 480
   Thr Phe Asp Ala Gln Ile Val Ile Ile Glu His Lys Ser Ile Ile Cys
                   485                 490                 495
   Pro Gly Tyr Asn Ala Val Leu His Ile His Thr Cys Ile Glu Glu Val
               500                 505                 510
   Glu Ile Thr Ala Leu Ile Cys Leu Val Asp Lys Lys Ser Gly Glu Lys
           515                 520                 525
   Ser Lys Thr Arg Pro Arg Phe Val Lys Gln Asp Gln Val Cys Ile Ala
       530                 535                 540
   Arg Leu Arg Thr Ala Gly Thr Ile Cys Leu Glu Thr Phe Lys Asp Phe
   545                 550                 555                 560
   Pro Gln Met Gly Arg Phe Thr Leu Arg Asp Glu Gly Lys Thr Ile Ala
                   565                 570                 575
   Ile Gly Lys Val Leu Lys Leu Val Pro Glu Lys Asp
               580                 585
<210> 56
   <211> 1317
   <212> DNA
   <213> Drosophila melanogaster
<400> 56
   atgtctggcg aggaaacgtc tgccgatcgc aatgtcgaga tctggaaaat caagaagctc      60
   atcaagagcc tggaaatggc ccgcggcaat ggaaccagca tgatttcttt gattattccg     120
   ccaaaggatc aaatctcgcg cgtcagcaag atgttggccg atgagtttgg aacggcgtcg     180
   aacatcaagt cgcgtgtaaa tcggttgtcc gtcctcggtg ccattacgtc ggtacagcac     240
   agactcaaat tatacaccaa agtgcctccc aacggtttgg tcatctactg cggcacaata     300
   gtcacagagg agggcaagga gaagaaggtg aacatagact ttgagccatt caagcccata     360
   aacacgtcgc tctacctctg cgacaacaag ttccacacgg aggccctcac tgccctgctc     420
   gccgacgaca acaaatttgg attcatcgtg atggatggta acggagcgct attcggtacc     480
   cttcagggca acacgcgcga ggtgctccac aagttcaccg tcgatctgcc gaagaagcac     540
   ggtcgtggtg gtcagtccgc ccttcgtttc gcccgtctgc gtatggagaa gcgccacaac     600
   tacgtgcgga aggtcgccga ggtggccacc cagctcttca tcacgaacga caagcccaac     660
   attgccggac tcatcctggc tggtagtgcg gatttcaaga ctgagcttag tcagtctgat     720
   atgttcgatc ctcgtttgca atcaaaagtc atcaagctgg tggacgtgtc gtatggtggg     780
   gaaaacggtt ttaaccaggc cattgaactg gcggccgaat cattgcagaa cgttaaattc     840
   atacaggaga agaaactcat tggtcgctac tttgatgaaa tttctcagga tactggcaaa     900
   tactgttttg gagtggagga cactttgcgg gcactggaac ttggctctgt agagactctc     960
   atttgttggg agaacctgga tattcaacgt tatgttctca agaatcatgc caactcgacg    1020
   tcaacgacag tattacattt gacgcccgag caggaaaagg acaagtcgca cttcactgac    1080
   aaggagagcg gggtagaaat ggagctgatt gagtctcagc cgctgctgga atggctggca    1140
   aacaactaca aaatgttcgg cgccacactg gagattatca cggataagtc ccaggaagga    1200
   agtcagttcg tgcgaggttt cggtggaatc ggcggtatct tacgctacaa ggtggatttc    1260
   cagagtatgc agctcgatga attggacaat gatggcttcg atctagatga ttactag       1317
<210> 57
   <211> 438
   <212> PRT
   <213> Drosophila melanogaster
<400> 57
   Met Ser Gly Glu Glu Thr Ser Ala Asp Arg Asn Val Glu Ile Trp Lys
   1               5                   10                  15
   Ile Lys Lys Leu Ile Lys Ser Leu Glu Met Ala Arg Gly Asn Gly Thr
               20                  25                  30
   Ser Met Ile Ser Leu Ile Ile Pro Pro Lys Asp Gln Ile Ser Arg Val
           35                  40                  45
   Ser Lys Met Leu Ala Asp Glu Phe Gly Thr Ala Ser Asn Ile Lys Ser
       50                  55                  60
   Arg Val Asn Arg Leu Ser Val Leu Gly Ala Ile Thr Ser Val Gln His
   65 70 75 80
   Arg Leu Lys Leu Tyr Thr Lys Val Pro Pro Asn Gly Leu Val Ile Tyr
   85 90 95
   Cys Gly Thr Ile Val Thr Glu Glu Gly Lys Glu Lys Lys Val Asn Ile
   100 105 110
   Asp Phe Glu Pro Phe Lys Pro Ile Asn Thr Ser Leu Tyr Leu Cys Asp
   115 120 125
   Asn Lys Phe His Thr Glu Ala Leu Thr Ala Leu Leu Ala Asp Asp Asn
   130 135 140
   Lys Phe Gly Phe Ile Val Met Asp Gly Asn Gly Ala Leu Phe Gly Thr
   145 150 155 160
   Leu Gln Gly Asn Thr Arg Glu Val Leu His Lys Phe Thr Val Asp Leu
   165 170 175
   Pro Lys Lys His Gly Arg Gly Gly Gln Ser Ala Leu Arg Phe Ala Arg
   180 185 190
   Leu Arg Met Glu Lys Arg His Asn Tyr Val Arg Lys Val Ala Glu Val
   195 200 205
   Ala Thr Gln Leu Phe Ile Thr Asn Asp Lys Pro Asn Ile Ala Gly Leu
   210 215 220
   Ile Leu Ala Gly Ser Ala Asp Phe Lys Thr Glu Leu Ser Gln Ser Asp
   225 230 235 240
   Met Phe Asp Pro Arg Leu Gln Ser Lys Val Ile Lys Leu Val Asp Val
   245 250 255
   Ser Tyr Gly Gly Glu Asn Gly Phe Asn Gln Ala Ile Glu Leu Ala Ala
   260 265 270
   Glu Ser Leu Gln Asn Val Lys Phe Ile Gln Glu Lys Lys Leu Ile Gly
   275 280 285
   Arg Tyr Phe Asp Glu Ile Ser Gln Asp Thr Gly Lys Tyr Cys Phe Gly
   290 295 300
   Val Glu Asp Thr Leu Arg Ala Leu Glu Leu Gly Ser Val Glu Thr Leu
   305 310 315 320
   Ile Cys Trp Glu Asn Leu Asp Ile Gln Arg Tyr Val Leu Lys Asn His
   325 330 335
   Ala Asn Ser Thr Ser Thr Thr Val Leu His Leu Thr Pro Glu Gln Glu
   340 345 350
   Lys Asp Lys Ser His Phe Thr Asp Lys Glu Ser Gly Val Glu Met Glu
   355 360 365
   Leu Ile Glu Ser Gln Pro Leu Leu Glu Trp Leu Ala Asn Asn Tyr Lys
   370 375 380
   Met Phe Gly Ala Thr Leu Glu Ile Ile Thr Asp Lys Ser Gln Glu Gly
   385 390 395 400
   Ser Gln Phe Val Arg Gly Phe Gly Gly Ile Gly Gly Ile Leu Arg Tyr
   405 410 415
   Lys Val Asp Phe Gln Ser Met Gln Leu Asp Glu Leu Asp Asn Asp Gly
   420 425 430
   Phe Asp Leu Asp Asp Tyr
   435
<210> 58
   <211> 1314
   <212> DNA
   <213> Saccharomyces cerevisiae
<400> 58
   atggataacg aggttgaaaa aaatattgag atctggaagg tcaagaagtt ggtccaatct 60
   ttagaaaaag ctagaggtaa tggtacttct atgatttcct tagttattcc tcctaagggt 120
   ctaattccac tgtaccaaaa aatgttaaca gatgaatatg gtactgcctc gaatattaaa 180
   tctagggtta atcgtctttc cgttttatct gctatcactt ccacccaaca aaagttgaag 240
   ctatataata ctttgcccaa gaacggttta gttttatatt gtggtgatat catcactgaa 300
   gatggtaaag aaaaaaaggt cacttttgac atcgaacctt acaaacctat caacacatcc 360
   ttatatttgt gtgataacaa atttcataca gaagttcttt cggaattgct tcaagctgac 420
   gacaagttcg gttttatagt catggacggt caaggtactt tgtttggttc tgtgtccggt 480
   aatacgagaa ctgttttaca taaatttact gtcgatctgc caaaaaagca tggtagaggt    540
   ggtcaatctg cgcttcgttt tgctcgttta agagaagaaa aaagacataa ttatgtgaga    600
   aaggtcgccg aagttgctgt tcaaaatttt attactaatg acaaagtcaa tgttaagggt    660
   ttaattttag ctggttctgc tgactttaag accgatttgg ctaaatctga attattcgat    720
   ccaagactag catgtaaggt tatttccatc gtggatgttt cttatggtgg tgaaaacggt    780
   ttcaaccagg ctatcgaact ttctgccgaa gcgttggcca atgtcaagta tgttcaagaa    840
   aagaaattat tggaggcata ttttgacgaa atttcccagg acactggtaa attctgttat    900
   ggtatagatg atactttaaa ggcattggat ttaggtgcag tcgaaaaatt aattgttttc    960
   gaaaatttgg aaactatcag atatacattt aaagatgccg aggataatga ggttataaaa   1020
   ttcgctgaac cagaagccaa ggacaagtcg tttgctattg acaaagctac cggccaagaa   1080
   atggacgttg tctccgaaga acctttaatt gaatggctag cagctaacta caaaaacttc   1140
   ggtgctacct tggaattcat cacagacaaa tcttcagaag gtgcccaatt tgtcacaggt   1200
   tttggtggta ttggtgccat gctgcgttac aaagttaatt ttgaacaact agttgatgaa   1260
   tctgaggatg aatattatga cgaagatgaa ggatccgact atgatttcat ttaa         1314
<210> 59
   <211> 437
   <212> PRT
   <213> Saccharomyces cerevisiae
<400> 59
   Met Asp Asn Glu Val Glu Lys Asn Ile Glu Ile Trp Lys Val Lys Lys
   1               5                   10                  15
   Leu Val Gln Ser Leu Glu Lys Ala Arg Gly Asn Gly Thr Ser Met Ile
               20                  25                  30
   Ser Leu Val Ile Pro Pro Lys Gly Leu Ile Pro Leu Tyr Gln Lys Met
           35                  40                  45
   Leu Thr Asp Glu Tyr Gly Thr Ala Ser Asn Ile Lys Ser Arg Val Asn
       50                  55                  60
   Arg Leu Ser Val Leu Ser Ala Ile Thr Ser Thr Gln Gln Lys Leu Lys
   65                  70                  75                  80
   Leu Tyr Asn Thr Leu Pro Lys Asn Gly Leu Val Leu Tyr Cys Gly Asp
                   85                  90                  95
   Ile Ile Thr Glu Asp Gly Lys Glu Lys Lys Val Thr Phe Asp Ile Glu
   100 105 110
   Pro Tyr Lys Pro Ile Asn Thr Ser Leu Tyr Leu Cys Asp Asn Lys Phe
   115 120 125
   His Thr Glu Val Leu Ser Glu Leu Leu Gln Ala Asp Asp Lys Phe Gly
   130 135 140
   Phe Ile Val Met Asp Gly Gln Gly Thr Leu Phe Gly Ser Val Ser Gly
   145 150 155 160
   Asn Thr Arg Thr Val Leu His Lys Phe Thr Val Asp Leu Pro Lys Lys
   165 170 175
   His Gly Arg Gly Gly Gln Ser Ala Leu Arg Phe Ala Arg Leu Arg Glu
   180 185 190
   Glu Lys Arg His Asn Tyr Val Arg Lys Val Ala Glu Val Ala Val Gln
   195 200 205
   Asn Phe Ile Thr Asn Asp Lys Val Asn Val Lys Gly Leu Ile Leu Ala
   210 215 220
   Gly Ser Ala Asp Phe Lys Thr Asp Leu Ala Lys Ser Glu Leu Phe Asp
   225 230 235 240
   Pro Arg Leu Ala Cys Lys Val Ile Ser Ile Val Asp Val Ser Tyr Gly
   245 250 255
   Gly Glu Asn Gly Phe Asn Gln Ala Ile Glu Leu Ser Ala Glu Ala Leu
   260 265 270
   Ala Asn Val Lys Tyr Val Gln Glu Lys Lys Leu Leu Glu Ala Tyr Phe
   275 280 285
   Asp Glu Ile Ser Gln Asp Thr Gly Lys Phe Cys Tyr Gly Ile Asp Asp
   290 295 300
   Thr Leu Lys Ala Leu Asp Leu Gly Ala Val Glu Lys Leu Ile Val Phe
   305 310 315 320
   Glu Asn Leu Glu Thr Ile Arg Tyr Thr Phe Lys Asp Ala Glu Asp Asn
   325 330 335
   Glu Val Ile Lys Phe Ala Glu Pro Glu Ala Lys Asp Lys Ser Phe Ala
   340 345 350
   Ile Asp Lys Ala Thr Gly Gln Glu Met Asp Val Val Ser Glu Glu Pro
   355 360 365
   Leu Ile Glu Trp Leu Ala Ala Asn Tyr Lys Asn Phe Gly Ala Thr Leu
   370 375 380
   Glu Phe Ile Thr Asp Lys Ser Ser Glu Gly Ala Gln Phe Val Thr Gly
   385 390 395 400
   Phe Gly Gly Ile Gly Ala Met Leu Arg Tyr Lys Val Asn Phe Glu Gln
   405 410 415
   Leu Val Asp Glu Ser Glu Asp Glu Tyr Tyr Asp Glu Asp Glu Gly Ser
   420 425 430
   Asp Tyr Asp Phe Ile
   435
<210> 60
   <211> 1137
   <212> DNA
   <213> Thermus thermophilus
<400> 60
   ttgcgcctcg cttcgcaatc tgctatcctg gtaaaggtat ggacctggaa cgcctcgcgc      60
   aacgcctgga aggcctcagg gggtatcttt gacatccccc aaaaggaaac ccgtctaaaa     120
   gagctggagc ggcgcctcga ggacccctcc ctctggaacg atcccgaggc cgcccgcaag     180
   gtgagccagg aggccgcccg cctccggcgc accgtggaca ccttccgctc cctggaaagc     240
   gacctccagg gccttttgga gctcatggag gagcttcccg ccgaggaacg ggaggccctc     300
   aagcccgagc tggaggaggc cgcgaagaag ctggacgagc tctaccacca gaccctcctc     360
   aacttccccc acgcggagaa gaacgccatc ctcaccatcc agcccggggc cgggggcacg     420
   gaggcctgcg actgggcgga gatgctccta aggatgtaca cccgcttcgc cgagcgccag     480
   ggcttccagg tggaggtggt ggacctcacc cctgggcccg aggcgggcat tgactacgcc     540
   cagatcctgg tcaaggggga gaacgcctac ggcctccttt cccccgaggc cggggtgcac     600
   cgcctggtgc gcccttcccc ctttgacgcc tcgggccgcc gccacacctc cttcgccggg     660
   gtggaggtga tccccgaggt ggacgaggag gtggaggtgg tgctcaagcc cgaggagctc     720
   cgcattgacg tgatgcgggc ctcggggccc gggggccagg gggtgaacac cacggactcg     780
   gcggtgcggg tggtccacct gcccacgggg atcaccgtga cctgccagac cacgcggagc     840
   cagatcaaga acaaggaact cgccctcaag atcctcaagg cccgcctcta cgagctggag     900
   cggaagaagc gggaggaaga gctcaaggcc ctgaggggcg aggtgcggcc catagagtgg     960
   ggaagccaga tccggagcta cgtcctggac aagaactacg tcaaggacca ccgcaccggg    1020
   ctcatgcgcc acgacccgga aaacgtcctg gacggggacc tcatggacct gatctgggcg    1080
   ggcctggagt ggaaggcggg ccgccgccag gggacggagg aggtggaggc ggagtag       1137
<210> 61
   <211> 378
   <212> PRT
   <213> Thermus thermophilus
<400> 61
   Met Arg Leu Ala Ser Gln Ser Ala Ile Leu Val Lys Val Trp Thr Trp
   1               5                   10                  15
   Asn Ala Ser Arg Asn Ala Trp Lys Ala Ser Gly Gly Ile Phe Asp Ile
               20                  25                  30
   Pro Gln Lys Glu Thr Arg Leu Lys Glu Leu Glu Arg Arg Leu Glu Asp
           35                  40                  45
   Pro Ser Leu Trp Asn Asp Pro Glu Ala Ala Arg Lys Val Ser Gln Glu
       50                  55                  60
   Ala Ala Arg Leu Arg Arg Thr Val Asp Thr Phe Arg Ser Leu Glu Ser
   65                  70                  75                  80
   Asp Leu Gln Gly Leu Leu Glu Leu Met Glu Glu Leu Pro Ala Glu Glu
                   85                  90                  95
   Arg Glu Ala Leu Lys Pro Glu Leu Glu Glu Ala Ala Lys Lys Leu Asp
               100                 105                 110
   Glu Leu Tyr His Gln Thr Leu Leu Asn Phe Pro His Ala Glu Lys Asn
           115                 120                 125
   Ala Ile Leu Thr Ile Gln Pro Gly Ala Gly Gly Thr Glu Ala Cys Asp
       130                 135                 140
   Trp Ala Glu Met Leu Leu Arg Met Tyr Thr Arg Phe Ala Glu Arg Gln
   145                 150                 155                 160
   Gly Phe Gln Val Glu Val Val Asp Leu Thr Pro Gly Pro Glu Ala Gly
                   165                 170                 175
   Ile Asp Tyr Ala Gln Ile Leu Val Lys Gly Glu Asn Ala Tyr Gly Leu
               180                 185                 190
   Leu Ser Pro Glu Ala Gly Val His Arg Leu Val Arg Pro Ser Pro Phe
           195                 200                 205
   Asp Ala Ser Gly Arg Arg His Thr Ser Phe Ala Gly Val Glu Val Ile
       210                 215                 220
   Pro Glu Val Asp Glu Glu Val Glu Val Val Leu Lys Pro Glu Glu Leu
   225                 230                 235                 240
   Arg Ile Asp Val Met Arg Ala Ser Gly Pro Gly Gly Gln Gly Val Asn
                   245                 250                 255
   Thr Thr Asp Ser Ala Val Arg Val Val His Leu Pro Thr Gly Ile Thr
               260                 265                 270
   Val Thr Cys Gln Thr Thr Arg Ser Gln Ile Lys Asn Lys Glu Leu Ala
           275                 280                 285
   Leu Lys Ile Leu Lys Ala Arg Leu Tyr Glu Leu Glu Arg Lys Lys Arg
       290                 295                 300
   Glu Glu Glu Leu Lys Ala Leu Arg Gly Glu Val Arg Pro Ile Glu Trp
   305                 310                 315                 320
   Gly Ser Gln Ile Arg Ser Tyr Val Leu Asp Lys Asn Tyr Val Lys Asp
                   325                 330                 335
   His Arg Thr Gly Leu Met Arg His Asp Pro Glu Asn Val Leu Asp Gly
               340                 345                 350
   Asp Leu Met Asp Leu Ile Trp Ala Gly Leu Glu Trp Lys Ala Gly Arg
           355                 360                 365
   Arg Gln Gly Thr Glu Glu Val Glu Ala Glu
       370                 375
<210> 62
   <211> 310
   <212> PRT
   <213> Alicyclobacillus acidocaldarius
<400> 62
   Met Pro Leu Asp Pro Val Ile Gln Gln Val Leu Asp Gln Leu Asn Arg
   1               5                   10                  15
   Met Pro Ala Pro Asp Tyr Lys His Leu Ser Ala Gln Gln Phe Arg Ser
               20                  25                  30
   Gln Gln Ser Leu Phe Pro Pro Val Lys Lys Glu Pro Val Ala Glu Val
           35                  40                  45
   Arg Glu Phe Asp Met Asp Leu Pro Gly Arg Thr Leu Lys Val Arg Met
       50                  55                  60
   Tyr Arg Pro Glu Gly Val Glu Pro Pro Tyr Pro Ala Leu Val Tyr Tyr
   65                  70                  75                  80
   His Gly Gly Gly Trp Val Val Gly Asp Leu Glu Thr His Asp Pro Val
                   85                  90                  95
   Cys Arg Val Leu Ala Lys Asp Gly Arg Ala Val Val Phe Ser Val Asp
               100                 105                 110
   Tyr Arg Leu Ala Pro Glu His Lys Phe Pro Ala Ala Val Glu Asp Ala
           115                 120                 125
   Tyr Asp Ala Leu Gln Trp Ile Ala Glu Arg Ala Ala Asp Phe His Leu
       130                 135                 140
   Asp Pro Ala Arg Ile Ala Val Gly Gly Asp Ser Ala Gly Gly Asn Leu
   145                 150                 155                 160
   Ala Ala Val Thr Ser Ile Leu Ala Lys Glu Arg Gly Gly Pro Ala Leu
                   165                 170                 175
   Ala Phe Gln Leu Leu Ile Tyr Pro Ser Thr Gly Tyr Asp Pro Ala His
               180                 185                 190
   Pro Pro Ala Ser Ile Glu Glu Asn Ala Glu Gly Tyr Leu Leu Thr Gly
           195                 200                 205
   Gly Met Met Leu Trp Phe Arg Asp Gln Tyr Leu Asn Ser Leu Glu Glu
       210                 215                 220
   Leu Thr His Pro Trp Phe Ser Pro Val Leu Tyr Pro Asp Leu Ser Gly
   225                 230                 235                 240
   Leu Pro Pro Ala Tyr Ile Ala Thr Ala Gln Tyr Asp Pro Leu Arg Asp
                   245                 250                 255
   Val Gly Lys Leu Tyr Ala Glu Ala Leu Asn Lys Ala Gly Val Lys Val
               260                 265                 270
   Glu Ile Glu Asn Phe Glu Asp Leu Ile His Gly Phe Ala Gln Phe Tyr
           275                 280                 285
   Ser Leu Ser Pro Gly Ala Thr Lys Ala Leu Val Arg Ile Ala Glu Lys
       290                 295                 300
   Leu Arg Asp Ala Leu Ala
   305                 310

## Description

The present invention relates to the use of a thermostable esterase of procaryotic origin for monitoring and/or tracking the synthesis of a protein, polypeptide or peptide in a cell-free translation system or in an *in vivo* expression system in which the synthesis of a protein, polypeptide or peptide can occur, wherein said monitoring and/or tracking comprises the detection of the function or activity of said esterase; preferably said function or activity is the enzymatic function of said esterase. The present invention discloses the use of a vector comprising a nucleic acid molecule coding for a thermostable esterase of procaryotic origin and expressing an esterase fusionprotein. Moreover, the present invention discloses the use of a vector comprising a nucleic acid molecule coding for a thermostable esterase of procaryotic origin and comprising in frame at least one multiple cloning site for a further protein/polypeptide /peptide, to be expressed in form of a fusion protein comprising said esterase (esterase activity) and said further proteinaceous peptide structure. The present invention discloses the use of a protein, polypeptide or peptide encoded by the vectors disclosed in the context of the present invention. Also disclosed is a method for monitoring and/or tracking the synthesis of a protein, polypeptide or peptide in a cell-free translation system or in an *in vivo* expression system, comprising the step of detecting the function of a thermostable esterase of procaryotic origin. Moreover, the present invention relates to uses of the vectors of the present invention or the nucleic acid molecules comprised therein for monitoring and/or tracking the synthesis of a protein, polypeptide or peptide in a cell-free translation system or in an *in vivo* expression system, whereby the monitoring and/or tracking comprises the detection of the function of said esterase.

Cell-free (coupled transcription/)translation systems for the in vitro synthesis of proteins are used either for the production of (functionally active) proteins or for studying of protein biosynthesis (in vitro) (Spirin (2002), Cell-Free Translation Systems. Springer Verlag, Berlin.). Normally, the cell-free (coupled transcription/)translation systems are derived from prokaryotic cells such as *E. coli* cells (e.g. Zubay (1973), Imm. Rev. Genet. Vol. 7, page 267) or from eukaryotic cells such as rabbit reticulocytes (e.g. Pelham (1976), Eur. J. Biochem. Vol. 131, page 289) and wheat germ cells (e.g. Spirin (1990), American Society for Microbiology, 56-70; Stiege (1995), J. Biotechnol. 41:81-90). The use of this systems became a standard technology in laboratory praxis (e. g., Baranov (1989), Gene, 84, 463-436; Endo (1992), J. Biotechnol., 25, 221-230) and the proteins produced by this systems are widely employed in biochemical, biostructural and pharmaceutical uses, not only in fundamental research but also in the biochemical, chemical and pharmaceutical industry.

To achieve high yields of the desired proteins (to be expressed) and in order to facilitate their isolation, optimization of the employed (coupled transcription/)transiation systems is required. In particular, in often used (coupled transcription/)translation systems the monitoring of the synthesis of the proteins is necessary.

Accordingly, there is a need for useful reporter proteins/molecules/groups and protein tags. Up to now, several representatives of this reporters have been tried, however, with limitations and down sides.

One example of a marker/monitor reporter to detect protein expression, in particular in bacterial or eukaryotic in vitro translation systems/cell-free translation systems, is the green fluorescent protein (GFP) (Kolb (1996), Biotechnology Letters, 18, 1447-1452.). Others comprise, firefly luciferase (Kolb (1994), EMBO J., 13, 3631-3637.), dihydrofolate reductase (DHFR) (Endo (1992), J. Biotechnol., 25, 221-230.; Kudlicki (1992), Biochem., 206, 389-393.), chloramphenicol acetyl transferase (CAT) (Kigawa (1991), J. Biochem. (Tokyo), 110, 166-168) and β-galactosidase (Noti (1980), J. Bacteriol., 144, 291-299.). Several modifications of these markers, which are often colorimetrically and/or fluorimetrically assessed, are employed in the art.

Especially green fluorescent protein (GFP), in particular in form of enhanced green fluorescent protein (eGFP), is one of the most commonly used reporter group. This protein requires time (several hours) for maturation (Coxon (1995), Chem. Biol., 2, 119-121.) and the fluorophor is formed post-translationally by oxidation with molecular oxygen. Therefore, the direct in-situ monitoring of protein expression (in cell-free systems, like (coupled transcription/)translation systems) is not possible. Moreover, the sensitivity of the detection of (e)GFP-labelled proteins is quite low and has also quantitative limits: About 100 mg/ml can be visualized directly with the naked eye, 0.1 mg/ml can be detected by fluorometer, and 1 µg of GFP is visible as a band in electrophoresis gel (Chekulayeva (2001), Biochem Biophys Res Commun., 280,914-917).

Similarly, even though the activity of firefly luciferase can be measured directly in cell-free translation systems (Kolb (2000), J. Bio.l Chem., 275, 16597-16601) it dramatically looses enzymatic activity at temperatures above 30°C. However, often higher temperatures are required (e.g. 37 °C in *E. coli* cell free extracts (see also in the appended examples)) and therefore, temperature sensitivity of reporter proteins is of great disadvantage.

For the detection of chloramphenicol acetyl transferase activity, a radioactive substrate and expensive equipments are required (Young (1985), DNA, 4, 469-475) and therefore said detection is complicated.

Further to other difficulties in the usage of the above listed reporters, their main disadvantage (for their practical use) is their lack of thermostability as they are derived from organisms living at normal temperature ranges (10 - 40 °C; mesophilic organisms). Along with temperature limitation, direct monitoring of the other above listed enzymatic activities in complex (coupled transcription/)translation mixtures is not possible.

Bonning proposes the use of a eucaryotic juvenile hormone esterase as a reporter enzyme in the baculovirus expression system (J. of Virolog. Meth. 51, 1, 1995, 103-113) and describes the expression of such an esterase and beta-galactosidase from the basic protein promoter of Autographa californica nuclear polyhedrosis virus compared to the p10 protein and polyhedron promoters (J. of General Virol., Society for General Microb., Spencers Wood, GB, 75, 1995, 1551-1556). Looms describes the functional protective role for mucin glycosylated repetitive domains (Europ. J. of Biochem. 266, 1, 1999, 105-111), Farrell the secretion of cytoplasmic and nuclear proteins from animal cells using novel secretion modules (Proteins: Structure, Function and Genetics, Alan R. Liss, US 41, 1, 2000, 144-153) and Manco the esterase from Bacillus acidocaldarius with sequence similarity to hormone-sensitive lipase subfamily (Biochem. J. 332, 1, 1998, 203-212).

Thus, the technical problem underlying the present invention is the provision of reliable means and methods for the assessment of the capabilities of a given cell-free translation system and/or cell free transcription/translation systems in vitro protein/peptide synthesis.

The solution to the above technical problem is achieved by providing the embodiments characterized in the claims.

Accordingly, the present invention relates to the use of a thermostable esterase of procaryotic origin for monitoring and/or tracking the synthesis of a protein, polypeptide or peptide in a cell-free translation system or in an *in vivo* expression system in which the synthesis of a protein, polypeptide or peptide can occur, wherein said monitoring and/or tracking comprises the detection of the function of said esterase.

The present invention solves the above identified technical problem since, as documented herein below and in the appended examples, it was surprisingly found that thermostable esterases of prokaryotic origin (or enzymatic thermostable prokaryotic esterase activities of polypeptides or functional fragments of thermostable prokaryotic esterases comprising esterase activity) may be employed as markers for the determination of the efficacy and/or function of cell-free translation systems or in *in vivo* expression systems. Accordingly, and in particular embodiments, the present invention allows for the expression of a fusion construct comprising a desired moiety "X" and a thermostable procaryotic esterase moiety. In context of the present invention, the term "(thermostable prokaryotic) esterase moiety" refers to a full length esterase, as well as to a fragment thereof displaying esterase activity/esterase function.

It was found that the synthesis of a protein, polypeptide or peptide in a cell-free translation system or in an *in vivo* expression system can be easily and efficiently be detected when said protein, polypeptide or peptide is synthesized with, preferably, a covalently attached/bound thermostable esterase of procaryotic origin (or a thermostable procaryotic esterase activity). Accordingly, the present invention provides, in one embodiment, for fusionproteins/fusionpolypeptides to be expressed in cell-free translation systems, whereby said fusionproteins/fusionpolypeptides comprise a thermostable procaryotic esterase activity as one part of said fusionproteins/fusionpolypeptides and the protein/polypeptide/peptide to be expressed or desired to be expressed in the translation system as at least one further part. As will be detailed below, the fusionproteins/fusionpolypeptides are, accordingly, expressed in said translation system in the format "esterase-X" or "X-esterase", whereby "esterase" denotes the esterase (or esterase-activity) as defined herein and "X" denotes the a protein, polypeptide or peptide desired to be expressed in the translation system. Accordingly, the desired protein, polypeptide or peptide (to be expressed from a desired "target gene") may be covalently bound to the N- or C-terminus of the herein defined esterase (or a functional fragment of said esterase, displaying esterase activity/ esterase function) In the appended, not limiting example, as esterase/esterase activity esterase 2 of A. acidocaldarius (Est2) is employed and "X" is exemplified by green fluorescent protein (GFP). The person skilled in the art is readily in the position to replace said GFP by any desired protein/polypeptide or peptide "X" without deviating from the gist of the present invention.

The terms "esterase-X" and "X-esterase" are not limited to fusion proteins which comprise merely the esterase (or esterase activity) and the protein, polypeptide or peptide to be expressed. Said terms also comprise, inter alia, the possibility that also "linker" structures are comprised in said fusionproteins/fusionpolypeptides. Also comprised in context of this invention is the possibility that the esterase (or esterase activity) be expressed in context of fusion polypeptides whereby not only one protein, polypeptide or peptide is covalently expressed with said esterase. Therefore, the invention also provides for the use of an esterase for monitoring/tracking the syntheses of multiple proteins or polypeptides or peptides. Accordingly, also polypeptide structures, in form of fusionproteins/fusionpolypeptides, may be expressed in the cell-free translation system in the format "esterase-X-X' ", "X-X'-esterase" or "X'-esterase-X". In this respect, "X" denotes one particular protein/polypeptide/peptide to expressed and "X' " denotes a further protein/polypeptide/peptide. Also, the esterase (or esterase activity), X and X' may be separated by "linkers/linker structures", preferably by cleavable linker structures. As will be detailed below, such linkers/linker structures are known in the art and consist preferably of chemically and/or enzymatically cleavable structures. In context of this invention, it is of note that "X" does not only relate to full-length proteins desired to be synthesized in the cell-free translation systems but may also denote fragments of full-length proteins/polypeptides, preferably said fragments are "functional fragments", i.e. fragments comprising, when expressed a certain activity. Said activity may be, but is not limited to, an enzymatic activity of said fragment. Yet, the present invention is also useful in the monitoring/tracking of the in vitro synthesis of "peptides". Such peptides may comprise a minimal amount of amino acid residues, but comprise, preferably at least 10 amino acid residues, more preferably at least 12 amino acid residues, more preferably at least 15 amino acid residues, more preferably at least 20 amino acid residues, more preferably at least 30 amino acid residues, more preferably at least 40 amino acid residues and most preferably at least 50 amino acid residues. Such peptides to be expressed may, inter alia, be useful in immunization approaches.

In context of the present invention, the meaning of the term "protein(s)" may also include "peptide(s)" or "polypeptide(s)". The meaning of the terms "protein(s)", "peptide(s)" or "polypeptide(s)" are well known in the art (see ,e.g., Stryer (1995), Biochemistry, 4th edition). As known in the art, the term "peptide" comprises joined amino acid residues, whereby the alpha-carboxyl group of one amino acid is joined to the alpha-group of another amino acid by a peptide bond (amide bond); see also Stryer ((1995), loc. cit.). In accordance with the invention, the term "peptide(s)" comprises any such joined amino acid residues, whereby at least three, preferably at least five, most preferably at least seven amino acids (amino acid residues) are linked via said peptide bond (amide bond). The term polypeptide comprises, in accordance with this invention, at least 15 joined amino acid residues, more preferably at least 20 amino acid residues. Accordingly, joined amino acid residues comprising 3 to 14 amino acid residues are to be considered in accordance with this invention as "peptide" whereas joined amino acid residues comprising 15 or more amino acid residues are considered as polypeptides. The term "protein" is used as synonym with the term "polypeptide", whereas the term "protein" also may comprise a specific biological, biochemical or pharmaceutical function exerted by said protein. However, the person skilled in the art is aware that a protein is a polypeptide. The terms "protein", "peptide" and "polypeptide" also comprise molecules comprising at least one unnaturally occurring amino acid residue or at least one unusual amino acid residue and is not limited to proteinaceous structures comprising the twenty normally occurring amino acid residues; see also Stryer ((1995), loc. cit.).

The proteins, polypeptides and peptides as mentioned herein are the desired gene products to be produced by the target genes employed in the *in vitro* translation or *in vivo* expression systems as discussed and/or described herein. The term "target gene" , accordingly, means a gene to be expressed, in particular in the *in vitro* systems, preferably in the *in vitro* translation systems or the *in vivo* expression systems as discussed and described herein and as also known in the art. An exemplified *in vivo* expression system may be, e.g. a system based on prokaryotic hosts, like *E. coli.*

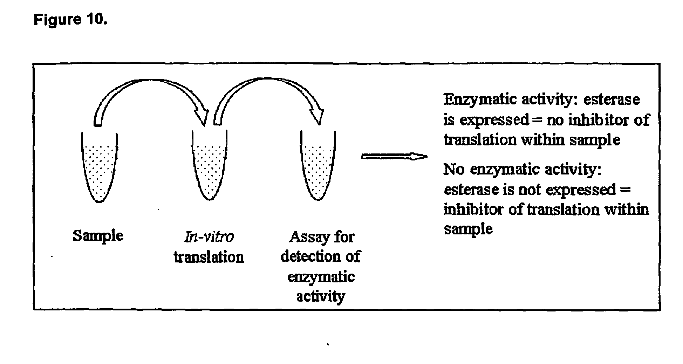

In context of the present invention, it was also found that the synthesis of a protein, polypeptide or peptide in a cell-free translation system can be easily and efficiently be detected when said protein, polypeptide or peptide to be synthesized is an esterase (or an esterase activity) or when said protein structure/polypeptide/peptide to be synthesized is covalently linked to thermostable procaryotic esterase/esterase activity. Therefore, in context of the present invention, it is envisaged that the esterase (or esterase activity) is either solely expressed in the employed cell-free translation system or that said esterase (esterase activity) is expressed in form of a fusion construct as described herein. In case the esterase is solely expressed, the use of an esterase for monitoring and/or tracking the synthesis of a protein, polypeptide or peptide in a cell-free translation system may be employed for determining the efficacy of said cell-free translation system per se. Accordingly, in one embodiment, the present invention provides for a method for screening substances that influence the function of protein biosynthesis in cell-free (coupled transcription/) translation systems. Said influence may be either the inhibiting or enhancing of the transcription step of in vitro biosynthesis of proteins and/or the inhibiting or enhancing the translation step of in vitro biosynthesis of proteins. An example of a method for screening the inhibitory effect of a substance on the translation step within a cell-free (coupled transcription/) translation system is shown in Figure 10.

The appended examples document that the present invention provides for a unique monitoring/tracking system for the expression of proteins, polypeptides or peptides in in vitro translation system. Furthermore, also a unique monitoring/tracking system for the expression of proteins, polypeptides or peptides in cellular expression systems is provided by the present invention. However, the present invention is particularly useful for the monitoring and/or tracking of the expression of proteins, polypeptides or peptides in *in vitro* translation systems/cell free translation systems. Cellular expression systems to be employed with respect to the present invention are known in the art and comprise, inter alia, bacterial cells (e.g. cells form *E. coli*) or eukaryotic cells (e.g. Yeast cells, CHO cells, HELA cells). A person skilled in the art is immediately able to replace in vitro translation systems as employed herein above by said cellular expression systems known in the art. The synthesis of the desired protein, polypeptide or peptide may be performed by heterologous or homologous expression in said systems. Again, the esterase/esterase activity as provided herein is used in this context as marker system for the monitoring and/or tracking of protein-bio synthesis or peptide-biosynthesis.

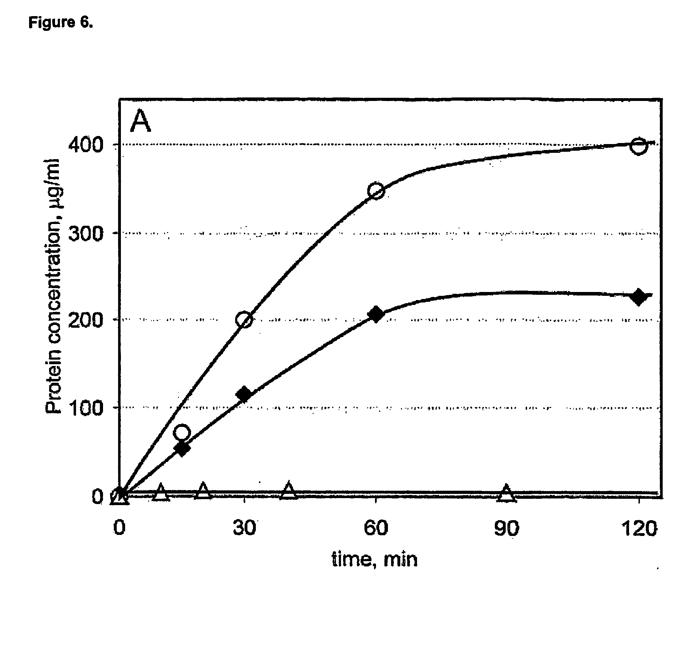

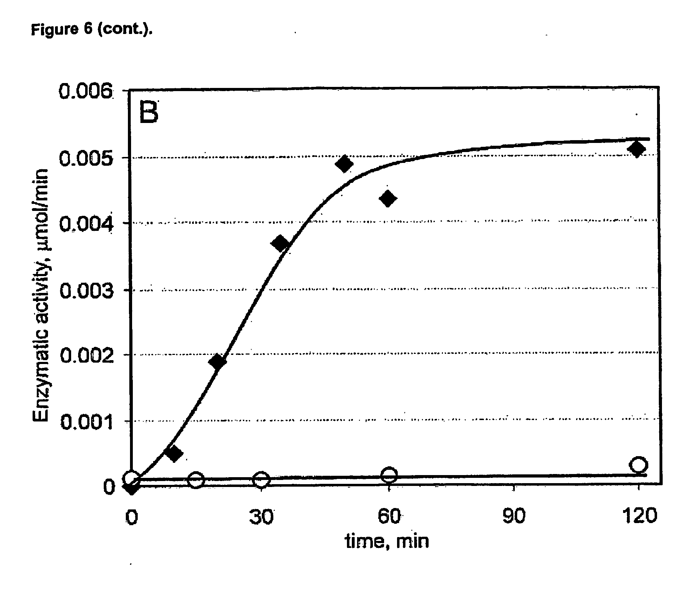

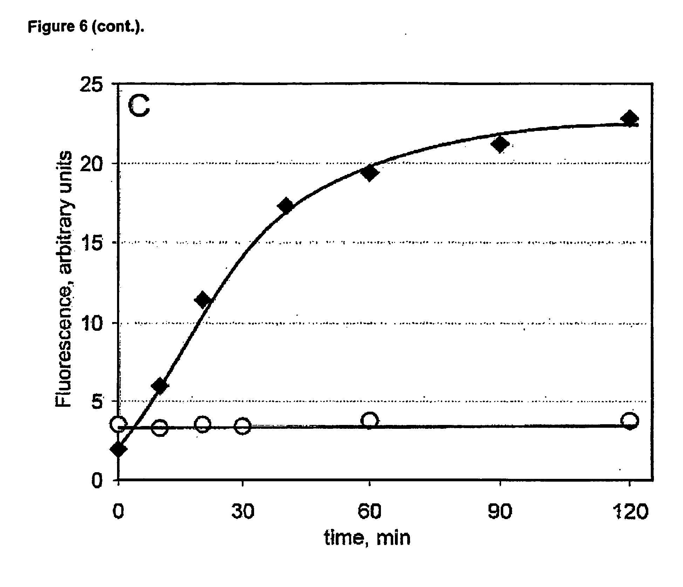

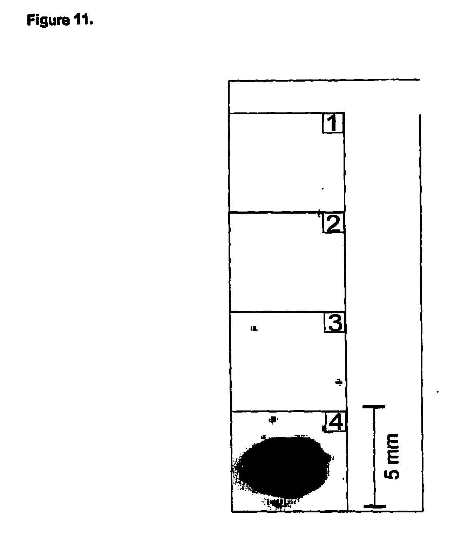

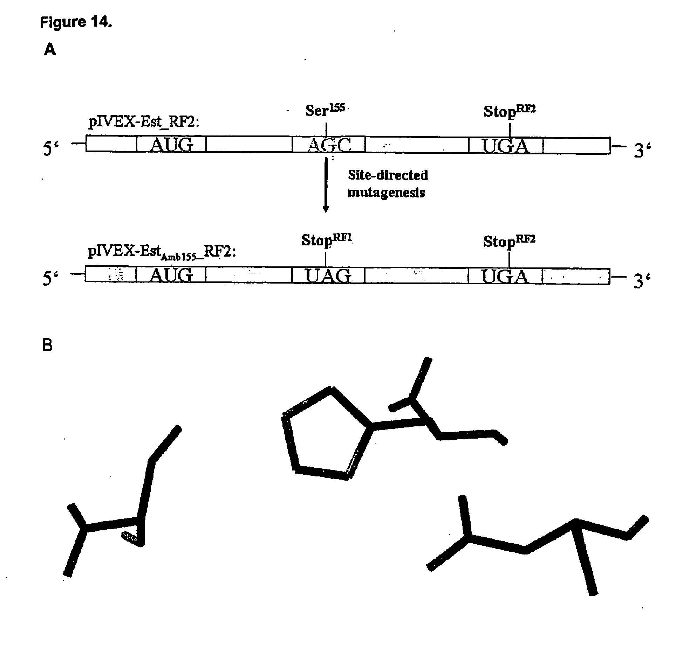

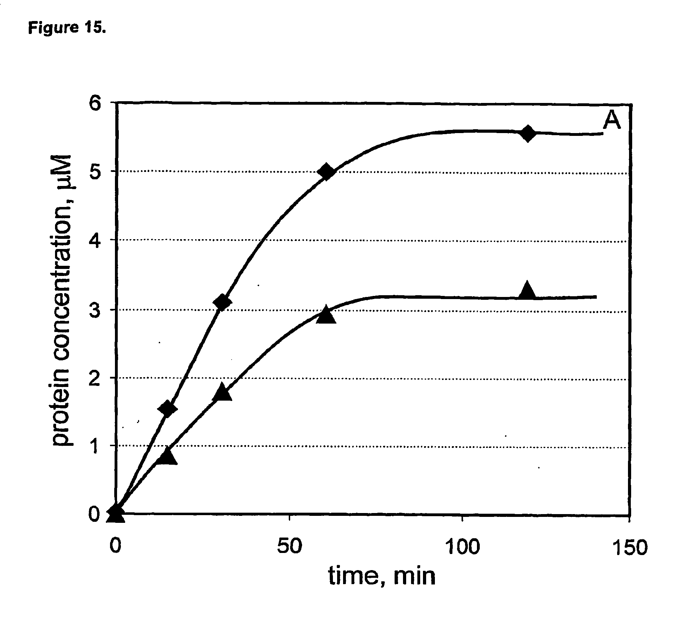

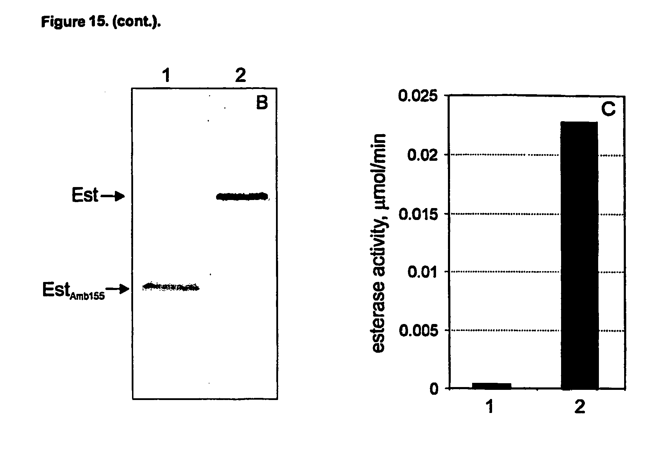

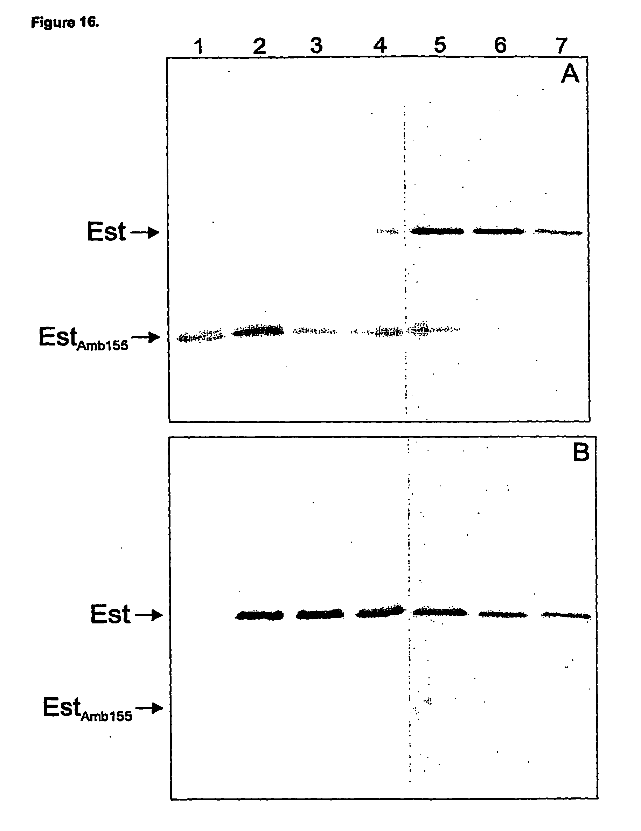

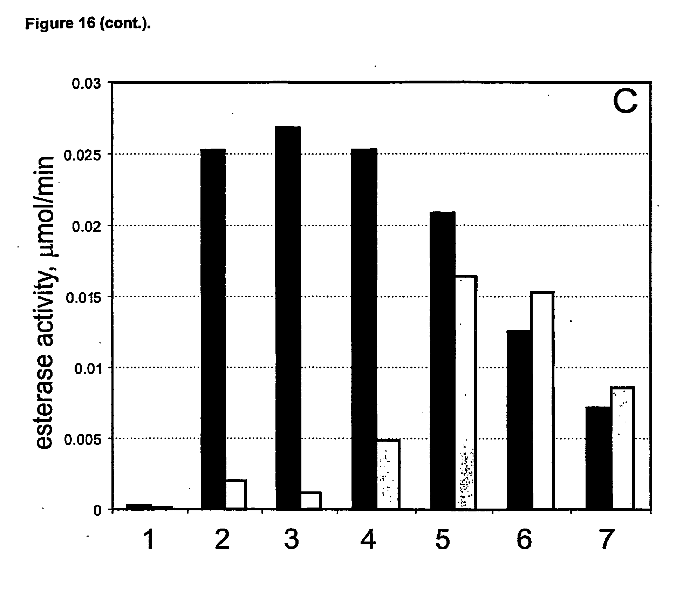

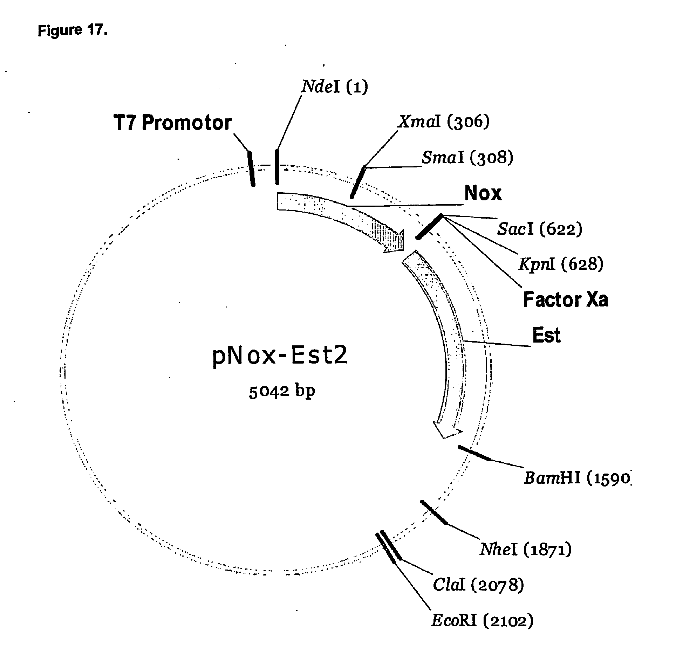

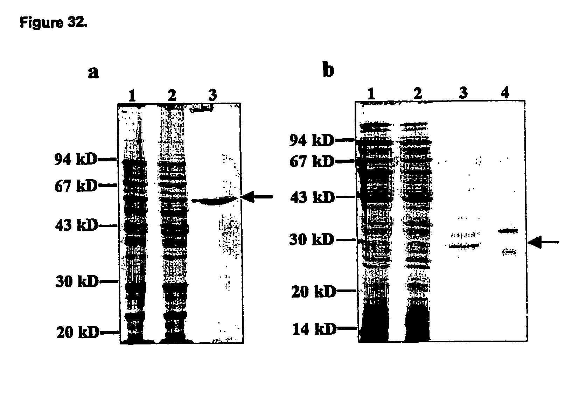

The examples further show that the esterase 2 from *Alicyclobacillus acidocaldarius* (Est2) can be synthesized with similar efficiency in a heterologous cell-free transcription/translation system (derived from *E. coli*) as an abundant homologous protein (elongation factor Ts from *E. coli*; Figure 6A), even the codon usage of the esterase gene was not adjusted to the codon usage of *E. coli* (Figure 6A). The synthesized esterase has high enzymatic activity (Figure 6B) and even a 1000 fold dilution of the translation mixture which results in 10⁻⁸ M final esterase concentration provides detectable esterase-activity. The examples also document that beside standard photometric action the Est2-activity is also fluorimetrically detectable (Example 6, Figure 6C). Further, the examples show that Est2 can be used for monitoring and/or tracking a synthesized protein (in the particular case the Est2 itself is monitored and/or tracked), in a gel after polyacrylamide gel electrophoresis (Example 6, Figure 6C). It is further demonstrated herein that esterases, like Est2, can be used for monitoring and/or tracking a protein to be synthesized, even if the protein to be monitored and/or tracked is not the esterase itself. Said protein to be monitored and/or tracked may be (heterologously) expressed (*in vitro* or *in vivo*), and/or (affinity) purified. Examples of these applications are provided herein, e.g. by Examples 17 to 22, Figures 17 to 32. These non-limiting examples show the monitoring and/or tracking of the proteins NADH oxidase (Nox) from *Thermus thermophilus*, elongation factor Tu from *Thermus thermophilus*, elongation factor Ts from *Thermus thermophilus, human* exportin-t and putative nuclease S2001 from *Sulfolobus solfataricus* by Est2 during their *in vitro* and/or *in vivo* expression (Examples 17 to 21, Figures 17 to 31 ) and/or their subsequent affinity purification (Examples 22, Figure 32). It is of particular note that a person skilled in the art is able to replace the exemplified proteins (to be monitored and/or tracked by the Est2 employed within the present application) by any other protein, polypeptide or peptide, which is desired to be monitored and/or tracked, e.g. during its (heterologous) expression (*in vitro* or *in vivo*), and/or (affinity) purification. Furthermore, the Examples show that the monitoring and/or tracking of the synthesis of an esterase (esterase-function) or a fragment thereof in a cell-free translation system can be used for detection of inhibitory or enhancing effects of substances on the function of protein biosynthesis in cell-free (coupled transcription/) translation systems. Thereby, esterase (Est2) can be used from screening of substances that influence the function of protein biosynthesis. Therefore, an esterase (Est2) is synthesized in cell-free (coupled transcription/) translation systems and the esterase activity during synthesis is detected. By adding substances to be screened to the cell-free (coupled transcription/) translation system in which the synthesis of the esterase (Est2) occurs, the effect of the added substance to protein biosynthesis can be investigated by detecting changes of the esterase activity (Figure 10). The examples further demonstrate the usage of Est2 to monitor and/or track the synthesis of alloproteins. (Example 12, Figure 11; Example 16, Figure 15 and 16). In particular it was shown that Est2 incorporates biotinylated puromycin at high yield in the presence of antibodies directed against release factor 1 of *Thermus thermophilus* (SEQ ID NO: 4) in a cell-free coupled transcription translation system (Example 12, Figure 11). In the particular case, the synthesis of the Est2-puromycin-biotin conjugate was performed on strepavidin-coded glass plates and the Est2-activity was detected directly on said strepavidin-coded glass plates having immobilized the synthesized esterase-puromycin-biotin conjugate (Figure 11, Spot 4). The examples further show the use of an esterase for monitoring and/or tracking the synthesis of a protein in a cell-free translation system from *E. coli*, having the release factor 1 contained in that cell-free translation system inactivated. This inactivation was achieved by addition of antibodies directed against release factor 1 from *Thermus thermophilus* which are capable to deplete the release factor 1 from *E. coli* (SEQ ID NO: 6) from the cell-free translation system by precipitation. It was demonstrated that in presence of suppressor tRNA^{SerCUA} and in the absence of release factor 1 an artificially introduced nonsense codon (replacing serine 155 of the Est2 which is essential for the function of Est2) was suppressed. Example 16, Figure 14 to 16. In this particular case, esterase activity was only detectable when the (functional) full length Est2 was synthesized. The synthesis of the functional Est2 only takes place, when the release factor 1 from *E. coli* was depleted from the cell-free translation system and thereby the suppressor seryl-tRNA^{SerCUA} was able to bind to the introduced nonsense codon to deliver the essential serine 155 (Figure 15B/C (2); Figure 16). The esterase activity was not detectable when the active release factor 1 obviates the binding of the suppressor seryl-tRNA^{SerCUA} to the artificially introduced nonsense codon and forces the synthesis of a non-functional Est2-fragment, due to termination (Figure 15B/C (1); Figure 16A (1)).

As detailed above and exemplified herein, the present invention provides for the use of an enzyme, i. e. a thermostable esterase of procaryotic origin, preferably a thermostable esterase isolated or obtained from thermophilic bacteria, more preferentially from *Alicyclobacillus acidocaldarius*, most preferably the esterase 2 from *Alicyclobacillus acidocaldarius* (Est2; Manco (1998), Biochem. J., 332, 203-212) as a reporter enzyme for monitoring and/or tracking of protein synthesis in, preferably, *in vitro* (coupled transcription/)translation systems. However, the use as a reporter enzyme in *in vivo* expression systems, e.g. eukaryotic or prokaryotic cells, preferably in prokaryotic cells, is also envisaged and exemplified herein. *In vivo* expression systems may be prokaryotic systems, like the *E. coli* expression system employed in the examples appended for the expression of heterologous fusion proteins like "X-esterase" or "esterase-X" as defined herein. However, similar heterologous expression of fusion proteins ("X-esterase" or "esterase-X") as defined herein is also envisaged in eukaryotic cells, like yeast cells, plant cells, or animal cells. These animal cells may e.g. be insect cells or mammalian cells. Corresponding expression systems *(in vivo* expression systems) are known in the art and comprise the expression in CHO cells, COS cells, HELA cells and the like.

The sequences coding for Est2 are known in the art and, e.g. obtainable from Hemilä (1994), Biochim. Biophys Acta 1210, 249-253. Furthermore, said sequences are documented herein under SEQ ID. No. 1 (coding sequence) and by the expressed amino acid sequence shown in SEQ ID NO. 2 or SEQ ID NO. 62. It is evident that the person skilled in the art may modify said sequences for specific purposes. For example, as also done herein, specific further/additional restriction sites may be introduced. Corresponding examples are given in the Est2 sequences comprised in the plasmids provided herein and shown in SEQ ID. NO 8, 9 or 10.
The term "isolated from" is not limited to direct isolation of said esterase from the corresponding species but relates, in particular, to its recombinant expression in the prokaryotic cell-free translation systems. As exemplified herein and shown in the appended examples, particular preferred is a cell-free translation system derived from *E. coli.*
Whenever employed herein, the term "cell-free translation system" is not limited to systems, wherein solely translation occurs. The term also, and in a preferred embodiment, comprises all cell-free coupled transcription/translation systems in which not only translation from a given RNA/mRNA occurs, but also the transcription step, e.g. the transcription from a given vector and/or DNA, like cDNA (peGFP-Est2 (SEQ ID NO:8)) takes place. The term "transcription" refers to the synthesis of RNA/mRNA, capable to code for the protein to be synthesized, by the cellular transcription machinery using a DNA, for example a cDNA as a template. The mode of operation and the composition of the cellular transcription machinery is known to a person skilled in the art. The term "translation" refers to the synthesis of a protein, polypeptide or peptide, whereby the transcription product (RNA/mRNA) acts as a template for the cellular translation machinery. Again, the mode of operation and the composition of the cellular translation machinery is known to a person skilled in the art.

In accordance with this invention, the term "esterase" relates to an enzyme with the enzymatic function or activity of a polypeptide (or of a fragment of such a polypeptide) which is capable of the cleavage of an ester into an alcohol and an carboxylic acid. In context of the present invention, the term "alcohol" refers to a compound carrying at least one hydroxyl group and the term "carboxylic acid" refers to a compound carrying at least one carboxyl group. Said "esterase" preferably refers to a protein comprising the consensus sequence HGGG and GXSXG as described in Hemilä ((1994), Biochemica et Biophysica kcta 1210, 249-253). Esterases are known in the art and comprise but are not limited to i.e. esterases as disclosed in Hemilä (1994), loc. cit.. As detailed below and as shown in the appended examples, a particular preferred esterase to be used in context of this invention is a thermostable esterase of prokaryotic origin. A preferred example of such an esterase is the esterase 2 from *Alicyclobacillus acidocaldaius*, as described herein below and as shown in (Manco, G. (1998), Biochem. J 332 ( Pt 1), 203-212). The coding sequence of said esterase 2 is shown in SEQ ID NO: 1, the corresponding amino acid sequence is shown in SEQ ID NO: 2 or SEQ ID NO. 62. It is preferred that the amino acid sequence of the esterase 2 from *Alicyclobacillus acidocaldarius* to be employed within the present invention is that of SEQ ID NO. 62.

The term "esterase" as employed herein does not only comprise full-length esterases, but also functional fragments of esterases which are capable of the cleavage of an ester into an alcohol and an carboxylic acid. Such a "functional fragment" may be of any length, however, preferably such functional fragments comprise at least 50, more preferably at least 60, more preferably at least 80 and more preferably at least 100 amino acid residues.

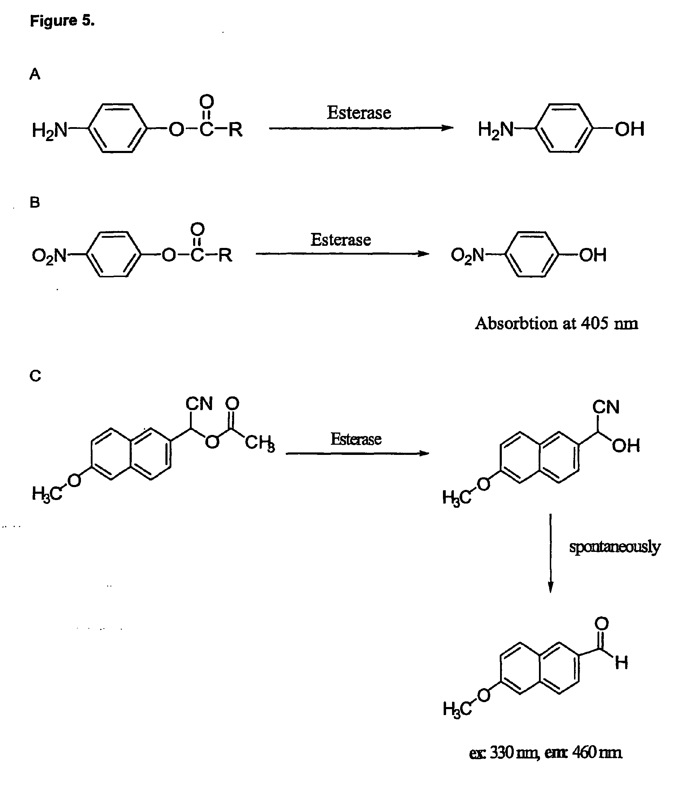

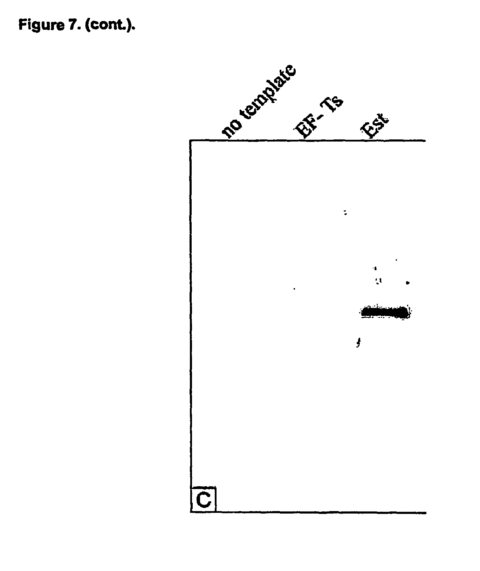

In context of the invention, particular preferred esterases are single chain esterases. However, it is also envisaged that individual, single chained polypeptides are employed in context of this invention which are derived from bi-or multichained esterases or from (esterase) complexes. These single chained polypeptides to be employed in context of this invention comprise the esterase activity or at least a part of said activity. Accordingly, as used herein, the term "esterase" relates to any polypeptide which can be expressed in cell-free translation systems and which comprise an esterase activity which may be measured. The measurement of "esterase activity" is performed by methods known in the art and as detailed herein, in particular in the appended examples. Such methods for the detection of "esterase activity" comprise photometric detection, wherein e.g. the esterase-catalysed hydrolysis of p-nitrophenyl acetate to the corresponding alcohol is performed (see, inter alia, Fig. 5B, Fig. 6B, example 6), electrochemical detection, wherein e.g. the esterase-catalysed hydrolysis of p-aminophenyl acetate to the corresponding alcohol is performed (see, inter alia, Fig. 5A) and fluorescent detection (see, inter alia, Fig. 5C; example 6, Fig. 6C), wherein e.g. the esterase hydrolyses 5-(and-6)-carboxy-2',7'- dichlorofluorescein diacetate which leads to the appearance of a fluorescent product. Furthermore it is exemplified herein that the detection of an esterase is also possible in the gels after sodium dodecyl sulfate polyacrylamide gel electrophoresis; see, inter alia, Fig. 7C.

As pointed out above, a particular preferred esterase of the present invention is the esterase 2 of *Alicyclobacillus acidocaldarius*.

The preferred esterase (Est2) from *Alicyclobacillus acidocaldarius* to be employed in the inventive uses and methods is a thermostable enzyme that consists of one polypeptide chain and possesses a broad substrate specificity (Manco, G. (1998) loc. cit.). Due to high thermostability, practically instant folding and refolding and easily detectable activity, this esterase has a potential application as a reporter for in vitro and in vivo protein expression systems. The tertiary structure of the esterase was determined by X-ray crystallography (De Simone, G. (2000) J Mol. Biol 303, 761-771). Serine 155, located in the Ser-His-Asp catalytic triad (Fig. 14B), is essential for hydrolytic activity (De Simone, G. (2000) J Mol. Biol 303, 761-771) It is encoded by the ACG triplet at the corresponding position of the est2 mRNA (Hemila (1994), Biochim. Biophys. Acta 1210, 249-253). As already mentioned before and exemplified herein below, the coding sequence for serine 155 was substituted to a RF1-dependent stop codon (UAG) and the resulting construct was used to test the conditions for efficient termination and/or suppression at UAG stop codon.

Many investigators dealing with mechanism of termination and suppression of termination codons use SDS-PAGE as a criterion for monitoring of suppression events. The possibility of increased translation error rates due to high concentrations of unnatural suppressor tRNAs were usually disregarded. The construction of Est2 mRNA(amber 155) from the template pEst2_amber 155 (see herein below) allows to monitor in parallel the efficiency of the UAG suppression by a band shift in SDS-PAGE and the accumulation of esterase activity in the in vitro translation mixture. This assay is suitable for estimation of optimal conditions to achieve highly efficient suppression in different in vitro translation mixtures. Such assessment seems to be very important since translation systems may individually differ from each other due to different source and preparation method.

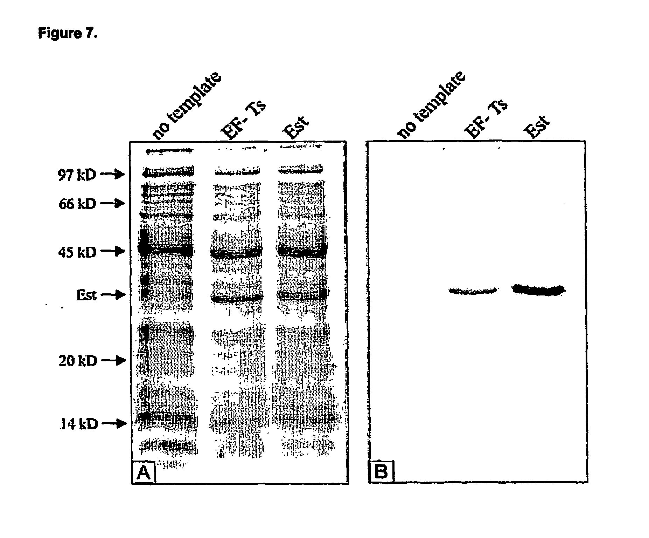

The 34 kD esterase described herein is a thermostable, single chain protein that folds into a one domain structure with one active center that possess a lipase-like Ser-His-Asp catalytic triad (De Simone, (2000) J. Mol. Biol, 303, 761-771.). The overall fold, typical for α/β hydrolases, shows a central eight-stranded mixed β-sheet surrounded by five helices with a helical cap on a top of the C-terminal end of the central β-sheet. The N and C-terminal ends of the protein are not involved in catalytic center of the enzyme and are exposed on the esterase surface (De Simone (2000) loc. cit.) providing a possibility for the protein to be fused with other polypeptides without altering the esterase native fold.
Esterase 2 from thermophilic bacteria *Alicyclobacillus acidocaldarius* can easily be produced up to 200 µg/ml by coupled in vitro transcription/translation system derived from *E. coli*, without any codon usage adjustment and keeping its activity. The activity of the produced esterase can be monitored directly in the translation mixture. Accordingly, this is an example how an esterase can successfully be employed for monitoring/tracking of biosynthesis. The examples provided herein in context of esterase 2 apply, mutatis mutandis, for other thermostable ersterases of prokaryotic origin. The photometric assay presented in figure 5B allows the detection of 10-12 moles of esterase/esterase activity in 100 µl assay volume. Use of micro plates allows to increase this detection limit by a factor of 10 to 100, reaching the sensitivity comparable with radioisotope labeling. The utilization of carboxyfluorescein diacetates as the esterase substrates allows fluorescent detection (Fig. 6C) that can be used for various applications in cell biology, biochemistry as well as pharmaceutical research. For example a fusion of the esterase with polypeptides allows the cellular localization by confocal microscopy. A remarkable feature of the esterase 2 from *Alicyclobacillus acidocaldarius* is its fast folding into a stable, active, single domain structure allowing refolding and detection of the esterase activity in polyacrylamide gels after SDS electrophoresis and removal of the SDS. The sensitivity of this activity detection is well-comparable with the sensitivity of the detection of 14C-labelled proteins by autoradiography (Fig. 7). Therefore, within the scope of the present invention is also the monitoring/tracking of protein biosynthesis with gel-technology, i.e. 2D-gels or even 3D-gels as well as gel-transfer technologies. However, as detailed in the appended examples, and described above, also a simple detection system on the gel per se is provided. Stability of esterase enzymes, like esterase 2 at wide temperature range (10-75°C) and activity over a broad pH (5-8) allows the use of heat or acidic precipitation as a simple and rapid purification step for successive isolation of the esterase fused proteins.

A preferred esterase to be used, in context of this invention is the esterase 2 described above and esterase/esterase activity which are homologous to said esterase. Accordingly, the esterase/esterase activity to be employed in the uses and methods provided herein may be selected form the group consisting of:
(a) an esterase encoded by a nucleotide sequence comprising a nucleotide sequence as shown in SEQ ID NO: 1;
(b) an esterase encoded by a nucleotide sequence coding for a polypeptide comprising an amino acid sequence as shown in SEQ ID NO: 2 or 62;
(c) an esterase encoded by a nucleotide sequence of a nucleic acid molecule that hybridizes to the complement strand of a nucleic acid molecule comprising an nucleotide sequence as defined in (a) or (b) and which catalyses the cleavage of an ester into an alcohol and an carboxylic acid;
(d) an esterase which comprises an amino acid sequence as shown in SEQ ID NO: 2 or 62;
(e) an esterase which comprises an amino acid sequence which is at least 60% identical to the full length amino acid sequence as shown in SEQ ID NOS: 2 or 62; and
(f) an esterase encoded by a nucleotide sequence which is degenerated to a nucleotide sequence as defined in any one of (a) to (c).
SEQ ID NO: 1 refers to the coding nucleotide sequence of esterase 2 from *Alicyclobacillus acidocaldarius* as described in Manco ((1998) loc. cit.). SEQ ID NOs: 2 or 62 refer to the amino acid sequence of the esterase 2 from *Alicyclobacillus acidocaldarius.* Within SEQ ID NO: 2, the internal methionine residues (Met (M)), encoded by their corresponding nucleotide residues of SEQ ID NO: 1, are indicated as X.
In context of the present invention, the term "nucleic acid(s)" and/or "nucleic acid molecule(s)" encompasses all forms of naturally occurring types of nucleic acid(s) and/or nucleic acid molecules as well chemically synthesized nucleic acids and also encompasses nucleic acid analogs and nucleic acid derivatives such as e. g. locked DNA, PNA, oligonucleotide thiophosphates and substituted ribo-oligonucleotides. Furthermore, the term "nucleic acid" and/or "nucleic acid molecules(s)" also refers to any molecule that comprises nucleotides or nucleotide analogs.
Preferably, the term "nucleic acid(s)" and/or "nucleic acid molecule(s)" refers to oligonucleotides or polynucleotides, including deoxyribonucleic acid (DNA) and ribonucleic acid (RNA). The "nucleic acids" and/or "nucleic acid molecule(s)" may be made by synthetic chemical methodology known to one of ordinary skill in the art, or by the use of recombinant technology, or may be isolated from natural sources, or by a combination thereof. The DNA and RNA may optionally comprise unnatural nucleotides and may be single or double stranded. "Nucleic acid(s)" and/or "nucleic acid molecule(s)" also refers to sense and anti-sense DNA and RNA, that is, a nucleotide sequence which is complementary to a specific sequence of nucleotides in DNA and/or RNA.
Furthermore, the term "nucleic acid(s)" and/or "nucleic acid molecule(s)" may refer to DNA or RNA or hybrids thereof or any modification thereof that is known in the state of the art (see, e.g., US 5525711, US 4711955, US 5792608 or EP 302175 for examples of modifications). Such nucleic acid molecule(s) are single- or doublestranded, linear or circular, natural or synthetic, and without any size limitation. For instance, the nucleic acid molecule(s) may be genomic DNA, cDNA, mRNA, antisense RNA, ribozyme or a DNA encoding such RNAs or chimeroplasts. Preferably, said nucleic acid molecule(s) is/are in the form of a plasmid or of viral DNA or RNA. Nucleic acid molecule(s) may also be oligonucleotide(s), wherein any of the state of the art modifications such as phosphothioates or peptide nucleic acids (PNA) are included.

In the context of the present invention the term "hybridizes" refers to hybridization under conventional hybridization conditions, preferably under stringent conditions, as for instance described in Sambrook and Russell (2001), Molecular Cloning: A Laboratory Manual, CSH Press, Cold Spring Harbor, NY, USA. In an especially preferred embodiment, the term "hybridizes" refers to hybridization that occurs under the following conditions: Hybridization buffer: 2 x SSC; 10 x Denhardt solution (Fikoll 400 + PEG + BSA; ratio 1: 1: 1); 0.1% SDS; 5 mM EDTA; 50 mM Na2HP04; 250, ug/ml of herring sperm DNA; 50 ug/ml of tRNA; or 0.25 M of sodium phosphate buffer, pH 7.2; 1 mM EDTA 7% SDS Hybridization temperature T = 60 °C Washing buffer: 2 x SSC; 0. 1 % SDS Washing temperature T = 60°C. It is disclosed and described herein that polynucleotides which hybridize to the complement strand of a nucleic acid molecule, comprising a nucleotide sequence as defined herein, can, in principle, encode a polypeptide having esterase activity from any organism expressing such polypeptides or can encode modified versions thereof. Polynucleotides which hybridize with the polynucleotides as defined in connection with the invention can for instance be isolated from genomic libraries or cDNA libraries of bacteria, fungi, plants or animals. Preferably, such polynucleotides are of procaryotic origin, particularly preferred from *Alicyclobacillus acidocaldarius*. Furthermore, the esterase contained in said cell-free translation system may also be at least 50%, more preferably at least 60%, more preferably at least 70%, more preferably at least 80%, even more preferably at least 90% and most preferably at least 95% identical to the full length amino acid sequences as shown in SEQ ID NO: 2 or 62.

The thermostable prokaryotic esterase (esterase activity) is normally, in context of this invention, used in form of one part of a fusionprotein, fusionpolypeptide or fusionpeptide, as detailed herein. Accordingly, in a most preferred embodiment of the invention, a use of a thermostable esterase of procaryotic origin in monitoring/tracking the synthesis of a desired protein/polypeptide/peptide is described, whereby said esterase is covalently attached/bound/fused to said protein/polypeptide/peptide. Said covalent attachment/binding/fusion may, on the protein level, be at the N- as well as at the C-terminus of the protein/polypeptide/peptide the expression/synthesis or which is to be monitored. Accordingly, and as further described below and illustrated in the appended examples, the use of the esterase as described herein is in particular envisaged in the provision of nucleic acid molecules (e.g. DNA, RNA, vectors and the like) wherein nucleic acid molecule is provided which comprises a coding sequence for an esterase (or an esterase activity) or a functional fragment thereof and a (further) coding sequence for the protein/polypeptide/peptide who's expression/synthesis in the in vitro system is to be monitored/tracked in accordance with this invention. Said nucleic acid molecule, coding for a thermostable procaryotic esterase (esterase activity) and the desired protein/polypeptide/peptide (also denoted herein as "X" or "X'") is than expressed in the cell-free system as described herein. Accordingly, the coding sequence of X/X' is in frame with the coding sequence of said esterase/esterase activity or said functional fragment of said esterase/esterase activity. The nucleic acid sequence, therefore, codes for an esterase-X/X-esterase fusion construct as detailed herein. Further embodiments on corresponding nucleic acid molecules as well as vectors are provided herein below.

The term "monitoring and/or tracking the synthesis of a protein, polypeptide or peptide" means that, inter alia, changes of the amount of a protein, polypeptide or peptide, in particular the increase of the amount of protein, polypeptide or peptide can be detected and quantitatively and/or qualitatively determined, during, before and/or after the synthesis of said protein, polypeptide or peptide. The corresponding detection of changes is carried out by measuring the esterase/esterase activity as described herein. Further, the term "monitoring and/or tracking the synthesis of a protein, polypeptide or peptide" means that the efficacy of the synthesis of a protein, polypeptide or peptide can be determined. The term also means that the synthesis rate of the protein, polypeptide or peptide can be determined, in particular in the absence or presence of inhibitors or enhancers of protein biosynthesis. Accordingly, with the systems and methods provided herein, also inhibitors or activators of translation systems may be determined. The term "monitoring and/or tracking the synthesis of a protein, polypeptide or peptide" also relates to the determination of the functionality of the protein, polypeptide or peptide can be determined during, before and/or after its synthesis. "Functionality" refers to the ability of the protein, polypeptide or peptide to exert its function and/or activity. In particular, the term "determining the functionality" of a protein, polypeptide or peptide means that it is determined (quantitatively or qualitatively) to what extent, the protein, polypeptide or peptide exerts its function and/or activity. The term "function" also relates to a physiological function within an organism. The term "monitoring and/or tracking the synthesis of a protein, polypeptide or peptide" also means that a protein, polypeptide or peptide can be localized during, before and/or after the synthesis of said protein, polypeptide or peptide. "Localization" of a protein, polypeptide or peptide means that a particular place, where a certain amount of said protein, polypeptide or peptide exists, is identified. This particular place may be, but is not limited to, in form of a vial, a gel, a blot, a column, a membrane, a slide (e.g. out of glass, polystyrene etc.) a liquid, a droplet, a cell, a tissue, beads and the like. However, said "localization", as described above may also comprise the localization of a protein in a cellular context, for example whereby said esterase is detected within the context of a synthesized esterase-fusion protein/-fusion construct in a cell. The term "localization" in this context also comprises, inter alia, the detection of the presence of the esterase moiety of the fusion construct described herein. Corresponding examples relate, inter alia, to the transfection of a cell with a vector described herein and the detection of a synthesized esterase-fusionprotein/-fusion construct, for example by microscopical means.
All the above recited "monitoring/tracking steps" are, in accordance with the present invention, based on the detection of a specific activity of a thermostable procaryotic esterase, i.e. the thermostable prokaryotic esterase activity, more preferably an Est2 activity. Corresponding embodiments are clearly evident from this specification as well as from the appended examples.
Yet, it is envisaged that not only the function of thermostable prokaryotic esterase or esterase activity, in particular in context of the herein described fusion constructs "X-esterase" or "esterase-X" (and the like), be detected in order to carry out the monitoring step and/or tracking step provided herein. Said monitoring and/or tracking step may also comprise the detection of the presence of said esterase, e.g. by immunological means, like microscopical techniques or immunolocalisation methods, like, inter alia, Western blots. Furthermore, the detection via radioactive labels (and the like) of the presence of the esterase moiety in the or on the fusion constructs (comprising at least one of the proteins, polypeptides or peptides desired to be synthesized (or synthesized) in the herein described cell-free systems and the moiety comprising the esterase and/or esterase activity) is envisaged. Examples for the detection of the function and/or the presence of thermostable prokaryotic esterase or esterase activity are provided herein and in the appended examples.

The use of a thermostable esterase of procaryotic origin as described herein, has several advantages compared to the use of the reporters in the prior art:
First, thermostable esterases of procaryotic origin, in particular the esterase 2 from *Alicyclobacillus acidocaldarius*, are stable and active over a wide range of temperature (10-75°C) and pH values (pH 5-8).

It was surprisingly found and exemplified herein that especially the detection of products of thermostable procaryotic esterases-catalysed reactions are very sensitive. The examples show that even at concentrations of, for example, ∼10⁻⁸ M, a detection of this product is still possible (example 6, Fig. 6). Therefore, the use of thermostable esterases of procaryotic origin, in particular the esterase 2 from *Alicyclobacillus acidocaldarius* provides for a highly sensitive monitoring and/or tracking of protein synthesis, in particular in cell-free translation systems. For example, this detection might be performed by photometric, electrochemical or fluorescent methods, as exemplified herein. In an example for photometric detection, the esterase-catalysed hydrolysis of p-nitrophenyl acetate to the corresponding alcohol is performed (see, inter alia, Fig. 5B, Fig. 6B, example 6). In an example for electrochemical detection, the esterase-catalysed hydrolysis of p-aminophenyl acetate to the corresponding alcohol is performed (see, inter alia, Fig. 5A). In an example for fluorescent the esterase hydrolyses 5-(and-6)-carboxy-2',7'-dichlorofluorescein diacetate which leads to the appearance of a fluorescent product (Fig. 6C, example 6).
Furthermore it is exemplified herein that the detection of a thermostable esterase of procaryotic origin might also be possible in gels after sodium dodecyl sulfate polyacrylamide gel electrophoresis (PAGE; see, inter alia, Fig. 7C, example 6). Furthermore, the use of thermostable esterases of procaryotic origin, in particular of the esterase 2 from *Alicyclobacillus acidocaldarius* (-34.4 kD), benefits from the fact, that such esterases have normally low molecular masses and a fast folding, globular, single domain structure having a very high enzymatic activity, in particular in cell-free translation systems as shown herein.
In addition, a maturation of such esterases, in particular of the esterase 2 from *Alicyclobacillus acidocaldarius*, is not required. This is in stark contrast to other reporter proteins, like, inter alia, GFP.

In summary, it is demonstrated herein and in particular in the appended examples that the presence of an esterase at the N- or C-terminus of a protein, polypeptide or peptide allows the use of said esterase for concomitant purification and detection of proteins, polypeptides or peptides, especially in the corresponding generation of these proteins, polypeptides or peptides in *in vitro* translation systems. As demonstrated herein, the esterase can be fused with a target protein, polypeptide or peptide resulting in a product that possesses activities and/or features of esterase as well as the additional, conjugated proteins, polypeptides or peptides. The additional activity may be, but is not limited to, an enzymatic, hormonal, signal activity and the like. However, said additional activity and/or feature may also comprise the marker and/or structural function of the (additional) conjugated protein, polypeptide or peptide. Therefore the corresponding (additional) function of the herein described fusion construct/fusion protein correspondes to the function or activity of the gene product (or a fragment thereof) as encoded by the target gene to be expressed. The presence of the esterase on the N- or C-terminus of the target protein, polypeptide or peptide permits quantitative determination of expression levels by esterase activity measurement, since esterase translation is possible after completion of a target gene. This could be used for a rapid optimization of *in vitro* or *in vivo* expression conditions.

The thermostable esterases of prokaryotic origin to be employed in context of this invention, in particular Est2, can be in vitro synthesized by (coupled transcription/)translation using a corresponding plasmid. An adjustment of the codon usage is not always required for in vivo as well as in vitro synthesis. Accordingly, the esterases, in particular Est2, as described herein, can be employed preferably in a prokaryotic system, more preferably in a cell free prokaryotic system and most preferably in a cell free system from E. coli. However, also the use of thermostable esterases of prokaryotic origin, in accordance with this invention, in *in vivo* expression systems, like E. coli systems, is envisaged.
As pointed out above, the esterase part can also be immobilized, inter alia, on solid surfaces by linking the esterase with affinity tags such as oligonucleotides, biotin, chemically and photochemicaly reactive groups and/or chemical structures that do not occur in natural polypeptides, for example, using puromycin-termination technology (Nemoto (1999), FEBS Lett. 462, 43-6; see, inter alia, example 12, Fig. 11).

Moreover, the use of thermostable esterases of prokaryotic origin as provided herein also provides for a reporter group for screening protein biosynthesis-inhibitory activity in combinatorial libraries and physiological extracts. Again, details are also provided in the examples and, inter alia, Figure 10. It is obviously evident for a person skilled in the art that not only the distinct disclosed esterases, like the Est2, can be used in context of the invention, but also other thermostable esterases of prokaryotic origin; in particular members of the class of carboxylesterases. Known carboxylesterases are and comprise i.e. the carboxylesterases as disclosed in Hemilä ((1994), loc. cit.

As pointed out above, in context of the disclosure of this invention the esterase/esterase activity is preferably used in the monitoring/tracking of protein-, polypeptide-, or peptide synthesis in vitro, in particular in cell-free systems for translation. However, as detailed above, the inventive use of esterase/esterase activity is also envisaged in the context of cellular expression. Said cellular expression may take place in eukaryotic as well as prokaryotic host cells transfected with recombinant constructs capable of expressing fusion constructs/fusion proteins ("X-esterase"/"esterase-X") as defined and exemplified herein.

Cell-free translation systems are well known in the art and the uses and methods provided herein can readily be employed in such systems. Known "in vitro" translation systems comprise, but are not limited to "in vitro" translation systems from prokaryotic cells such as *E. coli* cells (e.g. Zubay (1973), Imm. Rev. Genet. Vol. 7, page 267) and from eukaryotic cells such as rabbit reticulocytes (e.g. Pelham (1976), Eur. J. Biochem. Vol. 131, page 289) and wheat germ cells (e.g. Spirin (1990), American Society for Microbiology, 56-70; Endo (1992), J. Biotechnol., 25, 221-230; Stiege (1995), J. Biotechnol. 41:81-90). More details on preferred "cell-free translation systems are provided herein below and in the appended examples. Preferred in vitro translation systems in context of the inventive uses and methods provided herein are systems in which transcripton and translation can occur, i.e. cell-free coupled transcription/translation systems.

Yet, such systems may be of prokaryotic or eukaryotic origin or may even be a mixture of cell-free translation systems. In context of this invention the esterase/esterase activity is preferably used in monitoring/tracking of protein expression in prokaryotic systems. As shown in the appended examples, the system and methods provided herein work particularly well in cell-free translation systems of *E. coli* origin. However, also translation systems like wheat germ extract cell-free translation systems or rabbit reticulocyte lysates may readily be employed in context of this invention.

The cell-free translation system, to be employed within the present invention may comprise:
- a cell-free extract;
- ribonucleotide triphosphates, like ATP, CTP, GTP, UTP, etc.;
- a RNA polymerase;
- magnesium ions; and/or
- a template plasmid.
The cell-free system also may comprise additional amino acids, for example labelled amino acids or unnatural amino acids. Also comprised may be (additional) tRNA, like suppressor seryl-tRNA^{SER(CUA)}. Also, as shown below, (additional) leucine may be comprised, preferably labelled leucine. Preferably, the magnesium ions comprised in the cell-free translation system to be employed in context of this invention are at a concentration at which RNA is transcribed from DNA and RNA translates into protein. More preferably, the magnesium ions are in form of MgCl₂, e.g. at a concentration of 9-12 mM.

Preferably, the cell-free coupled transcription/translation systems, as employed in context of this invention, may comprise the ingredients as listed below:
- 30S cell-free extract from E. coli (enzyme- and und ribosomal fraction);
- MgCl₂ 9-12 mM;
- DTT 10 mM;
- Amino acids, 200 µM each (For labelling, each amino acid can be applied as a 14C amino acid with a concentration of 100 µM (e.g. 14C-leucine))
- Rifampicin 0,02 mg/ml reaction mixture,
- Bulk-tRNA 600µg/ml reaction mixture,
- ATP,CTP,GTP,UTP, 1mM each,
- Phosphoenolpyruvate 10 mM;
- Acetylphosphate 10 mM;
- Pyruvatekinase 8 µg/ml reaction mixture;
- Plasmid 2 pmol/ml reaction mixture;
- T7 Polymerase 500 Units/ml reaction mixture;
- HEPES pH 7,6, 50 mM;
- Potassium acetate70 mM;
- Ammonium chloride 30 mM;
- EDTA pH 8,0 , 0,1 mM;
- Sodium azide 0,02 %;
- Polyethyleneglycol 4000 2 %;
- Protease inhibitors: aprotinin 10 µg/ml reaction mixture, leupeptin 5 µg/ml reaction mixture, pepstatin 5 µg/ml reaction mixture; and
- Folic acid 50 µg/ml reaction mixture.

The above recited cell-free coupled transcription/translation system is merely an illustrative example of a cell-free system to be employed in context of this invention. Corresponding examples are also given in the experimental part.

Generally, the composition of cell-free translation systems, in particular cell-free coupled transcription/translation systems is well known in the art. Said systems are also commercially available, e. g..from Promega GmbH. Most preferably, and also shown in the experimental part, said cell-free coupled transcription/translation systems may be comprised in evaluation size transcription/translation kits purchased from RiNA GmbH (Berlin, Germany).

The cell-free translation systems to be employed in context of the present invention may (further) comprise a labelled amino acid. By incorporation of said labelled amino acid, it is possible to monitor and/or track the synthesis of a protein or to identify the location (e.g. in a polyacrylamide gel) of said protein. Preferably, the labelled amino acid is a radioactively labelled amino acid, more preferably the labelled amino acid is [¹⁴C]leucine, [¹⁴C]valine and/or [¹⁴C]isoleucine, most preferably the labelled amino acid is [¹⁴C]leucine.

The cell-free translation system as employed in the present invention, may also be of eukaryotic origin. In this case, a wheat germ extract cell-free translation system or a rabbit reticulocyte lysate cell-free translation system would be preferred, but a cell-free translation system based on lysates from oocytes or eggs (e.g. oocytes from Xenopus) may be also applicable. These eukaryotic systems may preferably be used for the expression of eukaryotic genes or mRNA and are also well known in the art.

Another embodiment of the cell-free translation system as employed in the present invention refers to a cell-free translation system that comprises a nonsense codon suppressing agent. Also comprised may be an inhibitor of release factors, like an anti-release factor antibody which precipitates and/or crosslinks a release factor in said cell-free translation system. Other inhibitors of release factors are known in the art and comprise, inter alia, aptamers directed against release factors; Szkaradkiewicz (2002) FEBS Itrs 514, 90-95. Also thermo-sensitive release factors have been employed in this context and are also envisaged in context of this invention; see, inter alia, Short (1999) Biochem. 38, 8808-8819.

The term "nonsense-codon suppressing agent" as used herein relates to an agent that is capable to bind to the A-site of a ribosome programmed by a stop codon. Said stop codons are known in the art and may be UAA, UAG or UGA, preferably, UAG. The nonsense-codon suppressing agent itself may be covalently bound to the elongating peptide-chain or may be delivering a substance that is bound to the elongating peptide chain. Said nonsense-codon suppressing agent may prevent normal termination accomplished by release factors or termination factors or said nonsense-codon suppressing agents may replace normal termination accomplished by release factors or termination factors. Preferably, said nonsense-codon suppressing agent that delivers a substance to be bound to the elongating peptide-chain prevents normal termination. Said nonsense-codon suppressing agent, being itself covalently bound to the elongating peptide-chain, replaces normal termination.

The nonsense-codon suppressing agent, delivering the substance to be bound to the polypeptide-chain, may be a aminoacyl-tRNA, preferably a suppressor aminoacyl-tRNA, more preferably a suppressor aminoacyl-tRNA^{(CUA)}. The nonsense-codon suppressing agent to be covalently bound to the polypeptide chain may be, inter alia and preferably, puromycine or a derivative thereof as defined herein below.

As discussed above, the cell-free system may also comprise, if desired, a component which is capable of inhibiting and/or negatively interfering with a release factor comprised in said cell-free translation system. Such a cell-free translation system is particularly useful when alloproteins (as detailed below) are desired to be synthesized in said cell-free system

The term "anti-RF antibody", in particular "anti-RF1 antibody" as employed herein refers to an antibody, a plurality of antibodies and/or a serum comprising such antibodies which is/are able to specifically bind to, interact with and/or detect RFs, preferably RF1, more preferably RF1 from *E. coli* or a fragment thereof. In context of the present invention, said "anti-RF antibody" must be capable of precipitating (in the in vitro system) the RF and/or must be capable of crosslinking said RF. The "precipitation" and/or crosslinking" leads to an inactivation of the RF, inter alia, due to the formation of larger RF- antibody complexes. The term "precipitates and/or crosslinks", accordingly, refers to the capability of an anti-release factor antibody to bind and to inactivate a release factor. Therefore, said binding leads to an inactivation of said release factors which is equivalent of a depletion of said release factor (from cell-free translation systems). The term "inactivation" refers to making said release factors incapable to bind to the A-site of the ribosome and thereby incapable to cause termination of the peptide-chain and its release from the ribosomal complex. The precipitating and/or deactivating activity of anti-RF polyclonal antibodies can be measured by the residual RF activity in the in vitro translation system, by testing the hydrolysis of a peptide from peptidyl-tRNA located in the P-site (Freistroffer (2000), Proc Natl Acad Sci U S A. 97, 2046-51). or by a gel electrophoresis followed by Western blotting, which is being a common laboratory praxis.

Corresponding antibodies directed against an release factor may easily be prepared as demonstrated in the appended examples and as known in the art. Said antibodies and/or sera may, inter alia, be prepared by immunization of a non-human vertebrate with purified and/or recombinantly produced "release factors". In the appended examples, it is documented how, for example a polyclonal serum against release factor 1 (RF1) of *Thermus thermophilus* (*T. th.;* SEQ ID NO: 4) can be prepared. In the corresponding example, a heterologuesly expressed, recombinantly produced RF1 was used in the immunization protocol. The preparation of antibodies, either monoclonal or polyclonal, is well known in the art; see, inter alia Harlow/Lane ("Antibodies: A laboratory manual" (1988), CSHL, New York).
The person skilled in the art readily in the position to deduce whether an antibody and/or antibody molecule or a serum directed against a given release factor is capable of precipitating and/or crosslinking said release factor.

The term "anti-RF antibody" also relates to a serum, in particular a purified serum, i.e. a purified polyclonal serum. The antibody molecule is preferably a full immunoglobulin, like an IgG, IgA, IgM, IgD, IgE, IgY (for example in yolk derived antibodies). The term "antibody" as used in this context of this invention also relates to a mixture of individual immunoglobulins. Furthermore, it is envisaged that the antibody/antibody molecule is a fragment of an antibody, like an F(ab), F(abc), Fv Fab' or F(ab)₂. Furthermore, the term "antibody" as employed in the invention also relates to derivatives of the antibodies which display the same specificity as the described antibodies. Such derivatives may, inter alia, comprise chimeric antibodies or single-chain constructs. Yet, most preferably, and as shown in the examples, said "anti-RF antibody" relates to a serum. Also a purified (polyclonal) serum and, preferably, to a non-purified crude polyclonal serum. The antibody/serum is obtainable, and preferably obtained, by the method described herein and illustrated in the appended examples or by other methods known in the art.

As exemplified in the experimental part, said anti-RF antibody, in particular said anti-RF1 antibody, may specifically deplete one particular RF (e.g. RF1 (e.g. having the amino acid sequence of SEQ ID NO: 6)) keeping (an-)other RF(s) (e.g. RF2 (e.g. having the amino acid sequence of SEQ ID NO: 44)) active. In this case, a nonsense-codon suppressing agent (e.g. suppressor tRNA) can bind to the corresponding STOP-codon (e.g. UAG) of the first RF (e.g. RF1) and the second RF (e.g. RF2) is still capable to accomplish normal termination at the corresponding second STOP-codon (e.g. UGA). Said first STOP-codon may be an artificial STOP-codon lying inside of the open reading frame of a mRNA to be translated. Said second STOP-codon may lie at the end of said open reading frame.

The term "release factor" as used herein relates to any factor(s) that is/are capable to bind to the A-site of a ribosome programmed by a stop codon, whereby the stop codon is defined as mentioned herein above. By binding to said A-site, said release factor causes termination of the elongation of a peptide-chain during translation process, and thereby leads to a release of the nascent peptide-chain from the ribosomal complex. Preferably, the term "release factor" refers to release factors that are contained in cell-free translation systems. In context of the present invention, the term "release factor" also relates to a fragment of a release factor as defined herein. The term "fragment" (of a release factor) as used herein relates to fragments of a length of at least 30, at least 40, at least 50, more preferably at least 60, ever more preferably at least 65 amino acid residues of a (native) RF as defined herein. The amino acid sequence of RFs are known in the art and also specified herein below. Preferably, said fragment comprises at least such stretch of amino acids that (polyclonal) antibodies may be raised against this fragments and that these obtained antibodies are capable to precipitate and/or crosslink a release factor in a cell-free translation system.

The proteins, polypeptides or peptide to be synthesized in the cell-free translation system as employed herein or in the *in vivo* expression system as defined herein, may, inter alia, be selected from the group consisting of enzymes, hormones, lectins, metabolic proteins, pheromones, proteins of signal transduction pathways, signal proteins, transporter molecules, proteins involved in translation and /or transcription processes, structural proteins, antibodies, antibody fragments, antibody parts, single-chain antibodies (scFvs), diabodies, markers (marker proteins), reporters (reporter proteins) and the like. Also envisaged are proteinaceous compounds, like toxins, e.g. ricin and the like. Also growth factors and cytokines are envisaged to be expressed, monitored and tracked by the methods provided herein. Also fragments of these proteins may be expressed and "monitored and/or tracked" by the uses and methods provided herein. In context of further embodiments of the invention, in particular the herein disclosed methods for immobilization of proteins/polypeptides/peptides and their corresponding (potential) recovery, other examples of proteins/polypeptides/peptides are provided are provided which may also be monitored/tracked by the method/uses provided herein. The person skilled in the art will readily understand that the uses and methods of the present invention can be widely employed and that the proteins/polypeptides/peptides to be synthesized in the cell-free system or in vivo expression system may be of or may be derived from any organism or may be of complete synthetic or recombinant origin. Accordingly, the present invention is not limited to a specific "X"/"X'" in the fusion construct/fusion protein as defined herein ("X-esterase"/"esterase-X"). Also synthetic and/or non-naturally occurring proteinaceous structures may be expressed, monitored and/or tracked by the means and methods provided herein.

Said protein, polypeptide/polypeptides/peptides to be synthesized is (during its synthesis) covalently bound to an esterase as defined in context of this invention. The construct of the covalently bound protein, polypeptide/polypeptides/peptides and the esterase may be in the form of a fusionprotein, fusionpolypeptide or fusionpeptide. The embodiments described below for the fusion constructs encoded by the inventive vectors apply for this fusionprotein, fusionpolypeptide or fusionpeptide, mutatis mutandis.
Besides the above recited naturally occurring, yet recombinantly produced proteins to be synthesized in the cell-free system or *in vivo* expression systems in combination with esterase/esterase acitivity, it is also envisaged that the preset uses and methods be employed in the production of alloproteins. Also the synthesis of such alloproteins may be monitored and/or tracked by the methods provided herein.

In context of the present invention, the term "alloproteins" refers to proteins that are achieved by applying the subject-matter of the present invention. Said term also refers to proteins having covalently bound a non-proteinaceous molecule which usually is not part of (the )naturally occurring protein(s). Said alloprotein may, for example, comprise a puromycin and/or derivative thereof as defined herein. Furthermore, said proteinaceous molecule may comprise an unnatural amino acid, e.g. as described in Gilmore (1999), Topics in Current Chemistry, 202, 77-99. Furthermore, said molecule being covalently bound to and comprised in the alloprotein, might be a functional substituent. Various functional substituents of proteins are well-known in the art. For instance, these functional substituents may be oligosaccharides, lipids, fatty acids, phosphates, acetates or other functional groups naturally occurring to modify polypeptide chains of functional proteins. (Eisele (1999), Bioorganic and Medicinal Chemistry 7,193-224). Furthermore, said molecule might be a residue of a puromycin (-derivative) as defined herein and/or a puromycin (derivative) as defined herein itself and/or the puromycin derivative of the present invention itself.

The alloproteins produced by the method of the present invention, may be used in a wide variety of applications, for example the preparation of synthetic enzymes (Corey (1987), Science, 238, 1401-1403), gene therapy (Zanta (1999), Proc. Natl. Acad. Sci. U.S.A., 96, 91-96), construction of protein microarray (Niemeyer (1994), Nucleic Acid Res., 22, 5530-5539), creation of molecular scale devices (Keren (2002), Science, 297, 72-75), and development of immunological assays (Niemeyer (2003), Nucleic Acids Res., 31, e90).

The method for the production of alloproteins, as described herein, offers the possibility that any desired chemical structure including different dyes, affinity tags, spin labels etc. may be covalently conjugated with proteins at a high yield. This opens the way for variety of applications. For example, puromycin modified with an azide group may be used to covalently attach to the C-terminus of proteins for subsequent one site addressed Staudinger reaction (Kohn (2004), Angew. Chem. Int. Ed Engl., 43, 3106-3116) and utilization of puromycin carrying α-thio-ester group may allow to use the protein ligation technology (Lovrinovic (2003), Chem. Commun. (Camb.), 822-823). This conjugation technology can further be used to develop concepts for preparation of protein arrays and novel tools to study protein interactions (Ramachandran (2004), Science, 305, 86-90).

The alloproteins described herein may as proteinaceous part comprise proteins. These proteins may, inter alia, be selected from the group consisting of enzymes, hormones, pheromones, structural proteins and the like. It is also envisaged that said alloproteins only comprise fragments, like functional, active fragments of said enzymes, hormones, pheromones, structural proteins and the like. Also proteinaceous toxins are envisaged. The person skilled in the art is readily in the position to understand that the embodiments provided herein are easily transferable to other proteins, polypeptides or peptides. The person skilled in the art can, e.g. replace the "GFP", "eGFP", "esterase-GFP", or "esterase-eGFP", as employed as "detectable marker" in the appended examples by any desired protein, polypeptide or peptide, without deferring from the gist of the present invention. Said proteins may act as core-proteins and/or starting proteins for the alloproteins to be produced in the cell-free translation system or *in vivo* expression system as employed herein and corresponding additional chemical structures may be added to said proteinaceous part. Accordingly, for example a hormone may be produced which comprises at least, e.g. one additional unnatural amino acid or (e.g.) a puromycin-derivative as defined herein.

Said alloproteins may also be conjugates of proteins and nucleic acids having specific sequences. Said conjugates allow to link the properties of these two distinct groups of biopolymers within one molecule. Therefore, said conjugates can be used in a wide variety of applications, where said linkage of said properties of these two distinct groups of biopolymers is advantageous. These applications are well known in the art and may, for instance, include the preparation of synthetic enzymes (Corey (1987), Science, 238, 1401-1403), gene therapy (Zanta (1999), Proc. Natl. Acad. Sci. U.S.A., 96, 91-96), construction of protein microarray (Niemeyer (1994), Nucleic Acid Res., 22, 5530-5539), creation of molecular scale devices (Keren (2002), Science, 297, 72-75), and development of immunological assays (Niemeyer (2003), Nucleic Acids Res., 31, e90).

As an example also provided in the experimental part of this invention, the alloproteins produced by the method of the present invention, e.g. by using the cell-free translation system or the in vivo systems disclosed herein, comprises the above described esterases/esterase activity as marker or tracking molecule.

The alloprotein may comprise a covalently-bound puromycine or a derivative thereof and/or wherein said protein, polypeptide or peptide to be synthesized comprises an amino acid, delivered by suppressor aminoacyl-tRNA. Preferred, said suppressor aminoacyl-tRNA may be suppressor aminoacyl-tRNA^{Ser(CUA)}.

Preferably, the alloprotein comprises a C-terminal, covalently-bound puromycine or a derivative thereof.

The puromycin derivative which are employed within the present invention may be particularly useful in the use of an esterase, the vectors, the methods and the kit. For example, the puromycin derivative of the present invention may be useful in mRNA display. The yields of the mRNA-protein coupling in mRNA display (Roberts, (1997) JW Proc Natl. Acad. Sci. U.S.A. 94, 122297-302) are usually low. The reason is the low tolerance of the ribosomal A-site for 5'-extended puromycin-nucleic acid conjugates and the high selectivity of this site for EF-Tu.GTP dependent delivery of the aminoacyl-tRNA (Starck (2002), RNA, 8 890-903) RNA molecules that are longer than 5-6 nucleoitde residues can not enter the ribosomal A-site in EF-TuGTP independent manner. There is, however, a possibility to circumvent this problem by using the puromycin derivative of the present invention. By the provision of said puromycin derivative, instead of covalent attachment of RNA to the 5'-position of puromycin an alternative strategy by which the RNA (mRNA) or other functional groups are attached directly or via a linker to the nculeobases of puromycine-derived olignucleotides (e.g. CpCpPu or CpPu) can be used. Example for this type of conjugation is provided in the experimental part of this invention

The potential residues of said puromycin derivative have been described above and same applies here for the advantageous puromycin attachment sites. Likewise, the linkers between the puromycin (-derivative) and the residues which may, inter alia be employed have been described above in context of other embodiments.

In the puromycin derivative, the covalently attached residue may be selected from the group consisting of a Cy3-fluorophore, biotin or another affinity tag, a reactive group for affinity labelling or any other reporter group. Further, the residue may be a(n) (other) spectroscopic reporter.
It is evident for the skilled artesian that other residues may be employed in context of this invention.

In a further preferred embodiment of the herein provided use of a puromycin derivative, the linker is an aliphatic amine, in particular an aliphatic amine forming an amide with a fatty acid.

Another preferred cell-free translation system to be employed in the context of this invention is a cell-free translation system, wherein said nonsense-codon suppressing agent is puromycin or a derivative thereof and/or a suppressor tRNA. Said suppressor tRNA may be, e_{.}g. suppressor tRNA^{Ser} (CUA).

The nonsense-codon suppressing agent may also be e.g. selected from the group consisting of:
(a) Puromycin;
(b) 5'-OH-CpPuromycin;
(c) 5'-OH-CpCpPuromycin;
(d) a puromycin derivative as defined in (a) to (c) having a residue covalently attached directly or via a linker to its 5'-position;
(e) a puromycin derivative as defined in (a) to (d) having a residue covalently attached directly or via a linker to the element N⁴ of the cytosine-residue of an 5' attached cytidine-residue; and
(f) a puromycin derivative as defined in (a) to (e) having a residue covalently attached directly or via a linker to the element C⁵ of the cytosine-residue of an 5' attached cytidine-residue;
whereby a nonsense-codon suppressing agent as defined in (e) is preferred.

For example, the residue to be covalently attached to the puromycin (or a derivate thereof), may be selected from the group consisting of nucleic acids like DNA, RNA, locked DNA, PNA, oligonucleotide-thiophosphates and substituted ribooligonucleotides and other nucleic acids. It is also envisaged that other residues, like peptides or "tags" can be attached to said puromycin to be integrated in a protein during its in vitro synthesis.

In further examples, the residue, covalently attached to said puromycin or said derivative thereof, may be selected from the group consisting of a Cy3-fluorosphore, biotin or an other affinity tag, a reactive group for affinity labelling or any other reporter group (for review see Gilmore (1999), Topics in Current Chemistry, 202, 77-99). The reactive group, for instance, can be an a-zide group to use for subsequent one site addressed Staudinger reaction (Kohn (2004), Angew. Chem. Int. Ed Engl., 43, 3106-3116) or an α-thio-ester group to use for the protein ligation technology (Lovrinovic (2003), Chem. Commun. (Camb. ), 822-823). In further examples, the residue, covalently attached to said puromycin or said derivative thereof, may be also be a(n) (other) spectroscopic reporter.

In the context of the present invention, the term "linker" refers to a molecule capable to connect said puromycin (-derivative) and said residue covalently.
For example, the linker between the puromycin (-derivative) and said residue may be an aliphatic amine derivative, preferably forming an amide with a fatty acid attached to a polyoxyamine. Preferably, said linker may comprise the following molecule: 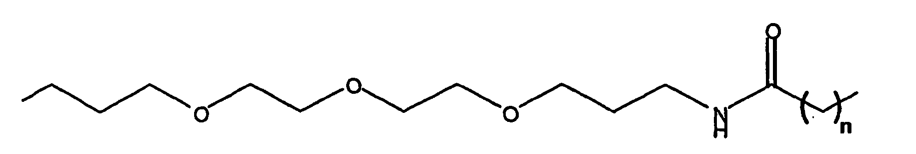 or 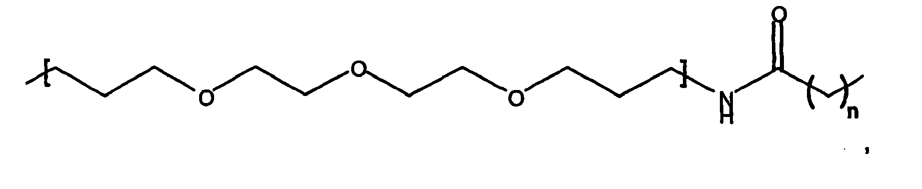 wherein the part of the molecule indicated in squared brackets may be of different length, e.g. may be elongated by additional or shortened by less carbon residues and/or oxygen residues.
Preferably, in said linker, (n) is at least 3, preferably at least 5 carbon residues. Yet, the amount of carbon residues (n) may most preferably be 5 or 9.
Further, the linker between the puromycin (-derivative) and said residue may act as a "place holder" that warrants the undisturbed entrance of the puromycin (-derivative) into the A-site of the ribosome.

"Nonsense-codon suppressing agent" are known in the art, as documented above. However, the "nonsense-codon suppressing agent" comprised in a cell-free translation system of the present invention or as described herein may also be selected from the group consisting of:
(a) 5'-OH-GpCpPuromycin;
(b) 5'-OH-GpCpCpPuromycin;
(c) 5'-OH-GpApCpCpPuromycin;
(d) 5'-OH-GpCpApCpCpPuromycin;
(e) 5'-OH-GpCpCpApCpCpPuromycin; 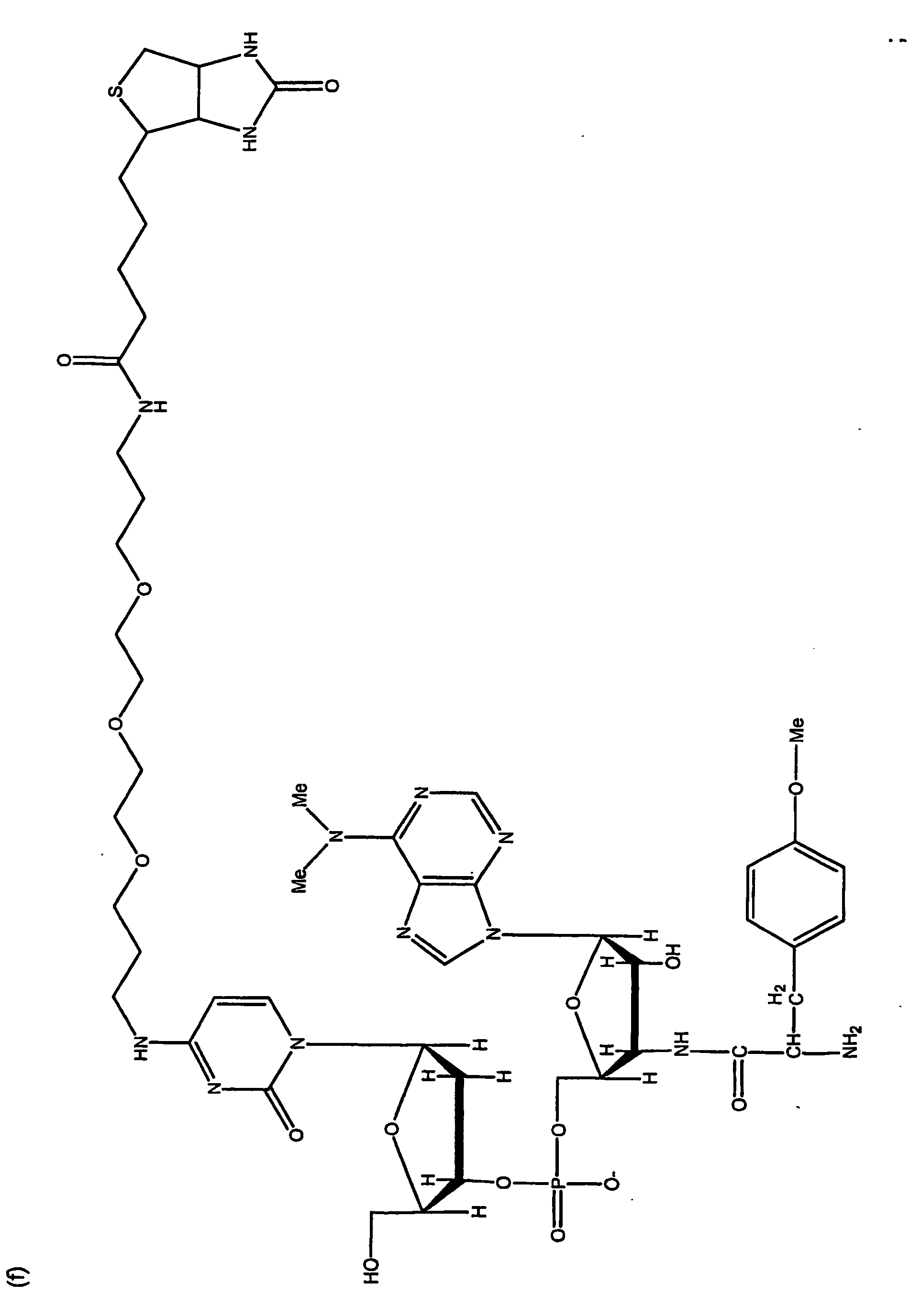 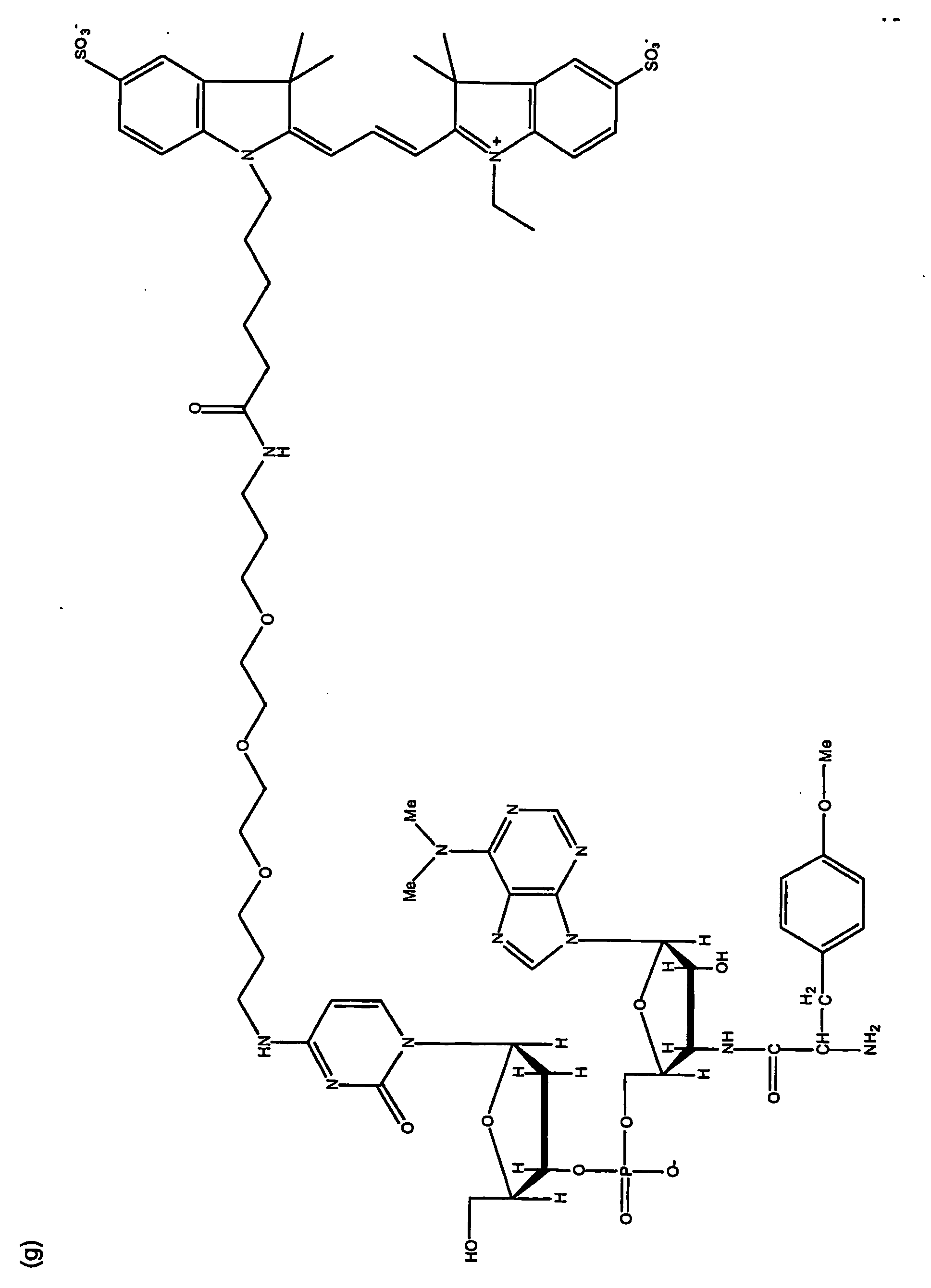 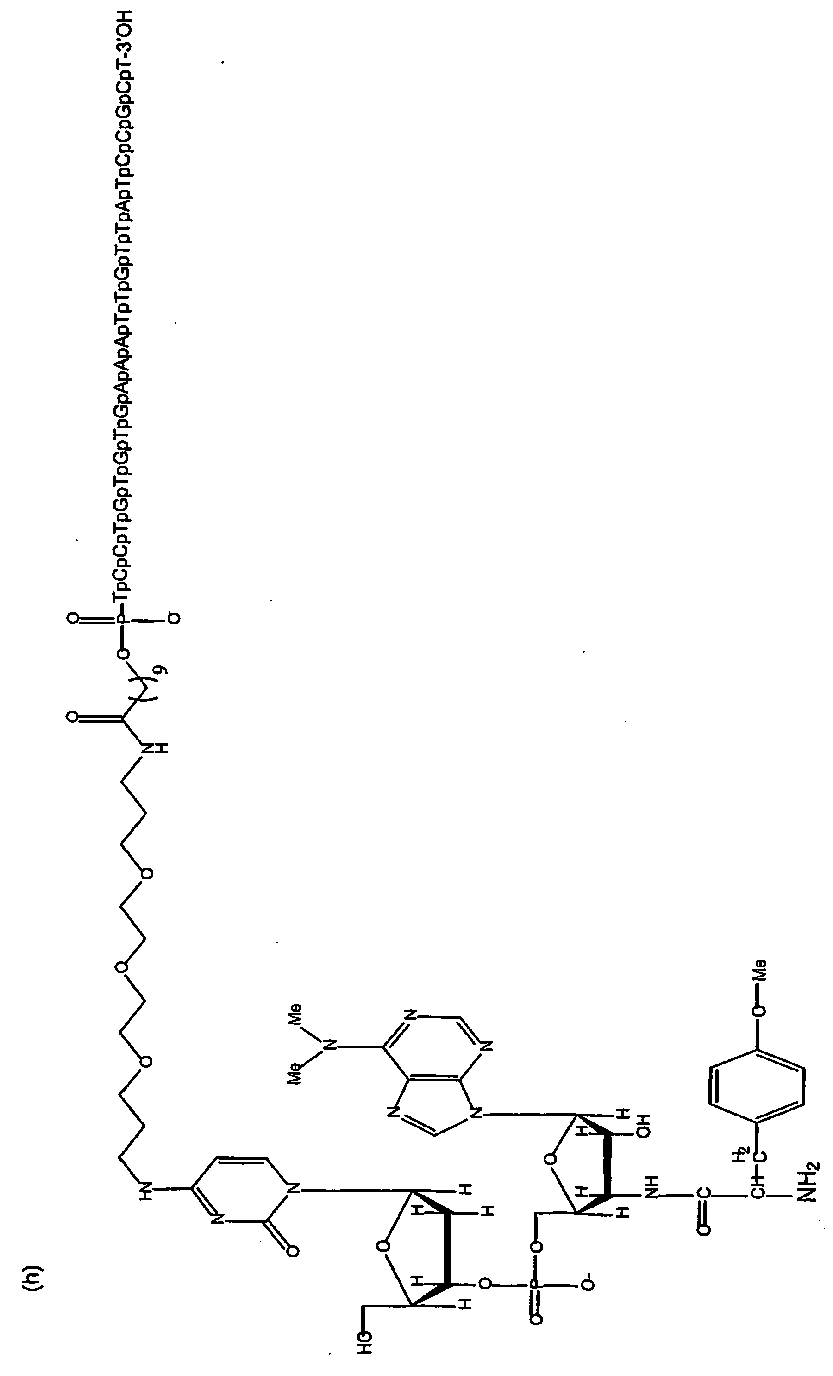
; and

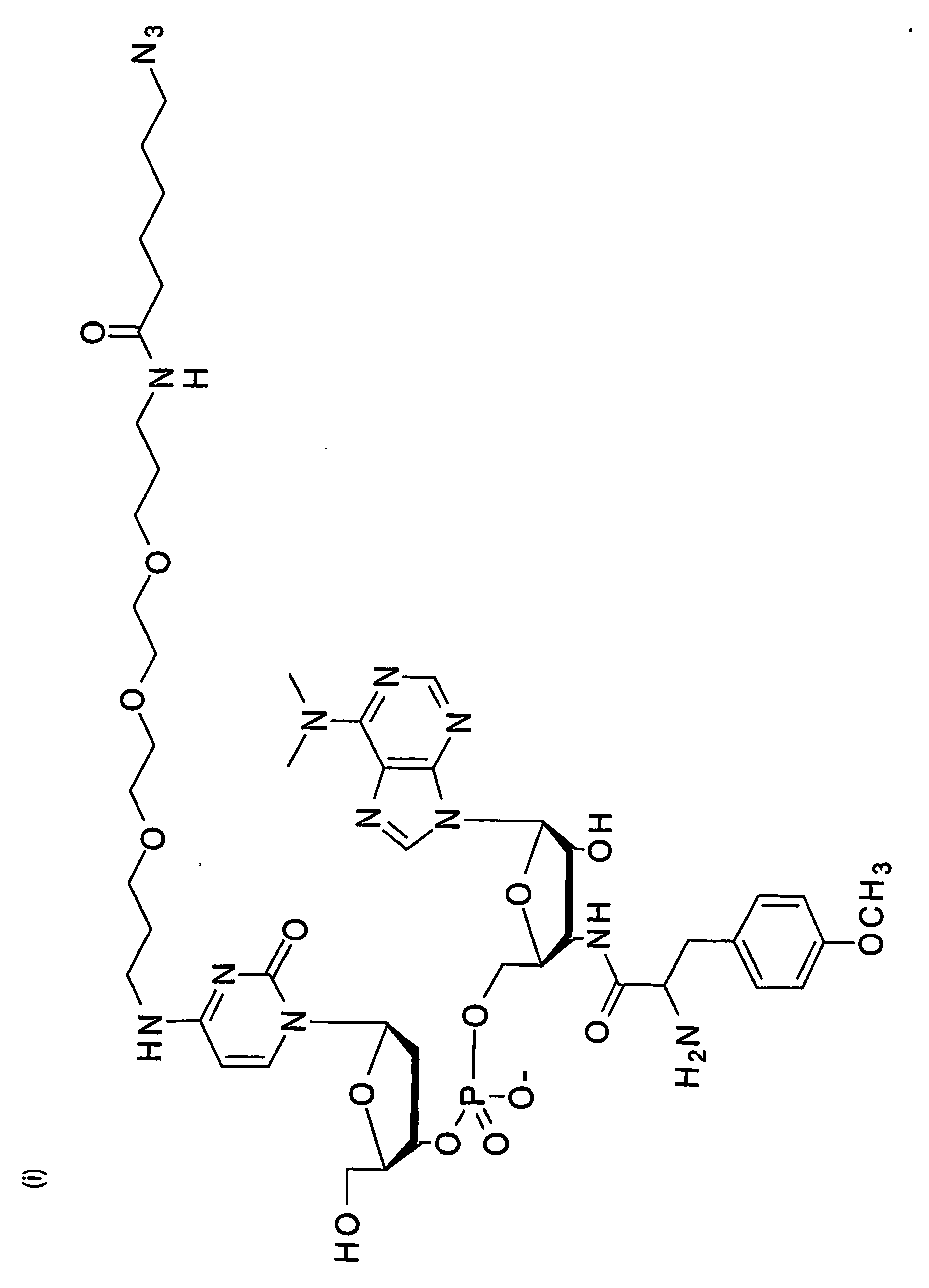

As already pointed out above, the cell-free translation system described herein and, inter alia, to be employed in the context of this invention may comprise a component which is capable of inhibiting and/or negatively interfering with a release factor comprised in said cell-free translation system. In context of this invention, it was found that an antibody directed against such (a) release factor(s) is particularly useful in inhibiting the function of such a factor. Such an anti-release factor antibody is to be used which is capble of specifically inactivating said release factor, e.g. by precipitation and/or crosslinking, whereas or other components remain intact, is of procaryotic or eukaryotic origin, more preferable it is of prokaryotic origin, even more preferably it is from *E.coli*.
For example, a release factor to be inactivated from *E. coli* may be the release factor 1, the release factor 2, the release factor homolog 1, the release factor homolog 2, the release factor homolog 3 or the release factor homolog 4. Said release factors may be encoded by the nucleotide sequences as shown in SEQ ID NOs: 5, 43, 45, 47 or 49 and/or may have the amino acid sequences as shown in SEQ ID NOs: 6, 44, 46, 48, 50 or 51. Most preferred, and also shown in the experimental part, said release factor contained in said cell-free translation system and to be inactivated is the release factor 1 from *E. coli*. Said most preferred release factor may be encoded by the nucleotide sequence as shown in SEQ ID NO: 5 and/or may have the amino acid sequence as shown in SEQ ID NO: 6.

The release factor contained and to be inactivated in the cell-free translation system of the present invention may be different from the release factor, against which the antibody to be employed was directed and/or generated. For example, the release factor contained and to be inactivated in the cell-free translation system of the present invention may be from *E. coli.* Accordingly, sad translation system comprises RF1 from *E. coli.* Yet, as shown in the examples, the anti-release factor antibody, precipitating and/or crosslinking said release factor, was generated against a release factor from *Thermus thermophilus*, namely against RF1 from *Thermus thermophilus*. Said RF1 from *Thermus thermophilus* may be encoded by the nucleotide sequence as shown in SEQ ID NO: 3 and/or may have the amino acid sequence as shown in SEQ ID NO: 4.).

In an eukaryotic context, the release factor to be inactivated by a specific crosslinking and/or precipitating antibody may be from rabbit, fruit fly or yeast. Preferably, said release factor to be inactivated by antibodies is a rabbit RF. For instance, said release factor is the release factor 1 or the release factor 3 from rabbit, release factor 1 from fruit fly or the release factor 1 or the peptide chain release factor 1 from yeast. Said exemplified release factors may be encoded by the nucleotide sequences as shown in SEQ ID NOs: 52, 54, 56 or 58, respectively, and/or may have the corresponding amino acid sequences as shown in SEQ ID NOs: 53, 55, 57 or 59. Corresponding antibodies may be prepared by methods known in the art, for example by the generation of a polyclonal serum against said release factors. "inactivating antibodies to be employed in the cell-free translation system of the present invention are, as described herein, antibodies and/or antibody molecules which are capable of precipitating and or crosslinking the release factor(s) comprised in the cell-free translation system of the present invention. Said "inactivation" may be a complete or a partial inactivation. As pointed out above, said "inactivation" leads to an inactivation of the function of said release-factors of at least 60%, more preferably of at least 70%, more preferably of at least 80% and more preferably of at least 90%. The corresponding inactivation of the release-factors by the addition of the precipitating and/or crosslinking antibodies and/or antibody molecules can be measured by methods known in the art. For example, the precipitating and/or deactivating activity of anti-RF polyclonal antibodies can be measured by the residual RF activity in the in vitro translation system, by testing the hydrolysis of a peptide from peptidyl-tRNA located in the P-site (Freistroffer Proc Natl Acad Sci U S A. (2000) 97, 2046-51, or by a gel electrophoresis followed by Western blotting, which is being a common laboratory praxis.

In particular, the release factor contained in said cell-free translation system and, potentially, to be inactivated may be selected from the group consisting of:
(a) a release factor encoded by a nucleotide sequence comprising a nucleotide sequence as shown in any one of SEQ ID NOS: 3, 5, 5, 43, 45, 47, 49, 52, 54, 56, 58 and 60;
(b) a release factor encoded by a nucleotide sequence coding for a polypeptide comprising an amino acid sequence as shown in any one of SEQ ID NOS: 4, 6, 44, 46, 48, 50, 51, 53, 55, 57, 59 and 61;
(c) a release factor which is encoded by a nucleotide sequence of a nucleic acid molecule that hybridizes to the complement strand of a nucleic acid molecule comprising a nucleotide sequence as defined in (a) or (b) and which releases a translation product from a ribosome in a cell-free translation system;
(d) a release factor which comprises an amino acid sequence as shown in any one of SEQ ID NOS: 4, 6, 44, 46, 48, 50, 51, 53, 55, 57, 59 and 61;
(e) a release factor which comprises an amino acid sequence which is at least 40% identical to the full length amino acid sequence as shown in any one of SEQ ID NOS: 4, 6, 44, 46, 48, 50, 51, 53, 55, 57, 59 and 61; and
(f) a release factor encoded by a nucleotide sequence which is degenerated to a nucleotide sequence as defined in any one of (a) to (c).

It is immediately evident form the above that the inhibition of the release factor is only and merely one embodiment of the uses and methods of the present invention. The use of thermostable prokaryotic esterase/esterase activity as disclosed herein is in no means limiting to the herein also described preparation and synthesis of alloproteins, synthesis of which is to be monitored and/or tracked in accordance with this invention. Yet, as also detailed below, the synthesis of alloproteins in combination with thermostable esterase of prokaryotic origin is particularly useful in the preparation of alloprotein-esterase fusion constructs. As shown below, the use of esterase in this context also provides for means and methods to immobilize (allo-) proteins, inter alia on solid surfaces. Further embodiments are provided below.

As detailed above, the invention is based on the advantageous use of thermostable prokaryotic esterase/esterase activity in the (in vitro) synthesis of proteins, also alloproteins. In a most preferred embodiment, said proteins/alloproteins and the like are produced in the format of an esterase-fusion construct, preferably in the format "X-esterase" or "esterase-X", i.e. the desired target protein, polypeptide or peptide being located N- or C-terminall of the esterase activity/esterase function bearing moiety. It is within the skills of the artisan to liberate the expressed target protein, polypeptide or peptide from the esterase moiety after expression. For example, the fusion construct to be expressed, monitored and/or tracked in accordance with the means and methods of the invention may additionally comprise a (proteolytically) cleavable tag which may be used to separate the desired "X" or "X'" from the esterase moiety. Corresponding examples are provided herein and are illustrated in the appended examples. Accordingly, known cleavage methods may be employed, like chemical or enzymatic methods. It is also envisaged that the expression product "X-esterase"/"esterase-X" comprises known cleavage sites (cleavage tags), sites between the "X" and the esterase moiety. Such cleavage sites are, inter alia, disclosed in Stevens (2003, Drug Discovery World, 4, 35-48) and LaVallie (1994, Enzymatic and chemical cleavage of fusion proteins. In Current Protocols in Molecular Biology. pp. 16.4.5-16.4.17, John Wiley and Sons, Inc, New York, NY); and comprise, but are not limited to, hydroxylamine cleavage (cleavage between Asn-Gly), enterokinase cleavage (cleavage after Asp-Asp-Asp-Asp-Lys), Factor Xa protease cleavage (cleavage after Ile-Glu/Asp-Gly-Arg) and the like. In context of the present invention, factor Xa protease cleavage is preferred, but also other cleavages are envisaged.

In the context of the invention also disclosed is the use of vectors, whereby said vectors are characterized in comprising a nucleic acid molecule coding for a thermostable esterase of prokaryotic origin and expressing a corresponding esterase fusionprotein. Preferably, said vector comprises a nucleic acid molecule coding for a thermostable esterase of prokaryotic origin and comprising in frame at least one multiple cloning site for a part X of an esterase-X fusionprotein, whereby the fusionprotein to be encoded may be of the format "X-esterase" or "esterase-X". Corresponding examples are given below and in the append examples. Vectors useful in particular in cell-free systems are known in the art and comprise but are not limited to plasmids, cosmids as well as viral vectors and bacteriophages or another vector used e.g. conventionally in genetic engineering. Said vectors may, besides the esterase activity and the preotein/polypeptide "X" desired to be expressed comprise further genes such as marker genes which allow for the further selection. The vector as disclosed in the context of the invention is, accordingly, an expression vector, in which the nucleic acid molecule coding for a thermostable esterase/esterase activity of prokaryotic origin is operatively linked to expression control sequence(s) allowing expression in prokaryotic or eukaryotic cell-free systems as described herein. The term "operatively linked", as used in this context, refers to a linkage between one or more expression control sequences and the coding region in the polynucleotide to be expressed (preferably "X-esterase" and "esterase-X") in such a way that expression is achieved under conditions compatible with the expression control sequence.

The use of a polynucleotide as defined above, coding for the fusion protein ("X-esterase"/"esterase-X") defined herein, whereby said polynucleotide is fused to a heterologous polynucleotide ("X"), preferably encoding a heterologous polypeptide ("X") is to be employed in context of this invention. This heterologous polypeptide may, inter alia, be a marker, like a green fluorescent protein or HA, as shown in the appended examples. However, every desired protein to be expressed may be employed as "X" in the herein defined fusion construct "X-esterase"/"esterase-X". Preferably, said nucleic acid molecule(s)/polynucleotide(s) disclosed in the context of the present invention is part of a vector. Said vector is preferably a gene expression vector. Therefore, also disclosed herein is a vector comprising the nucleic acid molecule coding for an esterase fusion construct as disclosed herein. Said vector is capable of expression. Such a vector may be, e.g., a plasmid, cosmid, virus, bacteriophage or another vector used e.g. conventionally in genetic engineering, and may comprise further genes such as marker genes which allow for the selection of said vector in a suitable host cell and under suitable conditions or in suitable expression/translation systems.

The nucleic acid molecules coding for such esterase-fusion constructs may be inserted into expression vectors, like several commercially available vectors. Nonlimiting examples include plasmid vectors compatible with mammalian cells, such as pGEM-T (Promega), pIVEX 2.3d (Roche Diagnostics), pUC, pBluescript (Stratagene), pET (Novagen), pREP (Invitrogen), pCRTopo (Invitrogen), pcDNA3 (Invitrogen), pCEP4 (Invitrogen), pMC1 neo (Stratagene), pXT1 (Stratagene), pSG5 (Stratagene), EBO-pSV2neo, pBPV-1, pdBPVMMTneo, pRSVgpt, pRSVneo, pSV2-dhfr, pUCTag, pIZD35, pLXIN and pSIR (Clontech) and pIRES-EGFP (Clontech). In the context of the present invention also a specific vector is disclosed, denoted pEst2, as also shown in SEQ ID No. 9. Further nonlimiting examples include baculovirus vectors (in particular useful in *in vitro* translation systems or *in vivo* expression systems based on insect cells) such as pBlueBac, BacPacz Baculovirus Expression System (CLONTECH), and MaxBacTM Baculovirus Expression System, insect cells and protocols (Invitrogen), which are available commercially and may also be used to produce high yields of biologically active protein. (see also, Miller (1993), Curr. Op. Genet. Dev., 3, 9; O'Reilly, Baculovirus Expression Vectors: A Laboratory Manual, p. 127). In addition, prokaryotic vectors such as pcDNA2 and yeast vectors such as pYes2 are nonlimiting examples of other vectors suitable for use with the present invention. For vector modification techniques, see Sambrook and Russel (2001), loc. cit. Vectors can contain one or more replication and inheritance systems for cloning or expression, one or more markers for selection in the host, e. g., antibiotic resistance, and one or more expression cassettes. The coding sequences inserted in the vector can be synthesized by standard methods, isolated from natural sources, or prepared as hybrids. Ligation of the coding sequences to transcriptional regulatory elements (e. g., promoters, enhancers, and/or insulators) and/or to other amino acid encoding sequences can be carried out using established methods.

The vectors disclosed in the context of the present invention are characterised in that they carry a nucleic acid molecule encoding a thermostable procaryotic esterase (thermostable prokaryotic esterase-activity) or a functional fragment thereof and that said vectors also comprise an additional cloning site, mostly an multiple cloning site, wherein a nucleic acid molecule coding for the desired protein, polypeptide or peptide (to be expressed in a given cell-free translation system) be introduced. Said introduction is desired to lead to a nucleic acid molecule, comprised in said vector, which is capable of expressing the protein, polypeptide or peptide desired in form of a fusionprotein, fusionpolypeptide or fusionpeptide, wherein the further part of said fusion molecule is the esterase (esterase activity) or a functional fragment thereof. Accordingly, the vector of the present invention, is a vector to be employed in the in vitro synthesis (in cell-free translation systems) of fusionproteins, fusionpolypeptides or fusionpeptides in the format "X-esterase" or "esterase-X". The embodiments described above for such fusion constructs apply here, mutatis mutandis. Accordingly, said inventive vector is also designed to lead to the expression of a fusion-construct "X-esterase" or "esterase-X", whereby said "X" being the desired protein, polypeptide or peptide to be expressed in the system and whereby "esterase" denotes the esterase/esterase-activity used for monitor and/or track the efficacy of the translation system, as detailed above.

Accordingly, it is also desired and possible that the disclosed vectors, comprising a nucleic acid molecule encoding said esterase and/or esterase-activity (or a functional fragment thereof) express molecules, whereby at least one further, additional peptide, polypeptide or peptide is expressed. Here it is referred to the embodiment above, relating to "X" and "X'". The components "X", "X'" etc. to be expressed (in frame) with at least one esterase and/or esterase activity may, like in the construct "X-esterase"/"esterase-X", be separated by a linker/linker structure. Such a construct may, e.g. be characterized as "X'-X-esterase", "X-X'-esterase", "esterase-X'-X" or "esterase-X-X'". Most preferably, said linker/linker structure is a cleavable linker, e.g. a linker which may be , e.g. chemically or enzymatically cleaved. Further embodiments described above for such linkers, apply here, mutatis mutandis.

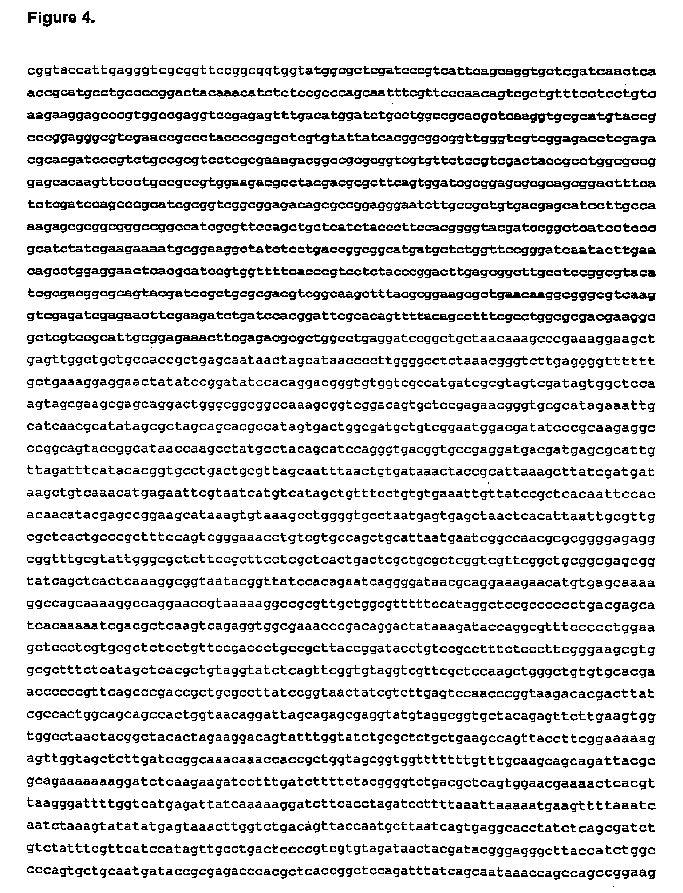

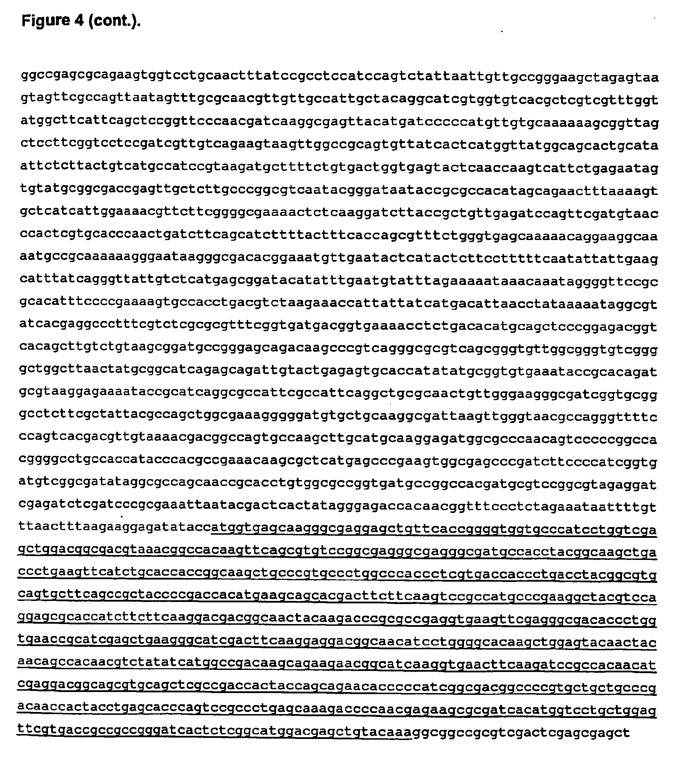

Vectors disclosed in the context of the invention are also illustrated herein and in the appended examples. One example of such a vector is given in SEQ ID NO: 8, and figure 4. Namely, said vector comprises a nucleic acid molecule coding for Est2 and said "X" (or said "X'") is eGFP. It is immediately evident for a person skilled in the art that the nucleic acid molecule coding eGFP can easily replaced (by recombinant means) with another nucleic acid molecule coding for the protein, polypeptide or peptide desired to be expressed in a given cell-free translation system.

Furthermore, the vectors may, in addition to the nucleic acid sequences disclosed in the context of the invention, i.e. a a nucleic acid molecule coding for esterase-fusion constructs as provided herein, comprise expression control elements, allowing proper expression of the coding regions in suitable hosts or in *in vitro* translation systems. Such control elements are known to the artisan and may include a promoter, translation initiation codon, translation and insertion site or internal ribosomal entry sites (IRES) (Owens, Proc. Natl. Acad. Sci. USA 98 (2001), 1471-1476) for introducing an insert into the vector. Preferably, the nucleic acid molecule disclosed in the context of the invention is operatively linked to said expression control sequences allowing expression in eukaryotic or prokaryotic cells or in *in vitro* translation systems.
Control elements ensuring expression in eukaryotic and prokaryotic cells are well known to those skilled in the art. As mentioned above, they usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in for example mammalian host cells comprise the CMV-HSV thymidine kinase promoter, SV40, RSV-promoter (Rous sarcome virus), human elongation factor 1α-promoter, CMV enhancer, CaM-kinase promoter or SV40-enhancer.

For the expression in prokaryotic cells, a multitude of promoters including, for example, the tac-lac-promoter, the lacUV5 or the trp promoter, has been described. Beside elements which are responsible for the initiation of transcription such regulatory elements may also comprise transcription termination signals, such as

SV40-poly-A site or the tk-poly-A site, downstream of the polynucleotide. In this context, suitable expression vectors are known in the art such as Okayama-Berg cDNA expression vector pcDV1 (Pharmacia), pRc/CMV, pcDNA1, pcDNA3 (In-Vitrogene), pSPORT1 (GIBCO BRL) or pGEM-T (Promega), or prokaryotic expression vectors, such as lambda gt11. In the appended examples, pGEM-T (Promega) and pIVEX2.3d (Roche Diagnostics, Mannheim, Germany) were employed. It is in the routine working skills of the artesian to adapt and generate desired gene expression vectors commercially available vectors.

An expression vector disclosed in the context of this invention is at least capable of directing the expression of the fusion nucleic acids and fusion proteins described herein. Suitable origins of replication include, for example, the Col E1, the SV40 viral and the M 13 origins of replication. Suitable promoters include, for example, the T7 promoter (as employed in the appended examples), the cytomegalovirus (CMV) promoter, the lacZ promoter, the gal10 promoter and the Autographa californica multiple nuclear polyhedrosis virus (AcMNPV) polyhedral promoter. Suitable termination sequences include, for example, the T7 terminator, the bovine growth hormone, SV40 terminator, lacZ terminator and AcMNPV terminator polyhedral polyadenylation signals. Examples of selectable markers include neomycin, ampicillin, and hygromycin resistance and the like. Specifically-designed vectors allow the shuttling of DNA between different host cells, such as bacteria-yeast, or bacteria-animal cells, or bacteria-fungal cells, or bacteria-invertebrate cells.

The vectors disclosed herein are particular useful in cell-free translation systems. To ligate said nucleic acid molecule "in frame" means, that the nucleic acid molecule coding for least the esterase (esterase function) or a fragment thereof as well as the nucleic acid molecule coding for the desired protein, polypeptide or peptide is transcribed in a manner that the resulting (mRNA) represents the open reading frames for both, said esterase (esterase function) or a fragment thereof and said desired protein, polypeptide or peptide. Therefore, the "in frame" means, that the resulting product of the expression vector is a fusionprotein, fusionpolypeptide or fusionpeptide, comprising at least the esterase (esterase function) or a fragment thereof and the desired protein, polypeptide or peptide or a fragment thereof, wherein the esterase (esterase function) or a fragment thereof as well as the desired protein, polypeptide or peptide is functional. What is disclosed herein for the described vector also applies to nucleic acid molecules comprised in said vectors.

The present application in addition discloses the use of a host transformed with a vector disclosed in the context of the present invention or to a host comprising the herein disclosed nucleic acid molecule and coding for a fusion protein as defined herein ("X-esterase"/"esterase-X"). Said host may be produced by introducing said vector or nucleotide sequence into a host cell which upon its presence in the cell mediates the expression of a protein encoded by the nucleotide sequence of the invention or comprising a nucleotide sequence or a vector disclosed herein, wherein the nucleotide sequence and/or the encoded polypeptide is foreign to the host cell.
By "foreign" it is meant that the nucleotide sequence and/or the encoded polypeptide is either heterologous with respect to the host, this means derived from a cell or organism with a different genomic background, or is homologous with respect to the host but located in a different genomic environment than the naturally occurring counterpart of said nucleotide sequence. This means that, if the nucleotide sequence is homologous with respect to the host, it is not located in its natural location in the genome of said host, in particular it is surrounded by different genes. In this case the nucleotide sequence may be either under the control of its own promoter or under the control of a heterologous promoter. The location of the introduced nucleic acid molecule or the vector can be determined by the skilled person by using methods well-known to the person skilled in the art, e.g., Southern Blotting. The vector or nucleotide sequence according to the invention which is present in the host may either be integrated into the genome of the host or it may be maintained in some form extrachromosomally. In this respect, it is also to be understood that the nucleotide sequence of the invention can be used to restore or create a mutant gene via homologous recombination.

Said host may be any prokaryotic or eukaryotic cell. Suitable prokaryotic/bacterial cells are those generally used for cloning like E. coli, Salmonella typhimurium, Serratia marcescens or Bacillus subtilis. Said eukaryotic host may be a mammalian cell, an amphibian cell, a fish cell, an insect cell, a fungal cell or a plant cell. Said prokaryotic cell may be bacterial cell (e.g., E coli strains BL21 (DE3), HB101, DH5a, XL1 Blue, Y1090 and JM101). Eukaryotic recombinant host cells are also useful. Examples of eukaryotic host cells include, but are not limited to, yeast, e.g., Saccharomyces cerevisiae, Schizosaccharomyces pombe, Kluyveromyces lactis or Pichia pastoris cells, cell lines of human, bovine, porcine, monkey, and rodent origin, as well as insect cells, including but not limited to, Spodoptera frugiperda insect cells and Drosophila-derived insect cells. Also fish and amphibian cells, like Xenopus cells or zebra fish cells (including eggs of amphibians and fishes), may be employed. Mammalian species-derived cell lines suitable for use and commercially available include, but are not limited to, L cells, CV-1 cells, COS-1 cells (like ATCC CRL 1650), COS-7 cells (like ATCC CRL 1651), HeLa cells (like ATCC CCL 2), C1271 (like ATCC CRL 1616), BS-C-1 (like ATCC CCL 26), CHO cells (like ATCC CRL1859, ATCC CRL 1866) and MRC-5 (like ATCC CCL 171). Also HEK293 cells may be employed and are useful as host cells in accordance with this invention.

The invention also provides for the use of a vector as described herein or of a nucleic acid molecule comprised in said vector (and comprises a coding sequence for a fusion protein as defined herein and comprising a thermostable procaryotic esterase/esterase activity (or a functional fragment thereof)) for monitoring and/or tracking the synthesis of said protein, polypeptide or peptide in a cell free translation system or in an *in vivo* expression system. As detailed herein and in the appended examples, said monitoring and/or tracking comprises the detection of the function of said esterase/esterase activity.

Furthermore, also disclosed in the context of the invention is the use of a protein, polypeptide or peptide encoded by a vector disclosed in the context of the invention. What is described above for the fusion constructs encoded by the vectors disclosed herein applies for the disclosed protein, polypeptide or peptide, mutatis mutandis.

In a further embodiment, the present invention provides for a method for monitoring and/or tracking the synthesis of a protein, polypeptide or peptide in a cell-free translation system, comprising the step of detecting the function of a thermostable esterase of prokaryotic origin. Said function of an esterase/esterase activity can be measured as discussed herein above, e.g. with the use of specific substrates. Again, corresponding embodiments are provided herein and in the appended examples.

As discussed above, the present invention is also useful in tagging proteins with thermostable esterase/a thermostable esterase activity of prokaryotic origin. Correspondingly, such a tagged protein/polypeptide/peptide, comprising such an esterase/esterase activity may be isolated, e.g. from the cell-free translation system with the help of specific esterase-interaction partners. Such an interaction partner/binding partner may, inter alia, be an antibody/antibody molecule or fragment thereof, specifically interacting with/binding to said esterase. In general, as employed herein, the term "antibody" may comprise purified serum, i.e. a purified polyclonal serum. The antibody molecule may also be a full immunoglobulin, like an IgG, IgA, IgM, IgD. IgE, IgY (for example in yolk derived antibodies). The term "antibody" as used in this context of this invention also relates to a mixture of individual immunoglobulins. Furthermore, it is envisaged that the antibody/antibody molecule is a fragment of an antibody, like an F(ab), F(abc), Fv Fab' or F(ab)2. Furthermore, the term "antibody" as employed in the invention also relates to derivatives of the antibodies which display the same specificity as the described antibodies. Such derivatives may, inter alia, comprise chimeric antibodies or single-chain constructs.

However, as illustrated herein, also an inhibitor of such an esterase may function as a corresponding interaction partner.

These and other embodiments are disclosed and encompassed by the description and examples of the present inversion. Further literature concerning any one of the Embodiments in accordance with the present invention may be retrieved from public libraries, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, hftp://www.fmi.ch/biology/research-tools.html, hftp://www.tigr.org/, are known to the person skilled in the art and can also be obtained using, e.g., hftp://www.lycos.com. An overview of patent information in biotechnology and a survey of relevant sources of patent information useful for retrospective searching and for current awareness is given in Berks, TIBTECH 12 (1994), 352-364.

The present invention is further described by reference to the following non-limiting figures and examples.

The Figures show:

### Figure 1.

Plasmid map of pIVEX 2.3d-Est2 (pEst2).

### Figure 2.

Nucleotide sequence of pIVEX 2.3d-Est2 (pEst2; SEQ ID NO: 9). Bolded letters are the coding sequence (SEQ ID NO: 1 of the esterase 2 (Est2; SEQ ID NO: 2 or 62) from *Alicyclobacillus acidocaldarius*

### Figure 3.

Plasmid map of pIVEX_eGFP-Est2 (peGFP-Est2).

### Figure 4.

Nucleotide sequence of pIVEX_eGFP-Est2 (peGFP-Est2; SEQ ID NO: 8). Bolded letters are the coding sequence (SEQ ID NO: 1 of the esterase 2 (SEQ ID NO: 2 or 62), underlined letters are the coding sequence of the enhanced green fluorescent protein.

### Figure 5.

Different substrates an their esterase-catalysed reactions for the (A) electrochemical, (B) optical and (C) fluorescence detection of the esterase activity.

### Figure 6.

*In vitro* synthesis of the esterase 2 from *Alicyclobacillus acidocaldarius* in *E. coli* translation system. The templates for the protein biosynthesis were: open circles, EF-Ts gene, filled squares pEst2; open triangles - control without template.
**A:** accumulation of newly synthesized protein measured by [¹⁴C]Ieucine incorporation.
**B:** activity of the esterase determined by hydrolysis of p-nitrophenyl acetate,
**C:** activity of the esterase determined by hydrolysis of 5-(and-6)-carboxy-2',7'-dichlorofluorescein diacetate.

### Figure 7.

SDS-PAGE of the *in vitro* translated polypeptides. The templates are indicated on the top of corresponding lane. Aliquots of 2 µl translation reaction mixture were withdrawn after 90 min. incubation time. EF-Ts, synthesis of elongation factor Ts from *E. coli*; Est, synthesis of esterase 2 from *Alicyclobacillus acidocaldarius.*
**A:** Coomasie blue-stained polyacrylamide gel,
**B:** radioactive image of the gel,
**C:** staining for esterase activity.

Positions of marker proteins and of the esterase 2 are indicated by arrows.

### Figure 8.

Affinity purification of expressed esterase with covalently linked protein.
**1:** Inhibitor possessing high affinity to esterase is attached via a spacer to a matrix.
**2:** Esterase covalently linked to a protein is bound to the matrix, residual proteins are washed out.
**3a:** The esterase is eluted from the column and the amount of the co-purfied protein X is determined by the linked esterase activity.
**3b:** linked peptide X is cleaved and washed of the column.

### Figure 9.

Esterase as an affinity tag for purification of the *in vitro* synthesized proteins. The *in vitro* transcription/translation was performed for 90 min. with the pEst2 as a template.
**A:** radioactive image of the SDS-PAGE of 4µl samples. The mobilities of eGFP-esterase, esterase and eGFP are indicated by arrows.
**B:** esterase activity (grey bars) and eGFP fluorescence (black bars) in the samples. The samples at subsequent purification steps were: 1-translation mixture after 90 min. incubation time; 2- translation mixture after 90 min. incubation followed by treatment with TFK-matrix SDS-PAGE, samples were then taken from the supernatant. 3- material released from the TFK-matrix by treatment with Factor Xa protease, 4- material which remained bound to the TFK-matrix after protease treatment and was released by treatment with 1% SDS at 95°C. After dialysis at 25°C the activity of the esterase was determined.

### Figure 10.

Scheme for screening of protein biosynthesis inhibitory activity in a celt-free translation assay.

### Figure 11.

Immobilization of Esterase-Puromycin conjugates produced on streptavidin coated glass surface. Translation reaction was performed as described in Experimental section. Plate 1 is a control onto which 1µL purified esterase, prepared by purification from *E. coli* cells overexpressing the enzyme (Arkov (2002), J Bacteriol. 184, 5052-5057.). On the plates 2, 3, and 4 one µL translation mixture containing no puromycin derivative, Biotin-CpPuromycin and Biotin-CpPuromycin together with anti-RFI antibodies, respectively. After 90 min. at 37°C the plates were rinsed by tap-water and the esterase activity was determined by 2-naphthyl acetate assay and developed with Fast Blue BB salt.

### Figure 12.

Plasmid map of pEst2_amb155.

### Figure 13.

Nucleotide sequence of pEst2_amb155 (SEQ ID NO: 10). Bolded letters are the coding sequence of esterase 2 S155amber mutant.

### Figure 14.

Substitution of the sense codon for catalytically important Serine 155 (AGC) into nonsense codon (UAG) in the mRNA for the esterase 2 from *Alicyclobacillus acidocaldarius*
**A:** Scheme of mutated mRNA
**B:** The esterase catalytic triad (Ser-His-Asp) structural organization (De Simone (2000), J Mol. Biol 303, 761-771).

### Figure 15.

In vitro synthesis of the pEst2_Amb155.
**A:** Kinetics of *in vitro* transcription/translation of the pEst2_Amb155; filled triangles: pEst2 (control) as a template; filled squares: pEst2_Amb155 as a template. The concentration of the newly synthesized proteins was determined by measurement of the TCA precipitatable [¹⁴C]leucine radioactivity in aliquots withdrawn at indicated time intervals.
**B:** Radioactive image of the SDS-PAGE of 2 µl samples from the *in vitro* translation mixture after 120 min. incubation time. Lane 1 - pEst2_Amb155 as a template; lane 2 - pEst2 (control) as a template.
**C:** Esterase activity of the *in vitro* synthesized polypeptides determined by hydrolysis of p-nitrophenyl acetate. Bar 1 - pEst2_Amb155 as a template; bar 2 - pEst2 (control) as a template.

### Figure 16.

Suppression of amber stop codon by suppressor Ser-tRNA^{Ser(CUA)} in the presence of antibodies against RF1. Samples from the *in vitro* translation system programmed by pEst2_Amb155 were withdrawn after 160 minutes of incubation in the presence of [¹⁴C]leucine.
**A:** Translation was carried out in the absence of anti-RF1 antibodies. Radioactive image of the gel after SDS-PAGE separation of 2 µl samples from the *in vitro* translation mixture is presented. Concentration of added suppressor tRNA^{Ser(CUA)} was as follows: lane - no tRNA^{Ser} added, lane 2 - 24 nM, lane 3 - 120 nM, lane 4- 600 nM, lane 5 - 2.5 µM, lane 6 -10 µM, lane 7 - 25 µM.
**B:** Translation was carried out in the presence of anti-RF1 antibodies. Radioactive image of the gel after SDS-PAGE separation of 2 µl samples from the *in vitro* translation mixture is presented. Concentration of added suppressor tRNA^{Ser} was as described in **(A).**
**C:** Enzymatic activity of the *in vitro* produced esterase determined by hydrolysis of p-nitrophenyl acetate. Samples of 1 µl were used for detection. Grey bars, translation was carried out in the absence of anti-RF1 antibodies, black bars, translation was carried out in the presence of anti-RF1 antibodies. Concentration of added suppressor tRNA^{Ser(CUA)} was as described in **(A).**

### Figure 17.

Plasmid map of pNox-Est2.

### Figure 18.

Nucleotides sequence of pNox-Est2 (SEQ ID NO: 11).
Bold letters are gene of Nox. Italic letters are gene of Est2 (SEQ ID NO: 1). Bold and italic letters are the coding sequence of Factor Xa cleavage site.

### Figure 19.

SDS-PAGE of *in vitro* and *in vivo* expression of Nox-Est2 fusion protein with Esterase activity staining (bands marked by arrows) and coomassie blue G-250 staining of total protein.
**A:** *in vitro* expression of Nox-Est2 fusion protein. The position of the Nox-Est2 fusion protein is indicated by arrow.
**B:** *in vivo* expression of Nox-Est2 fusion protein. Lane 1: protein molecular weight standard; lane 2: cell extracts. Nox-Est2 fusion protein is indicated by black arrow.

### Figure 20.

Plasmid map of pTu-Est2.

### Figure 21.

Nucleotides sequence of pTu-Est2 (SEQ ID NO: 12).
Bold letters are gene of EF-Tu. Italic letters are gene of Est2 (SEQ ID NO: 1). Bold and italic letters are the coding sequence of Factor Xa cleavage site.

### Figure 22.

SDS-PAGE of *in vitro* and *in vivo* expression of EF-Tu-Est2 fusion protein with Esterase activity staining and protein coomassie blue G-250 staining.
**A:** *in vitro* expression of EF-Tu-Est2 fusion protein. Lane 1: protein molecular weight standard; Lane 2: *in vitro* expression mixture. EF-Tu-Est2 fusion protein was pointed out by the black arrow.
**B:** *in vivo* expression of EF-Tu-Est2 fusion protein. EF-Tu-Est2 fusion protein was pointed out by the black arrow.

### Figure 23.

Plasmid map of pTs-Est2.

### Figure 24.

Nucleotides sequence of pTs-Est2 (SEQ ID NO: 13).
Bold letters are gene of EF-Ts. Italic letters are gene of Est2 (SEQ ID NO: 1). Bold and italic letters are the coding sequence of Factor Xa cleavage site.

### Figure 25.

SDS-PAGE of *in vitro* and *in vivo* expression of EF-Ts-Est2 fusion protein using esterase activity and protein coomassie blue G-250 staining.
**A:** *in vitro* expression of EF-Ts-Est2 fusion protein. Lane 1: protein molecular weight standard; lane 2: *in vitro* expression mixture. EF-Ts-Est2 fusion protein is indicated by a black arrow.
**B:** *in vivo* expression of EF-Ts-Est2 fusion protein. EF-Ts-Est2 fusion protein is indicated by a black arrow.

### Figure 26.

Plasmid map of pExp-Est2.

### Figure 27.

Nucleotides sequence of pExp-Est2 (SEQ ID NO: 14).
Bold letters are a gene of Exportin-t. Italic letters are agene of Est2 (SEQ ID NO: 1). Bold and italic letters are the coding sequence of Factor Xa cleavage site.

### Figure 28.

SDS-PAGE of *in vitro* expression of Exportin-t-Est2 fusion protein. The fusion protein detected by enzymatic staining for esterase is indicated by arrow.
Positions of protein molecular weight standard was indicated by bars the total protein bands were stained by coomassie blue G-250.

### Figure 29.

Plasmid map of pET-52001-Est2.

### Figure 30.

Nucleotides sequence of pET-52001-Est2 (SEQ ID NO: 15).
Bold letters are gene of putative nuclease S2001. Italic letters are gene of Est2 (SEQ ID NO: 1). Bold and italic letters are the coding sequence of Factor Xa cleavage site.

### Figure 31.

SDS-PAGE of *in vivo* expression of S2001-Est2 fusion protein with Esterase activity and protein coomassie blue G-250 staining.
Lane 1: protein molecular weight standard; lane 2: cell extract of *in vivo* expression of S2001-Est2 fusion protein. The S2001-Est2 fusion protein is indicated by an arrow.

### Figure 32.

Purification of recombinant Nox-Est2 fusion protein on TFK-Sepharose.
a: SDS-PAGE of: (1) molecular weight standards, (2) proteins in the break-through volume of the Nox-Est2 fusion protein overexpressing *E. coli* cellular extract, (3) Nox-Est2 fusion protein eluted by 1,1,1-Trifluoro-3-(2-hydroxy-ethylsulfanyl)-propan-2-one (F₃C-CO-CH₂-S-CH₂CH₂-OH). The arrow indicate the position of Nox-Est2 fusion protein, the bars the molecular masses of protein standards. The gels were stained by coomassie blue and esterase activity staining.
b: Purification of recombinant Nox-Est fusion protein. SDS-PAGE of samples from different steps of NADH oxidase purification. The positions of the molecular weight standards are shown by bars. After SDS-PAGE, the polyacrylamide gel was treated by activity staining of esterase, then stained with coomassie blue G-250. Lane 1: cell extracts; lane 2: break-through peak; lane 3: Nox eluted from TFK column via amino acid sequence specific cleavage by Factor Xa; Lane 4: Factor Xa from commercial source. The arrow indicate the position of NADH oxidase (Nox).

The Examples illustrate the invention.

### Example 1: Preparation of the plasmid pIVEX 2.3d-Est2 (pEst2) comprising a cDNA encoding the esterase 2 (Est2) from Alicyclobacillus acidocaldarius

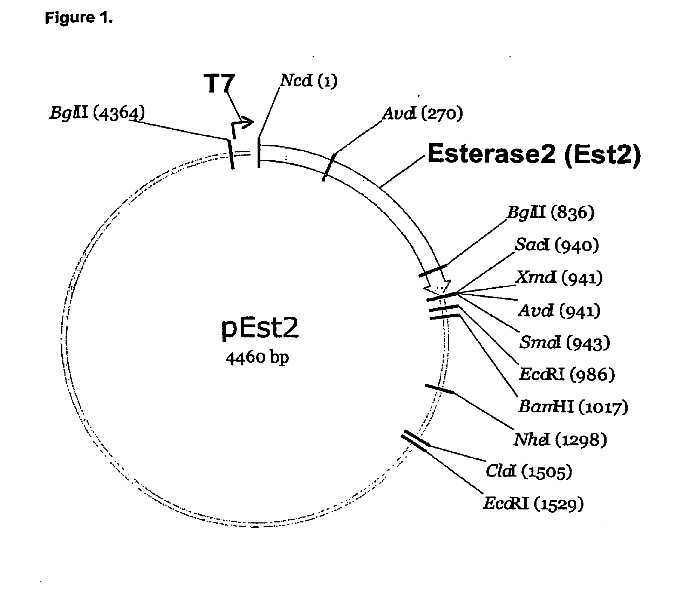

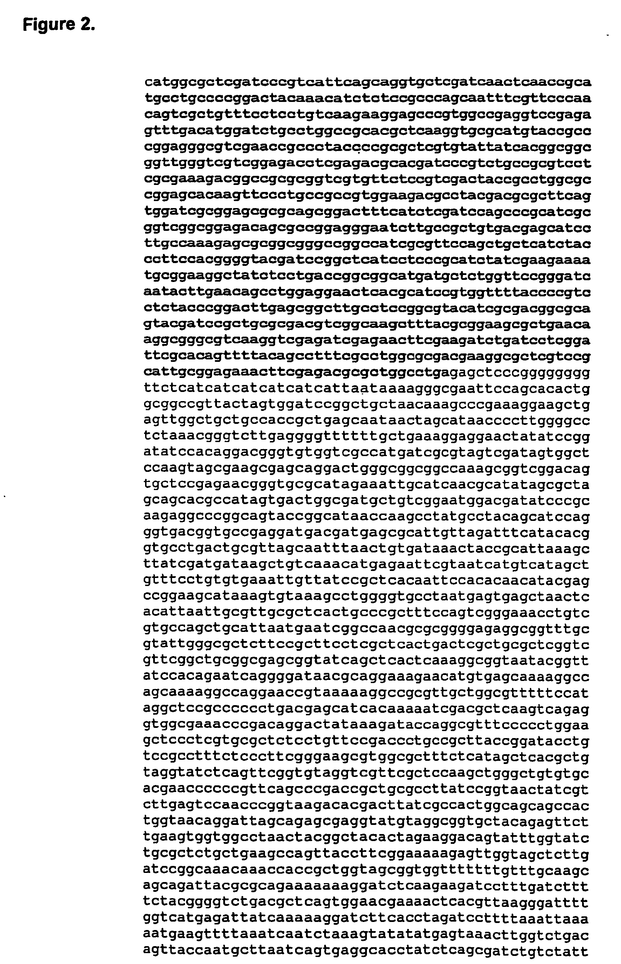

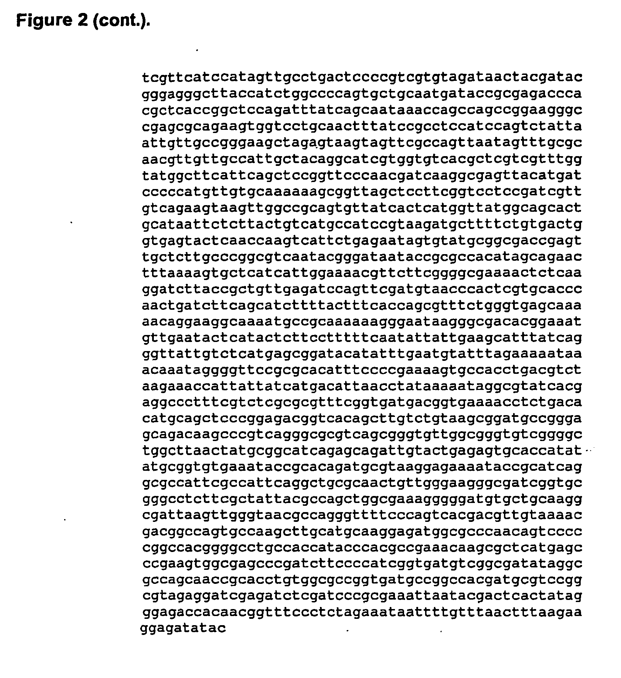

Plasmid pT7SCll containing esterase 2 (Est2) was kindly provided by G. Manco, Napples, Italy (Manco (1998), *Biochem. J,* 332 (Pt 1), 203-212.). The Est2 gene was amplified by using pT7SCll-esterase as template, recombinant Taq-polymerase, and two synthetic oligonucleotides, estfor (5'- CCATG**G**CGCTCGATCCCGTCATTCAGC -3'; SEQ ID NO: 16) and estrev (5'- GAGCTC**CTA**GGCCAGCGCGTCTCG -3'; SEQ ID NO: 17) in a 30-cycle polymerase chain reaction (1 min at 95°C, 30 sec 60°C, and 1 min at 72°C).
The primer estfor was designed to introduce a *Nco*I restriction site (underlined) at the initiation site which also leads to a C to G exchange (bold) in the coding sequence (proline at position 2 changes to alanine). This amino acid replacement has no effect on structure or function of the enzyme. Primer estrev introduces a *Sac*I restriction site (underlined) downstream from the UAG stop codon (bold). The PCR product was eluted from an agarose gel and ligated into pGEM-T vector (Promega) and completely sequenced to verify that only desired mutations were introduced. The obtained plasmid was then digested with *Nco*I and *Sac*I, the cloned fragment was eluted from an agarose gel and ligated into *Nco*I-*Sac*I-linearized *in vitro*-translation-vector pIVEX2.3d (Roche Diagnostics, Mannheim, Germany). The resulting plasmid, pIVEX2.3d-Est2 (pEst2), was used for *in vitro* translation. A map of pEst2 is shown in Fig. 1, the corresponding nucleotide sequence is shown in Fig. 2 (SEQ ID NO: 9).

### Example 2: Preparation of the plasmid pIVEX2.3d-eGFP-Est2 (peGFP-Est2) comprising a cDNA encoding a fusion protein comprising eGFP, esterase 2 (Est2) from Alicyclobacillus acidocaidarius and a cleavable linker.

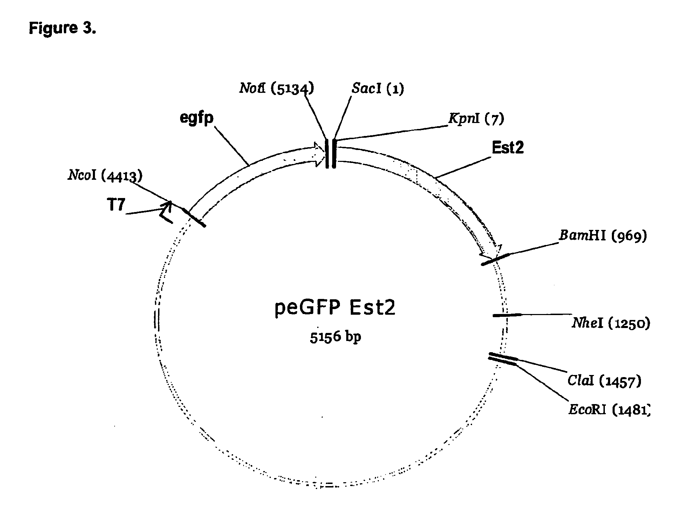

The pIVEX2.3d-eGFP-Est2 (peGFP-Est2) plasmid was constructed as follows. The gene of enhanced green fluorescence protein (eGFP) was amplified from the plasmid pSL1180-eGFP (Genbank, accession number pEGFP-1-U55761, provided by G. Krauss, Bayreuth) by PCR with the primers eGFP_for (5'-CCATGGTGAGCAAGGGCG-3'; SEQ ID NO: 18) and eGFP_rev (5'-GCGG CCGCCTTTGTACAGCTCGTCCAT-3'; SEQ ID NO: 19). The primers introduce the *NcoI* and the *NotI* cleavage sites (underlined letters) upstream and downstream of the eGFP, respectively. The PCR product was sequenced and cloned into the pIVEX2.3d vector resulting in the pIVEX2.3d-eGFP (peGFP) plasmid. The Est2 was amplified with primers Est2CT_for (5'-GAGCTCGGTACC**ATTGAGGGTCGC**GGTTCCGGCGGTGGTATGGCGCTCGATC CC-3'; SEQ ID NO: 20) and Est2CT_rev (5'-GGATCC**TCA**GGCCAGCGC-3'; SEQ ID NO: 21). The primers create the *SacI* and the *BamHI* cleavage sites (underlined letters) upstream and downstream of the Est2, respectively. The primer Est2CT_for contains the cleavage site of protease Factor Xa coding sequence (bold letters) and the primer Est2CT_rev contains the UAG stop codon (bold letters). The PCR product was sequenced and cloned into the peGFP plasmids. The resulting plasmid, peGFP-Est2, was used for *in vitro* translation. A map of peGFP-Est2 is shown in Fig. 3, the corresponding nucleotide sequence is shown in Fig. 4 (SEQ ID NO: 8).

### Example 3: Purification of the plasmids pEst2 and peGFP-Est2

The plasmids for coupled in vitro transcription/translation were purified with modified PEG method (Nicoletti (1993), Biotechniques, 14, 532-4, 536.). Therefor, the 12.5 ml cell culture, containing the plasmid was harvested and resuspended in 240 µL of 25 mM Tris/HCl pH 8.0, 50 mM glucose, 10 mM EDTA. Then 600 µL of 0.2 N NaOH, 1 % (w/v) SDS was added. The tube was gently turned over for several times and incubated for 4 minutes at room temperature. 450 µL of 3.6 M NaOAc, pH 5.0 was added and the suspension was gently mixed by turning over the tube for 20 times and incubated for 4 minutes at room temperature. Cell debris was removed by centrifugation at 16,000 g for 10 minutes and the supernatant was mixed with 400 µL of 40 % PEG 6000 and kept on ice for one hour. The sample was centrifuged at 16,600 g for 10 minutes and the pellet was completely dissolved in 150 µL ddH₂O. After addition of 300 µL of saturated. NH₄Ac the suspension was incubated for 15 minutes on ice and then centrifuged at 16,600 g for 10 minutes at room temperature. The supernatant was mixed with 300 µL of isopropanol and incubated for 15 minutes at room temperature. After centrifugation at 16,600 g for 10 min, the DNA pellet was washed two times with 75% ethanol, dried and dissolved in distilled water.

### Example 4: The SDS-PAGE of the present invention

Protein pattern of the reaction mixture was analysed by SDS-PAGE (Schagger (1987), Anal. Biochem., 166, 368-379.). Aliquots were mixed with the sample buffer, incubated 5 min. at 95°C and loaded onto 10% polyacrylamide gel. After the run the gels were fixed with 15% formaldehyde in 60% methanol and stained with Coomassie Blue G-250.

For imaging radioactivity, the dried gels were exposed to an imaging plate for radioactivity analysis with the PhosphorImager SI (Molecular Dynamics, Sunnyvale, USA).
Activity staining of the esterase in polyacrylamide gels after electrophoretic separation was performed according to (Higerd (1973), J. Bacteriol., 114, 1184-1192.) with Fast Blue BB Salt and β-naphthyt-acetate.

### Example 5: The esterase activity assays of the present invention

Determination of esterase activity was performed as described (Manco (1998), Biochem. J., 332, 203-212.) with minor modifications.
Aliquots of 1 µl transcription/translation mixture were added to 1 ml of 50 mM phosphate buffer pH 7.5 containing 0.025 mM p-nitrophenyl acetate. The production of p-nitrophenoxide was monitored at 405 nm in 1 cm path-length cells with UV-Spectral photometer DU 640 (Beckman, Fullerton, USA) at 25°C. Initial rates were calculated by linear least-square analysis of time courses comprising less than 10% of the total substrate turnover.
Esterase activity was also determined by fluorescence assay. At each time interval 1 µl was withdrawn from transcription/translation mixture and added to 1 ml of 50 mM phosphate buffer pH 7.5 with 0.025 mM 5-(and-6)-carboxy-2',7'-dichlorofluorescein diacetate. Production of 5-(and-6)-carboxy-2',7'-dichlorofluorescein was measured at 25°C by Luminescence spectrometer LS50B (Perkin Elmer, Boston, USA) with λₑₓ at 500 nm and λₑₘ at 525 nm.
Staining of esterase activity in polyacrylamide gels was performed as described herein above (Example 4).

### Example 6: In vitro synthesis of the esterase 2 (Est2) from Alicyclobacillus acidocaldarius in an E. coli cell-free translation system

Esterase 2 from *Alicyclobacillus acidocaldarius* was synthesized by coupled *in vitro* transcription/translation system derived form *E. coli.* Although, the codon usage of the esterase gene was not adjusted to the codon usage of *E. coli,* the synthesis of the thermostable esterase proceeds with similar efficiency in this heterologous system as the synthesis of one most abundant *E. coli* proteins, the elongation factor Ts. Fig. 6 demonstrates the *in vitro* [¹⁴C]Ieucine incorporation into the esterase (Fig. 6A) with the simultaneous monitoring of the esterase activity (Fig. 6B). The system produces the target proteins linearly up to 60 minutes of incubation. The estimated yield for the EF-Ts and the esterase was approximately 350 and 200 micrograms of the protein per 1 ml of the reaction mixture, respectively (Fig. 6A). The *in vitro* produced esterase possesses high enzymatic activity (Fig. 6B). Thus, even thousand fold dilution of the *in vitro* synthesized esterase in the assay mixture, which results in 10⁻⁸ M final esterase concentration, provides well-detectable initial rates of the enzymatic activity. In contrast, the level of esterase activity in the absence of esterase gene is very close to the background (Fig. 6B) providing evidence for the absence of endogenous *E. coli* esterase activity in the translation system. Besides the standard photometric detection the fluorescence measurement of the esterase activity was carried out. Enzymatic hydrolysis of the 5-(and-6)-carboxy-2',7'-dichlorofluorescein diacetate by the esterase leads to appearance of the fluorescent product (Fig. 6C). Kinetics of esterase production detected by fluorescence coincide with ones determined photometrically or by polypeptide-incorporated radioactivity.

The SDS-PAGE of the total protein from the reaction mixture with subsequent detection of radioactivity distribution and staining of the gel for esterase activity were also performed for *in vitro* synthesized esterase in comparison with EF-Ts. The protein samples analyzed by SDS-PAGE contain many endogenous *E. coli* proteins (Fig. 7A), which are, however, not labeled with [¹⁴C]Ieucine (Fig. 7B). The autoradiogram of the gel (Fig. 7B) reveals distinct bands at the position of the esterase (MW -34.4 kD) and of the control protein EF-Ts (MW ∼31.6 kD) with more than 90% of incorporated radioactivity belonged to the full-length products in both cases. The faster migrating bands are probably incomplete polypeptides translated from truncated mRNAs. The *in situ* activity staining of the esterase in polyacrylamide gel detects only one band that corresponds to the full-length esterase. This is in contrast with the lack of esterase activity in the lines related to EF-Ts and in the control without template (Fig. 7C).

The kits used for *in vitro* translation experiments within the present application were evaluation size transcription/translation kits from RiNA GmbH, (Berlin, Germany, kindly provided by Dr. W. Stiege) and the reaction was performed at 37 °C according to the manual provided by the supplier. [¹⁴C]Leucine (17,3 mCi/mmol) was added up to 0.5 mM. The template (control vector, peEst2 or peGFP-Est2) was added up to 5 nM. The reaction was started by transferring the reaction tube to the thermo shaker at 37 °C with 500 rpm agitation. Aliquots of 3µl were withdrawn at different time intervals (up to 2 hours) and the newly synthesized protein was determined by radioactivity measurement in 10% trichloroacetic acid precipitate.
As an example, the detailed protocol of the in vitro translations performed within the present application is listed below.

Protocol for an *in vitro* translation (RiNA GmbH Kits):
For preparation of a 30 µl reaction mixture the following components should be combined on ice (given in order of mixing):
1. 5.1 µl of 1 mM [¹⁴C]Leu (54 mCi/mmol, Amersham)
2. 1 µl 10 mM Leu (supplied with the Kit)
3. 0.5 µl of RNase free water (supplied with the Kit)
4. 2.4 µl of E-mix (red lid, supplied with the Kit)
5. 9 µl of T-mix without Leu (blue lid, supplied with the Kit)
6. 10.5 µl of S-mix (yellow lid, supplied with the Kit)
7. 1.5 µl of the 100 nM template plasmid
The reaction mixture should be incubated at 37°C for up to 2 hours with agitation (500 rpm). Aliquots can be withdrawn at any desired time intervals. The reaction can be performed without radioactivity (Leu should be substituted with the same amount of water and T-mix with Leu (supplied with the Kit) should be used instead of one without).

For instance, the in vitro translation system (without RF depleating agents (e. g. Antibodies against RF1 from *Thermus thermophilus*) and nonsense codon suppressing agents (e. g. puromycin derivatives and/or suppressor tRNAs) of the protocol listed above comprises the following ingredients:
- 30S cell-free extract from E. coli (enzyme- and und ribosomal fraction);
- MgCl₂ 9-12 mM;
- DTT 10 mM;
- Amino acids, 200 µM each (For labelling, each amino acid can be applied as a 14C amino acid with a concentration of 100 µM (e.g. 14C-leucine))
- Rifampicin 0,02 mg/ml reaction mixture,
- Bulk-tRNA 600pg/ml reaction mixture,
- ATP,CTP,GTP,UTP, 1mM each,
- Phosphoenolpyruvate 10 mM;
- Acetylphosphate 10 mM;
- Pyruvatekinase 8 µg/ml reaction mixture;
- Plasmid 2 pmol/ml reaction mixture;
- T7 Polymerase 500 Units/ml reaction mixture;
- HEPES pH 7,6, 50 mM;
- Potassium acetate70 mM;
- Ammonium chloride 30 mM;
- EDTA pH 8,0 , 0,1 mM;
- Sodium azide 0,02 %;
- Polyethyleneglycol 4000 2 %;
- Protease inhibitors: aprotinin 10 µg/ml reaction mixture, leupeptin 5 µg/ml reaction mixture, pepstatin 5 µg/ml reaction mixture; and
- Folic acid 50 µg/ml reaction mixture.

### Example 7 which is not part of this invention: Affinity purification of the eGFP-Esterase fusion protein by immobilization on a TFK-coated matrix

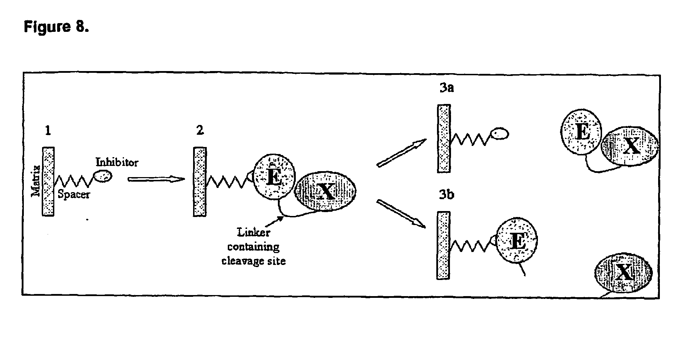

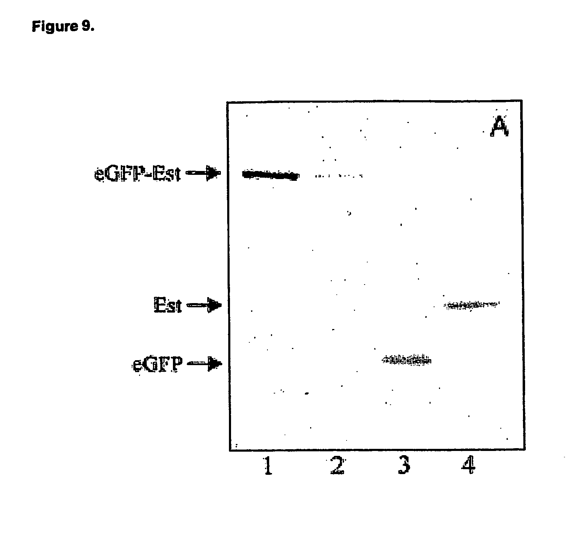

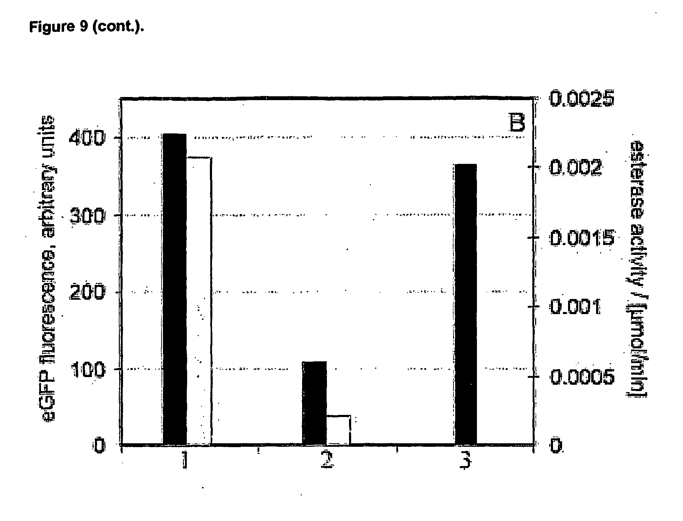

Esterase 2 from *Alicyclobacillus acidocaldarius* can be used as an affinity tag for purification of protein esterase fusions (Fig. 8). This is demonstrated in experiments presented in Fig. 9. The synthesis of eGFP-esterase fusion protein is demonstrated by SDS-PAGE and autoradiography of the [¹⁴C]Ieucine labeled protein (Fig 9A, lane 1). Trifluoromethyl-alkyl ketones are efficient competitive inhibitors of the esterases with the inhibition constant in µM range. Immobilized trifluoromethyl-alkylketones can be, therefore, used for affinity purification of esterases (Hanzlik (1987), J. Biol. Chem., 262, 13584-13591.). After addition of TFK-Sepharose to translation mixture the eGFP-esterase is almost completely removed from the supernatant (Fig. 9A, lane 2). Cleavage of eGFP from affinity matrix was achieved via the build-in protease sensitive linker by Factor Xa protease. Therefore, after this step the eGFP polypeptide appears in the supernatant (Fig. 9A; lane 3). For release of esterase from affinity matrix harsh conditions (95°C, 1 % SDS) had to be used. In the figure 9B the esterase activity and florescence of eGFP are demonstrated in different fractions of the affinity-purification steps. The synthesized fusion protein possess both activities (Fig. 9B; bar 1). After treatment with the TFK-Sepharose the supernatant has strongly diminishes esterase activity and low eGFP fluorescence due to immobilization of the fusion protein (Fig. 9B; bar 2). After protease cleavage the fluorescent eGFP appears in the supernatant whereas the esterase, as expected, remains attached to the matrix. Correspondingly, no esterase activity can be detected in the supernatant after protease treatment (Fig 9B; bar 3).

For affinity purification of the eGFP-Esterase fusion protein, the peGFP-Est2 plasmid was expressed *in vitro* as described above. The fluorescence at 507 nm of eGFP-esterase fusion protein was monitored at λₑₓ = 488 nm and 25°C directly in the translation mixture without dilution using a 150 µl quartz cell. The esterase activity was monitored by photometric assay in parallel with eGFP fluorescence assay. Then 200 µl of the translation mixture was incubated with 25 µl of TFK-matrix (trifluoromethyl ketone Sepharose CL-6B, prepared as described (Hanzlik (1987), J. Biol. Chem., 262, 13584-13591.)) equilibrated with 100 mM Na-phosphate, pH 7.5 at 37 °C for 4 hours. The TFK-matrix was spun down and the supernatant was analyzed for the eGFP fluorescence and the esterase activity. The remaining pellet of TFK-matrix was washed with 3 ml of 100 mM Na-phosphate pH 7.5 with 100 mM NaCl. Then the TFK-matrix was resuspended in 175 µl of 40 mM Tris, 200 mM NaCl, 4 mM CaCl₂, pH 8.0 and treated with 20 µg Factor Xa protease for 15 h at 23 °C. The TFK-matrix was spun down and the supernatant was analyzed for the eGFP fluorescence and the esterase activity. The remaining material was removed from TFK-matrix by boiling it for 5 min at 95 °C in 10 % SDS. The aliquots from each step of purification were also analysed by SDS-PAGE.

### Example 8: Cloning of the release factor 1 of Thermus thermophilus (T.th.RF1)

Degenerated primers were used to amplify a *prfA* specific probe from *Thermus thermophilus* genomic DNA by PCR. Preparation of *Th. thermophilus* genomic DNA and subsequent genomic PCR followed conventional protocols for mesophilic bacteria (Sambrook (2001), Molecular Cloning: A Laboratory Manual. Cold Spring Harbor Laboratory Press, NY, Vol. 1, 2, 3). A 50 mg bacterial pellet in an Eppendorf tube was resuspended in 565 µl TE buffer, 30 µl 10% SDS and 5 µl 20 mg/ml Proteinase K was added and incubated for 1 h at 37° C. Lysis was performed after addition of 100 µl 5 M NaCl and repeated uptaking and emptying the bacteria with a needle-equipped syringe by shearing forces. After addition of 80 µl of 10% Hexadecyltrimethylammoniumbromid (CTAB) in 0.7 M NaCl, 10 min incubation at 65° C and extraction with 700 µl Chloroform/Isoamylalkohol and 5 times with 700 µl Phenol/Chloroform/Isoamylalcolhol 25:24:1, genomic DNA was precipitated with Isoamylalcohol. 1µg DNA from the genomic DNA preparation was used in a 100 µl PCR reaction containing 5 µl of each 10 µM primer, 10 µl 15 mM MgCl_2 , 10 µl 10x Taq Pol buffer (100mM Tris-HCl pH 8.8, 500 mM KCI, 15 mM MgCl₂), 1 µl 1U/µl Taq Polymerase, and cycled 30 times with 30 s at 95°, 30 s at 60° and 60 s at 72° after an initial 5 min denaturation at 95°.
With the amplified fragment, a 3.0 kbp fragment could be identified carrying the complete *prfA* sequence. The fragment was cloned into the plasmid pBluescript KS+ and the resulting plasmid pBlueK4b was sequenced. The sequence identified is identical to that of the literature (Ito (1997), Biochimie. 79, 287-292) and is shown in SEQ ID NO: 3. The corresponding amino acid sequence is shown in SEQ ID No: 4.

### Example 9: Overexpression of the release factor 1 of Thermus thermophilus in E. coli and purification of the same

The *T.th.*RF1 protein was heterologous overexpressed in *E.coli* and purified to homogeneity as judged by SDS PAGE and Maldi mass spectroscopy. In brief, after cell lysis of RF1-overproducing *E.coli* cells by Lysozyme and/or Parr bomb treatment, the S100 supernatant from the ultracentrifugation was concentrated by AMS-precipitation, dialyzed against 50 mM Tris/HCl pH 7.5, 10 mM MgCl₂, 1 mM β-mercaptoethanol, 5% glycerol and used for Q-Sepharose FF (Amersham-Pharmacia) ionexchange chromatography running a gradient from 0 to 500 mM NaCl. RF1-containing fractions were pooled, 15 min at 65°C heat-treated removing most *E.coli* proteins - including heterologous *E.coli* RF1 - AMS-precipitated, dialyzed against 10 mM K-Phosphate buffer pH 6.8 and used for hydroxyapatite chromatography (Merck) running a gradient from 10 to 500 mM K-Phosphate pH 6.8. Pooled RF1-fractions were ammonium sulfate-precipitated, dialyzed against 100 mM sodium acetate pH 5.75 and used for a final ionexchange chromatography on EMD-SO₃ -Tentakel (Merck) running a gradient from 0 to 2 M KCI. Recovered *Thermus thermophilus* RF1 was ammonium sulfate-precipitated, dialyzed against 100 mM Tris/HCl pH 7.5, 100 mM KCl, 5% glycerol, and stored at -20°C after adding an equal volume pure Glycerol.

### Example 10: Preparation of polyclonal antibodies against the release factor 1 of Thermus thermophilus

Heterologous, in *E.coli* overexpressed and purified *Thermus thermophilus* RF1 was used to immunize two rabbits following the standard one month immunization-protocol at Eurogentec (Seraing, Belgium): A first immunization used the glycerinated protein mixed with incomplete Freund's adjuvant and a intradermic multisite injection at the rabbits back at day 0. Three boost immunizations at day 14, 30 and 60 followed with a small bleeding after 45 days and termination of the rabbits and final bleeding after 70 days. Vacutainer tubes were used to process blood samples and remove agglutinated blood clots.
After three boosts serum was collected, centrifuged and the polyclonal antibodies were stored at -20 °C.

### Example 11: Preparation and radioactive labelling of the puromycine-derivatives of the present invention

The puromycin derivatives used herein were synthesized by standard phosphoramidite chemistry (Berg, Tymoczko, Stryer, Biochemistry, 5th Edition, Freeman Co. New York, 2001 pp. 148-149) by Purimex, Staufenberg, Germany. The synthesis of the puromycin dinucleotide 5'-dC(N⁴-TEG-NH2)pPuromycin-3' was accomplished by coupling of N⁴ alkylamino synthon (dC(N4-TEG-NH-TFA)-phosphoramidit, provided by ChemGenes, Wilmington, MA01887 U.S.A., Cat-No. CLP-1329, Formula: 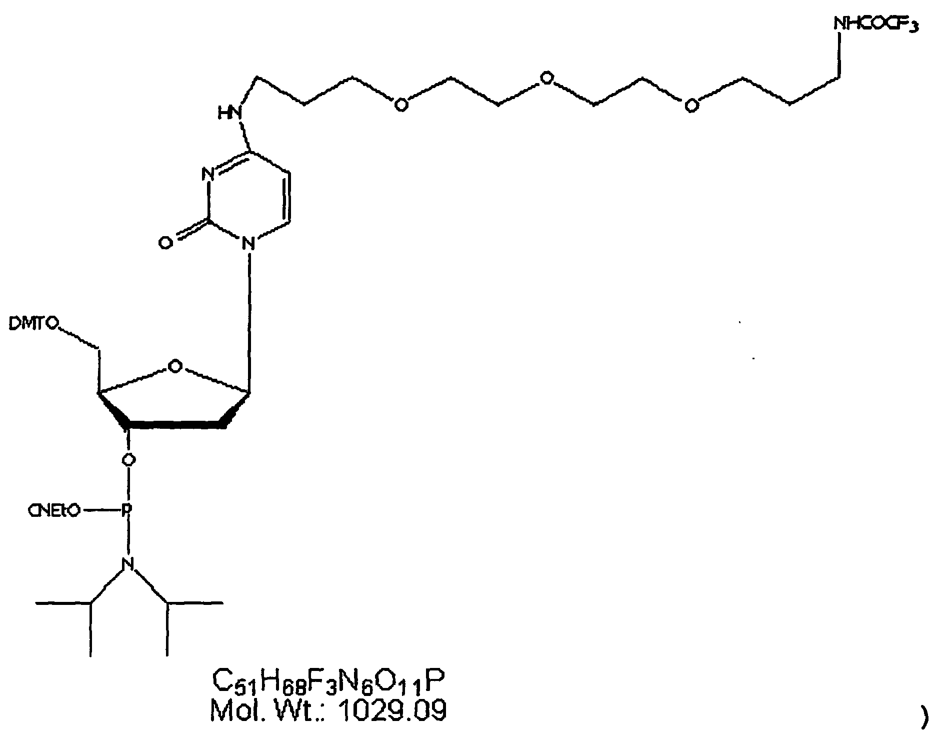 with 5'-Dimethoxytrityl-N-trifluroacetyl-puromycin,2'-succinyl-lcaa(long chain alkylamino)-CPG (provided by GlenResearch, Sterling, VA 20164 U.S.A., Cat.No. 20-4040-xx, Formula:

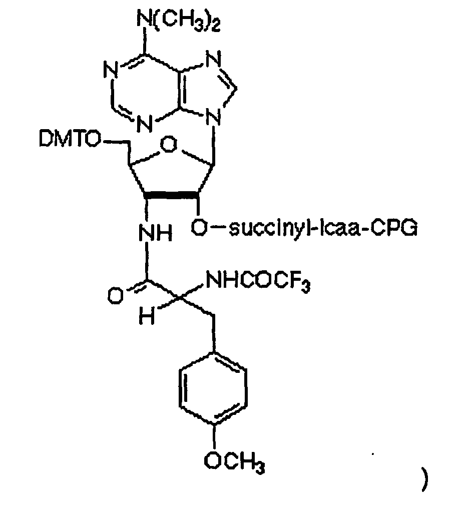

The resulting 5'-dimethoxytrityl-protected dinucleotide was cleaved from the CPG matrix by 32% ammonium hydroxide and left under this condition at 65°C for 1h in order to achieve total deprotection. Subsequently the dinucleotide was purified by HPLC and the trityl group was removed by treatment with 80% aqueous acetic acid solution. The final purification was achieved by two HPLC steps. The sample was concentrated by evaporation and desalted by passing through a SepPac cartridge. Concentration of the puromycin derivatives in the *in vitro* translation assay was 7µM, unless otherwise indicated.
To monitor the incorporation of puromycin-derivatives during translation reactions, the used puromycin derivatives were labelled with [γ₋³²P]ATP at the 5'-end by ³²P in the following way.
The reaction mixture (10 µl) contains 0.8 U/ul T4-polynucleotide kinase, 4 µM ATP, 0.2 µM [γ-³²P]ATP (10 µCi, 4950 mCi/mmol, Hartmann Analytic, Braunschweig, Germany), 4 µM puromycin-modified oligonucleotide (Purimex, Staufenberg, Germany) in T4-polynucleotid kinase buffer (70 mM Tris/HCl (pH 7.6), 10 mM MgCl₂, 5 mM DTT). Phosphorylation was carried out for 30 minutes at 37°C. Then entire volume of the reaction mixture was mixed with 6.6 µl of 100 µM puromycin derivative. The resulting solution (50 µM, 301 mCi/prnol) was used for *in vitro* translation experiments.

### Example 12: Depletion of the release factor 1 from E. coli in an E. coli cell-free translation system by precipitating and/or crosslinking said release factor 1 from E. coli with polyclonal antibodies against the release factor 1 of Thermus thermophilus and thereby increasing the incorporation of puromycine and/or its derivatives at the C-terminal nonsense codon of the esterase 2 (Est2) from Alicyclobacillus acidocaldarius

To further demonstrate the use of the esterase 2 of the present invention for monitoring and/or tracking the synthesis of a protein, polypeptide or peptide in a system in which the synthesis of a protein, polypeptide or peptide can occur, the following Experiment was performed. In order to improve C-terminal incorporation of puromycin, the E. coli release factor 1 (RF1) responsible for termination at the UAA and UAG stop codons was inactivated in the *in vitro* translation system from *E. coli* by rabbit antibodies specific against *Thermus thermophilus* RF1. Template DNA that encoded mRNA for synthesis of the esterase 2 from *Alicyclobacillus acidocaldarius* (Manco (1998), Biochem. J, 332 (Pt 1), 203-212.) and ended by UAG stop codon, was used.
The C-terminal labeling of the esterase was monitored by incorporation of Biotin-Puromycin into full-length esterase protein
Further, the attachment of Biotin-Puromycin to the esterase was demonstrated by immobilization of the resulting protein-puromycin-biotin conjugate to the surface of streptavidin-coated glass plates (Fig. 11).
At position 1 of Fig. 11, 1µL esterase purified from overexpressing *E. coli* strain (Manco (2000), Arch. Biochem. Biophys., 373, 182-192.) was applied to streptavidin-coated glass plates. At positions 2, 3 and 4 *in vitro* translation, programmed by esterase gene, was performed directly "on spot" in 1 µL translation mixture containing no puromycin derivative on the streptavidine-coated glass plates. The translation mixture without Biotin-CpPuromycin and RF1 antibody was placed on spot 2, translation mixture in the presence of Biotin-CpPuromycin on spot 3 and the translation mixture with both, Biotin-CpPuromycin and RF1 antibody on spot 4. After translation performed for 90 min. at 37°C in a cell free translation System as described herein the unbound components were removed by rinsing the plates with tap-water. The residual activity of biotinylated esterase bound to the streptavidine coated glass plates (Greiner Bio-One, Frickenhausen, Germany) was determined by applying 2 µL solution composed of 20 mg of 2-naphthyl acetate dissolved in 1 mL of acetone and 150 mg of Fast Blue BB salt suspended in 4 mL 100 mM Tris/HCl pH 7.5, to the surface of the plate. Esterase containing spots became brown-coloured after few minutes. The reaction was stopped by rinsing the gel in tap water.
As demonstrated in Fig. 11, the control experiments on spots 1 and 2 show no esterase activity. Only very little esterase activity could be detected on spot 3 where the translation was performed in the presence of biotin-puromycin. Obviously, the competition of the puromycin-derivative with RF1 for the AUG triplet-coded A-site prevented an effective incorporation. Only after inactivation of RF1 with antibodies (Fig 11, plate 4) significant amount of esterase has been immobilized.
The in vitro synthesis of the esterase 2 was performed according to the example 6, but in the presence of antibodies against RF1 of *T. thetmophilus* and a biotinylated puromycin derivative. As an example, a detailed protocol of the performed in vitro translation is listed below.

Protocol for *in vitro* translation (RiNA GmbH Kits) in the presence of antibodies against RF1 of *T. thetmophilus* and Puromycin derivatives:
For preparation of a 30 µl reaction mixture the following components should be combined on ice (given in order of mixing):
   1. 1.5 µl of the 100 nM template plasmid
   2. 2.4 µl of E-mix (red lid, supplied with the Kit)
   3. 9 µl of T-mix with Leu (blue lid, supplied with the Kit)
   4. 10.5 µl of S-mix (yellow lid, supplied with the Kit)
   5. 2 µl of rabbit anti-RF1 antiserum
   6. 4 µl of 50 µM solution of puromycin derivative (radioactively labelled)
The reaction mixture should be incubated at 37°C for up to 2 hours with agitation (500 rpm). Aliquots can be withdrawn at any desired time intervals.

For instance, the in vitro translation system (without RF depleating agents (e. g. Antibodies against RF1 from *Thermus thermophilus*) and nonsense codon suppressing agents (e. g. puromycin derivatives and/or suppressor tRNAs) of the protocol listed above comprises the same ingredients as listed herein-above (Example 6).

### Example 14: Preparation of the plasmid pEst2_amb155 comprising a cDNA encoding an AGC¹⁵⁵→TAG¹⁵⁵-mutated esterase 2 (Est2) from Alicyclobacillus acidocaldarius

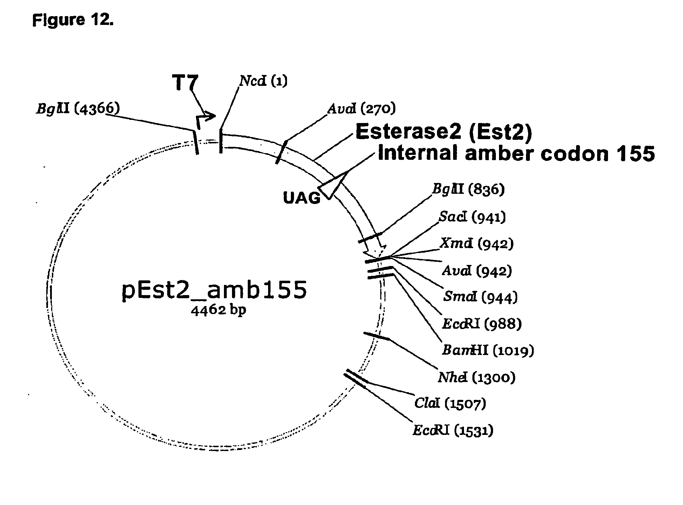

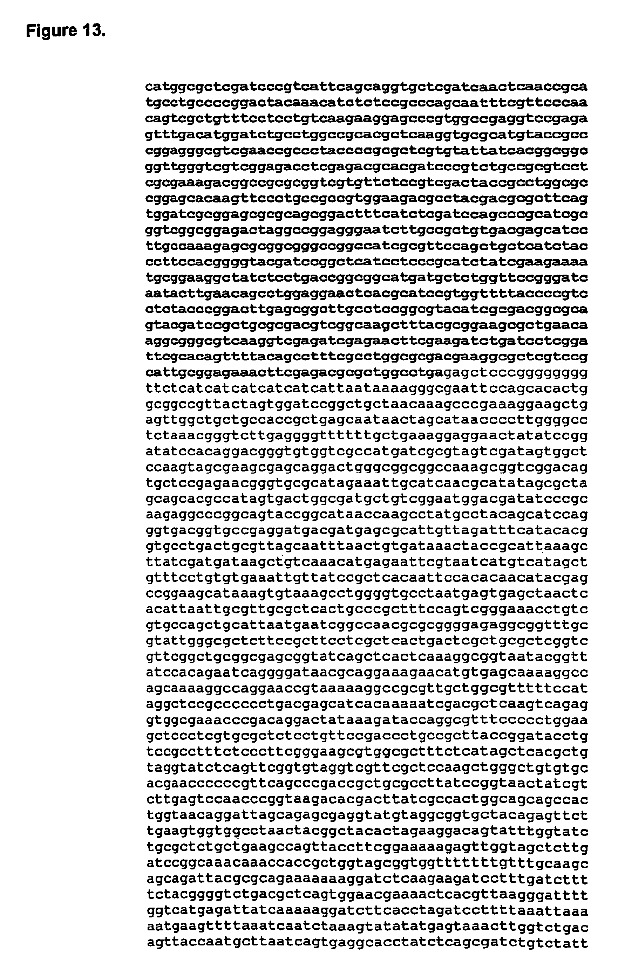

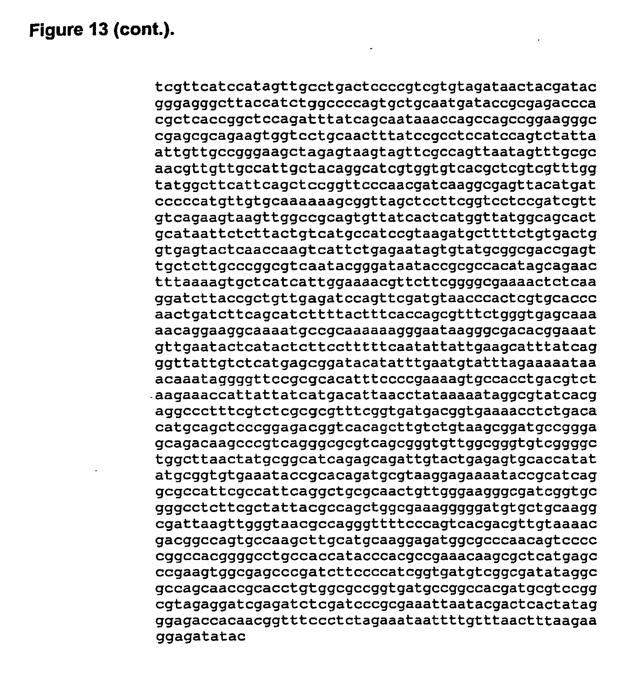

The ACG triplet in the est2 mRNA of *Alicyclobacillus acidocaldarius* esterase 2 coding for serine-155 was replaced by the RF1 stop codon UAG (amber), while the stop codon at the end of the mRNA was substituted for UGA (opal) codon that promotes RF2-dependent termination (Fig. 10A). Therefore, site-directed mutagenesis was performed on the esterase gene (Est2) in pIVEX_Est2 (pEst2) plasmid by the overlap extension method. Two separate PCR reactions was carried out using (1) T7 promotor primer (5'-TAATACGACTCACTATAGGG-3'; SEQ ID NO: 22) and EstS115x_rev (5'-ATTCCCTCCGGC**CTA**GTCTCCGCCGACCGCGATGC-3'; SEQ ID NO: 23); (2) EstS115x_for (5'-CGGTCGGCGGAGAC**TAG**GCCGGAGGGAATCTTGCC-3'; SEQ ID NO: 24) and T7 terminator primer (5'- CTAGTTATTGCTCAGCGGTG-3'; SEQ ID NO: 25). The mutated codons are bolded and the serine codon at position 155 of amino acid sequence of the esterase was changed to RF1 stop codon (TAG) mutation. The PCR fragments were fused by another PCR using T7 promotor and T7 terminator primers. The fused PCR product was digested with *NcoI*/*SacI* and ligated into *NcoI*/*SacI* digested pIVEX vector. The ligation mixture was transformed into *E.coli* strain XL-1 Blue. The plasmid DNA was isolated from clones and sequenced before use. The resulting plasmid pEst2_amb155 was used for *in vitro* translation. A map of pEst2_amb155 is shown in Fig. 12, the corresponding nucleotide sequence is shown in Fig. 13 (SEQ ID NO: 10).
The plasmid was purified as described herein above (Example 3)

### Example 15: Preparation of suppressor tRNA^{Ser(CUA)}

Within the present application, the used suppressor tRNA^{SerCUA} was prepared as follows.
The gene of tRNA^{SerCuA} was constructed by PCR using primers tser-amberl (5'-GGAATTC**TAATACGACTCACTATA***GGAGAGATGCC*-3'; SEQ ID NO: 26), tSer-amber2 (5'-*GTCCGTTCAGCCGCTCCGGCATCTCTCCTATAGTG*-3'; SEQ 10 NO: 27), tSer-amber3 (5'-*CTCCGGTTTTAGAGACCGGTCCGTTCAGCCGCTCC*-3'; SEQ ID NO: 28), tSer-amber4 (5'-*CCGGTAGAGTTGCCCCTACTCCGGTTTTAGAGACG* 3'; SEQ ID NO: 29), tSer-amber5 (5'-*GAGAGGGGGATTTGAACCCCCGGTAGAGTTGCCCC-*3'; SEQ ID NO: 30), tSer-amber6 (5'-AAGCTTGGATGGATCACC*TGGCGGAGAGAGGGGGATTTGAAC-*3'; SEQ ID NO: 31). Bolded letters are T7 promotor and italic letters are the gene of tRNA^{SerCUA}. The mutated anticodon is underlined. The conditions of the performed PCR were 95°C denaturation for 30 seconds, 50°C annealing for 30 seconds and 72°C polymerization for 30 seconds; 25 cycles were performed. The primer concentration was about 1 nM. DNTP concentration was 0.4 mM. The sequence of suppressor tRNA is based on a tRNA^{ser} from E.coli (tRNA databank number DS1660) with a CUA mutation from position 34 to 36. The PCR product was cloned in a pGEM-T vector. The resulting plasmid ptSer-amber was sequenced and used as a template for the following PCR. The PCR was performed with primers tSer-amber1 and M13_rev (5'-CAGGAAACAGCTATGACC-3'; SEQ ID NO: 32). The PCR product was digested with BstNl for a CCA end and used as the template for *in vitro* transcription. The transcripts were purified by urea polyacrylamide gel electrophoresis and stored at -20°C.

### Example 16: Depletion of the release factor 1 from E. coli in an E. coli cell-free translation system by precipitating and/or crosslinking said release factor 1 from E. coli with polyclonal antibodies against the release factor 1 of Thermus thermophilus and thereby increasing the incorporation of an (unnatural) amino acid delivered by aminoacyl suppressor tRNA^{CUA} at an internal nonsense codon of an AGC¹⁵⁵→TAG¹⁵⁵-mutated esterase 2 (Est2) from Alicyclobacillus acidocaldarius

To further demonstrate the use of the esterase 2 of the present invention for monitoring and/or tracking the synthesis of a protein, polypeptide or peptide in a system in which the synthesis of a protein, polypeptide or peptide can occur, additionally, the following Experiment was performed.

Using the construct of Example 14 as a template for *in vitro* protein synthesis, the suppression of the amber codon was studied by SDS-PAGE of the full-length esterase 2 production (Fig. 15B) and by measurement of the catalytic activity of the *in vitro* synthesized esterase (Fig. 15C). Translation of est2 mRNA(Ser-155) and est2 mRNA(amber-155) as measured by [¹⁴C]Ieucine incorporation into polypeptide chain provides a protein of 34.4 and 17.3 kDa (Fig. 15B), respectively, approximately with the same efficiency (Fig. 15A). The amber mutation in position 155 leads to complete termination and synthesis of 17.3 kDa protein void of esterase activity (Fig. 15B; lane 1 and Fig. 15C).

Addition of increasing amounts of amber suppressor tRNA^{Ser(CUA)} to the translation mixture that is programmed by est2 mRNA(amber-155) gives rise to the synthesis of the full size polypeptide chain. The required concentration of tRNA^{Ser(CUA)} for production of the full-length protein in maximal yield is about 5 µM (Fig 16A). This value is in the range of concentrations of tRNA isoacceptors in *E. coli* cells (Dong (1996), J. Mol. Biol. 260, 649-663.. Although, the amber suppressor Ser-tRNA^{Ser(CUA)}, with an anticodon complementary to UAG and presented in complex with EF-Tu·GTP and has the optimal prerequisites to enter the UAG-programmed A-site, it has still to compete with endogenous RF1. As demonstrated in Fig. 16A and C this competition starts to be efficient only at µM concentration of Ser- tRNA^{Ser(CUA)}. The cellular concentration of RF1 is similar to that of tRNA isoacceptors (Dong (1996), J. Mol. Biol. 260, 649-663.; Adamski (1994), J. Mol. Biol 238, 302-308.). However, during preparations of cellular extracts for *in vitro* translation the concentrations of all cellular components drop as compared to the situation in cytoplasm. Whereas the tRNA concentration in the *in vitro* translation system was adjusted by addition of bulk tRNA to 50 µM, the concentration of RF1 becomes about 50 fold lower as compared with the situation *in vivo.* It follows, that the average final concentration of a single aminoacyl-tRNA isoacceptor and RF1 in the *in vitro* translation mixture is about 1µM and 20 nM, respectively. The need for high Ser tRNA^{Ser(CUA)} concentrations to compete for RF1 probably reflects the different affinity for the ribosomal A-site of these alternative substrates. At concentrations higher than 1 µM, however, Ser-tRNA^{Ser(CUA)} already starts to compete also with near-cognate aminoacyl-tRNAs for codon-specified binding to the A-site and the serine becomes misincorporated into several other positions of the polypeptide chain. This leads to accumulation of errors and loss of protein functionality, i.e. inactive enzyme (Fig. 16C). At very high tRNA^{Ser(CUA)} concentrations the yield of the 17.3 and 34 kDa polypeptides drops, probably due to frameshifting and premature termination, and the [¹⁴C]leucine radioactivity becomes distributed between polypeptides of different lengths (Fig. 16A, lines 6 and 7).
Completely different is the situation in the absence of RF1 that can be efficiently deactivated by addition of antibodies against *Thermus thermophilus* RF1 to the *E. coli in vitro* translation system. Absence of RF1 leads to stimulation of UAG suppression by near-cognate endogenous tRNAs (compare Fig. 16A, line 1 and Fig. 16B, line 1). Thus, in the absence of RF1 (Fig. 16B) the synthesis of 17 kDa polypeptide substantially decreases as compared to the translation in the complete system (Fig. 16A) and only a small amount of mostly inactive, full-length protein is synthesized.
As compared to the complete system (Fig. 16A), in the absence of RF1 the concentration of tRNA^{Ser(CUA)} required to achieve full UAG suppression and synthesis of active full-length esterase 2 from est2 mRNA(amber-155) drops dramatically (Fig. 16B). Already at 24 nM tRNA^{Ser(CUA)} in the translation mixture the synthesis of the full-length (34 kDa) polypeptide becomes efficient. The esterase synthesized under these conditions is fully active. The yield of the synthesized enzyme and its activity are identical to the esterase obtained by translation of the wild-type est2 mRNA (Ser-155). The yield of active esterase remains high up to 1 µM concentration of tRNA^{Ser(CUA)} (Fig. 16C, bars 2-5). Further increase of the suppressor tRNA concentration in the translation mixture results in drop of protein production along with loss of enzymatic activity. In the high tRNA^{Ser(CUA)} concentration range there is a coincidence between the data presented in Fig. 16A and 16B.

Thus, it was demonstrated that in the absence of RF1 the suppressor Ser-tRNA^{Ser(CUA)} is efficiently bound to the A-site of UAG-programmed ribosomes. This leads to complete suppression of UAG codon and to incorporation of the catalytically essential serine-155 into the enzyme. At high Ser- tRNA^{Ser(CUA)}EF-Tu·GTP concentrations, the competition with other aminoacyl-tRNA-EF-Tu·GTP ternary complexes leads to misreading of near-cognate codons and results in synthesis of error prone or incomplete polypeptide chains void of enzymatic activity. Thus, the use of est2 mRNA(amber 155) as a template and the possibility to deactivate the endogenous RF1 in the *in vitro* translation system by RF1 antibodies permits an optimal adjustment of RF1 and tRNA^{Ser(CUA)} concentrations to achieve a complete suppression and at the same time a maximal retention of enzymatic activity of the esterase.

The kits used for *in vitro* translation experiments within this example were evaluation size transcription/translation kits from RiNA GmbH (Berlin, Germany) and the reaction was performed according to the manual provided by the supplier. [¹⁴C]L-Leucine (54 mCi/mmol) was added up to 160 µM along with leucine resulting in 0.5 mM total concentration. The templates pEst2 and pEst2_amb155 were added up to 5 nM concentrations. The reaction was performed at 37 °C with agitation. Aliquots, 3µL, were withdrawn at different time intervals (up to 2 hours) and the newly synthesized protein was determined by radioactivity measurement in 10% trichloroacetic acid precipitate. Protein composition was analysed by SDS-PAG). The gels were fixed with 15% formaldehyde in 60% methanol and stained with Coomassie Blue G-250. The dried gels were exposed to an imaging plate for radioactivity analysis with the Phosphorlmager SI (Molecular Dynamics, Sunnyvale, USA).
The in vitro translations were performed according to the protocol shown in example 6 with minor modifications. As an example a detailed protocol of the in vitro translation performed in the presence of anti-RF1 (*T. thermophilus*) antibodies and suppressor tRNA is listed below.

Protocol for *in vitro* translation (RiNA GmbH Kits) in the presence of anti-RF1 (*T*. *thermophilus*) antibodies and suppressor tRNA:
For preparation of a 30 µl reaction the following components should be combined on ice (given in order of mixing):
   1. 5.1 µl of 926 µM [¹⁴C]Leu (54 mCi/mmol, Amersham)
   2. 1 µl 10 mM Leu (supplied with the Kit)
   3. 2.4 µl of E-mix (red lid, supplied with the Kit)
   4. 9 µl of T-mix without Leu (blue lid, supplied with the Kit)
   5. 10.5 µl of S-mix (yellow lid, supplied with the Kit)
   6. 2 µl of rabbit anti-RF1 antiserum
   7. 0.5 µl of 1.52 µM tRNA^{Ser(CUA)} (can be varied in 20 fold range)
   8. 0.1 µl of the 1.9 µM template plasmid
The reaction mixture should be incubated at 37°C for up to 2 hours with agitation (500 rpm). Aliquots can be withdrawn at any desired time intervals.

For instance, the in vitro translation system (without RF depleating agents (e. g. Antibodies against RF1 from *Thermus thermophilus*) and nonsense codon suppressing agents (e. g. puromycin derivatives and/or suppressor tRNAs) of the protocol listed above comprises the same ingredients as listed herein-above (Example 6).

### Example 17: Preparation of the plasmid pIVEX2.3d-Nox-Est2 (pNox-Est2) for expression of a fusion protein comprising NADH oxidase (Nox) from Thermus thermophilus, esterase 2 from Alicyclobacillus acidocaldarius and a Factor Xa-cleavable link.

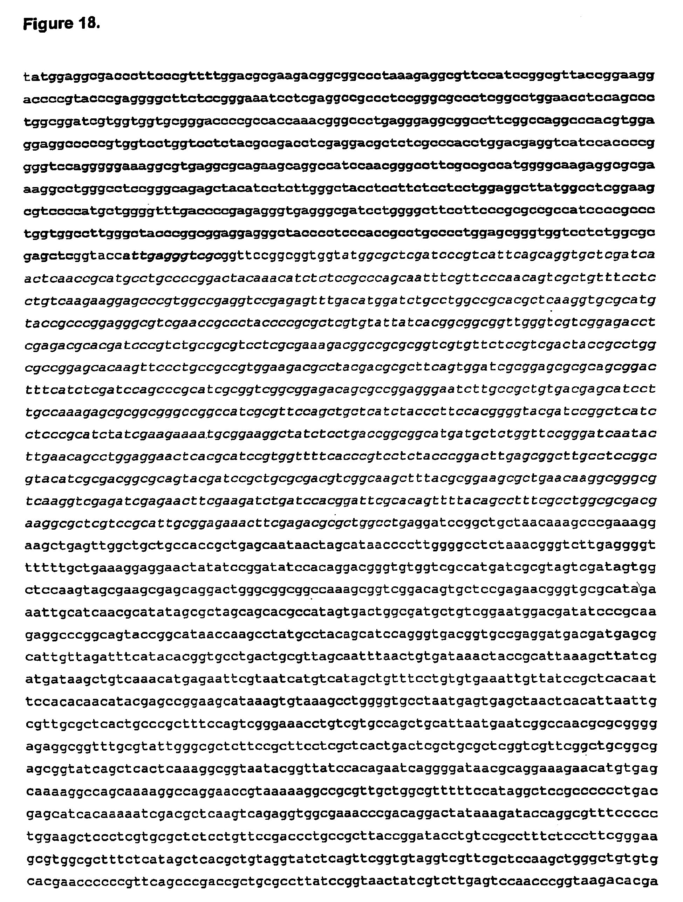

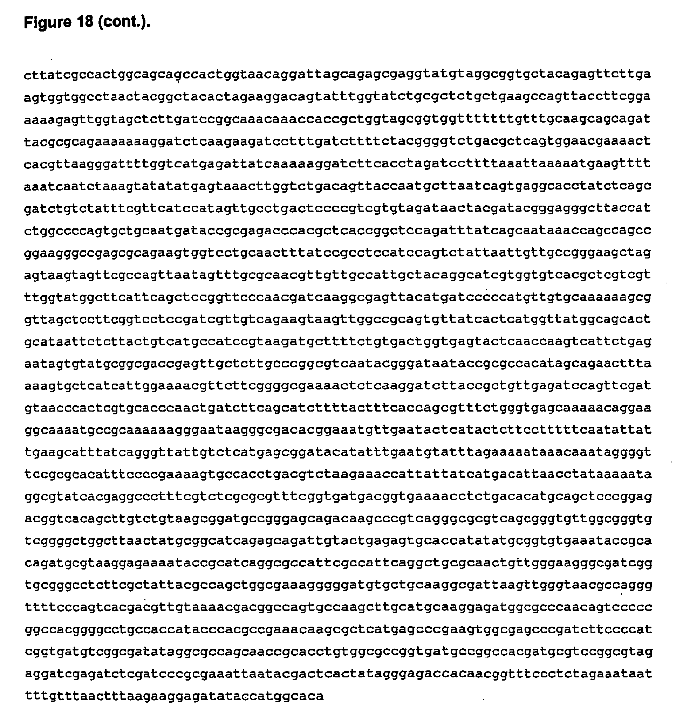

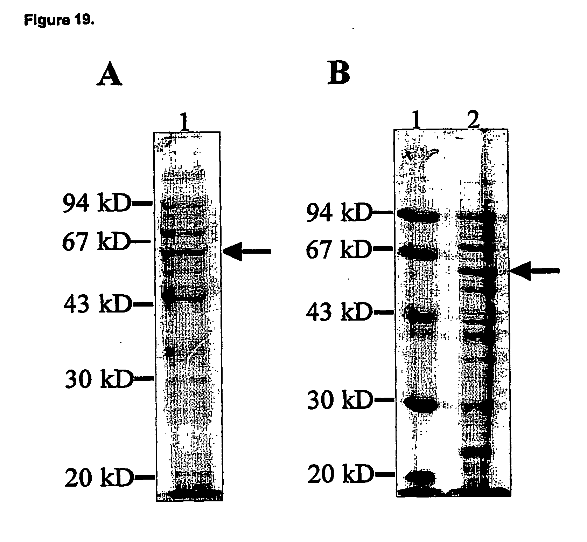

Pasmid pT7SCII, containing the gene of the esterase (Est2) was kindly provided by G. Manco, Napples, Italy (Manco (1998), Biochem. J, 332 (Pt 1), 203-212.). The gene was amplified by PCR with the primers Est2CT for (5'-GAGCTCGGTACC**ATTGAGGGTC**GCGGTTCCGGCGGTGGTATGGCGCTCGATC CC-3'; SEQ ID NO: 20) and **Est2CT_rev** (5'-GGATCC**TCA**GGCCAGCGC-3'; SEQ ID NO: 21). The primers create the *SacI* and the *BamHI* cleavage sites (underlined letters) upstream and downstream of the Est2, respectively. The primer **Est2CT_for** contains the cleavage site of protease Factor Xa coding sequence (bold letters) and the primer **Est2CT_rev** contains the UAG stop codon (bold letters). The PCR product was sequenced and cloned into the pIVEX2.3d vector through *SacI* and *BamHI* cleavage sites. The resulting plasmid pEst was used as a parental expression vector for further cloning.
The gene of NADH oxidase (Nox) from *Thermus thermophilus* was amplified from the plasmid pTthnadox310 (Lehrstuhl Biochemie, University of Bayreuth, Germany, UniPort Q60049) by PCR with the primers **Nox_for** (5'-CATATGGAGGCGACCCTTCCCGTTTTG-3'; SEQ ID NO: 33) and **Nox_rev** (5'-GAGCTCGCGCCAGAGGACCACCCGCTCCA GGG-3'; SEQ ID NO: 34). The primers introduce the *NdeI* and the *SacI* cleavage sites (underlined.letters) upstream and downstream of the Nox, respectively. The PCR product was sequenced and cloned into the pEst2 vector resulting in pNox-Est2 plasmid. The plasmid can be used for *in vitro* translation and *in vivo* expression. A map of pNox-Est2 is shown in Figure 17 and the corresponding nucleotide sequence is shown in Figure 18 (SEQ ID NO: 11). The *in vitro* expression of the Nox-Est2 fusion protein was achieved with EasyXpress Protein Synthesis Kits from Qiagen (Figure 19A). The *in vivo* expression of the Nox-Est2 fusion protein was achieved in E. *coli* strain BL21 (DE3) (Figure 19B).

### Example 18: Preparation of the plasmid pIVEX2.3d-EF-Tu-Est2 (pTu-Est2) comprising a cDNA encoding a fusion protein comprising elongation factor Tu from Thermus thermophilus, esterase 2 from Alicyclobacillus acidocaldarius and a factor Xa cleavable link and expression of the fusion protein.

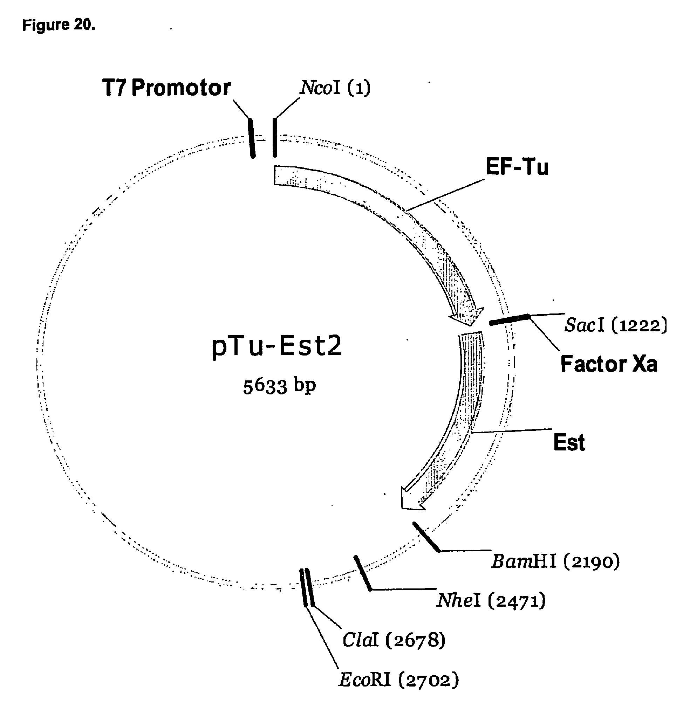

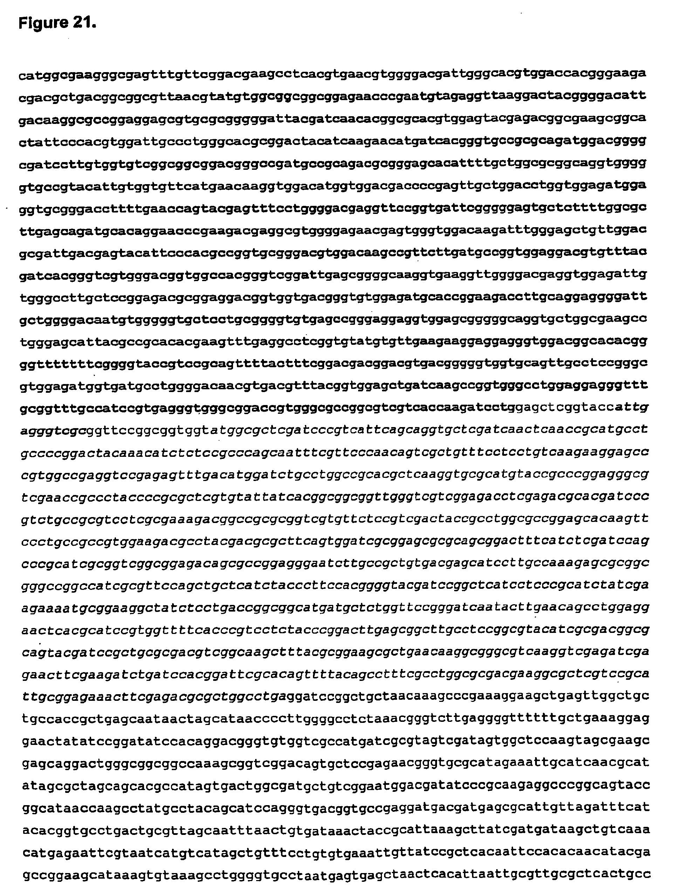

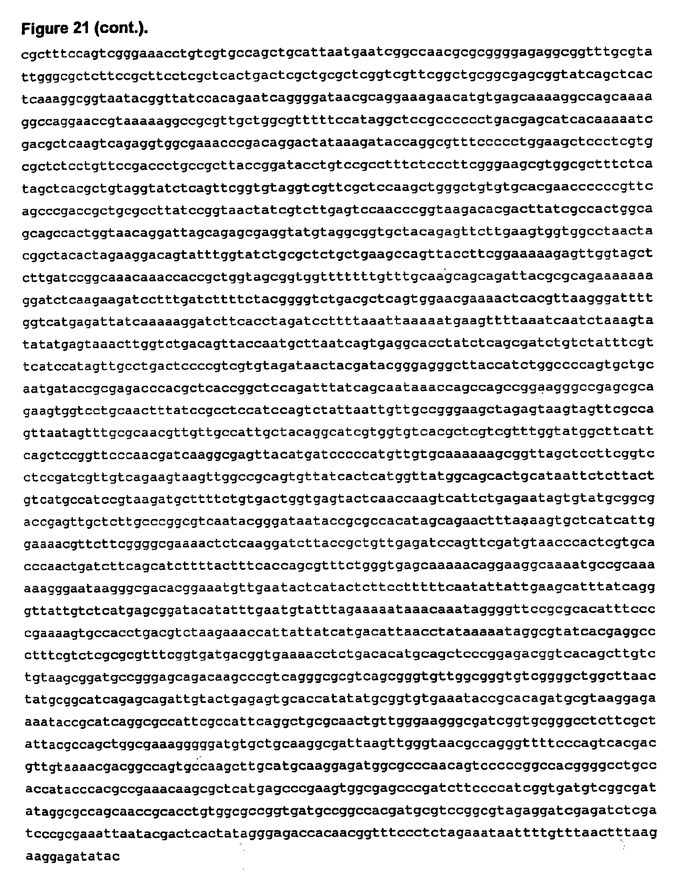

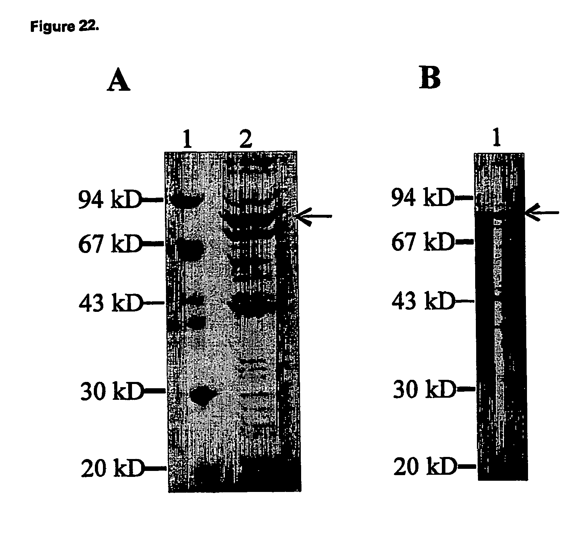

The gene of elongation factor Tu (EF-Tu) from *Thermus thermophilus* was amplified from the plasmid pGEM-T-EF-Tu (Lehrstuhl Biochemie, University of Bayreuth, Germany, UniPort P60338) by PCR with the primers Tu_for (5'-CCATGGCGAAGGGCGAGTTTGTTCGGACG-3'; SEQ ID NO: 35) and Tu_rev (5'-GAGCTCCAGGATCTTGGTGACGACGC CGGCGC-3'; SEQ ID NO: 36). The primers introduce the *NcoI* and the *SacI* cleavage sites (underlined letters) upstream and downstream of the EF-Tu, respectively. The PCR product was sequenced and cloned into the pEst2 vector resulting in pTu-Est2 plasmid. The plasmid can be used for *in vitro* translation and *in vivo* expression. A map of pTu-Est2 is shown in Figure 20 and the corresponding nucleotide sequence is shown in Figure 21 (SEQ ID NO: 12). The *in vitro* expression of the EF-Tu-Est2 fusion protein was achieved with EasyXpress Protein Synthesis Kits from Qiagen (Figure 22A). The *in vivo* expression of the Tu-Est2 fusion protein was achieved in *E. coli* strain BL21 (DE3) (Figure 22B).

### Example 19: Preparation of the plasmid pIVEX2.3d-EF-Ts-Est2 (pTs-Est2) for expression of a a fusion protein comprising elongation factor Ts from Thermus thermophilus, esterase 2 from Alicyclobacillus acidocaldarius and a factor Xa cleavable link.

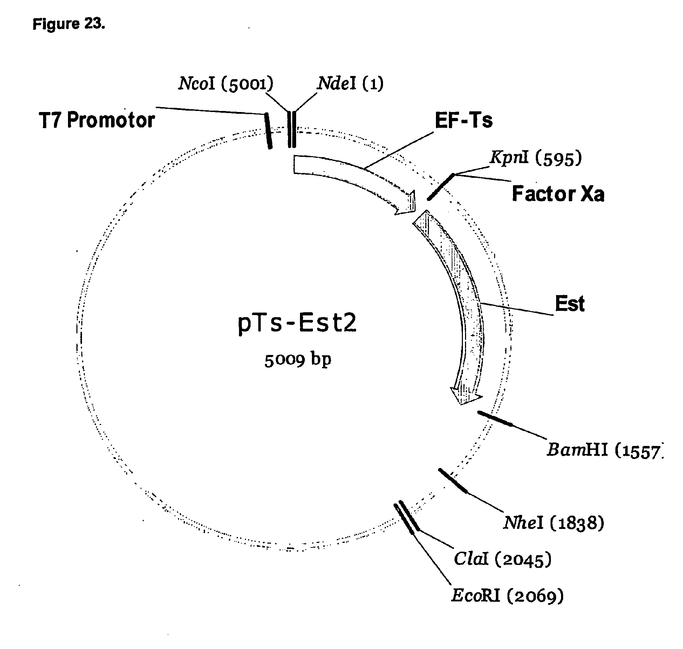

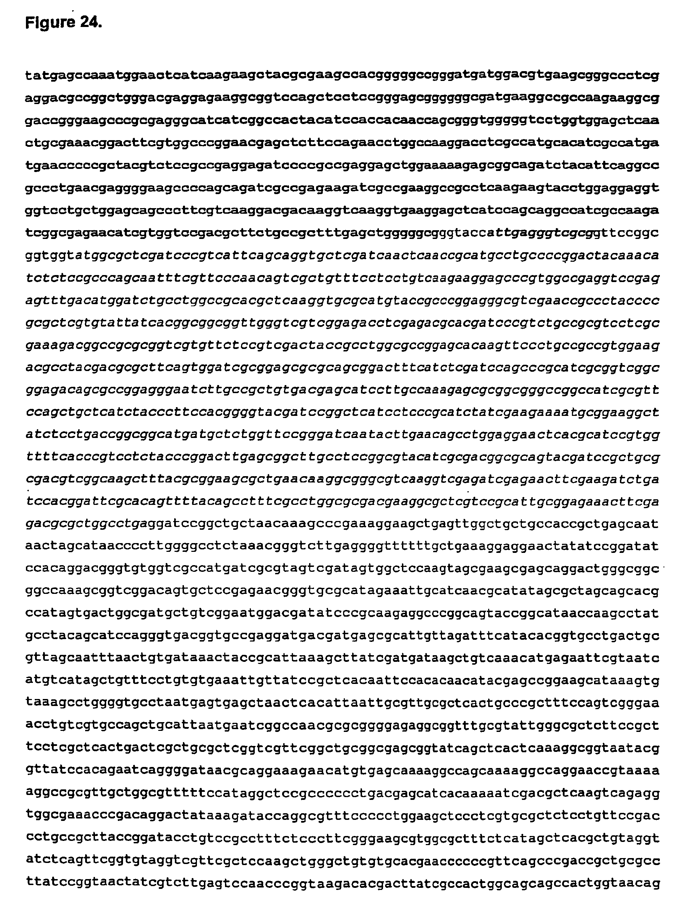

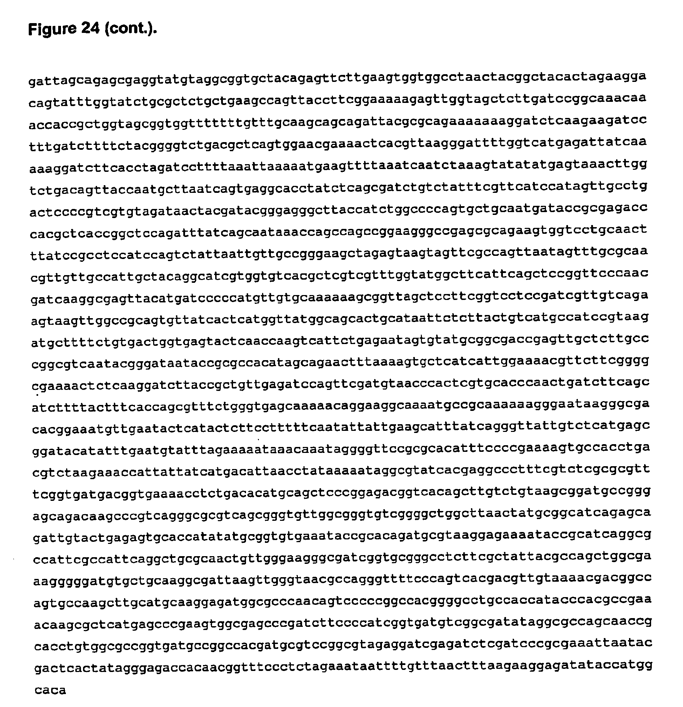

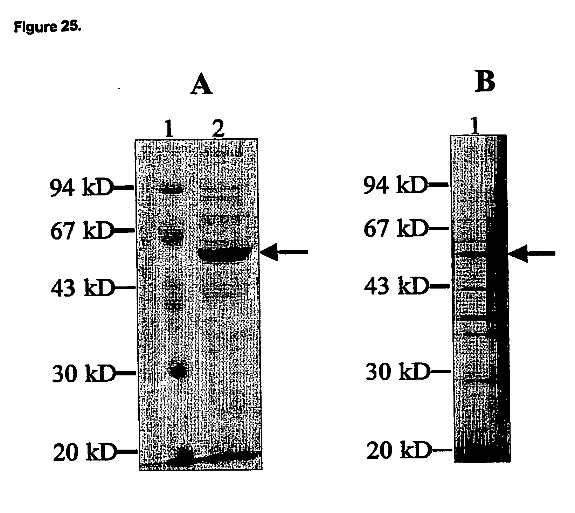

The gene of elongation factor Ts (EF-Ts) from *Thermus thermophilus* was amplified from the plasmid pET-Ts7 (Lehrstuhl Biochemie, University of Bayreuth, Germany, UniPort P43895) by PCR with the primers **Ts_for** (5'-CATATGAGCCAAATGGAACTCATCAAGAAGC-3'; SEQ ID NO: 37) and **Ts_rev** (5'-GGTACCCGCCCCCAGCTCAAAGCGG C-3'; SEQ ID NO: 38). The primers introduce the *NdeI* and the *KpnI* cleavage sites (underlined letters) upstream and downstream of the EF-Ts, respectively. The PCR product was sequenced and cloned into the pEst2 vector resulting in pTs-Est2 plasmid. The plasmid can be used for *in vitro* translation and *in vivo* expression. A map of pTs-Est2 is shown in Figure 23 and the corresponding nucleotide sequence is shown in Figure 24 (SEQ ID NO: 13). The *in vitro* expression of the EF-Ts-Est2 fusion protein was achieved with EasyXpress Protein Synthesis Kits from Qiagen (Figure 25A). The *in vivo* expression of the EF-Ts-Est2 fusion protein was achieved in *E. coli* strain BL21 (DE3) (Figure 25B).

### Example 20: Preparation of the plasmid pIVEX2.3d-Exportin-t-Est2 (pExp-Est2) for expression of human exportin-t, esterase 2 from Alicyclobacillus acidocaldarius and a factor Xa cleavable link fusion protein.

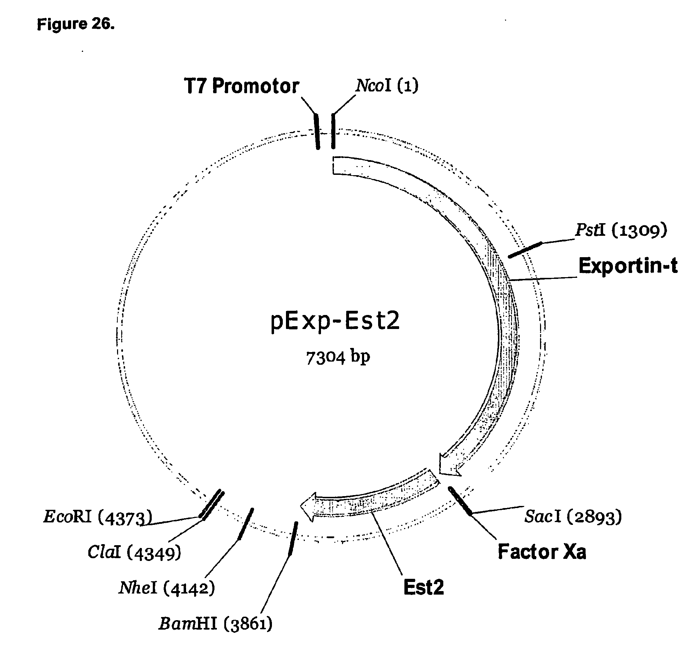

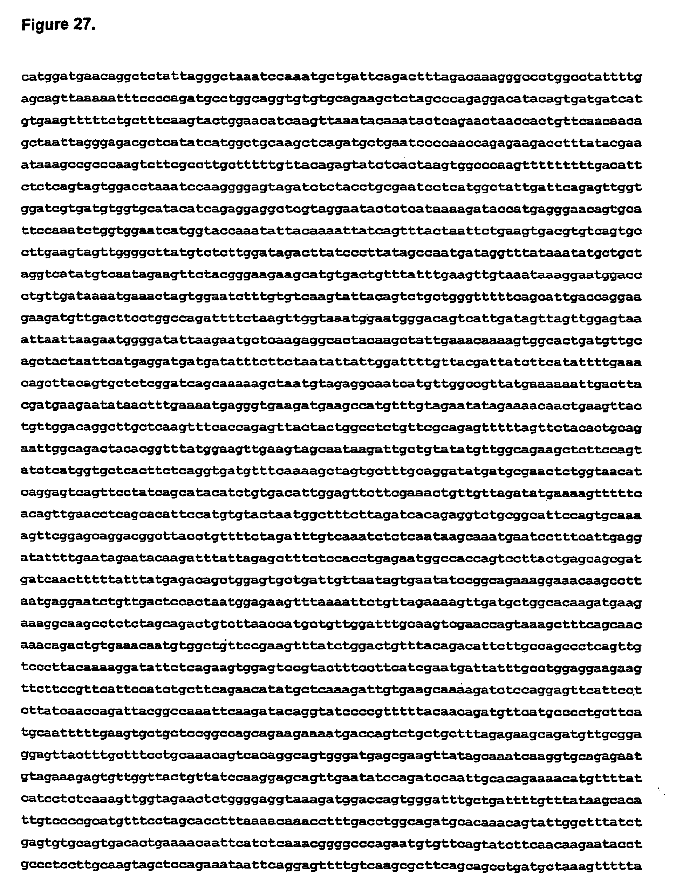

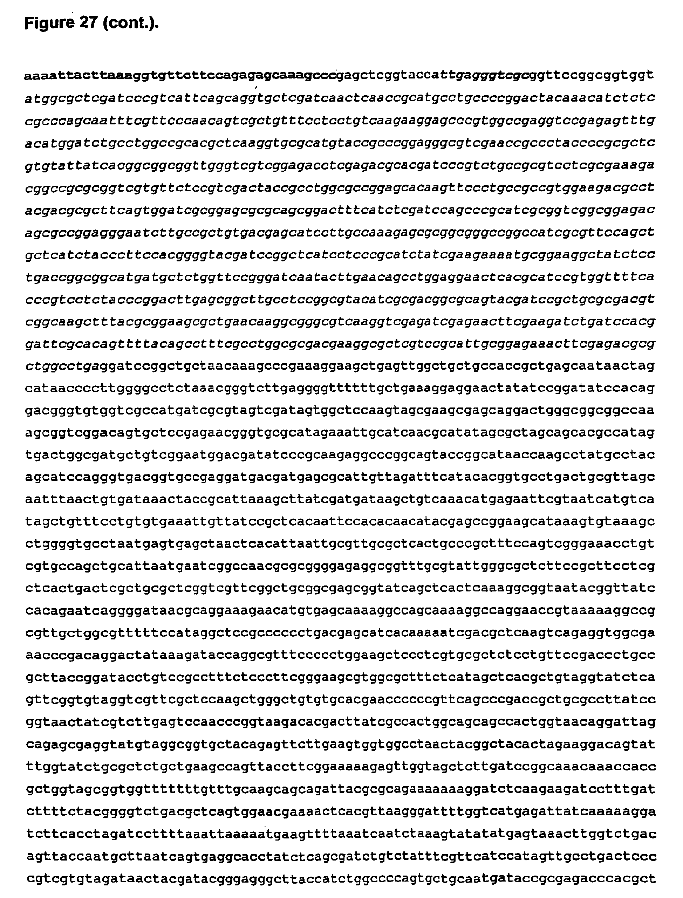

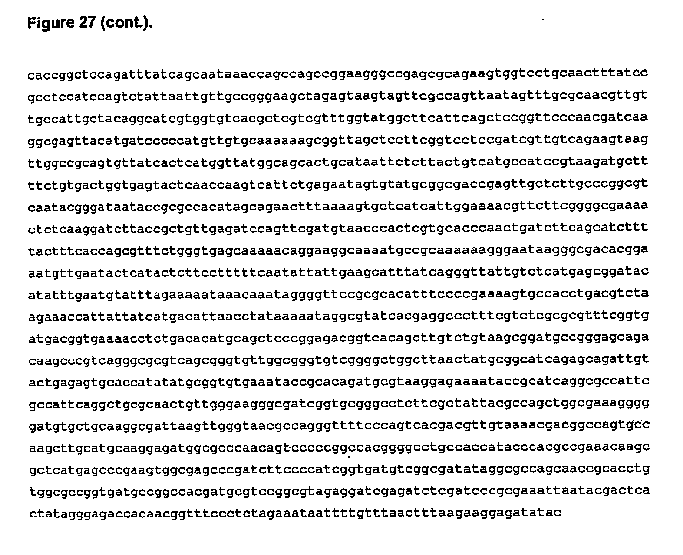

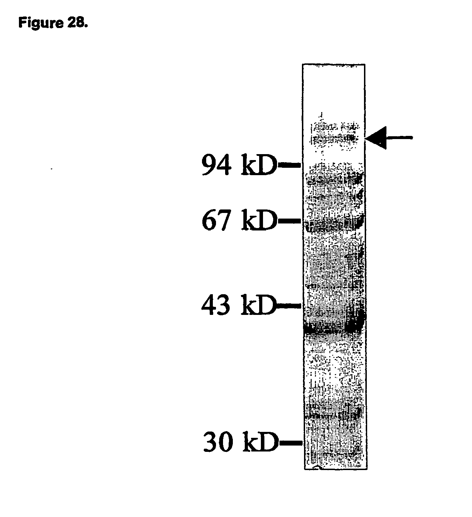

The gene of Exprotin-t from *Human* was amplified from the plasmid pExportin-t (Lehrstuhl Biochemie, University of Bayreuth, Germany, UniPort 043592) by PCR with the primers Exportin_for (5'- CCATGGATGAACAGGCTCTATTAGGGC-3'; SEQ ID NO: 39) and Exportin_rev (5'-GAGCTCGGGCTTTGCTCTCTGGAAGAACAC-3'; SEQ ID NO: 40). The primers introduce the *NcoI* and the *SacI* cleavage sites (underlined letters) upstream and downstream of the Exportin-t, respectively. The PCR product was sequenced and cloned into the pEst2 vector resulting in pExp-Est2 plasmid. The plasmid was used for *in vitro* translation and *in vivo* expression. A map of pExp-Est2 is shown in Figure 26 and the corresponding nucleotide sequence is shown in Figure 27 (SEQ ID NO: 14). The *in vitro* expression of the Exportin-t-Est2 fusion protein was achieved with EasyXpress Protein Synthesis Kits from Qiagen (Figure 28). The yield of the fusion protein expression is low, probably due to use of heterologous system where E.coli cells (or extracts) are expected to synthesize a human protein. On the other hand the sensitive detection with esterase assay allows for optimization of conditions to achieve higher expression.

### Example 21: Preparation of the plasmid pET28c-S2001-Est2 (pET-S2001-Est2) expression of a fusion protein consisting of putative nuclease S2001 from Sulfolobus solfataricus, esterase 2 from Alicyclobacillus acidocaldarius and a factor Xa cleavable link.

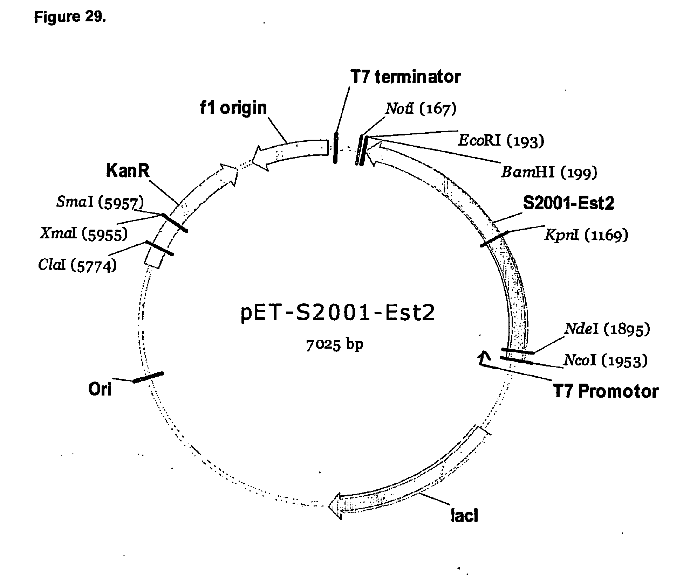

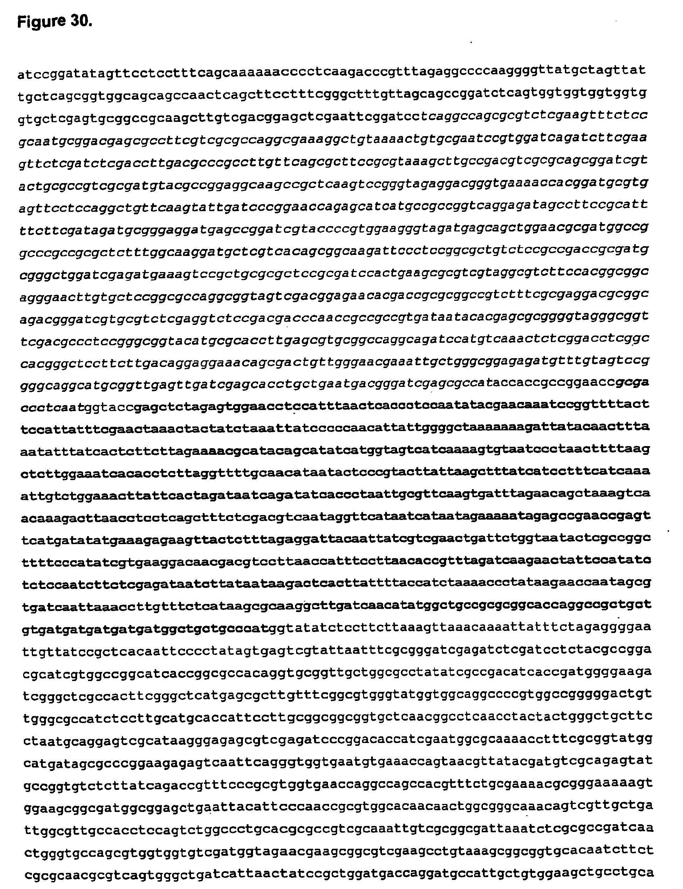

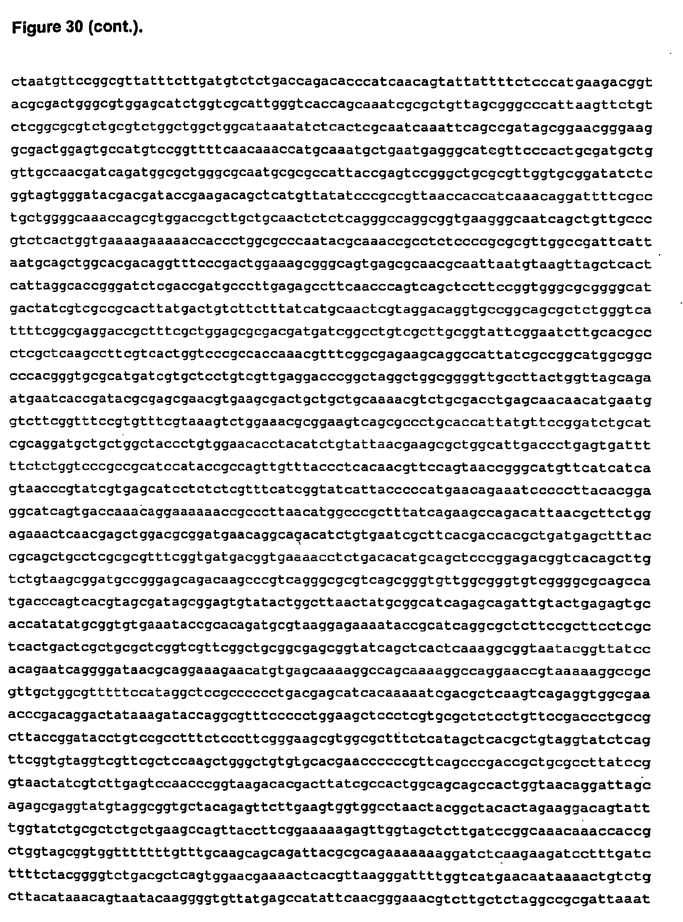

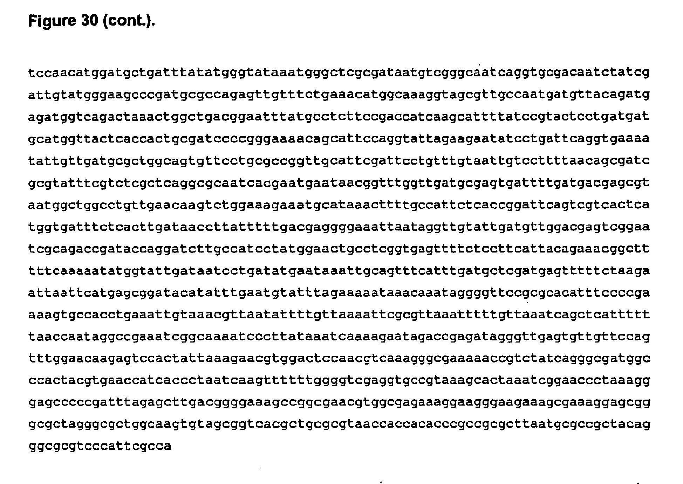

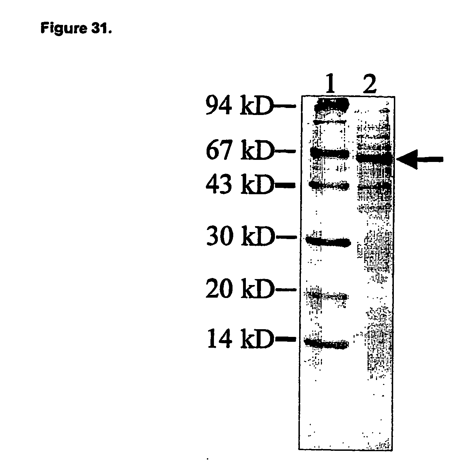

The gene of putative nuclease S2001 from *Sulfolobus solfataricus* was amplified from the plasmid pET28c-S2001 (Lehrstuhl Biochemie, University of Bayreuth, Germany, NCBI AAK 42190) by PCR with the primers **S2001_for** (5'-CCATGGGCAGCAGCCATCATCATCATCATCACAGCAGCGGCCTGGTGCCGCGC GGCAG-3'; SEQ ID NO: 41) and **S2001_rev** (5'-GGTACCGAGCTCTAGAGTGGAACCTCC-3'; SEQ ID NO: 42). The primers introduce the *NcoI* and the *KpnI* cleavage sites (underlined letters) upstream and downstream of the nuclease S2001, respectively. The PCR product was sequenced and cloned into the pEst2 vector resulting in pS2001-Est2 plasmid. The S2001-Est2 gene was cut from pS2001-Est2 plasmid by *NcoI* and *BamHI* and cloned into pET-28c expression vector. The resulting plasmid pET-S2001-Est2 can be used for *in vivo* expression in *E. coli* strain Rosetta (DE3, pLysS). A map of pET-S2001-Est2 is shown in Figure 29 and the corresponding nucleotide sequence is shown in Figure 30 (SEQ ID NO: 15). The *in vivo* expression of the S2001-Est2 fusion protein was - achieved in *E. coli* strain Rosetta (DE3, pLysS) (Figure 31).

### Example 22 which is not part of this invention: Affinity purification of in vivo expressed Nox-Est2 fusion protein

Cells that expressed the Nox-Est fusion protein were harvested and resuspended in 10 ml of 50 mM Tris-HCl pH 7.5, 300 mM NaCl, 1 mM 2-mercaptoethanol, 1 mM EDTA, 5% glycerol, disintegrated by mild ultrasonication and centrifuged at 20,000 g for 15 minutes at 4°C. The supernatant was loaded on the 5 ml TFK-Sepharose column which was equilibrated with the above buffer and washed with the buffer to remove all cellular proteins and other components. The Nox-Est fusion protein was then eluted with 5 ml 200 mM 1,1,1-Trifluoro-3-(2-hydroxy-ethylsulfanyl)-propan-2-one (F₃C-CO-CHᵣS-CHᵣCH₂-OH) in the same buffer. The pooled fractions were dialysed against 20 mM Tris-HCI pH 7.5, 50 mM NaCl, 1 mM 2-mercaptoethanol, 1 mM EDTA, 5% Glycerol. The protein concentration was determined by Roti^{®}-Nanoquant (Roth, Karlsruhe, Germany).
The fusion protein purified by this single step of affinity elution gave a single protein band with an apparent molecular weight of 58.5 kD based on coomassie blue stained SDS-PAGE (Figure 32a). 1.5 mg of purified fusion protein was obtained from 1 L cell culture. The esterase-fused protein can be isolated also by cleavage with factor Xa directly from the TFK-Sepharose column. After cleavage, the esterase part of fusion protein still remains bound to TFK-Sepharose while Factor Xa and Nox are eluted from the column (Figure 32b).

### Example 23: Materials employed in this study

Within the present application, materials were purchased as follows.
Taq polymerase was from Qiagen (Hilden, Germany), T4-DNA-Ligase from Promega (Mannheim, Germany), Factor Xa protease and restriction enzymes were from NewEngland Biolabs (Frankfurt, Germany). Fast Blue BB Salt, p-Nitrophenyl acetate and β-Naphthyl-acetate were from Fluka (Steinheim, Germany). 5-(and 6-) Carboxy-2',7'-dichlorofluoresceine diacetate was from Molecular probes (Eugene, USA). Other analytical grade chemicals were obtained from Roth (Karlsruhe, Germany). Radioactive [¹⁴C]leucine (54 mCi/mmol) was from Amersham, Life Sciences (Freiburg, Germany).

The present invention refers to the following nucleotide and amino acid sequences:
SEQ ID No. 1:
   Nucleotide sequence encoding esterase 2 from *Alicyclobacillus acidocaldarius:*
SEQ ID No. 2:
   Amino acid sequence of esterase 2 from *Alicyclobacillus acidoca*/*darius:*
   With respect to SEQ ID NO: 2, it is of note that the amino acid residues indicated with X (position 53, 64, 210 and 211) are methionine residues (Met (M)) as encoded by the corresponding codon triplet "ATG" as shown in SEQ ID NO:1. The amino acid sequence corresponding to SEQ ID NO: 2 and having methionine residues (Met (M)) at amino acid position 53, 64, 210 and 211 is shown in SEQ ID NO: 62 of the sequence listing.
SEQ ID No. 3:
   Nucleotide sequence encoding release factor 1 from *Thermus thermophilus*
SEQ ID No. 4:
   Amino acid sequence of release factor 1 from *Thermus thermophilus*
SEQ ID No. 5:
   Nucleotide sequence encoding peptide chain release factor 1 (RF-1) - Escherichia coli, Escherichia coli O6, Escherichia coli O157:H7, and Shigella flexneri.
SEQ ID No. 6:
   Amino acid sequenze of peptide chain release factor 1 (RF-1) - Escherichia coli, Escherichia coli O6, Escherichia coli O157:H7, and Shigella flexneri.
SEQ ID No. 7:
   Nucleotide sequence encoding the linker between Est2 and eGFP encoded by the plasmid peGFP-Est2 gagctcggtaccattgagggtcgcggttccggcggtggt
SEQ ID No. 8:
   Nucleotides sequence of the plasmid peGFP-Est2
SEQ ID No. 9:
   Nucleotide sequence of the plasmid pEst2

Sequences were retrieved from www.expasy.ch with entry from swiss prot database and entry from TrEMBL database.

## Claims

1. Use of a thermostable esterase of prokaryotic origin for monitoring and/or tracking the synthesis of a protein, polypeptide or peptide in a cell-free translation system or in an in vivo expression system in which the synthesis of a protein, polypeptide or peptide can occur, wherein said monitoring and/or tracking comprises the detection of the enzymatic function or activity of said esterase.

2. The use according to claim 1, wherein said esterase is a single chain esterase.

3. The use according to claim 1 or 2, wherein said esterase is from Alicyclobacillus acidocaldarius.

4. The use according to any one of claims 1 to 3, wherein said esterase is the esterase 2 from Alicyclobacillus acidocaldarius.

5. The use according to any one of claims 1 to 4, wherein said esterase is selected from the group consisting of:
(a) an esterase encoded by a nucleotide sequence comprising a nucleotide sequence as shown in SEQ ID NO: 1;
(b) an esterase encoded by a nucleotide sequence coding for a polypeptide comprising an amino acid sequence as shown in SEQ ID NO: 2 or SEQ ID NO: 62;
(c) an esterase encoded by a nucleotide sequence of a nucleic acid molecule that hybridizes to the complement strand of a nucleic acid molecule comprising an nucleotide sequence as defined in (a) or (b) and which catalyses the cleavage of an ester into an alcohol and an carboxylic acid;
(d) an esterase which comprises an amino acid sequence as shown in SEQ ID NO: 2 or SEQ ID NO: 62;
(e) an esterase which comprises an amino acid sequence which is at least 60% identical to the full length amino acid sequence as shown in SEQ ID NOS: 2 or SEQ ID NO: 62; and
(f) an esterase encoded by a nucleotide sequence which is degenerated to a nucleotide sequence as defined in any one of (a) to (c).

6. The use according to any one of claims 1 to 5, wherein said esterase is comprised in a fusionprotein, fusionpolypeptide and/or fusionpeptide to be synthesized.

7. The use according to any one of claims 1 to 6, wherein said cell-free translation system is a cell-free coupled transcription/translation system.

8. The use according to any one of claims 1 to 7, wherein said cell-free translation system is a cell-free translation system of prokaryotic and/or eukaryotic origin and wherein said in vivo expression system is a prokaryotic and/or eukaryotic system.

9. The use according to any one of claims 1 to 8, wherein said cell-free translation system is a cell-free translation system of prokaryotic origin and wherein said in vivo expression system is a prokaryotic system.

10. The use according to any one of claims 1 to 9, wherein said cell-free translation system is an E. coli cell-free translation system and wherein said in vivo expression system is an E. coli system.

11. The use according to any one of claims 1 to 10, wherein said cell-free translation system is a wheat germ extract cell-free translation system or a rabbit reticulocyte lysate cell-free translation system.

12. The use according to any one of claims 1 to 11, wherein said cell-free translation system (further) comprises:
- a cell-free extract;
- ribonucleotide triphosphates;
- a RNA polymerase;
- magnesium ions;
- a template plasmid;
- free amino acids; and/or
- aminoacyl-tRNAs.

13. The use according to any one of claims 1 to 12, wherein said cell-free translation system (further) comprises a radioactively labelled amino acid.

14. The use according to any one of claims 1 to 13, wherein said cell-free translation system (further) comprises [¹⁴C]leucine, [¹⁴C]valine and/or [¹⁴C]isoleucine.

15. The use according to any one of claims 1 to 14, wherein said cell-free translation system comprises a nonsense codon suppressing agent and/or an inhibitor of (a) release factor(s), like an anti-release factor antibody which precipitates and/or crosslinks said release factor(s) in said cell-free translation system.

16. The use according to any one of claims 1 to 15, wherein said protein, polypeptide or peptide to be synthesized is selected from the group consisting of enzymes, hormones, cytokines, pheromones, growth factors, signal proteins, structural proteins, toxins, markers, reporters and the like.

17. The use according to any one of claims 1 to 16, wherein said protein, polypeptide or peptide to be synthesized is covalently bound to an esterase as defined in any one of claims 1 to 6 or is an esterase as defined in any one of claims 1 to 6.

18. The use according to any one of claims 1 to 17, wherein said protein, polypeptide or peptide to be synthesized is an alloprotein.

19. The use according to any one of claims 1 to 18, wherein said protein, polypeptide or peptide to be synthesized is an alloprotein that comprises a covalently-bound puromycine or a derivative thereof and/or wherein said protein, polypeptide or peptide to be synthesized comprises an amino acid delivered by aminoacyl suppressor tRNA.

20. The use according to any one of claims 1 to 19, wherein said protein, polypeptide or peptide to be synthesized is an alloprotein that comprises a covalently-bound puromycine or a derivative thereof.

21. The use according to any one of claims 1 to 20, wherein said protein, polypeptide or peptide to be synthesized is a alloprotein that comprises a covalently-bound puromycine or a derivative thereof, wherein said covalently-bound puromycine or said derivative thereof is selected from the group consisting of:
(a) Puromycin;
(b) 5'-OH-CpPuromycin;
(c) 5'-OH-CpCpPuromycin;
(d) a puromycin derivative as defined in (a) to (c) having a residue covalently attached directly or via a linker to its 5'-position;
(e) a puromycin derivative as defined in (a) to (d) having a residue covalently attached directly or via a linker to the element N4 of the cytosine-residue of an 5' attached cytidine residue; and
(f) a puromycin derivative as defined in (a) to (e) having a residue covalently attached directly or via a linker to the element C5 of the cytosine-residue of an 5' attached cytidine-residue.

22. The use according to any one of claims 15 to 21, wherein said nonsense-codon suppressing agent comprises a puromycin derivative as defined in claim 21(a) to (d) having a residue covalently attached directly or via a linker to its element N4 of the cytosine-residue of an 5' attached cytidine residue.

23. The use according to claim 21 or 22, wherein said residue is selected from the group consisting of DNA, RNA, locked DNA, PNA, oligonucleotide-thiophosphates, substituted ribo-oligonucleotides, and proteins.

24. The use according to claim 21 or 22, wherein said residue is selected from the group consisting of a fluorophore (like Cy3), biotin or an other affinity tag, a reactive group for affinity labelling or any other reporter group.

25. The use of any one of claims 21 to 24, wherein said linker is an aliphatic amine derivative.

26. The use of any one of claims 15 to 25, wherein the nonsense-codon suppressing agent is selected from the group consisting of:
(a) 5'-OH-GpCpPuromycin;
(b) 5'-OH-GpCpCpPuromycin;
(c) 5'-OH-GpApCpCpPuromycin;
(d) 5'-OH-GpCpApCpCpPuromycin;
(e) 5'-OH-GpCpCpApCpCpPuromycin; 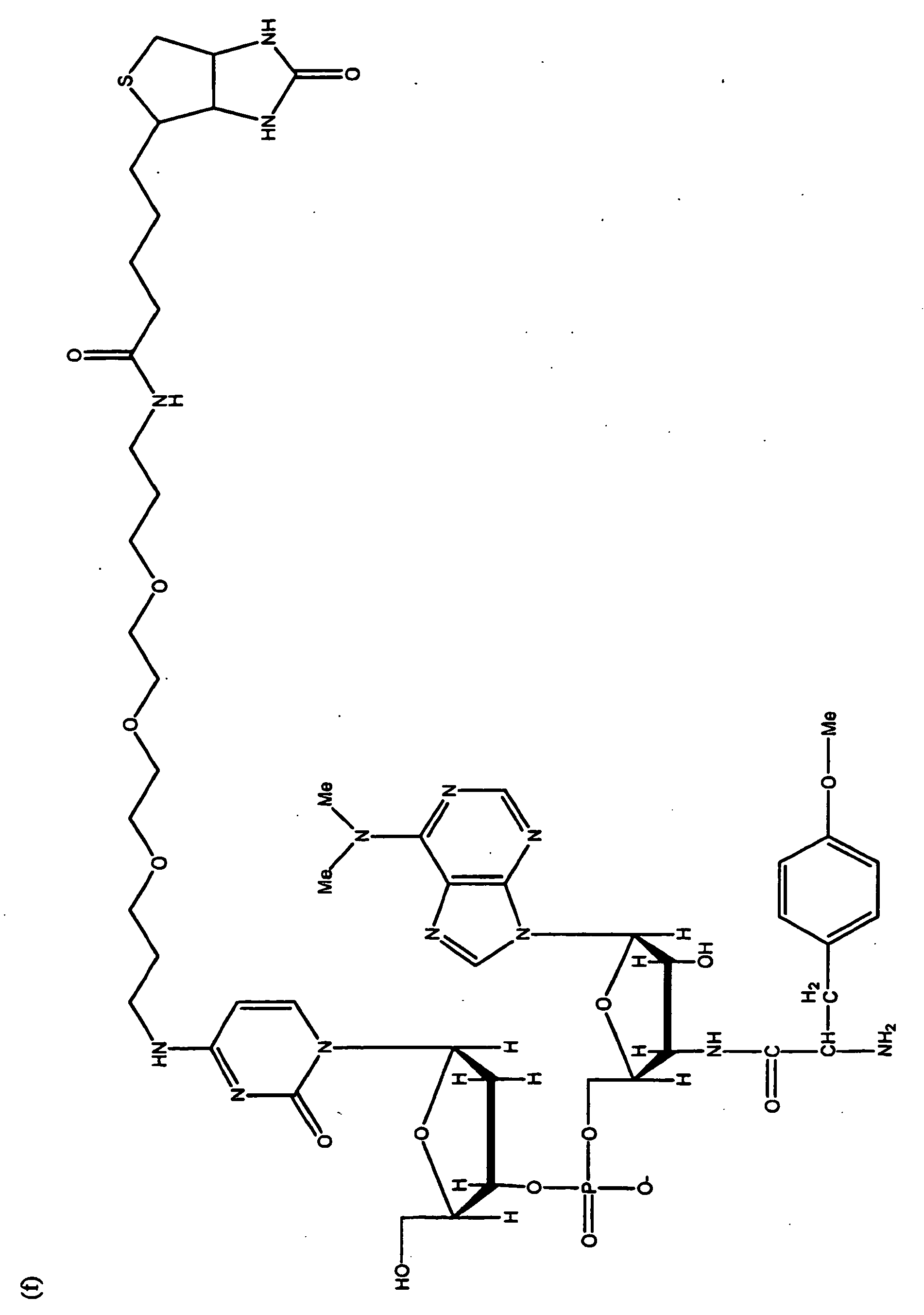 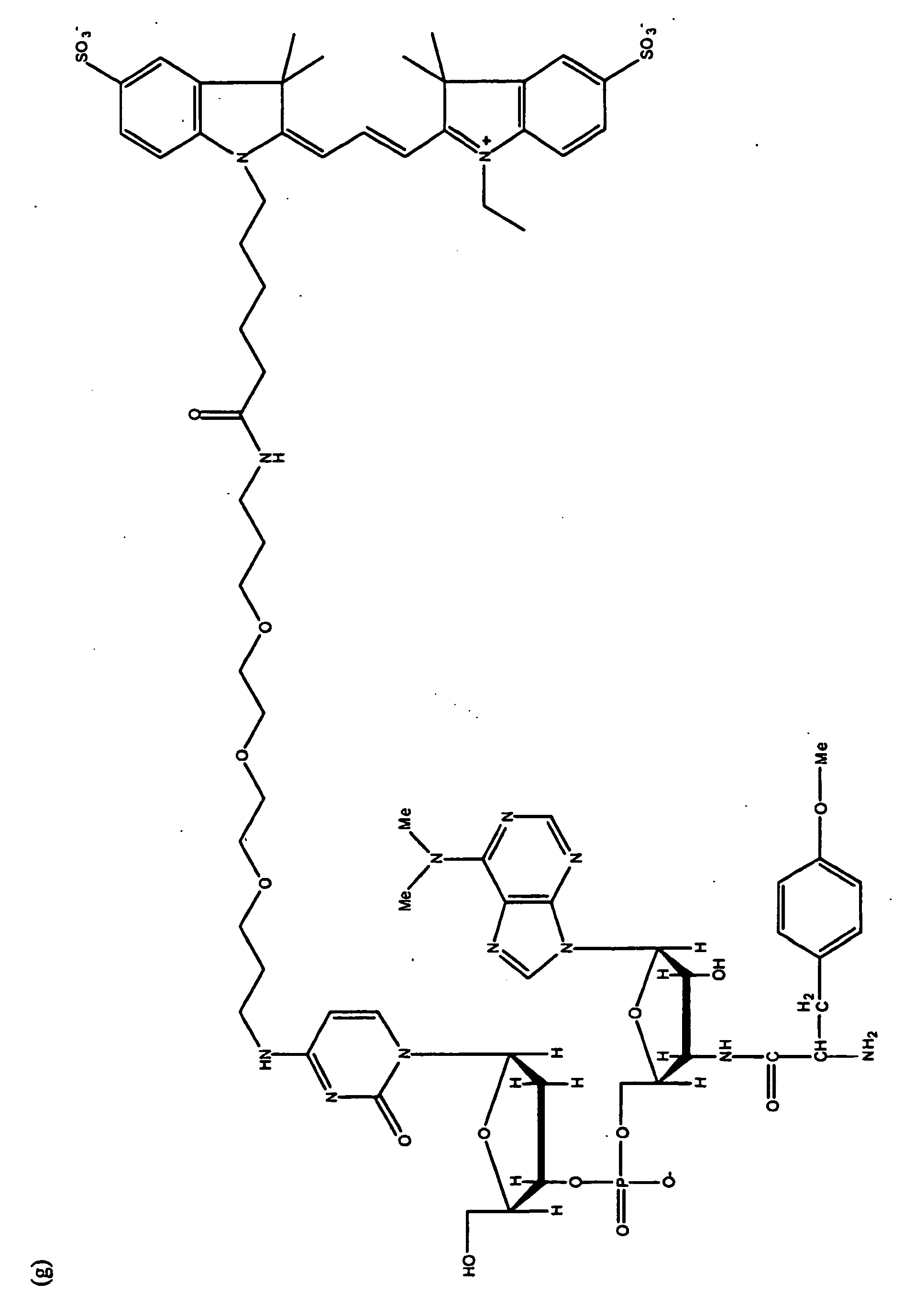 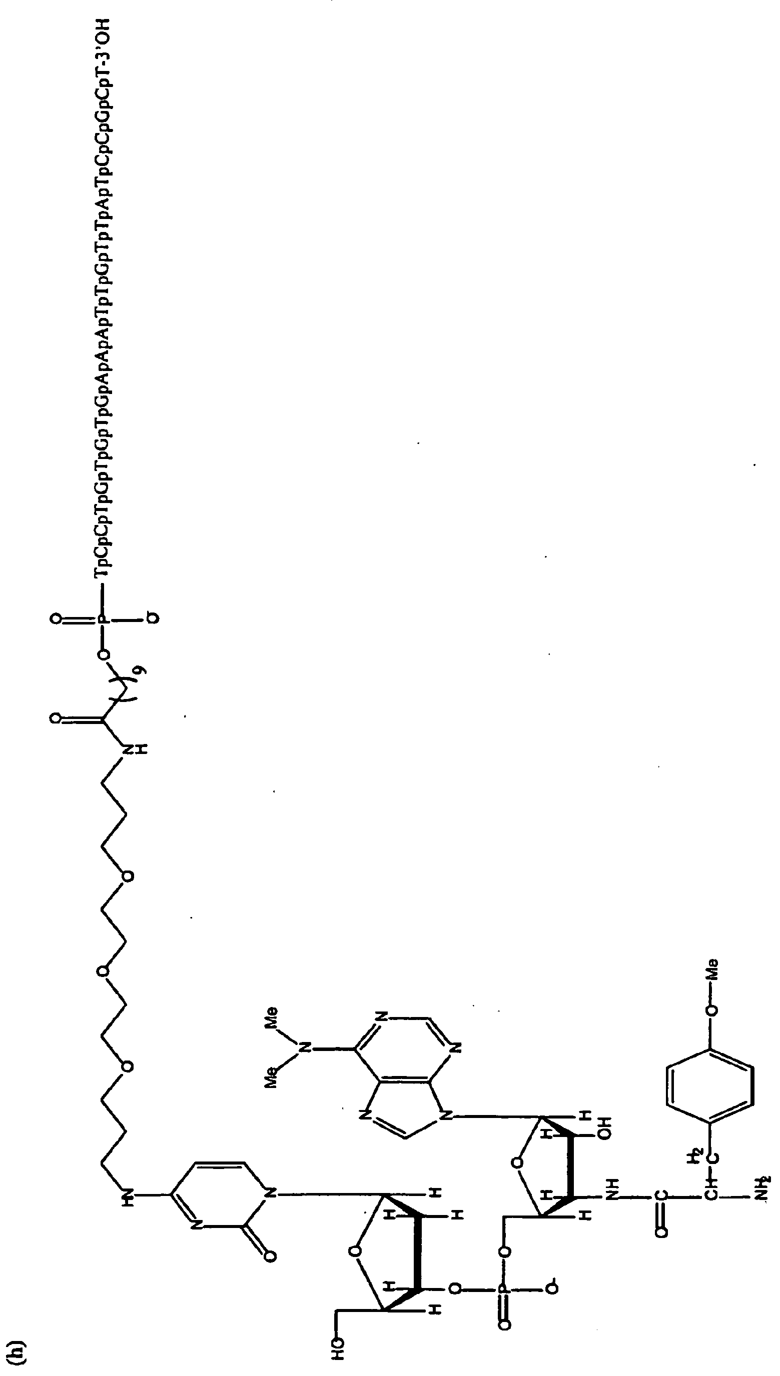 and

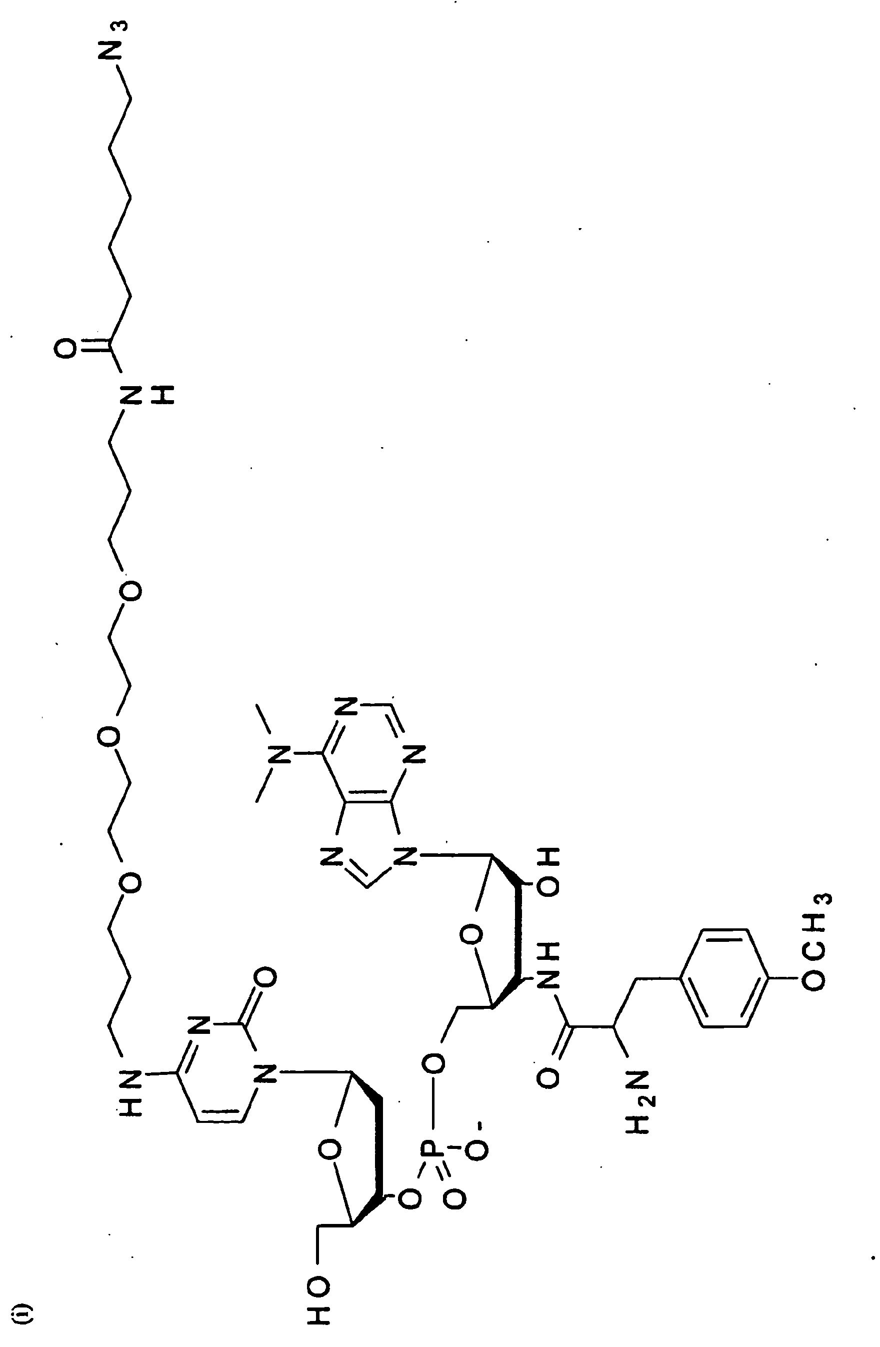

27. The use according to any one of claims I to 26, wherein said protein, polypeptide or peptide to be synthesized is a alloprotein that comprises a C-terminal, covalently-bound puromycine or a derivative thereof, as defined in any one of claims 21 to 26.

28. The use according to any one of claims 1 to 19, wherein said protein, polypeptide or peptide to be synthesized is a alloprotein that comprises an amino acid delivered by aminoacyl suppressor tRNA.

29. The use according to claim 28, wherein said protein, polypeptide or peptide to be synthesized is a alloprotein that comprises an amino acid delivered by aminoacyl suppressor tRNASer(CUA).

30. Use of
(a) a vector comprising a nucleic acid molecule coding for an esterase and expressing an esterase fusionprotein, wherein said esterase is defined as in any one of claims 1 to 5 or a functional fragment thereof;
(b) a vector comprising a nucleic acid molecule coding for an esterase and comprising in frame at least one multiple cloning site for a part X of an esterase-X fusionprotein, whereby the fusionprotein to be encoded may be of the format "X-esterase" or "esterase-X", wherein said esterase is defined as in any one of claims 1 to 5 or a functional fragment thereof;
(c) a nucleic acid molecule as defined in (a) or (b); or
(d) an esterase fusion protein, polypeptide or peptide encoded by a vector as defined in (a) or (b)
for monitoring and/or tracking the synthesis of a protein, polypeptide or peptide in a cell-free translation system or in an *in vivo* expression system, wherein the monitoring and/or tracking comprises the detection of the enzymatic function or activity of said esterase.

31. The use according to claim 30, wherein said nucleic acid molecule codes for a fusionprotein, whereby said fusion protein is in the format "X-esterase" or "esterase-X", whereby said X is a protein, polypeptide or peptide as defined in any one of claims 16 to 29 or a fragment thereof.

32. The use according to claim 30 or 31, wherein said nucleic acid molecule codes for a fusionprotein comprising a said esterase and GFP.

33. The use according to any one of claims 30 to 32, wherein said fusionprotein comprises a cleavable linker between said esterase and said fused protein.

34. The use according to claim 33, wherein said cleavable linker between said esterase and said fused protein is cleavable by a factor XA protease.

35. The use according to claim 33 or 34, wherein said cleavable linker between said esterase and said fused protein is encoded by a nucleotide sequence comprising SEQ ID NO: 7.

36. The use according to any one of claims 30 to 35, wherein said nucleic acid molecule comprises the nucleotide sequence of SEQ ID NO: 8.

37. A method for monitoring and/or tracking the synthesis of a protein, polypeptide or peptide in a cell-free translation system, comprising the step of detecting the enzymatic function or activity of an esterase as defined in any one of claims 1 to 5 or a functional fragment thereof.

38. The method according to claim 37, wherein said protein, polypeptide or peptide is defined as in any one of claims 16 to 24.

39. The use of any one of claims 1 to 36 or the method of claim 37 or 38, wherein said detection of the enzymatic function or activity of said esterase is an electrochemical detection.

40. The use or the method of claim 39, wherein said electrochemical detection comprises the hydrolysis of an ester of p-aminophenol to p-aminophenol.

41. The use or the method of claim 39 or 40, wherein said electrochemical detection comprises the hydrolysis of p-aminophenol acetate to p-aminophenol.

42. The use of any one of claims 1 to 36 and 39 to 41 or the method of claim 37 or 38, wherein said enzymatic function or activity to be detected is the cleavage of an ester into an alcohol and a carboxylic acid.

## Patentansprüche

1. Verwendung einer thermostabilen Esterase prokaryontischen Ursprungs zum Überwachen und/oder Verfolgen der Synthese eines Proteins, Polypeptids oder Peptids in einem zellfreien Translationssystem oder in einem *in vivo-*Expressionssystem, in dem die Synthese eines Proteins, Polypeptids oder Peptids erfolgen kann, wobei das Überwachen und/oder Verfolgen den Nachweis der enzymatischen Funktion oder Aktivität der Esterase umfasst.

2. Verwendung gemäß Anspruch 1, wobei die Esterase eine Einzelketten-Esterase ist.

3. Verwendung gemäß Anspruch 1 oder 2, wobei die Esterase von *Alicyclobacillus acidocaldarius* ist.

4. Verwendung gemäß einem der Ansprüche 1 bis 3, wobei die Esterase die Esterase 2 von *Alicyclobacillus acidocaldarius* ist.

5. Verwendung gemäß einem der Ansprüche 1 bis 4, wobei die Esterase ausgewählt ist aus der Gruppe bestehend aus:
(a) einer Esterase, die von einer Nucleotidsequenz codiert wird, die eine Nucleotidsequenz wie in SEQ ID NO: 1 gezeigt umfasst;
(b) einer Esterase, die von einer Nucleotidsequenz codiert wird, die ein Polypeptid codiert, das eine Aminosäuresequenz wie in SEQ ID NO: 2 oder SEQ ID NO: 62 gezeigt umfasst;
(c) einer Esterase, die von einer Nucleotidsequenz eines Nucleinsäuremoleküls codiert wird, das an den komplementären Strang eines Nucleinsäuremoleküls hybridisiert, das eine Nucleotidsequenz wie in (a) oder (b) definiert umfasst, und die die Spaltung eines Esters in einen Alkohol und eine Carbonsäure katalysiert;
(d) einer Esterase, die eine Aminosäuresequenz wie in SEQ ID NO: 2 oder SEQ ID NO: 62 gezeigt umfasst;
(e) einer Esterase, die eine Aminosäuresequenz umfasst, die zu mindestens 60 % mit der Volllängen-Aminosäuresequenz wie in SEQ ID NO: 2 oder SEQ ID NO: 62 gezeigt identisch ist; und
(f) einer Esterase, die von einer Nucleotidsequenz codiert wird, die zu einer Nucleotidsequenz wie in einem von (a) bis (c) definiert degeneriert ist.

6. Verwendung gemäß einem der Ansprüche 1 bis 5, wobei die Esterase in einem Fusionsprotein, Fusionspolypeptid und/oder Fusionspeptid umfasst ist, das synthetisiert werden soll.

7. Verwendung gemäß einem der Ansprüche 1 bis 6, wobei das zellfreie Translationssystem ein zellfreies, gekoppeltes Transkriptions-/Translationssystem ist.

8. Verwendung gemäß einem der Ansprüche 1 bis 7, wobei das zellfreie Translationssystem ein zellfreies Translationssystem prokaryontischen und/oder eukaryontischen Ursprungs ist und wobei das *in vivo*-Expressionssystem ein prokaryontisches und/oder eukaryontisches System ist.

9. Verwendung gemäß einem der Ansprüche 1 bis 8, wobei das zellfreie Translationssystem ein zellfreies Translationssystem prokaryontischen Ursprungs ist und wobei das *in vivo*-Expressionssystem ein prokaryontisches System ist.

10. Verwendung gemäß einem der Ansprüche 1 bis 9, wobei das zellfreie Translationssystem ein *E. coli*-zellfreies-Translationssystem ist und wobei das *in vivo-*Expressionssystem ein *E. coli*-System ist.

11. Verwendung gemäß einem der Ansprüche 1 bis 10, wobei das zellfreie Translationssystem ein Weizenkeimextrakt-zellfreies-Translationssystem oder ein Kaninchen-Reticulocytenlysat-zellfreies-Translationssystem ist.

12. Verwendung gemäß einem der Ansprüche 1 bis 11, wobei das zellfreie Translationssystem (ferner) umfasst:
- einen zellfreien Extrakt;
- Ribonucleotidtriphosphate;
- eine RNA-Polymerase;
- Magnesiumionen;
- ein Matrizenplasmid;
- freie Aminosäuren; und/oder
- Aminoacyl-tRNAs.

13. Verwendung gemäß einem der Ansprüche 1 bis 12, wobei das zellfreie Translationssystem (ferner) eine radioaktiv markierte Aminosäure umfasst.

14. Verwendung gemäß einem der Ansprüche 1 bis 13, wobei das zellfreie Translationssystem (ferner) [¹⁴C]-Leucin, [¹⁴C]-Valin und/oder [¹⁴C]-Isoleucin umfasst.

15. Verwendung gemäß einem der Ansprüche 1 bis 14, wobei das zellfreie Translationssystem ein Nonsense-Codon-unterdrückendes Agens und/oder einen Inhibitor eines Freisetzungsfaktors/von Freisetzungsfaktoren umfasst, wie einen Anti-Freisetzungsfaktor-Antikörper, der den (die) Freisetzungsfaktor(en) in dem zellfreien Translationssystem präzipitiert und/oder vernetzt.

16. Verwendung gemäß einem der Ansprüche 1 bis 15, wobei das Protein, Polypeptid oder Peptid, das synthetisiert werden soll, ausgewählt ist aus der Gruppe bestehend aus Enzymen, Hormonen, Cytokinen, Pheromonen, Wachstumsfaktoren, Signalproteinen, Strukturproteinen, Toxinen, Markern, Reportern und dergleichen.

17. Verwendung gemäß einem der Ansprüche 1 bis 16, wobei das Protein, Polypeptid oder Peptid, das synthetisiert werden soll, kovalent an eine Esterase wie in einem der Ansprüche 1 bis 6 definiert gebunden ist oder eine Esterase wie in einem der Ansprüche 1 bis 6 definiert ist.

18. Verwendung gemäß einem der Ansprüche 1 bis 17, wobei das Protein, Polypeptid oder Peptid, das synthetisiert werden soll, ein Alloprotein ist.

19. Verwendung gemäß einem der Ansprüche 1 bis 18, wobei das Protein, Polypeptid oder Peptid, das synthetisiert werden soll, ein Alloprotein ist, das ein kovalent gebundenes Puromycin oder Derivat davon umfasst, und/oder wobei das Protein, Polypeptid oder Peptid, das synthetisiert werden soll, eine Aminosäure umfasst, die durch eine Aminoacyl-Suppressor-tRNA bereitgestellt wird.

20. Verwendung gemäß einem der Ansprüche 1 bis 19, wobei das Protein, Polypeptid oder Peptid, das synthetisiert werden soll, ein Alloprotein ist, das ein kovalent gebundenes Puromycin oder Derivat davon umfasst.

21. Verwendung gemäß einem der Ansprüche 1 bis 20, wobei das Protein, Polypeptid oder Peptid, das synthetisiert werden soll, ein Alloprotein ist, das ein kovalent gebundenes Puromycin oder Derivat davon umfasst, wobei das kovalent gebundene Puromycin oder Derivat davon ausgewählt ist aus der Gruppe bestehend aus:
(a) Puromycin;
(b) 5'-OH-CpPuromycin;
(c) 5'-OH-CpCpPuromycin;
(d) einem Puromycinderivat wie in (a) bis (c) definiert, das einen Rest aufweist, der direkt oder über einen Linker kovalent an seine 5'-Position angehängt ist;
(e) einem Puromycinderivat wie in (a) bis (d) definiert, das einen Rest aufweist, der direkt oder über einen Linker kovalent an das Element N4 des Cytosinrests eines 5'-angehängten Cytidinrests angehängt ist; und
(f) einem Puromycinderivat wie in (a) bis (e) definiert, das einen Rest aufweist, der direkt oder über einen Linker kovalent an das Element C5 des Cytosinrests eines 5'-angehängten Cytidinrests angehängt ist.

22. Verwendung gemäß einem der Ansprüche 15 bis 21, wobei das Nonsense-Codonunterdrückende Agens ein Puromycinderivat wie in Anspruch 21 (a) bis (d) definiert umfasst, das einen Rest aufweist, der direkt oder über einen Linker kovalent an sein Element N4 des Cytosinrests eines 5'-angehängten Cytidinrests angehängt ist.

23. Verwendung gemäß einem der Ansprüche 21 oder 22, wobei der Rest ausgewählt ist aus der Gruppe bestehend aus DNA, RNA, locked DNA, PNA, Oligonucleotidthiophosphaten, substituierten Ribo-Oligonucleotiden und Proteinen.

24. Verwendung gemäß einem der Ansprüche 21 bis 22, wobei der Rest ausgewählt ist aus der Gruppe bestehend aus einem Fluorophor (wie Cy3), Biotin oder einem anderen Affinitäts-Tag, einer reaktiven Gruppe für Affinitätskennzeichnung oder einer beliebigen anderen Reportergruppe.

25. Verwendung gemäß einem der Ansprüche 21 bis 24, wobei der Linker ein aliphatisches Aminderivat ist.

26. Verwendung gemäß einem der Ansprüche 15 bis 25, wobei das Nonsense-Codonunterdrückende Agens ausgewählt ist aus der Gruppe bestehend aus:
(a) 5'-OH-GpCpPuromycin;
(b) 5'-OH-GpCpCpPuromycin;
(c) 5'-OH-GpApCpCpPuromycin;
(d) 5'-OH-GpCpApCpCpPuromycin;
(e) 5'-OH-GpCpCpApCpCpPuromycin;

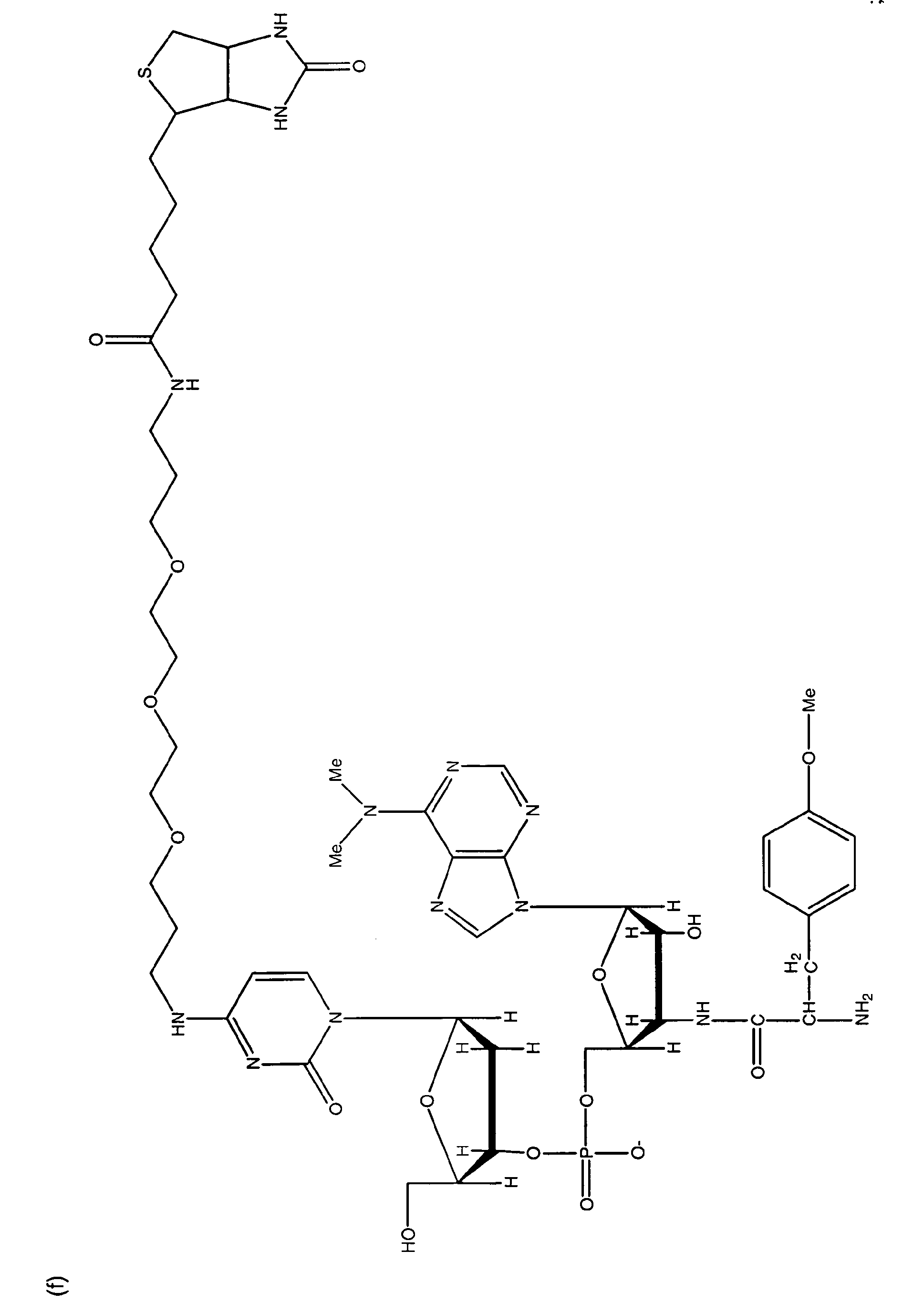

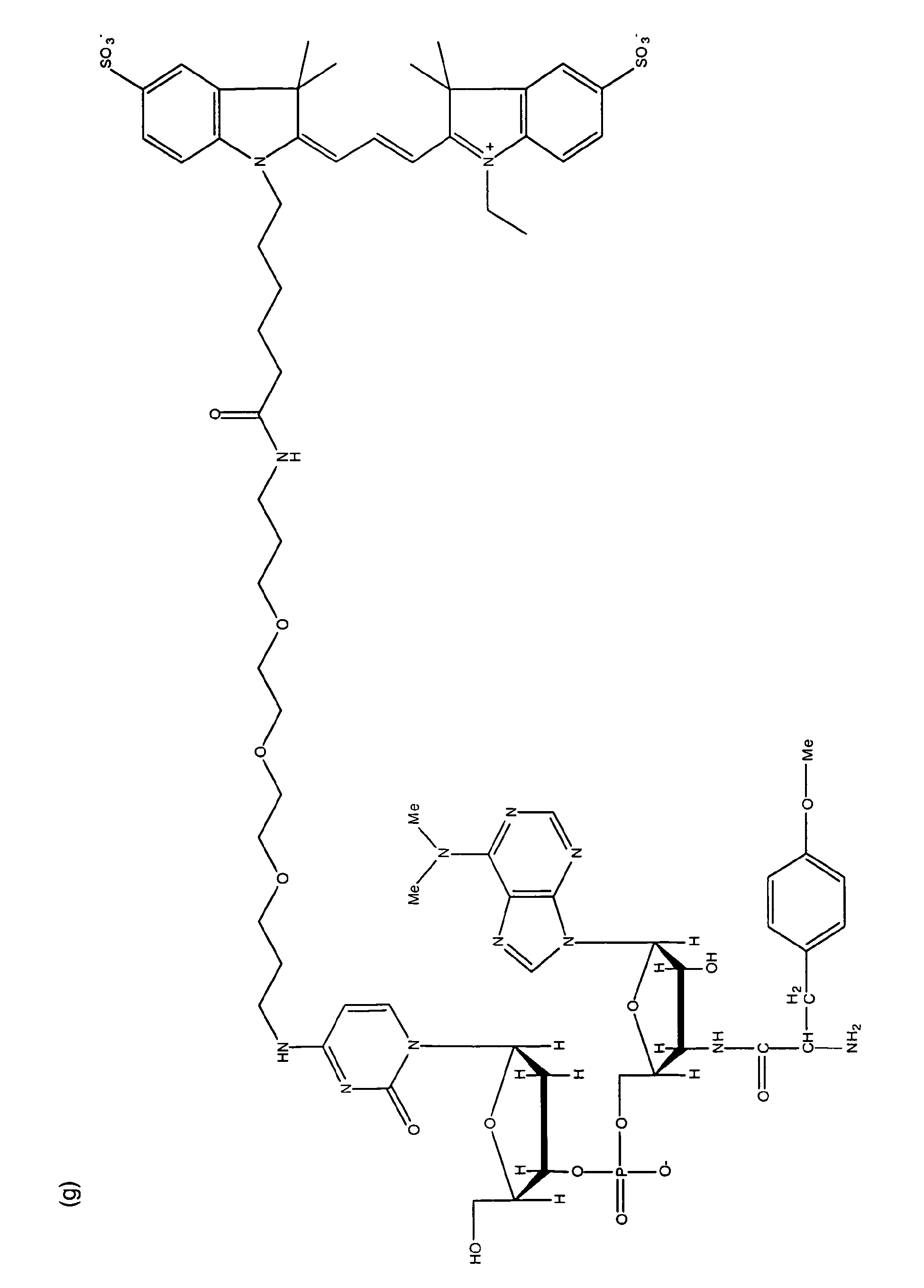

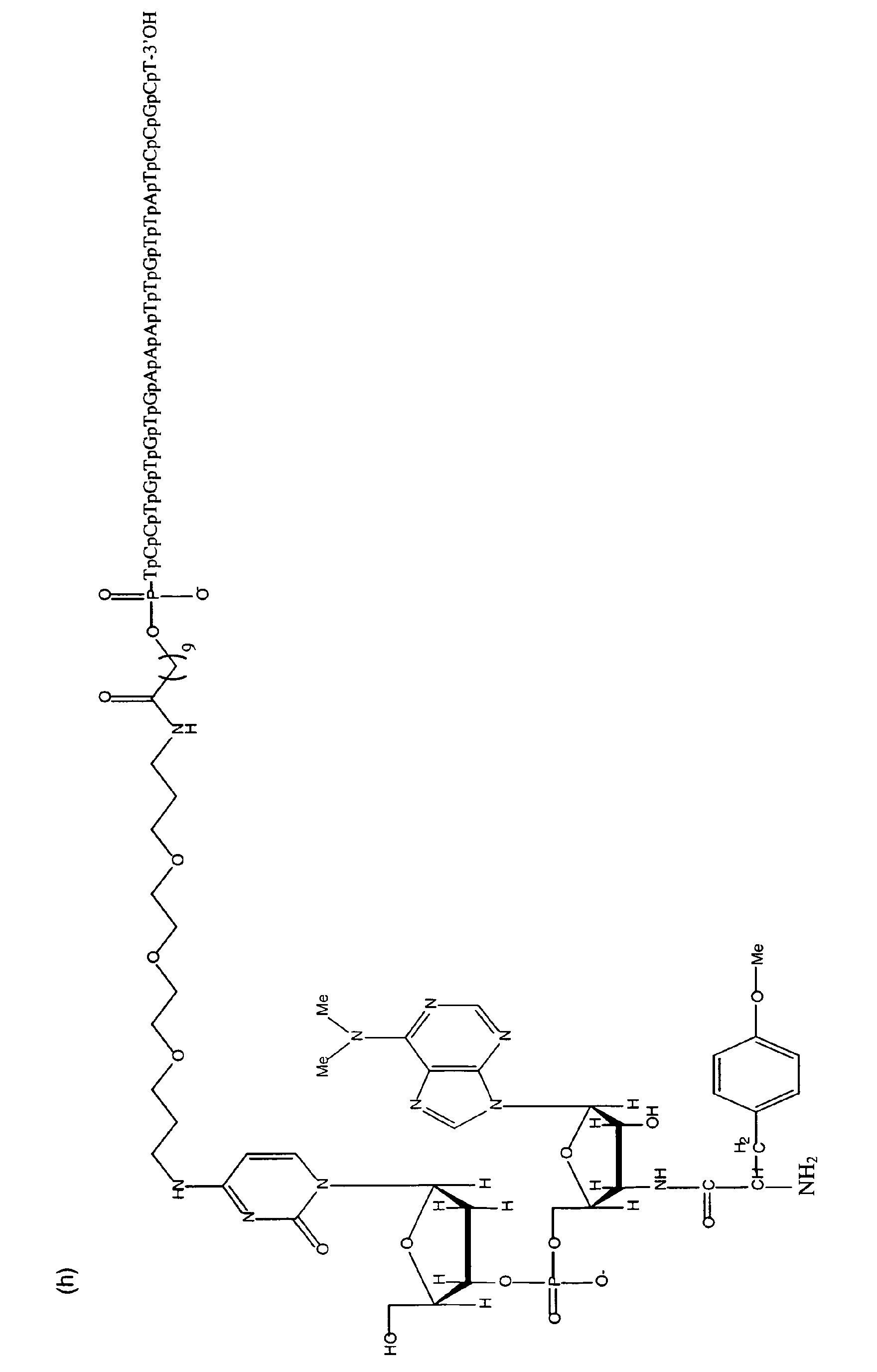

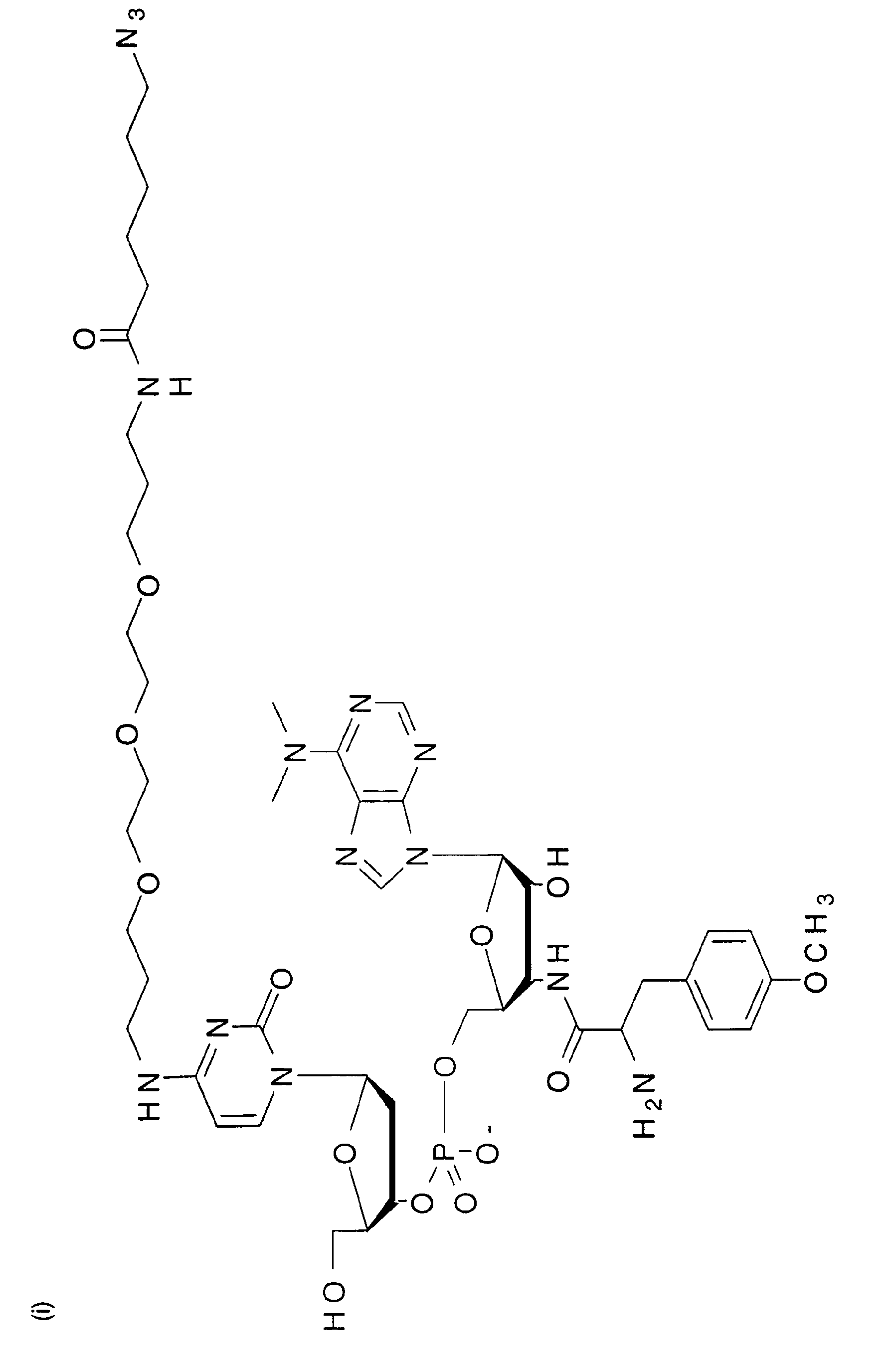

27. Verwendung gemäß einem der Ansprüche 1 bis 26, wobei das Protein, Polypeptid oder Peptid, das synthetisiert werden soll, ein Alloprotein ist, welches ein C-terminales, kovalent gebundenes Puromycin oder ein Derivat davon wie in einem der Ansprüche 21 bis 26 definiert umfasst.

28. Verwendung gemäß einem der Ansprüche 1 bis 19, wobei das Protein, Polypeptid oder Peptid, das synthetisiert werden soll, ein Alloprotein ist, das eine Aminosäure umfasst, die von einer Aminoacyl-Suppressor-tRNA bereitgestellt wird.

29. Verwendung gemäß Anspruch 28, wobei das Protein, Polypeptid oder Peptid, das synthetisiert werden soll, ein Alloprotein ist, das eine Aminosäure umfasst, die durch Aminoacyl-Suppressor-tRNASer (CUA) bereitgestellt wird.

30. Verwendung
(a) eines Vektors, der ein Nucleinsäuremolekül umfasst, das eine Esterase codiert, und der ein Esterase-Fusionsprotein exprimiert, wobei die Esterase wie in einem der Ansprüche 1 bis 5 definiert ist, oder ein funktionelles Fragment davon;
(b) eines Vektors, der ein Nucleinsäuremolekül umfasst, das eine Esterase codiert, und der im Leseraster mindestens eine multiple Clonierungsstelle für einen Teil X eines Esterase-X-Fusionsproteins umfasst, wobei das zu codierende Fusionsprotein im Format "X-Esterase" oder "Esterase-X" sein kann, wobei die Esterase wie in einem der Ansprüche 1 bis 5 definiert ist, oder ein funktionelles Fragment davon;
(c) eines Nucleinsäuremoleküls wie in (a) oder (b) definiert; oder
(d) eines Esterase-Fusionsproteins, -Polypeptids oder -Peptids, das von einem Vektor wie in (a) oder (b) definiert codiert wird
zum Überwachen und/oder Verfolgen der Synthese eines Proteins, Polypeptids oder Peptids in einem zellfreien Translationssystem oder in einem *in vivo-*Expressionssystem, wobei das Überwachen und/oder Verfolgen den Nachweis der enzymatischen Funktion oder Aktivität der Esterase umfasst.

31. Verwendung gemäß Anspruch 30, wobei das Nucleinsäuremolekül ein Fusionsprotein codiert, wobei das Fusionsprotein im Format "X-Esterase" oder "Esterase-X" ist, wobei das X ein Protein, Polypeptid oder Peptid wie in einem der Ansprüche 16 bis 29 definiert oder ein Fragment davon ist.

32. Verwendung gemäß einem der Ansprüche 30 oder 31, wobei das Nucleinsäuremolekül ein Fusionsprotein codiert, das die Esterase und GFP umfasst.

33. Verwendung gemäß einem der Ansprüche 30 bis 32, wobei das Fusionsprotein einen spaltbaren Linker zwischen der Esterase und dem fusionierten Protein umfasst.

34. Verwendung gemäß Anspruch 33, wobei der spaltbare Linker zwischen der Esterase und dem fusionierten Protein durch eine Faktor XA-Protease spaltbar ist.

35. Verwendung gemäß Anspruch 33 oder 34, wobei der spaltbare Linker zwischen der Esterase und dem fusionierten Protein durch eine Nucleotidsequenz codiert wird, die SEQ ID NO: 7 umfasst.

36. Verwendung gemäß einem der Ansprüche 30 bis 35, wobei das Nucleinsäuremolekül die Nucleotidsequenz von SEQ ID NO: 8 umfasst.

37. Verfahren zum Überwachen und/oder Verfolgen der Synthese eines Proteins, Polypeptids oder Peptids in einem zellfreien Translationssystem, umfassend den Schritt des Nachweisens der enzymatischen Funktion oder Aktivität einer Esterase wie in einem der Ansprüche 1 bis 5 definiert oder eines funktionellen Fragments davon.

38. Verfahren gemäß Anspruch 37, wobei das Protein, Polypeptid oder Peptid wie in einem der Ansprüche 16 bis 24 definiert ist.

39. Verwendung gemäß einem der Ansprüche 1 bis 36 oder Verfahren gemäß Anspruch 37 oder 38, wobei der Nachweis der enzymatischen Funktion oder Aktivität der Esterase ein elektrochemischer Nachweis ist.

40. Verwendung oder Verfahren gemäß Anspruch 39, wobei der elektrochemische Nachweis die Hydrolyse eines Esters von p-Aminophenol zu p-Aminophenol umfasst.

41. Verwendung oder Verfahren gemäß Anspruch 39 oder 40, wobei der elektrochemische Nachweis die Hydrolyse von p-Aminophenolacetat zu p-Aminophenol umfasst.

42. Verwendung gemäß einem der Ansprüche 1 bis 36 und 39 bis 41 oder Verfahren gemäß Anspruch 37 oder 38, wobei die enzymatische Funktion oder Aktivität, die nachgewiesen werden soll, die Spaltung eines Esters in einen Alkohol und eine Carbonsäure ist.

## Revendications

1. Utilisation d'une estérase thermostable d'origine procaryote pour surveiller et/ou suivre la synthèse d'une protéine, d'un polypeptide ou d'un peptide dans un système de traduction acellulaire ou dans un système d'expression *in vivo* dans lequel la synthèse d'une protéine, d'un polypeptide ou d'un peptide peut avoir lieu, où ladite surveillance et/ou ledit suivi comprend la détection de la fonction ou activité enzymatique de ladite estérase.

2. Utilisation selon la revendication 1, où ladite estérase est une estérase à une seule chaîne.

3. Utilisation selon la revendication 1 ou 2, où ladite estérase provient de l'Alicyclobacillus acidocaldarius.

4. Utilisation selon l'une quelconque des revendications 1 à 3, où ladite estérase est l'estérase 2 provenant de l'Alicyclobacillus acidocaldarius.

5. Utilisation selon l'une quelconque des revendications 1 à 4, où ladite estérase est choisie parmi le groupe consistant en :
(a) une estérase codée par une séquence nucléotidique comprenant une séquence nucléotidique comme représenté dans la SEQ ID NO: 1 ;
(b) une estérase codée par une séquence nucléotidique codant pour un polypeptide comprenant une séquence d'acides aminés comme représenté dans la SEQ ID NO: 2 ou SEQ ID NO: 62 ;
(c) une estérase codée par une séquence nucléotidique d'une molécule d'acide nucléique qui s'hybride au brin complémentaire d'une molécule d'acide nucléique comprenant une séquence nucléotidique telle que définie dans (a) ou (b) et qui catalyse le clivage d'un ester en un alcool et un acide carboxylique ;
(d) une estérase qui comprend une séquence d'acides aminés comme représenté dans la SEQ ID NO: 2 ou SEQ ID NO: 62 ;
(e) une estérase qui comprend une séquence d'acides aminés qui est au moins à 60 % identique à la séquence d'acides aminés de longueur totale comme représenté dans la SEQ ID NO: 2 ou SEQ ID NO: 62 ; et
(f) une estérase codée par une séquence nucléotidique qui est dégénérée en une séquence nucléotidique telle que définie dans l'une quelconque parmi (a) à (c).

6. Utilisation selon l'une quelconque des revendications 1 à 5, où ladite estérase est comprise dans une protéine de fusion, un polypeptide de fusion et/ou un peptide de fusion destiné à être synthétisé.

7. Utilisation selon l'une quelconque des revendications 1 à 6, où ledit système de traduction acellulaire est un système de transcription/traduction couplée acellulaire.

8. Utilisation selon l'une quelconque des revendications 1 à 7, où ledit système de traduction acellulaire est un système de traduction acellulaire d'origine procaryote et/ou eucaryote et où ledit système d'expression *in vivo* est un système procaryote et/ou eucaryote.

9. Utilisation selon l'une quelconque des revendications 1 à 8, où ledit système de traduction acellulaire est un système de traduction acellulaire d'origine procaryote et/ou ledit système d'expression *in vivo* est un système procaryote.

10. Utilisation selon l'une quelconque des revendications 1 à 9, où ledit système de traduction acellulaire est un système de traduction acellulaire *E. coli* et où ledit système d'expression *in vivo* est un système *E. coli*.

11. Utilisation selon l'une quelconque des revendications 1 à 10, où ledit système de traduction acellulaire est un système de traduction acellulaire d'extraits de germe de blé ou un système de traduction acellulaire de lysat de réticulocyte de lapin.

12. Utilisation selon l'une quelconque des revendications 1 à 11, où ledit système de traduction acellulaire comprend (en outre) :
un extrait acellulaire ;
des ribonucléotide triphosphates ;
une ARN polymérase ;
des ions magnésium ;
une matrice plasmidique ;
des acides aminés libres ; et/ou
des aminoacyl-ARNt.

13. Utilisation selon l'une quelconque des revendications 1 à 12, où ledit système de traduction acellulaire comprend (en outre) un aminoacide marqué radioactivement.

14. Utilisation selon l'une quelconque des revendications 1 à 13, où ledit système de traduction acellulaire comprend (en outre) la [¹⁴C] leucine, la [¹⁴C] valine et/ou la [¹⁴C] isoleucine.

15. Utilisation selon l'une quelconque des revendications 1 à 14, où ledit système de traduction acellulaire comprend un agent suppresseur du codon nonsens et/ou un inhibiteur d'un ou de plusieurs facteurs de libération, tel qu'un anticorps anti-facteur de libération qui précipite et/ou provoque la réticulation dudit ou desdits facteurs de libération dans ledit système de traduction acellulaire.

16. Utilisation selon l'une quelconque des revendications 1 à 15, où ladite protéine, ledit polypeptide ou ledit peptide devant être synthétisé est choisi parmi le groupe consistant en enzymes, hormones, cytokines, phéromones, facteurs de croissance, protéines signal, protéines structurales, toxines, marqueurs, reporteurs et analogues.

17. Utilisation selon l'une quelconque des revendications 1 à 16, où ladite protéine, ledit polypeptide ou ledit peptide devant être synthétisé est lié de manière covalente à une estérase telle que définie dans l'une quelconque des revendications 1 à 6 ou est une estérase telle que définie dans l'une quelconque des revendications 1 à 6.

18. Utilisation selon l'une quelconque des revendications 1 à 17, où ladite protéine, ledit polypeptide ou ledit peptide devant être synthétisé est une alloprotéine.

19. Utilisation selon l'une quelconque des revendications 1 à 18, où ladite protéine, ledit polypeptide ou ledit peptide devant être synthétisé est une alloprotéine qui comprend une puromycine liée de manière covalente ou un dérivé de celle-ci et/ou où ladite protéine, ledit polypeptide ou ledit peptide devant être synthétisé comprend un acide aminé délivré par l'aminoacyl suppresseur ARNt.

20. Utilisation selon l'une quelconque des revendications 1 à 19, où ladite protéine, ledit polypeptide ou ledit peptide devant être synthétisé est une alloprotéine qui comprend une puromycine liée de manière covalente ou un dérivé de celle-ci.

21. Utilisation selon l'une quelconque des revendications 1 à 20, où ladite protéine, ledit polypeptide ou ledit peptide devant être synthétisé est une alloprotéine qui comprend une puromycine liée de manière covalente ou un dérivé de celle-ci, où ladite puromycine liée de manière covalente ou un dérivé de celle-ci est choisi parmi le groupe consistant en :
(a) puromycine ;
(b) 5'-OH-CpPuromycine ;
(c) 5'-OH-CpCpPuromycine ;
(d) un dérivé de puromycine tel que défini dans (a) à (c) ayant un résidu lié de manière covalente directement ou par un lieur à sa position 5' ;
(e) un dérivé de puromycine tel que défini dans (a) à (d) ayant un résidu lié de manière covalente directement ou par ailleurs à l'élément N4 du résidu cytosine d'un résidu cytidine attaché en 5' ; et
(f) un dérivé puromycine tel que défini dans (a) à (e) ayant un résidu lié de manière covalente directement ou par un lieur à l'élément C5 du résidu cytosine d'un résidu cytidine attaché en 5'.

22. Utilisation selon l'une quelconque des revendications 15 à 21, où l'agent suppresseur de codon nonsense comprend un dérivé de puromycine tel que défini dans la revendication 21 (a) à (d) ayant un résidu fixé de manière covalente directement ou par un lieur à son élément N4 du résidu cytosine d'un résidu cytidine attaché en 5'.

23. Utilisation selon la revendication 21 ou 22, où ledit résidu est choisi parmi le groupe consistant en ADN, ARN, ADN bloqué, ANP, oligonucléotide thiophosphates, ribo-oligonucléotides substitués, et protéines.

24. Utilisation selon la revendication 21 ou 22, où ledit résidu est choisi parmi le groupe consistant en un fluorophore (Comme Cy3), une biotine ou un autre marqueur d'affinité, un groupe réactif pour marquer l'affinité ou un autre groupe reporteur quelconque.

25. Utilisation selon l'une quelconque des revendications 21 à 24, où ledit lieur est un dérivé d'amine aliphatique.

26. Utilisation selon l'une quelconque des revendications 15 à 25, où l'agent suppresseur de codon nonsens est choisi parmi le groupe consistant en :
(a) 5'-OH-GpCpPuromycine ;
(b) 5'-OH-GpCpCpPuromycine ;
(c) 5'-OH-GpApCpCpPuromycine ;
(d) 5'-OH-GpCpApCpCpPuromycine ;
(e) 5'-OH-GpCpCpApCpCpPuromycine ; 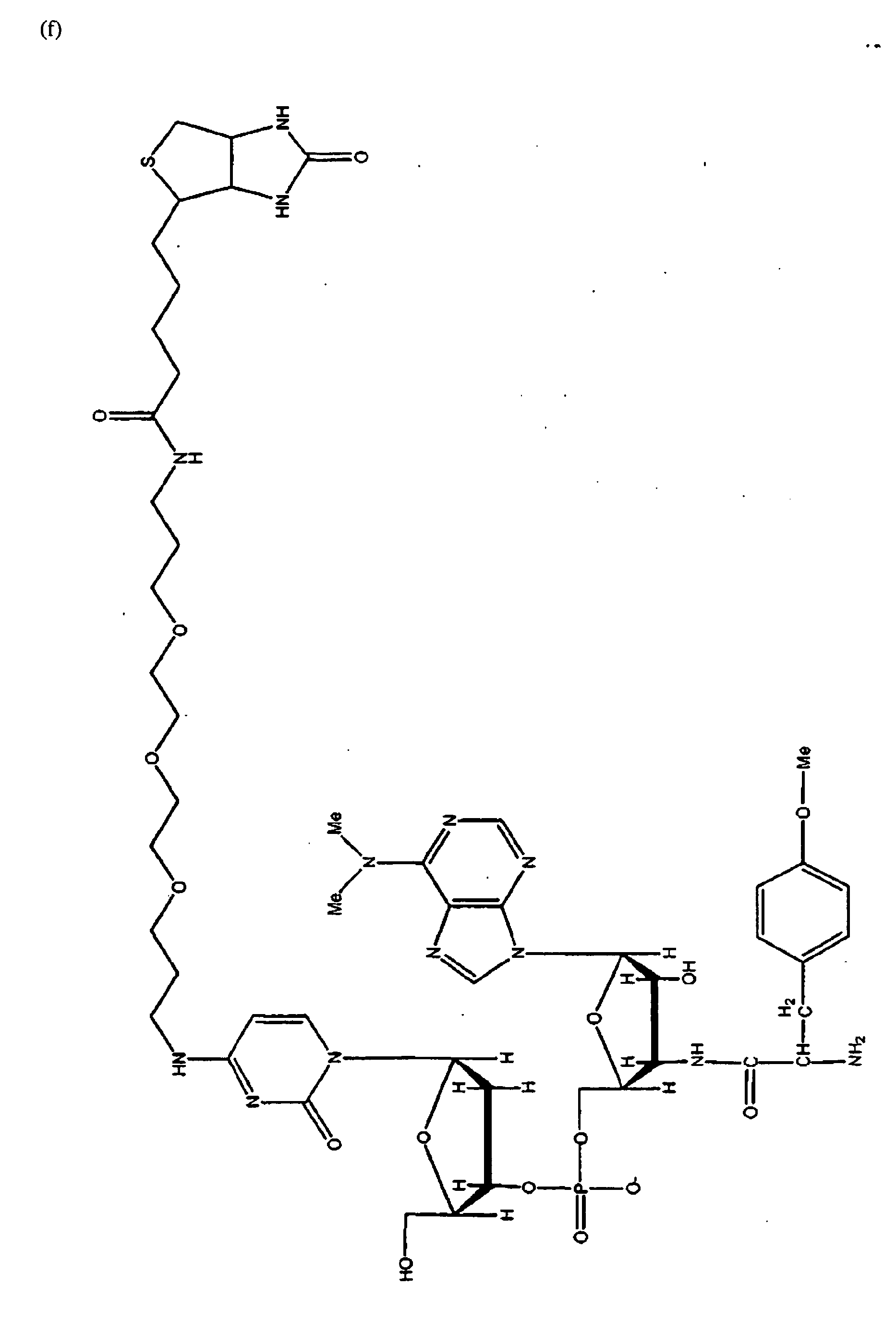 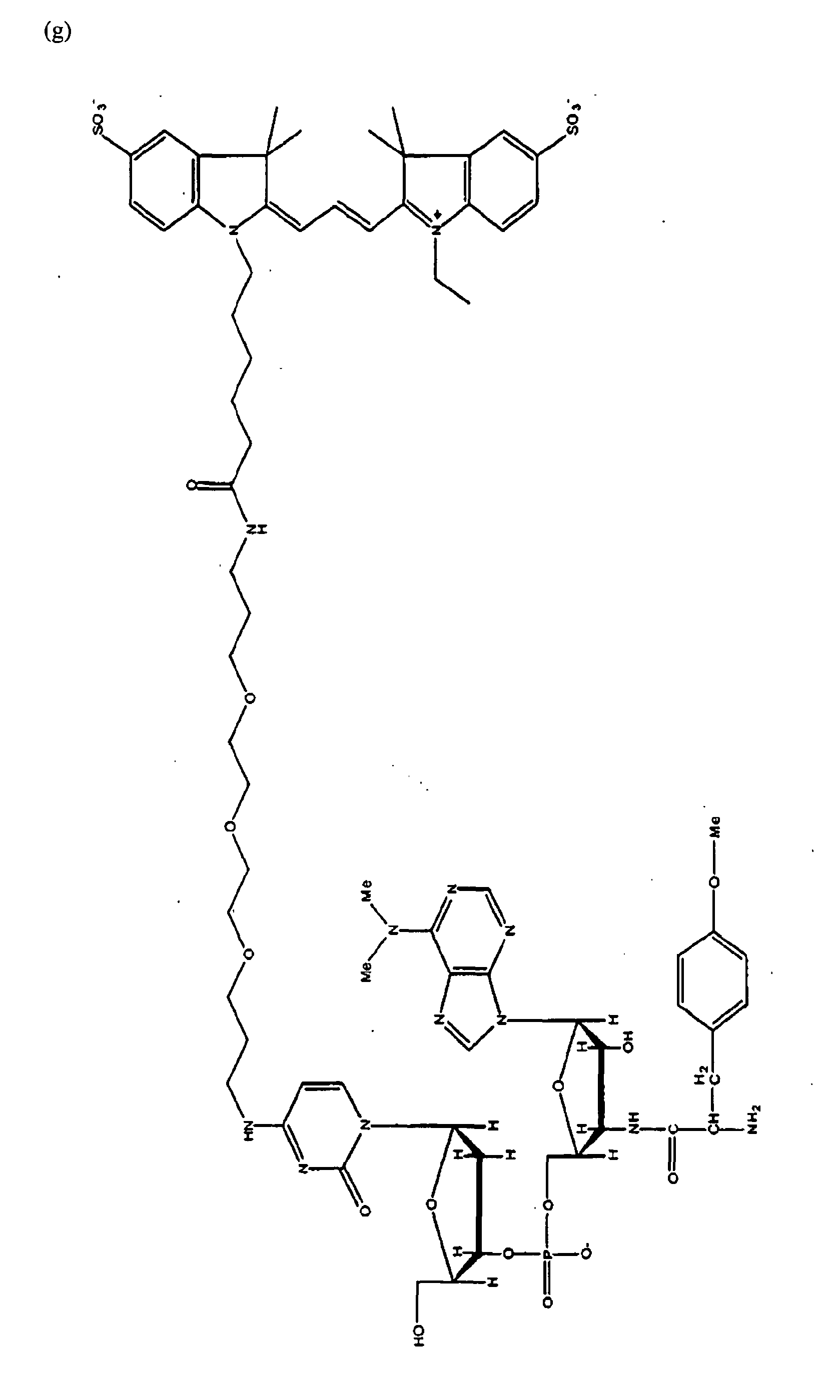 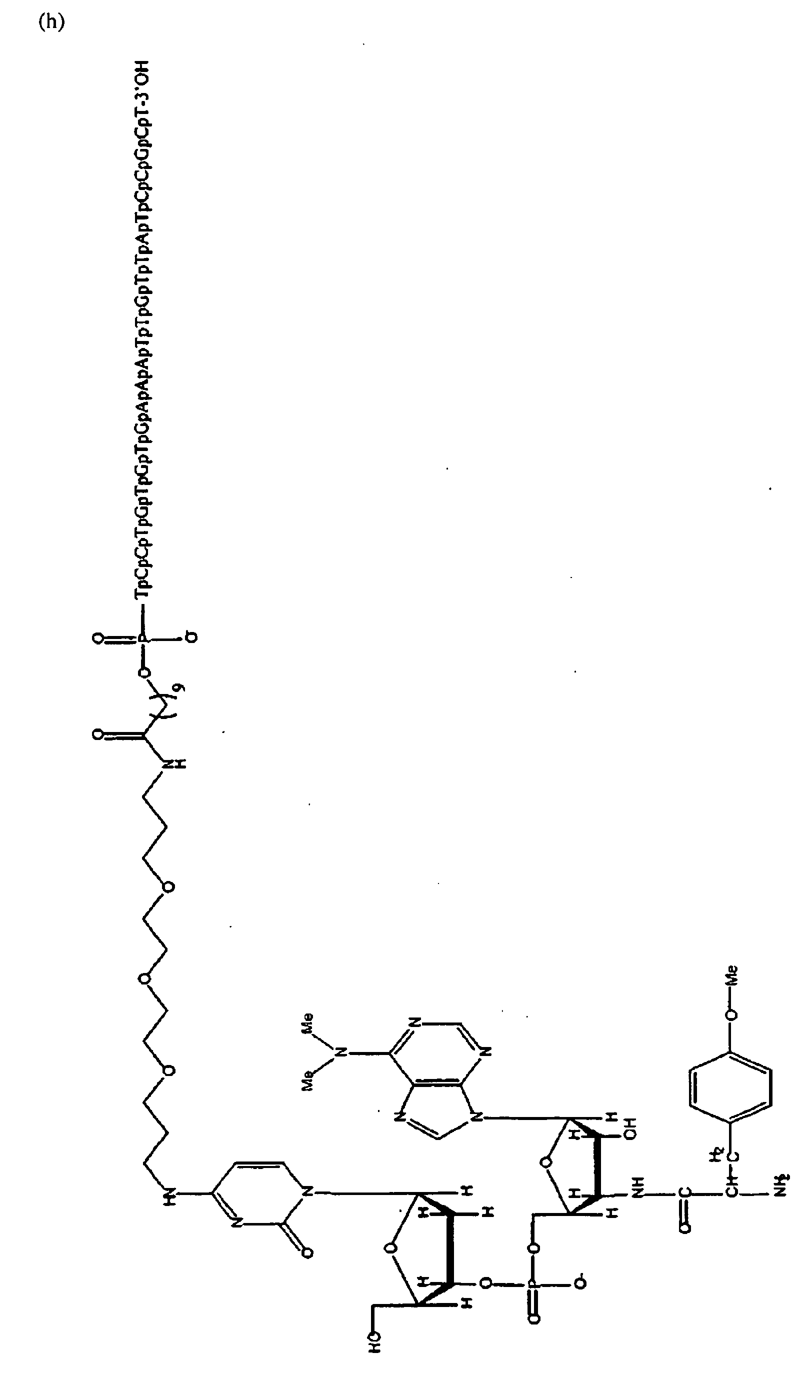 et (i)

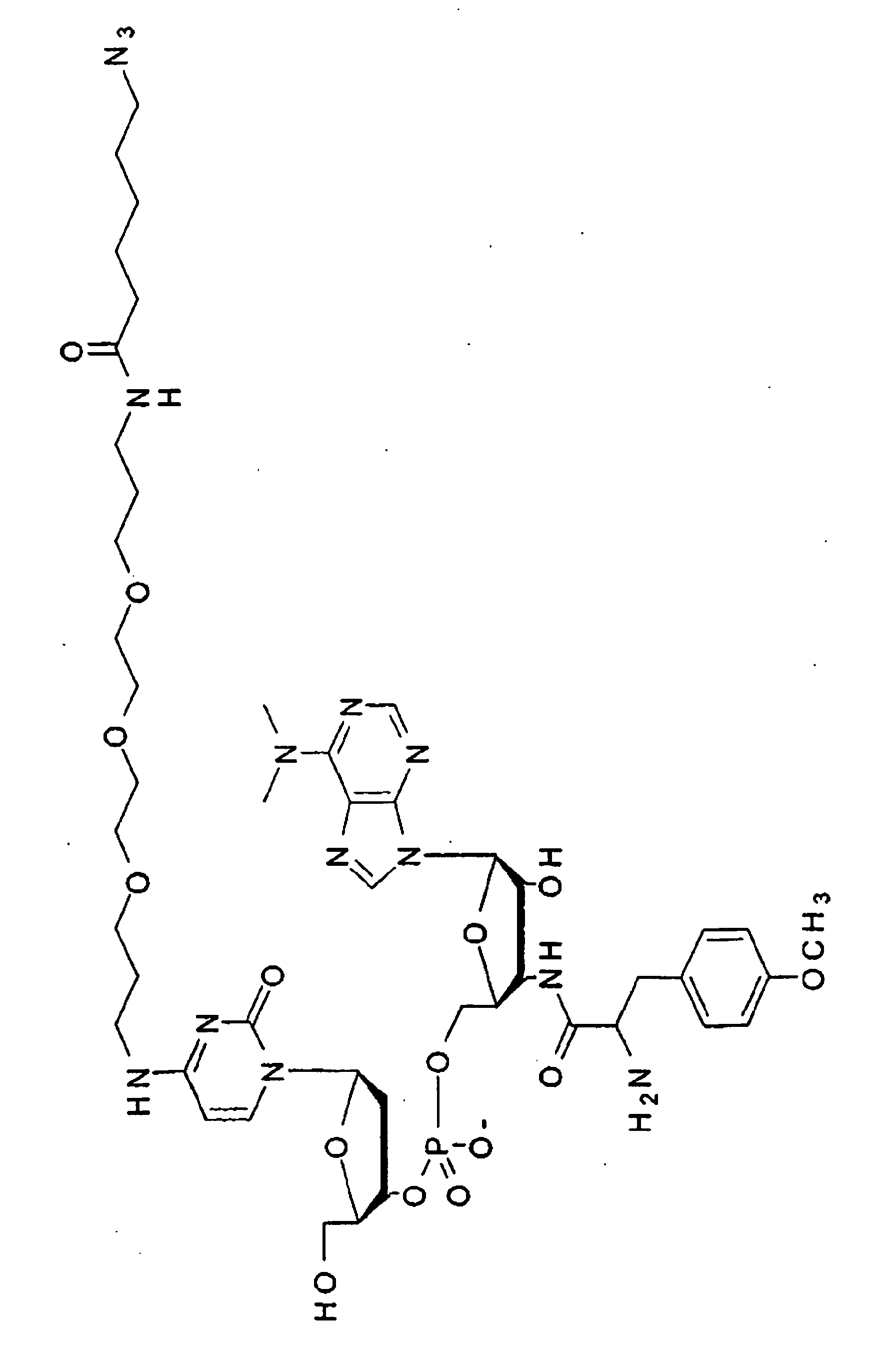

27. Utilisation selon l'une quelconque des revendications 1 à 26, où ladite protéine, ledit polypeptide ou ledit peptide devant être synthétisé est une alloprotéine qui comprend une puromycine C-terminale liée de manière covalente ou un dérivé de celle-ci, telle que définie dans l'une quelconque des revendications 21 à 26.

28. Utilisation selon l'une quelconque des revendications 1 à 19, où ladite protéine, ledit polypeptide ou ledit peptide devant être synthétisé est une alloprotéine qui comprend un acide aminé délivré par l'aminoacyle suppresseur ARNt.

29. Utilisation selon la revendication 28, où ladite protéine, ledit polypeptide ou ledit peptide devant être synthétisé est une alloprotéine qui comprend un acide aminé délivré par l'aminoacyle suppresseur ARNt Ser (CUA).

30. Utilisation de
(a) un vecteur comprenant une molécule d'acide nucléique codant pour une estérase et exprimant une protéine de fusion d'estérase, où ladite estérase est définie comme dans l'une quelconque des revendications 1 à 5 ou un fragment fonctionnel de celle-ci ;
(b) un vecteur comprenant une molécule d'acide nucléique codant pour une estérase et comprenant dans le cadre au moins un site de clonage multiple pour une partie X d'une protéine de fusion estérase-X, grâce à quoi la protéine de fusion devant être codée peut être du format "X-estérase" ou "estérase-X", où ladite estérase est définie comme dans l'une quelconque des revendications 1 à 5 ou un fragment fonctionnel de celle-ci ;
(c) une molécule d'acide nucléique telle que définie dans (a) ou (b) ; ou
(d) une protéine de fusion d'estérase, un polypeptide ou un peptide codé par un vecteur comme défini dans (a) ou (b)
pour surveiller et/ou suivre la synthèse d'une protéine, un polypeptide ou un peptide dans un système de traduction acellulaire ou dans un système d'expression *in vivo*, où la surveillance et/ou le suivi comprend la détection de la fonction ou activité enzymatique de ladite estérase.

31. Utilisation selon la revendication 30, à où ladite molécule d'acide nucléique code pour une protéine de fusion, grâce à quoi ladite protéine de fusion est dans le format "X-estérase" ou "estérase-X", grâce à quoi ladite partie X est une protéine, un polypeptide ou un peptide tel que défini dans l'une quelconque des revendications 16 à 29 ou un fragment de ces derniers.

32. Utilisation selon la revendication 30 ou 31, où ladite molécule d'acide nucléique code pour une protéine de fusion comprenant ladite estérase et GFP.

33. Utilisation selon l'une quelconque des revendications 30 à 32, où ladite protéine de fusion comprend un lieur clivable entre ladite estérase et ladite protéine condensée.

34. Utilisation selon la revendication 33, où ledit lieur clivable entre ladite estérase et ladite protéine condensée est clivable par une protéase de facteur XA.

35. Utilisation selon la revendication 33 ou 34, où ledit lieur clivable entre ladite estérase et ladite protéine condensée est codé par une séquence nucléotidique comprenant la SEQ ID NO: 7.

36. Utilisation selon l'une quelconque des revendications 30 à 35, où ladite molécule d'acide nucléique comprend la séquence nucléotidique de SEQ ID NO: 8.

37. Procédé pour surveiller et/ou suivre la synthèse d'une protéine, un polypeptide ou un peptide dans un système de traduction acellulaire, comprenant l'étape de détection de la fonction ou activité enzymatique d'une estérase telle que définie dans l'une quelconque des revendications 1 à 5 ou un fragment fonctionnel de celle-ci.

38. Procédé selon la revendication 37, où ladite protéine, ledit polypeptide ou ledit peptide est défini comme dans l'une quelconque des revendications 16 à 24.

39. Utilisation selon l'une quelconque des revendications 1 à 36 ou procédé selon la revendication 37 ou 38, où ladite détection de la fonction ou activité enzymatique de ladite estérase est une détection électrochimique.

40. Utilisation ou procédé selon la revendication 39, où ladite détection électrochimique comprend l'hydrolyse d'un ester de p-aminophénol en p-aminophénol.

41. Utilisation ou procédé selon la revendication 39 ou 40, où ladite détection électrochimique comprend l'hydrolyse de l'acétate de p-aminophénol en p-aminophénol.

42. Utilisation selon l'une quelconque des revendications 1 à 36 et 39 à 41 ou procédé selon la revendication 37 ou 38, où ladite fonction ou activité enzymatique devant être détectée est le clivage d'un ester en un alcool et un acide carboxylique.
